(19) Europäisches Patentamt
European Patent Office
Office européen des brevets

(11) EP 2 501 242 B1

(12) **EUROPEAN PATENT SPECIFICATION**

(45) Date of publication and mention of the grant of the patent:
**09.08.2017 Bulletin 2017/32**

(21) Application number: **10784586.9**

(22) Date of filing: **08.11.2010**

(51) Int Cl.:
***A23C 9/18*** *(2006.01)* ***C12N 9/10*** *(2006.01)*

(86) International application number:
**PCT/IB2010/055057**

(87) International publication number:
**WO 2011/061657 (26.05.2011 Gazette 2011/21)**

(54) **Method for producing powder milk**

Methode zur Herstellung von Milchpulver

Procédé de fabrication de lait en poudre

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2009 GB 0920089**
**18.11.2009 US 262285 P**

(43) Date of publication of application:
**26.09.2012 Bulletin 2012/39**

(83) **Declaration under Rule 32(1) EPC (expert solution)**

(73) Proprietor: **DuPont Nutrition Biosciences ApS**
**1411 Copenhagen K (DK)**

(72) Inventors:
- **LARSEN, Niels Erik**
**8250 Egå (DK)**
- **SØE, Jørn Borch**
**8321 Tilst (DK)**

(74) Representative: **D Young & Co LLP**
**120 Holborn**
**London EC1N 2DY (GB)**

(56) References cited:
**WO-A1-2009/024736 US-A1- 2007 026 106**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 501 242 B1

## Description

FIELD OF THE INVENTION

The present invention relates to a method for the production of powder milk, an enzymatically treated powder milk and uses of an enzyme for the treatment of powder milk to provide new and unexpected technical advantages.

BACKGROUND TO THE INVENTION

Powder milk (also referred to herein as 'dried milk') is a manufactured dairy product made by drying milk. The principal objective of drying is to increase its shelf life and avoid the need for refrigeration, due to the low moisture content. Powder milk and powdered dairy products may comprise dried whole milk, dried skim milk, dried buttermilk, dry whey products and dry dairy blends.

Typically, powder milk is made by spray drying non-fat skim milk, whole milk, buttermilk or whey. Pasteurized milk is first concentrated in an evaporator to about 50% milk solids. The resulting concentrated milk is sprayed into a heated chamber where the water almost instantly evaporates, leaving fine particles of powdered milk solids. The powder particles are separated from the air stream and recovered at the bottom of the dryer while the humid air is moved out of the evaporator.

Alternatively, the milk can be dried by drum drying (also known as roller drying). Milk is applied as a thin film to the surface of a heated drum (typically heated by steam). The evaporated water is drawn off, leaving dried milk solids which form a layer on the drum which is then scraped off. Powdered milk made this way tends to have a 'cooked' flavour, due to caramelization caused by greater heat exposure.

Another process used to produce powder milk is freeze drying, which preserves many nutrients in milk, compared to drum drying. However, this method is generally more expensive than drum or spray drying.

Powder milk products and processes used to produce them are described in general terms in "Milk and Dairy Products", R. Jost, publ. Wiley-VCH, Weinheim, 2007.

WO 2006/066590 describes a method of producing a milk powder using phospholipases, in particular phospholipase A. However, this document does not disclose or suggest that producing milk powder using this enzyme would prevent fouling of the equipment used in the method.

Lipid acyltransferases are known to be advantageous in food applications. Lipid acyltransferases have been found to have significant acyltransferase activity in foodstuffs. This activity has surprising beneficial applications in methods of preparing foodstuffs.

For instance, WO 2004/064537 discloses a method for the *in situ* production of an emulsifier by use of a lipid acyltransferase and the advantages associated therewith.

WO 2008/090395 teaches the expression of lipid acyltransferases in a (heterologous) host cell.

WO 2009/024862 describes a method for manufacturing UHT milk using a lipid acyltransferase and milk produced by the method.

The principal constituents of milk are water, fat, proteins, lactose (milk sugar) and minerals (salts). Milk also contains smaller amounts of other substances such as pigments, enzymes, vitamins, phospholipids (substances with fat-like properties), sterols and gases.

The many lipids of milk, together forming the 'milk fat', have a very complicated composition and structure, even more complicated than most other naturally occurring fats. Typically milk fat consists of triglycerides, di- and monoglycerides, fatty acids, sterols, carotenoids and vitamins (A, D, E and K). Other components include phospholipids, lipoproteins, glycerides, cerebrosides, proteins, nucleic acids, enzymes, metals and water.

Phospholipids are the most surface-active class, as they are amphipolar. As the molecular size is relatively large, they tend to form lamellar bilayers. Phospholipids of milk are generally seen in close connection with proteins, especially when located in the membrane(s) of milk fat globules. The main constituent of phospholipids in milk comprise lecithins, which are surface active at moderate hydrophilicity. Thus lecithin can be seen as a suspending and dispersing agent or as an emulsifier for O/W emulsions as well as for W/O emulsions.

Phospholipids comprise 0.8-1.0% of the natural milk fat. The main types of phospholipids/lecithin in milk are phosphatidylcholine and phosphatidylethanolamine.

Sterols are highly insoluble in water, and show very little surface activity. They easily associate with phospholipids. The cholesterol may be considered an unwanted ingredient in milk when considering the nutritional value of milk. Cholesterol comprises 0.3-0.4% of the natural milk fat.

SUMMARY OF THE INVENTION

Aspects of the present invention are presented in the claims and in the following commentary.

It has surprisingly been found that exposing milk or a fraction thereof to a lipid acyltransferase during production of powder milk results in the powder milk having improved flowability and rehydration properties, and also results in the powder milk having decreased free fatty acid content (compared with powder milk which during its manufacture has been treated with a phospholipase) and decreased cholesterol content.

According to a first aspect of the present invention there is provided a method of producing powder milk, said method comprising:

(a) contacting milk or a fraction thereof with a lipid acyltransferase enzyme; and
(b) drying the enzyme treated milk to produce powder milk.

According to a second aspect of the present invention there is provided powder milk obtained or obtainable by the method of the invention.

According to a third aspect of the present invention there is provided a milk product produced by rehydrating the powder milk of the invention.

According to a fourth aspect of the present invention there is provided a use of a lipid acyltransferase in the manufacture of powder milk for improving the rehydration properties of the powder milk. In one aspect, such improved rehydration properties of the powder milk comprise an improved wettability and/or lowered wetting time.

According to a fifth aspect of the present invention there is provided a use of a lipid acyltransferase in the manufacture of powder milk for reducing the cholesterol content of the powder milk.

According to a sixth aspect of the present invention there is provided a use of a lipid acyltransferase in the manufacture of powder milk for reducing the free fatty acid content of the powder milk compared with powder milk which during its manufacture has been treated with a phospholipase.
A reduction in cholesterol can be measured by Thin Layer Chromatography (TLC) and/or Gas Liquid Chromatography (GLC).

According to a seventh aspect of the present invention there is provided a use of a lipid acyltransferase in the manufacture of powder milk for improving the flowability of the powder milk.

According to an eighth aspect of the present.invention there is provided a use of a lipid acyltransferase in the manufacture of powder milk for reducing fouling of the equipment used in the manufacture of the powder milk.

## DETAILED DESCRIPTION OF PREFERRED EMBODIMENTS

As described above, in one aspect the present invention comprises a method of producing powder milk, comprising: (a) contacting milk or a fraction thereof with a lipid acyltransferase enzyme; and (b) drying the enzyme treated milk to produce powder milk.

## MILK

The term 'milk' as used herein may comprise milk from either animal or vegetable origin, and includes whole milk, skim milk, and semi-skim milk.

It is possible to use milk from animal sources such as buffalo, (traditional) cow, sheep, goat etc. either individually or combined. Vegetable milks such as soya milk may also be used, either alone or in combination with the animal milk. When vegetable milks are used in combination with animal milk, the combination typically comprises a low percentage (of vegetable milk) say below 15%, or below 20%, or below 25% v/v. The term milk preferably does not comprise cheese milk and cream.

The term 'essentially consists' as used herein, when referring to a product or composition, preferably means that the product or composition, may consist of other products or compositions but only to a maximum concentration of, preferably 10%, such as 5%, such as 3%, such as 2% or 1 %, or 0.5% or 0.1 %.

For the enzyme modification of milk and/or cream for example it may be preferable to use a temperature of less than about 30°C for example, suitably less than 20°C for example, suitably less than 10°C for example. Suitable temperatures of between 1-30°C may be used, such as between 3-20°C for example, such as between 1-10°C.

## INCUBATION

The milk is contacted according to the present invention with a lipid acyltransferase enzyme such that the enzyme is incubated therewith. Suitable lipid acyltransferase enzymes are described in more detail herein.

Suitably, the lipid acyltransferase is contacted with the milk and incubated therewith at a temperature of between about 0°C and about 70°C. In one embodiment, the lipid acyltransferase is contacted with the milk and incubated therewith at a temperature of between about 20°C and about 60°C, more preferably between about 30°C and about 50°C, still

more preferably between about 35°C and about 45°C, and most preferably about 40°C. In another embodiment, the lipid acyltransferase is contacted with the milk and incubated therewith at a temperature of between about 0°C and about 20°C, more preferably between about 5°C and about 15°C, and still more preferably between about 5°C and about 10°C.

Preferably the lipid acyltransferase is contacted with the milk and incubated therewith in a concentration of about 0.01 to about 1 mg enzyme / kg milk, more preferably about 0.01 to about 0.05 mg enzyme / kg milk, even more preferably about 0.01 to about 0.2 mg enzyme/ kg milk, still more preferably about 0.01 to about 0.1 mg enzyme/ kg milk, yet more preferably about 0.01 to about 0.05 mg enzyme/ kg milk, and most preferably about 0.05 mg enzyme/ kg milk.

Preferably the incubation time is effective to ensure that there is at least 10% transferase activity, more preferably at least 15%, 20%, 25%, 26%, 28%, 30%, 40%, 50%, 60% or 70% transferase activity.

The transferase activity is measured by the Transferase Assay referred to herein.

Suitably the incubation time may be from 1 minute up to 36 hours, preferably from 2 minutes up to 24 hours, more preferably from 5 minutes up to 18 hours, even preferably from 10 minutes up to 12 hours, and still more preferably from 20 minutes up to 8 hours.

In one embodiment the incubation time may be from about 20 minutes to about 2 hours, preferably from about 30 minutes to about 1 hour, more preferably about 35 minutes to about 45 minutes.

In another embodiment the incubation time may be from about 2 hours to about 36 hours, preferably from about 4 hours to about 24 hours.

Preferably the combination of temperature and the incubation time is effective to ensure that there is at least 5% transferase activity, preferably at least 10% transferase activity, preferably at least 15%, 20%, 25% 26%, 28%, 30%, 40% 50%, 60% or 75% transferase activity.

Suitably, the method may further comprise a step of removing the enzyme and/or denaturing the enzyme.

Suitably the enzyme for use in the present invention may be an immobilised enzyme.

The reaction may take place in any suitable vessel, non-limiting examples of which include a continuous flow reactor.

DRYING

Following treatment with an acyltransferase enzyme as described herein, the treated milk is dried to produce powder milk.

In one embodiment, the enzyme treated milk is dried by spray drying to produce powder milk. Suitable spray drying conditions described in general terms in "Milk and Dairy Products", R. Jost, publ. Wiley-VCH, Weinheim, 2007.

In this embodiment, the enzyme treated milk is suitably fed into the spray dryer at a temperature ranging from about 20°C to about 95°C, preferably from about 50°C to about 95°C, more preferably from about 60°C to about 80°C. In one alternative embodiment, the enzyme treated milk is suitably fed into the spray dryer at a temperature ranging from 35°C to 45°C, preferably about 40°C.

In this embodiment, the outlet air temperature of the spray dryer suitably ranges from about 50°C to about 150°C, preferably from about 70°C to about 130°C, more preferably from about 90°C to about 110°C, and most preferably about 100°C.

In this embodiment, the product outlet temperature of the spray dryer suitably ranges from about 20°C to about 80°C, preferably from about 30°C to about 70°C, more preferably from about 35°C to about 45°C, and most preferably about 40°C.

In another embodiment the enzyme treated milk is dried by roller drying (also known as drum drying) to produce powder milk. Suitable roller drying conditions are described in general terms in "Milk and Dairy Products", R. Jost, publ. Wiley-VCH, Weinheim, 2007.

In this embodiment, the temperature of the drum suitably ranges from about 90°C to about 150°C, more preferably from about 100°C to about 130°C.

ADVANTAGES

A surprising advantage conferred by the present invention is the greatly improved rehydration properties of the powder milk. In one aspect, such improved rehydration properties of the powder milk comprise an improved wettability and/or lowered wetting time. Wettability may be measured in accordance with IDF method 87:1979.

A further advantage of the present invention may be the reduction of fouling of the powder process plant (e.g. of the plant tubes and/or steel surfaces) when using the powder milk treated in accordance with the present invention compared with powder milk which has not been enzymatically treated and/or compared with powder milk which during its manufacture has been treated with a phospholipase (in particular either a phospholipase A1 enzyme classified as E.C. 3.1.1.32 or a phospholipase A2 enzyme classified as EC.3.1.1.4) (rather than the lipid acyltransferase as described

herein).

A further advantage of the present invention may be a reduction in free fatty acids in powder milk treated in accordance with the present invention compared with powder milk which during its manufacture has been treated with a phospholipase (in particular either a phospholipase A1 enzyme classified as E.C. 3.1.1.32 or a phospholipase A2 enzyme classified as EC.3.1.1.4) (rather than the lipid acyltransferase as described herein).

A further advantage of the present invention is a reduction in cholesterol content in the powder milk which may have major health benefits.

A further advantage of the present invention is the improved flowability of the powder milk.

Suitably the improvement in the rehydration properties and/or the improvement in the perceptible sensory difference and/or the improvement in smell and/or taste and/or the reduction in cholesterol content and/or the improved flowability of the powder milk and/or reduced fouling of the equipment used in its manufacture means an improvement when the enzymatically treated milk (treated with enzymes in accordance with the present invention) is compared with powder milk which has not been enzymatically treated and/or compared with powder milk which has been treated with a phospholipase (in particular either a phospholipase A1 enzyme classified as E.C. 3.1.1.32 or a phospholipase A2 enzyme classified as EC.3.1.1.4).

Suitably the improvement in the rehydration properties and/or the improvement in the perceptible sensory difference and/or the improvement in smell and/or taste and/or the reduction in cholesterol content and/or the improved flowability of the powder milk and/or reduced fouling of the equipment used in its manufacture may mean an improvement when the enzymatically treated milk (treated with enzymes in accordance with the present invention) is compared with powder milk which has been treated with one or more of the following phospholipases: Phospholipase A1 from *Fusarium oxysporum* (Lipopan F™) and/or a phospholipase from *Fusarium heterosporum* and/or a phospholipase A1 from *Fusarium venenatum* (YieldMax™) and/or a phospholipase from *Aspergillus niger* and/or a phospholipase A2 from *Streptomyces violaceoruber* and/or a phospholipase A2 from porcine pancreas and/or a phospholipase A2 from *Tuber borchii.*

HOST CELL

The host organism can be a prokaryotic or a eukaryotic organism.

In one embodiment of the present invention the lipid acyl transferase according to the present invention in expressed in a host cell, for example a bacterial cells, such as a *Bacillus* spp, for example a *Bacillus licheniformis* host cell.

Alternative host cells may be fungi, yeasts or plants for example.

It has been found that the use of a *Bacillus licheniformis* host cell results in increased expression of a lipid acyltransferase when compared with other organisms, such as *Bacillus subtilis.*

A lipid acyltransferase from *Aeromonas salmonicida* has been inserted into a number of conventional expression vectors, designed to be optimal for the expression in *Bacillus subtilis, Hansenula polymorpha, Schizosaccharomyces pombe* and *Aspergillus tubigensis,* respectively. Only very low levels were, however, detected in *Hansenula polymorpha, Schizosaccharomyces pombe* and *Aspergillus tubigensis.* The expression levels were below 1 μg/ml, and it was not possible to select cells which yielded enough protein to initiate a commercial production (results not shown). In contrast, *Bacillus licheniformis* was able to produce protein levels, which are attractive for an economically feasible production.

In particular, it has been found that expression in *B. licheniformis* is approximately 100-times greater than expression in B. *subtilis* under the control of aprE promoter or is approximately 100-times greater than expression in *S. lividans* under the control of an A4 promoter and fused to cellulose (results not shown herein).

The host cell may be any *Bacillus* cell other than *B.subtilis.* Preferably, said *Bacillus* host cell being from one of the following species: *Bacillus licheniformis; B. alkalophilus; B. amyloliquefaciens; B. circulans; B. clausii; B. coagulans; B. firmus; B. lautus; B. lentus; B. megaterium; B. pumilus* or B. *stearothermophilus.*

The term "host cell" - in relation to the present invention includes any cell that comprises either a nucleotide sequence encoding a lipid acyltransferase as defined herein or an expression vector as defined herein and which is used in the recombinant production of a lipid acyltransferase having the specific properties as defined herein.

Suitably, the host cell may be a protease deficient or protease minus strain and/or an α-amylase deficient or α-amylase minus strain.

The term "heterologous" as used herein means a sequence derived from a separate genetic source or species. A heterologous sequence is a non-host sequence, a modified sequence, a sequence from a different host cell strain, or a homologous sequence from a different chromosomal location of the host cell.

A "homologous" sequence is a sequence that is found in the same genetic source or species i.e. it is naturally occurring in the relevant species of host cell.

The term "recombinant lipid acyltransferase" as used herein means that the lipid acyltransferase has been produced by means of genetic recombination. For instance, the nucleotide sequence encoding the lipid acyltansferase has been inserted into a cloning vector, resulting in a *B. licheniformis* cell characterised by the presence of the heterologous lipid acyltransferase.

REGULATORY SEQUENCES

In some applications, a lipid acyltransferase sequence for use in the methods and/or uses of the present invention may be obtained by operably linking a nucleotide sequence encoding same to a regulatory sequence which is capable of providing for the expression of the nucleotide sequence, such as by the chosen host cell (such as a *B. licheniformis* cell).

By way of example, a vector comprising the nucleotide sequence of the present invention operably linked to such a regulatory sequence, i.e. the vector is an expression vector, may be used.

The term "operably linked" refers to a juxtaposition wherein the components described are in a relationship permitting them to function in their intended manner. A regulatory sequence "operably linked" to a coding sequence is ligated in such a way that expression of the coding sequence is achieved under conditions compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme having the specific properties as defined herein may also be achieved by the selection of regulatory regions, e.g. promoter, secretion leader and terminator regions that are not regulatory regions for the nucleotide sequence encoding the enzyme in nature.

Suitably, the nucleotide sequence of the present invention may be operably linked to at least a promoter.

Suitably, the nucleotide sequence encoding a lipid acyltransferase may be operably linked to at a nucleotide sequence encoding a terminator sequence. Examples of suitable terminator sequences for use in any one of the vectors, host cells, methods and/or uses of the present invention include: an $\alpha$-amylase terminator sequence (for instance, CGGGACTTACCGAAAGAAACCATCAATGATGGTTTCTTTTTTGTTCATAAA - SEQ ID No. 64), an alkaline protease terminator sequence (for instance, CAAGACTAAAGACCGTTCGCCCGTTTTTGCAATAAGCGGGCGAATCTTACATAAAA ATA - SEQ ID No. 65), a glutamic-acid specific terminator sequence (for instance, ACGGCCGTTAGATGTGACAGCCCGTTCCAAAAGGAAGCGGGCTGTCTTCGTGTAT TATTGT - SEQ ID No. 66), a levanase terminator sequence (for instance, TCTTTTAAAGGAAAGGCTGGAATGCCCGGCATTCCAGCCACATGATCATCGTTT - SEQ ID No. 67) and a subtilisin E terminator sequence (for instance, GCTGACAAATAAAAAGAAGCAGGTATGGAGGAACCTGCTTCTTTTTACTATTATTG - SEQ ID No. 119). Suitably, the nucleotide sequence encoding a lipid acyltransferase may be operably linked to an $\alpha$-amylase terminator, such as a B. *licheniformis* $\alpha$-amylase terminator.

PROMOTER

The promoter sequence to be used in accordance with the present invention may be heterologous or homologous to the sequence encoding a lipid acyltransferase.

The promoter sequence may be any promoter sequence capable of directing expression of a lipid acyltransferase in the host cell of choice.

Suitably, the promoter sequence may be homologous to a *Bacillus* species, for example *B. licheniformis.* Preferably, the promoter sequence is homologous to the host cell of choice.

Suitably the promoter sequence may be homologous to the host cell. "Homologous to the host cell" means originating within the host organism; i.e. a promoter sequence which is found naturally in the host organism.

Suitably, the promoter sequence may be selected from the group consisting of a nucleotide sequence encoding: an $\alpha$-amylase promoter, a protease promoter, a subtilisin promoter, a glutamic acid-specific protease promoter and a levansucrase promoter.

Suitably the promoter sequence may be a nucleotide sequence encoding: the LAT (e.g. the alpha-amylase promoter from *B. licheniformis,* also known as AmyL), AprL (e.g. subtilisin Carlsberg promoter), EndoGluC (e.g. the glutamic-acid specific promoter from *B. licheniformis*), AmyQ (e.g. the alpha amylase promoter from B. *amyloliquefaciens* alpha-amylase promoter) and SacB (e.g. the B. *subtilis* levansucrase promoter).

Other examples of promoters suitable for directing the transcription of a nucleic acid sequence in the methods of the present invention include: the promoter of the *Bacillus lentus* alkaline protease gene (aprH), ; the promoter of the *Bacillus subtilis* alpha-amylase gene (amyE); the promoter of the *Bacillus stearothermophilus* maltogenic amylase gene (amyM); the promoter of the *Bacillus licheniformis* penicillinase gene (penP); the promoters of the *Bacillus subtilis* xylA and xylB genes; and/or the promoter of the *Bacillus thuringiensis* subsp. *tenebrionis* CryIIIA gene.

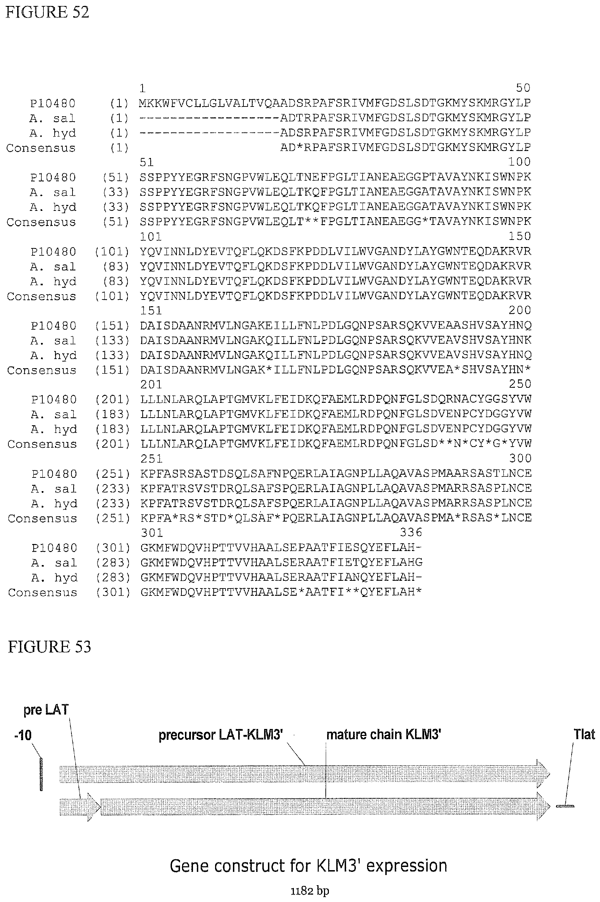

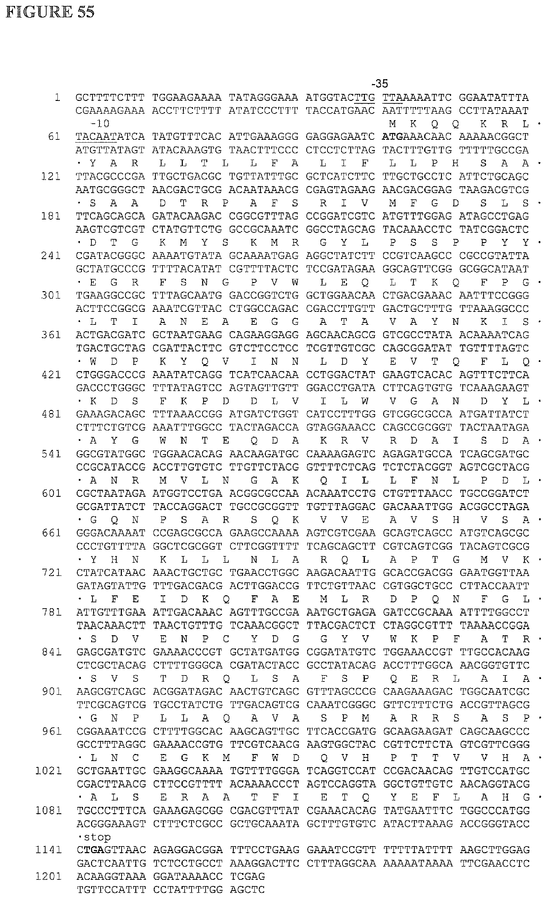

In a preferred embodiment, the promoter sequence is an $\alpha$-amylase promoter (such as a *Bacillus licheniformis* $\alpha$-amylase promoter). Preferably, the promoter sequence comprises the -35 to -10 sequence of the B. *licheniformis* $\alpha$-amylase promoter - see Figures 53 and 55.

The "-35 to -10 sequence" describes the position relative to the transcription start site. Both the "-35" and the "-10" are boxes, i.e. a number of nucleotides, each comprising 6 nucleotides and these boxes are separated by 17

nucleotides. These 17 nucleotides are often referred to as a "spacer". This is illustrated in Figure 55, where the -35 and the -10 boxes are underlined. For the avoidance of doubt, where "-35 to -10 sequence" is used herein it refers to a sequence from the start of the -35 box to the end of the -10 box i.e. including both the -35 box, the 17 nucleotide long spacer and the -10 box.

SIGNAL PEPTIDE

The lipid acyltransferase produced by a host cell by expression of the nucleotide sequence encoding the lipid acyltransferase may be secreted or may be contained intracellularly depending on the sequence and/or the vector used.

A signal sequence may be used to direct secretion of the coding sequences through a particular cell membrane. The signal sequences may be natural or foreign to the lipid acyltransferase coding sequence. For instance, the signal peptide coding sequence may be obtained form an amylase or protease gene from a *Bacillus* species, preferably from *Bacillus licheniformis.*

Suitable signal peptide coding sequences may be obtained from one or more of the following genes: maltogenic $\alpha$-amylase gene, subtilisin gene, beta-lactamase gene, neutral protease gene, prsA gene, and/or acyltransferase gene.

Preferably, the signal peptide is a signal peptide of *B. licheniformis* $\alpha$-amylase, Aeromonas acyltransferase (for instance, mkkwfvcllglialtvqa - SEQ ID No. 21), *B. subtilis* subtilisin (for instance, mrskklwisllfaltliftmafsnmsaqa - SEQ ID No. 22) or *B. licheniformis* subtilisin (for instance, mmrkksfwfgmltafmlvftmefsdsasa - SEQ ID No. 23). Suitably, the signal peptide may be the signal peptide of *B. licheniformis* $\alpha$-amylase.

However, any signal peptide coding sequence capable of directing the expressed lipid acyltransferase into the secretory pathway of a *Bacillus* host cell (preferably a *B. licheniformis* host cell) of choice may be used.

In some embodiments of the present invention, a nucleotide sequence encoding a signal peptide may be operably linked to a nucleotide sequence encoding a lipid acyltransferase of choice.

The lipid acyltransferase of choice may be expressed in a host cell as defined herein as a fusion protein.

EXPRESSION VECTOR

The term "expression vector" means a construct capable of *in vivo* or *in vitro* expression.

Preferably, the expression vector is incorporated in the genome of the organism, such as a *B. licheniformis* host. The term "incorporated" preferably covers stable incorporation into the genome.

The nucleotide sequence encoding a lipid acyltransferase as defined herein may be present in a vector, in which the nucleotide sequence is operably linked to regulatory sequences such that the regulatory sequences are capable of providing the expression of the nucleotide sequence by a suitable host organism (such as *B. licheniformis*), i.e. the vector is an expression vector.

The vectors of the present invention may be transformed into a suitable host cell as described above to provide for expression of a polypeptide having lipid acyltransferase activity as defined herein.

The choice of vector, e.g. plasmid, cosmid, virus or phage vector, genomic insert, will often depend on the host cell into which it is to be introduced. The present invention may cover other forms of expression vectors which serve equivalent functions and which are, or become, known in the art.

Once transformed into the host cell of choice, the vector may replicate and function independently of the host cell's genome, or may integrate into the genome itself.

The vectors may contain one or more selectable marker genes - such as a gene which confers antibiotic resistance e.g. ampicillin, kanamycin, chloramphenicol or tetracycline resistance. Alternatively, the selection may be accomplished by co-transformation (as described in WO 91/17243).

Vectors may be used *in vitro,* for example for the production of RNA or used to transfect or transform a host cell.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

LIPID ACYLTRANSFERASE

The nucleotide sequence encoding a lipid acyl transferase for use in any one of the methods and/or uses of the present invention may encode a natural lipid acyl transferase or a variant lipid acyl transferase.

The lipid acyl transferase for use in any one of the methods and/or uses of the present invention may be a natural lipid acyl transferase or a variant lipid acyl transferase.

For instance, the nucleotide sequence encoding a lipid acyl transferase for use in the present invention may be one as described in WO 2004/064537, WO 2004/064987, WO 2005/066347, WO 2006/008508, WO 2009/024862 or PCT/IB2009/054535.

The term "lipid acyl transferase" as used herein preferably means an enzyme that has acyltransferase activity (generally classified as E.C. 2.3.1.x, for example 2.3.1.43), whereby the enzyme is capable of transferring an acyl group from a lipid to one or more acceptor substrates, such as one or more of the following: a sterol; a stanol; a carbohydrate; a protein; a protein subunit; a sugar alcohol, such as ascorbic acid and/or glycerol - preferably glycerol and/or a sterol, such as cholesterol.

Preferably, the lipid acyl transferase for use in any one of the methods and/or uses of the present invention is a lipid acyltransferase that is capable of transferring an acyl group from a phospholipid (as defined herein) to a sugar alcohol, such as ascorbic acid and/or glycerol and/or a sterol, preferably glycerol or a sterol, most preferably a sterol (e.g. cholesterol).

For some aspects the "acyl acceptor" according to the present invention may be any compound comprising a hydroxy group (-OH), such as for example, polyvalent alcohols, including glycerol; sterols; stanols; carbohydrates; hydroxy acids including fruit acids, citric acid, tartaric acid, lactic acid and ascorbic acid; proteins or a sub-unit thereof, such as amino acids, protein hydrolysates and peptides (partly hydrolysed protein) for example; and mixtures and derivatives thereof. Preferably, the "acyl acceptor" according to the present invention is not water. Preferably, the "acyl acceptor" according to the present invention is a sugar alcohol, such as a polyol, most preferably glycerol. For the purpose of this invention ascorbic acid is also considered a sugar-alcohol.

The acyl acceptor is preferably not a monoglyceride.

The acyl acceptor is preferably not a diglyceride.

In one aspect, the lipid acyltransferase for use in any one of the methods and/or uses of the present invention is a lipid acyltransferase that may, as well as being able to transfer an acyl group from a lipid to glycerol, additionally be able to transfer the acyl group from a lipid to one or more of the following: a carbohydrate, a protein, a protein subunit, sterol and/or a stanol, preferably it is capable of transferring to both a sugar alcohol, such as ascorbic acid and/or glycerol, most preferably a sterol such as cholesterol, and/or plant sterols/stanols.

In some aspects, the lipid acyltransferase for use in any one of the methods and/or uses of the present invention is a lipid acyltransferase that is capable of esterifying at least about 10%, more preferably at least about 20%, 30%, 40%, 50%, 60% or 70% of the acyl acceptor.

In preferred aspects, the lipid acyltransferase for use in any one of the methods and/or uses of the present invention is a lipid acyltransferase that is capable of esterifying at least about 10%, more preferably at least about 20%, 30%, 40%, 50%, 60% or 70% of cholesterol present in the starting milk.

Preferably, the lipid substrate upon which the lipid acyltransferase acts is one or more of the following lipids: a phospholipid, such as a lecithin, e.g. phosphatidylcholine and/or phophatidylethanolamine.

This lipid substrate may be referred to herein as the "lipid acyl donor". The term lecithin as used herein encompasses phosphatidylcholine, phosphatidylethanolamine, phosphatidylinositol, phosphatidylserine and phosphatidylglycerol.

For some aspects, preferably the lipid acyl transferase for use in any one of the methods and/or uses of the present invention is a lipid acyltransferase that is incapable, or substantially incapable, of acting on a triglyceride and/or a 1-monoglyceride and/or 2-monoglyceride.

For some aspects, preferably the lipid acyl transferase for use in any one of the methods and/or uses of the present invention is a lipid acyltransferase that does not exhibit triacylglycerol lipase activity (E.C. 3.1.1.3) or does not exhibit significant triacylglycerol lipase activity (E.C. 3.1.1.3).

Triaclgycerol lipase activity based on tributyrin is measured according to Food Chemical Codex, 4th Edition, National Academy Press, 1996, p 803, with the modifications that the sample is dissolved in deionized water instead of glycine buffer, and the pH stat set point is 5.5 instead of 7. 1 Lipase Unit (LIPU) is defined as the quantity of enzyme which can liberate 1 $\mu$mol butyric acid per minute under these assay conditions.

The lipid acyl transferase for use in any one of the methods and/or uses of the present invention may be a lipid acyltransferase which is substantially incapable of acting on a triglyceride may have a LIPU of less than 5 / kg milk, more preferably a LIPU of less than 0.25 / kg milk, and most preferably a LIPU of less than 0.05 / kg milk.

Suitably, the lipid acyltransferase for use in any one of the methods and/or uses of the present invention is a lipid acyltransferase that may exhibit one or more of the following phospholipase activities: phospholipase A2 activity (E.C. 3.1.1.4) and/or phospholipase A1 activity (E.C. 3.1.1.32). The lipid acyl transferase may also have phospholipase B activity (E.C 3.1.1.5).

Suitably, for some aspects the lipid acyltransferase may be capable of transferring an acyl group from a phospholipid to a sugar alcohol, preferably glycerol and/or ascorbic acid.

Suitably, for some aspects the lipid acyltransferase may be capable of transferring an acyl group from a phospholipid to a stanol and/or sterol, preferably cholesterol.

For some aspects, preferably the lipid acyltransferase for use any one of the methods and/or uses of the present invention encodes a lipid acyltransferase that is capable of transferring an acyl group from a phospholipid to a sterol and/or a stanol to form at least a sterol ester and/or a stanol ester.

The lipid acyltransferase may be capable of transferring an acyl group from a lipid to a polyol such as glycerol, and/or a sterol such as cholesterol or plant sterol/stanols. Thus, in one embodiment the "acyl acceptor" according to the present invention may be glycerol and/or cholesterol or plant sterol/stanols.

In some aspects, the lipid acyltransferase for use in any one of the methods and/or uses of the present invention may comprise a GDSx motif and/ or a GANDY motif.

Preferably, the lipid acyltransferase enzyme is characterised as an enzyme which possesses acyltransferase activity and which comprises the amino acid sequence motif GDSX, wherein X is one or more of the following amino acid residues L, A, V, I, F, Y, H, Q, T, N, M or S.

Suitably, the nucleotide sequence encoding a lipid acyltransferase or lipid acyltransferase for use in any one of the methods and/or uses of the present invention may be obtainable, preferably obtained, from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas and Candida.* Preferably, the lipid acyltransferase is obtainable, preferably obtained, from an organism from the genus *Aeromonas.*

In some aspects of the present invention, the nucleotide sequence encoding a lipid acyltransferase for use in any one of the methods and/or uses of the present invention encodes a lipid acyltransferase that comprises an aspartic acid residue at a position corresponding to N-80 in the amino acid sequence of the *Aeromonas hydrophila* lipid acyltransferase shown as SEQ ID No. 34.

In some aspects of the present invention, the lipid acyltransferase for use in any one of the methods and/or uses of the present invention is a lipid acyltransferase that comprises an aspartic acid residue at a position corresponding to N-80 in the amino acid sequence of the *Aeromonas hydrophila* lipid acyltransferase shown as SEQ ID No. 34.

In addition or in the alternative, the nucleotide sequence encoding a lipid acyltransferase for use in any one of the methods and/or uses of the present invention encodes a lipid acyltransferase that may comprise the amino acid sequence shown as SEQ ID No. 16, or an amino acid sequence which has 75% or more homology thereto. Suitably, the nucleotide sequence encoding a lipid acyltransferase encodes a lipid acyltransferase that may comprise the amino acid sequence shown as SEQ ID No. 16.

In addition or in the alternative, the nucleotide sequence encoding a lipid acyltransferase for use in any one of the methods and/or uses of the present invention encodes a lipid acyltransferase that may comprise the amino acid sequence shown as SEQ ID No. 68, or an amino acid sequence which has 75% or more homology thereto. Suitably, the nucleotide sequence encoding a lipid acyltransferase encodes a lipid acyltransferase that may comprise the amino acid sequence shown as SEQ ID No. 68.

In one embodiment the lipid acyltransferase for use in any on of the methods and/or uses of the present invention has an amino acid sequence shown in SEQ ID No. 16 or SEQ ID No. 68, or has an amino acid sequence which has at least 75% identity therewith, preferably at least 80%, preferably at least 85%, preferably at least 95%, preferably at least 98% identity therewith.

Preferably, the lipid acyltransferase enzyme may be characterised using the following criteria:

the enzyme possesses acyl transferase activity which may be defined as ester transfer activity whereby the acyl part of an original ester bond of a lipid acyl donor is transferred to an acyl acceptor, preferably glycerol or cholesterol, to form a new ester; and

the enzyme comprises the amino acid sequence motif GDSX, wherein X is one or more of the following amino acid residues L, A, V, I, F, Y, H, Q, T, N, M or S.

Preferably, X of the GDSX motif is L or Y. More preferably, X of the GDSX motif is L. Thus, preferably the enzyme according to the present invention comprises the amino acid sequence motif GDSL.

The GDSX motif is comprised of four conserved amino acids. Preferably, the serine within the motif is a catalytic serine of the lipid acyl transferase enzyme. Suitably, the serine of the GDSX motif may be in a position corresponding to Ser-16 in *Aeromonas hydrophila* lipid acyltransferase enzyme taught in Brumlik & Buckley (Journal of Bacteriology Apr. 1996, Vol. 178, No. 7, p 2060-2064).

To determine if a protein has the GDSX motif according to the present invention, the sequence is preferably compared with the hidden markov model profiles (HMM profiles) of the pfam database in accordance with the procedures taught in WO 2004/064537 or WO 2004/064987.

Preferably the lipid acyl transferase enzyme can be aligned using the Pfam00657 consensus sequence (for a full explanation see WO 2004/064537 or WO 2004/064987).

Preferably, a positive match with the hidden markov model profile (HMM profile) of the pfam00657 domain family indicates the presence of the GDSL or GDSX domain according to the present invention.

Preferably when aligned with the Pfam00657 consensus sequence the lipid acyltransferase for use in the methods or uses of the invention may have at least one, preferably more than one, preferably more than two, of the

following, a GDSx block, a GANDY block, a HPT block. Suitably, the lipid acyltransferase may have a GDSx block and a GANDY block. Alternatively, the enzyme may have a GDSx block and a HPT block. Preferably the enzyme comprises at least a GDSx block. See WO 2004/064537 or WO 2004/064987 for further details.

Preferably, residues of the GANDY motif are selected from GANDY, GGNDA, GGNDL, most preferably GANDY.

Preferably, when aligned with the Pfam00657 consensus sequence the enzyme for use in the methods or uses of the invention have at least one, preferably more than one, preferably more than two, preferably more than three, preferably more than four, preferably more than five, preferably more than six, preferably more than seven, preferably more than eight, preferably more than nine, preferably more than ten, preferably more than eleven, preferably more than twelve, preferably more than thirteen, preferably more than fourteen, of the following amino acid residues when compared to the reference *A. hydrophilia* polypeptide sequence, namely SEQ ID No. 1: 28His, 29His, 30His, 31His, 32Gly, 33Asp, 34Ser, 35His, 130His, 131Gly, 132His, 133Asn, 134Asp, 135His, 309His.

The pfam00657 GDSX domain is a unique identifier which distinguishes proteins possessing this domain from other enzymes.

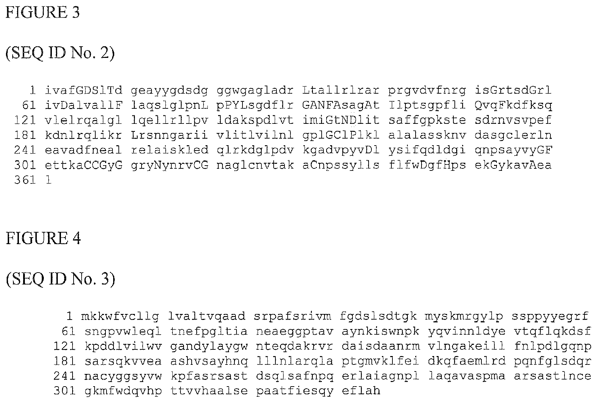

The pfam00657 consensus sequence is presented in Figure 3 as SEQ ID No. 2. This is derived from the identification of the pfam family 00657, database version 6, which may also be referred to as pfam00657.6 herein.

The consensus sequence may be updated by using further releases of the pfam database (for example see WO 2004/064537 or WO 2004/064987).

In one embodiment, the lipid acyl transferase enzyme for use in any one of the methods and/or uses of the present invention is a lipid acyltransferase that may be characterised using the following criteria:

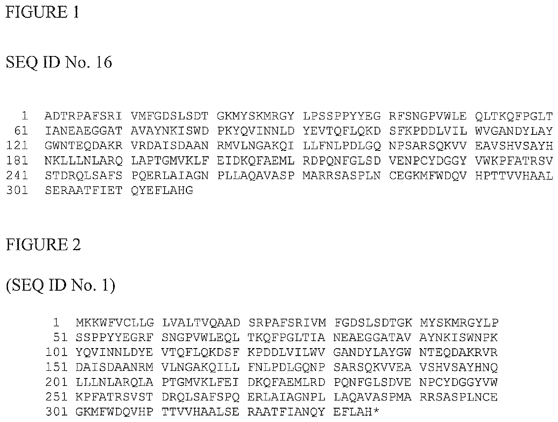

(i) the enzyme possesses acyl transferase activity which may be defined as ester transfer activity whereby the acyl part of an original ester bond of a lipid acyl donor is transferred to acyl acceptor, preferably glycerol or cholesterol, to form a new ester, preferably monoglyceride or cholesterol ester respectfully;
(ii) the enzyme comprises the amino acid sequence motif GDSX, wherein X is one or more of the following amino acid residues L, A, V, I, F, Y, H, Q, T, N, M or S;
(iii) the enzyme comprises His-309 or comprises a histidine residue at a position corresponding to His-309 in the *Aeromonas hydrophila* lipid acyltransferase enzyme shown in Figures 2 and 4 (SEQ ID No. 1 or SEQ ID No. 3).

Preferably, the amino acid residue of the GDSX motif is L.

In SEQ ID No. 3 or SEQ ID No. 1 the first 18 amino acid residues form a signal sequence. His-309 of the full length sequence, that is the protein including the signal sequence, equates to His-291 of the mature part of the protein, i.e. the sequence without the signal sequence.

In one embodiment, the lipid acyl transferase enzyme for use any one of the methods and uses of the present invention is a lipid acyltransferase that comprises the following catalytic triad: Ser-34, Asp-306 and His-309 or comprises a serine residue, an aspartic acid residue and a histidine residue, respectively, at positions corresponding to Ser-34, Asp-306 and His-309 in the *Aeromonas hydrophila* lipid acyl transferase enzyme shown in Figure 4 (SEQ ID No. 3) or Figure 2 (SEQ ID No. 1). As stated above, in the sequence shown in SEQ ID No. 3 or SEQ ID No. 1 the first 18 amino acid residues form a signal sequence. Ser-34, Asp-306 and His-309 of the full length sequence, that is the protein including the signal sequence, equate to Ser-16, Asp-288 and His-291 of the mature part of the protein, i.e. the sequence without the signal sequence. In the pfam00657 consensus sequence, as given in Figure 3 (SEQ ID No. 2) the active site residues correspond to Ser-7, Asp-345 and His-348.

In one embodiment, the lipid acyl transferase enzyme for use in any one of the methods and/or uses of the present invention is a lipid acyl transferase that may be characterised using the following criteria:

the enzyme possesses acyl transferase activity which may be defined as ester transfer activity whereby the acyl part of an original ester bond of a first lipid acyl donor is transferred to an acyl acceptor to form a new ester; and

the enzyme comprises at least Gly-32, Asp-33, Ser-34, Asp-134 and His-309 or comprises glycine, aspartic acid, serine, aspartic acid and histidine residues at positions corresponding to Gly-32, Asp-33, Ser-34, Asp-306 and His-309, respectively, in the *Aeromonas hydrophila* lipid acyltransferase enzyme shown in SEQ ID No. 3 or SEQ ID No. 1.

Suitably, the lipid acyltransferase enzyme for use in any one of the methods and/or uses of the present invention may be encoded by one of the following nucleotide sequences:

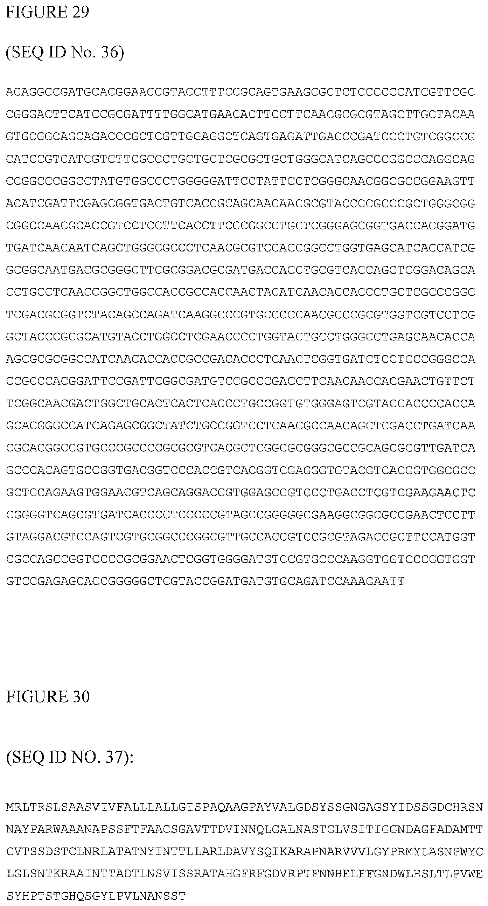

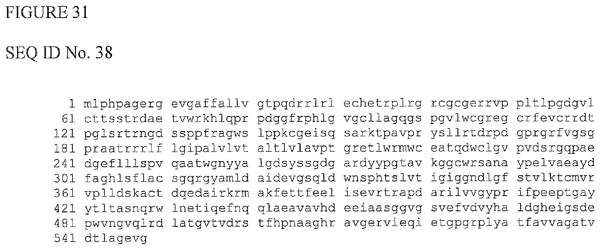

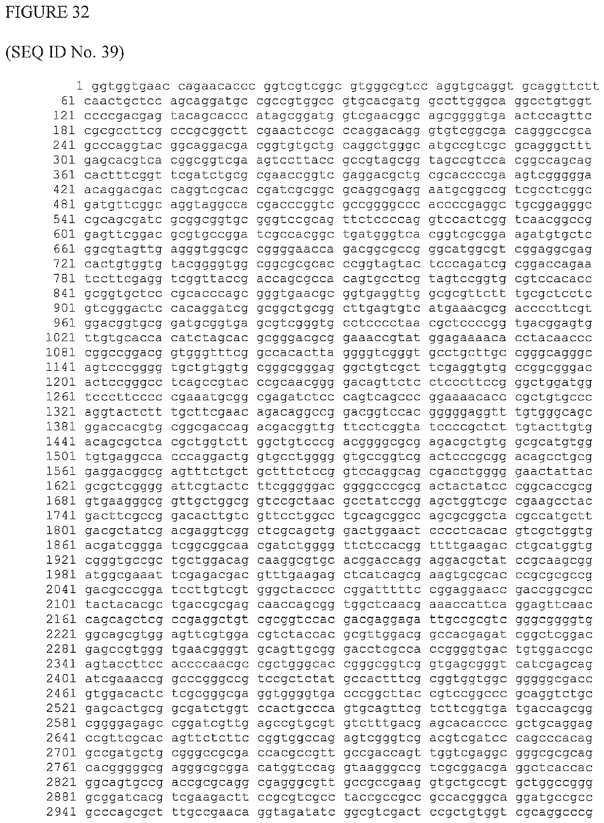

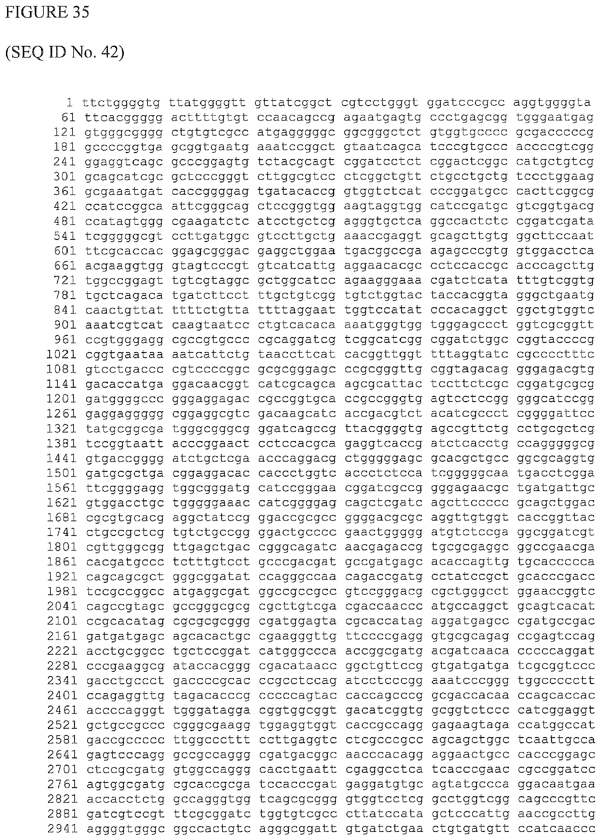

(a) the nucleotide sequence shown as SEQ ID No. 36 (see Figure 29);
(b) the nucleotide sequence shown as SEQ ID No. 38 (see Figure 31);
(c) the nucleotide sequence shown as SEQ ID No. 39 (see Figure 32);
(d) the nucleotide sequence shown as SEQ ID No. 42 (see Figure 35);

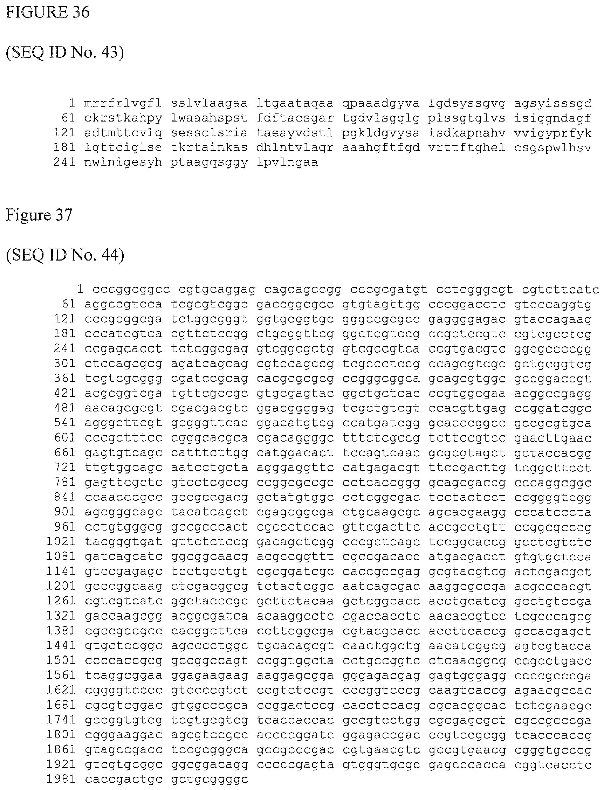

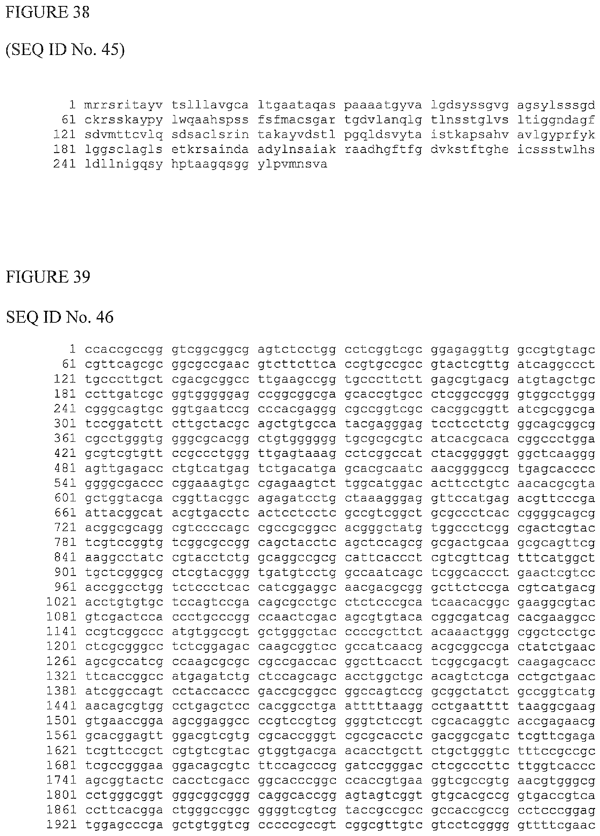

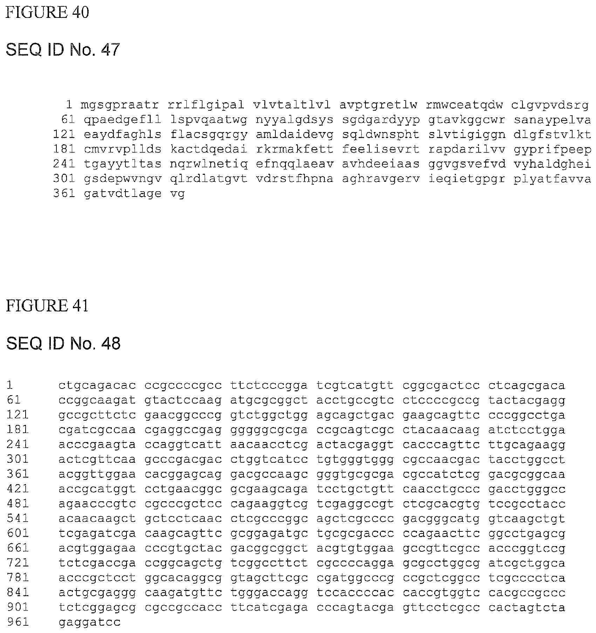

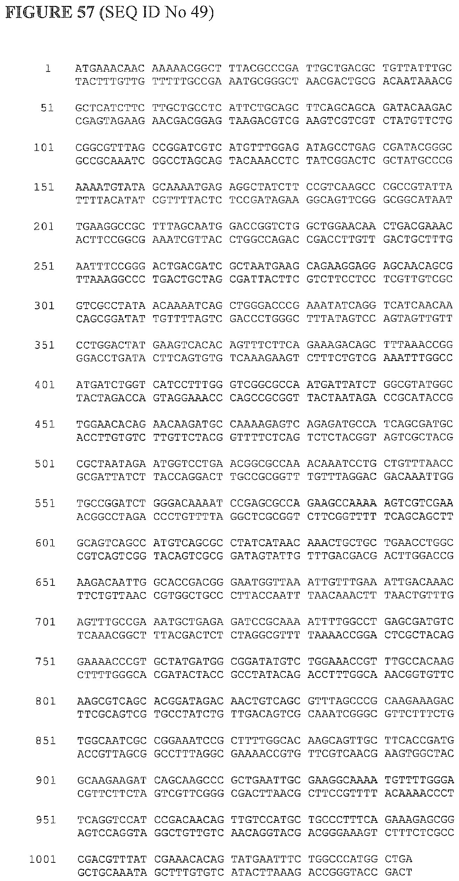

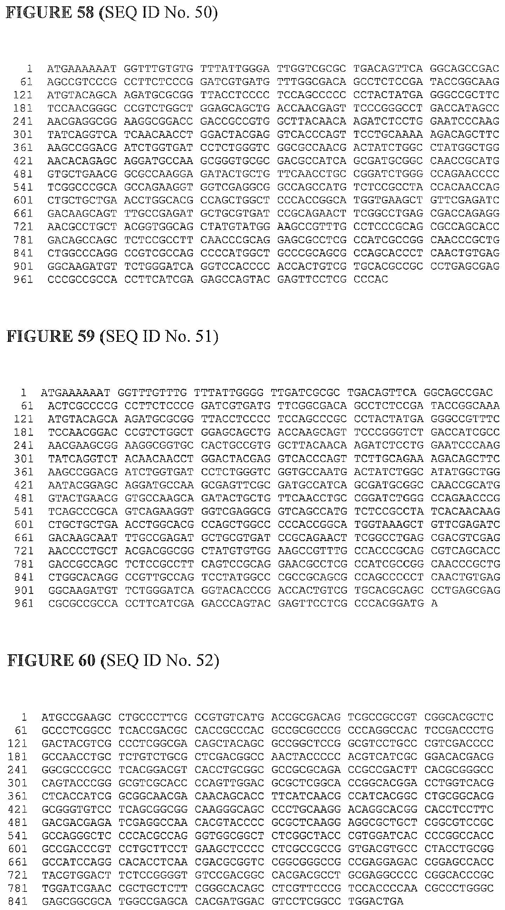

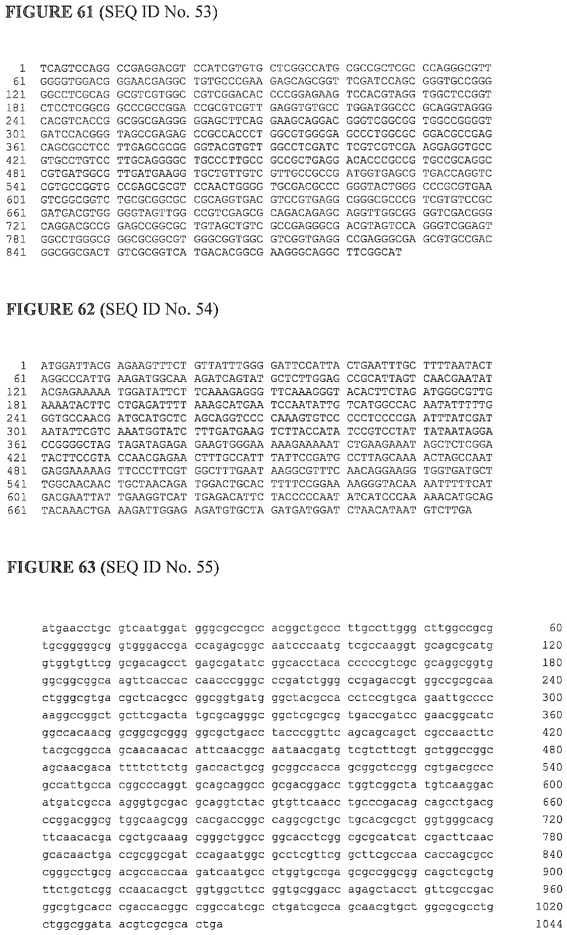

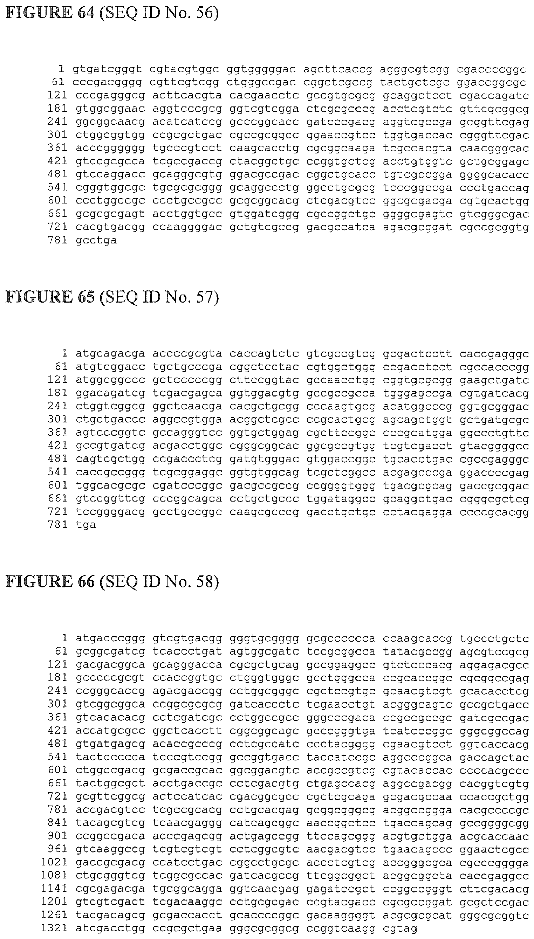

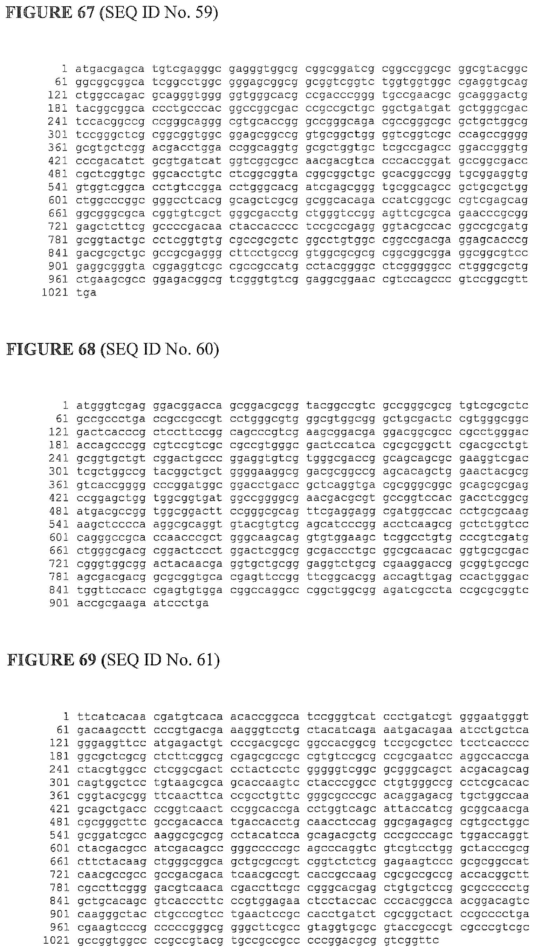

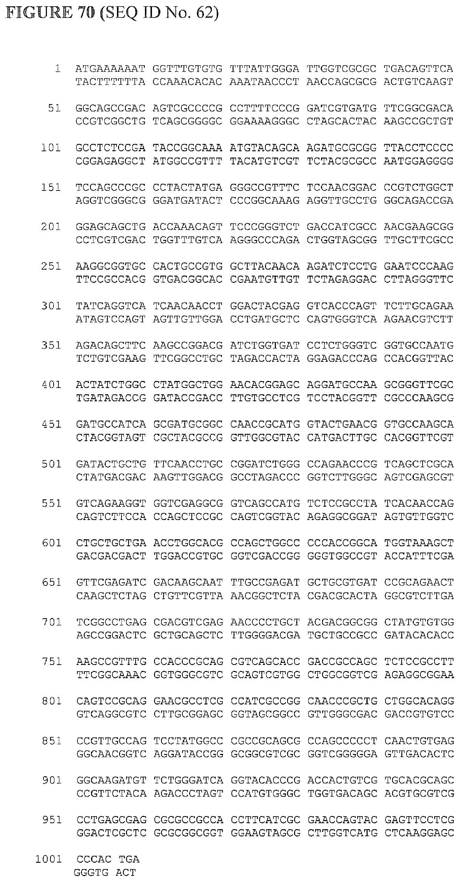

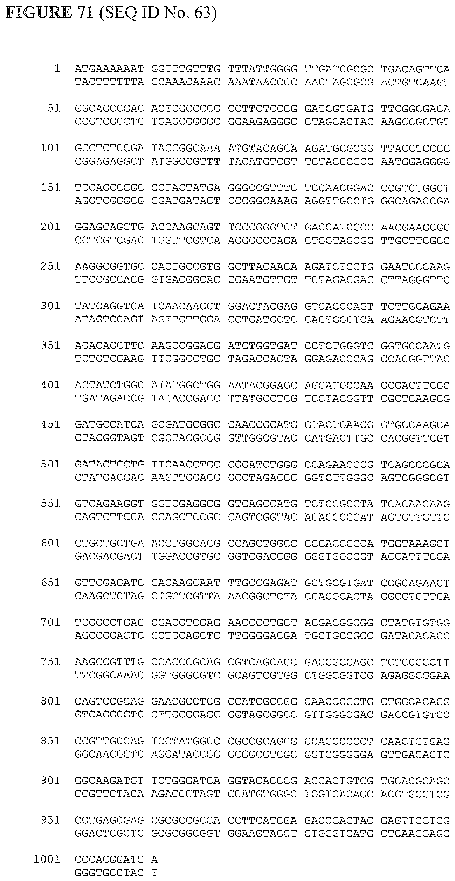

(e) the nucleotide sequence shown as SEQ ID No. 44 (see Figure 37);
(f) the nucleotide sequence shown as SEQ ID No. 46 (see Figure 39);
(g) the nucleotide sequence shown as SEQ ID No. 48 (see Figure 41);
(h) the nucleotide sequence shown as SEQ ID No. 49 (see Figure 57);
(i) the nucleotide sequence shown as SEQ ID No. 50 (see Figure 58);
(j) the nucleotide sequence shown as SEQ ID No. 51 (see Figure 59);
(k) the nucleotide sequence shown as SEQ ID No. 52 (see Figure 60);
(l) the nucleotide sequence shown as SEQ ID No. 53 (see Figure 61);
(m)the nucleotide sequence shown as SEQ ID No. 54 (see Figure 62);
(n) the nucleotide sequence shown as SEQ ID No. 55 (see Figure 63);
(o) the nucleotide sequence shown as SEQ ID No. 56 (see Figure 64);
(p) the nucleotide sequence shown as SEQ ID No. 57 (see Figure 65);
(q) the nucleotide sequence shown as SEQ ID No. 58 (see Figure 66);
(r) the nucleotide sequence shown as SEQ ID No. 59 (see Figure 67);
(s) the nucleotide sequence shown as SEQ ID No. 60 (see Figure 68);
(t) the nucleotide sequence shown as SEQ ID No. 61 (see Figure 69);
(u) the nucleotide sequence shown as SEQ ID No. 62 (see Figure 70);
(v) the nucleotide sequence shown as SEQ ID No. 63 (see Figure 71);
(w) or

a nucleotide sequence which has 70% or more, preferably 75% or more, identity with any one of the sequences shown as SEQ ID No. 36, SEQ ID No. 38, SEQ ID No. 39, SEQ ID No. 42, SEQ ID No. 44, SEQ ID No. 46, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62 or SEQ ID No. 63.

Suitably the nucleotide sequence may have 80% or more, preferably 85% or more, more preferably 90% or more and even more preferably 95% or more identity with any one of the sequences shown as SEQ ID No. 36, SEQ ID No. 38, SEQ ID No. 39, SEQ ID No. 42, SEQ ID No. 44, SEQ ID No. 46, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62 or SEQ ID No. 63.

In one embodiment, the nucleotide sequence encoding a lipid acyltransferase enzyme for use any one of the methods and uses of the present invention is a nucleotide sequence which has 70% or more, preferably 75% or more, identity with any one of the sequences shown as: SEQ ID No. 49, SEQ ID No. 50, SEQ ID No. 51, SEQ ID No. 62, and SEQ ID No. 63. Suitably the nucleotide sequence may have 80% or more, preferably 85% or more, more preferably 90% or more and even more preferably 95% or more identity with any one of the sequences shown as: SEQ ID No. 49, SEQ ID No. 50, SEQ ID No. 51, SEQ ID No. 62, and SEQ ID No. 63.

In one embodiment, the nucleotide sequence encoding a lipid acyltransferase enzyme for use in any one of the methods and uses of the present invention is a nucleotide sequence which has 70% or more, 75% or more, 80% or more, preferably 85% or more, more preferably 90% or more and even more preferably 95% or more identity the sequence shown as SEQ ID No. 49.

Suitably, the lipid acyl transferase enzyme for use in any one of the methods and/or uses of the present invention may be a lipid acyltransferase that comprises one or more of the following amino acid sequences:

(i) the amino acid sequence shown as SEQ ID No. 3
(ii) the amino acid sequence shown as SEQ ID No. 4
(iii) the amino acid sequence shown as SEQ ID No. 5
(iv) the amino acid sequence shown as SEQ ID No. 6
(v) the amino acid sequence shown as SEQ ID No. 7
(vi) the amino acid sequence shown as SEQ ID No. 8
(vii) the amino acid sequence shown as SEQ ID No. 9
(viii) the amino acid sequence shown as SEQ ID No. 10
(ix) the amino acid sequence shown as SEQ ID No. 11
(x) the amino acid sequence shown as SEQ ID No. 12
(xi) the amino acid sequence shown as SEQ ID No. 13
(xii) the amino acid sequence shown as SEQ ID No. 14
(xiii) the amino acid sequence shown as SEQ ID No. 1
(xiv) the amino acid sequence shown as SEQ ID No. 15
(xv) the amino acid sequence shown as SEQ ID No. 16
(xvi) the amino acid sequence shown as SEQ ID No. 17

(xvii) the amino acid sequence shown as SEQ ID No. 18
(xviii) the amino acid sequence shown as SEQ ID No. 34
(xix) the amino acid sequence shown as SEQ ID No. 35
(xx) the amino acid sequence shown as SEQ ID No. 68
(xxi) the amino acid sequence shown as SEQ ID No. 121
(xxii) the amino acid sequence shown as SEQ ID No. 122
(xxiii) the amino acid sequence shown as SEQ ID No. 123 or

an amino acid sequence which has 75%, 80%, 85%, 90%, 95%, 98% or more identity with any one of the sequences shown as SEQ ID No. 1, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14 or SEQ ID No. 15, SEQ ID No. 16, SEQ ID No. 17, SEQ ID No. 18, SEQ ID No. 34, SEQ ID No. 35, SEQ ID No. 68, SEQ ID No. 121, SEQ ID No. 122 or SEQ ID No. 123.

Suitably, the lipid acyl transferase enzyme for use in any one of the methods and uses of the present invention may be a lipid acyltransferase that comprises either the amino acid sequence shown as SEQ ID No. 3 or as SEQ ID No. 4 or SEQ ID No. 1 or SEQ ID No. 15 or SEQ ID No. 16, or SEQ ID No. 34, SEQ ID No. 35, SEQ ID No. 68, SEQ ID No. 121, SEQ ID No. 122 or SEQ ID No. 123 or comprises an amino acid sequence which has 75% or more, preferably 80% or more, preferably 85% or more, preferably 90% or more, preferably 95% or more, identity with the amino acid sequence shown as SEQ ID No. 3 or the amino acid sequence shown as SEQ ID No. 4 or the amino acid sequence shown as SEQ ID No. 1 or the amino acid sequence shown as SEQ ID No. 15 or the amino acid sequence shown as SEQ ID No. 16 or the amino acid sequence shown as SEQ ID No. 34 or the amino acid sequence shown as SEQ ID No. 35 or the amino acid sequence shown as SEQ ID No. 68 or the amino acid sequence shown as SEQ ID No. 121 or the amino acid sequence shown as SEQ ID No. 122 or the amino acid sequence shown as SEQ ID No. 123.

Suitably the lipid acyl transferase enzyme for use any one of the methods and/or uses of the present invention may be a lipid acyltransferase that comprises an amino acid sequence which has 80% or more, preferably 85% or more, more preferably 90% or more and even more preferably 95% or more identity with any one of the sequences shown as SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 1, SEQ ID No. 15, SEQ ID No. 16, SEQ ID No. 17, SEQ ID No. 18, SEQ ID No. 34 or SEQ ID No. 35, SEQ ID No. 68, SEQ ID No. 121, SEQ ID No. 122 or SEQ ID No. 123.

Suitably, the lipid acyl transferase enzyme for use any one of the methods and/or uses of the present invention may be a lipid acyltransferase that comprises one or more of the following amino acid sequences:

(a) an amino acid sequence shown as amino acid residues 1-100 of SEQ ID No. 3 or SEQ ID No. 1;
(b) an amino acid sequence shown as amino acids residues 101-200 of SEQ ID No. 3 or SEQ ID No. 1;
(c) an amino acid sequence shown as amino acid residues 201-300 of SEQ ID No. 3 or SEQ ID No. 1; or
(d) an amino acid sequence which has 75% or more, preferably 85% or more, more preferably 90% or more, even more preferably 95% or more identity to any one of the amino acid sequences defined in (a)-(c) above.

Suitably, the lipid acyl transferase enzyme for use in methods and uses of the present invention may comprise one or more of the following amino acid sequences:

(a) an amino acid sequence shown as amino acid residues 28-39 of SEQ ID No. 3 or SEQ ID No. 1;
(b) an amino acid sequence shown as amino acids residues 77-88 of SEQ ID No. 3 or SEQ ID No. 1;
(c) an amino acid sequence shown as amino acid residues 126-136 of SEQ ID No. 3 or SEQ ID No. 1;
(d) an amino acid sequence shown as amino acid residues 163-175 of SEQ ID No. 3 or SEQ ID No. 1;
(e) an amino acid sequence shown as amino acid residues 304-311 of SEQ ID No. 3 or SEQ ID No. 1; or
(f) an amino acid sequence which has 75% or more, preferably 85% or more, more preferably 90% or more, even more preferably 95% or more identity to any one of the amino acid sequences defined in (a)-(e) above.

In one aspect, the lipid acyl transferase enzyme for use any one of the methods and/or uses of the present invention is a lipid acyltransferase that may be the lipid acyl transferase from *Candida parapsilosis* as taught in EP 1 275 711. Thus in one aspect the lipid acyl transferase for use in the method and uses of the present invention may be a lipid acyl transferase comprising one of the amino acid sequences taught in SEQ ID No. 17 or SEQ ID No. 18.

Much by preference, the lipid acyl transferase enzyme for use in any one of the methods and uses of the present invention is a lipid acyltransferase that may be a lipid acyl transferase comprising the amino acid sequence shown as SEQ ID No. 16, or an amino acid sequence which has 75% or more, preferably 85% or more, more preferably 90% or more, even more preferably 95% or more, even more preferably 98% or more, or even more preferably 99% or more

identity to SEQ ID No. 16. This enzyme could be considered a variant enzyme.

In one aspect, the lipid acyltransferase enzyme for use any one of the methods and/or uses of the present invention is a lipid acyltransferase that may be a lecithin:cholesterol acyltransferase (LCAT) or variant thereof (for example a variant made by molecular evolution)

Suitable LCATs are known in the art and may be obtainable from one or more of the following organisms for example: mammals, rat, mice, chickens, *Drosophila melanogaster,* plants, including Arabidopsis and *Oryza sativa,* nematodes, fungi and yeast.

In one embodiment the lipid acyltransferase enzyme for use any one of the methods and/or uses of the present invention is a lipid acyltransferase that may be the lipid acyltransferase obtainable, preferably obtained, from the E. coli strains TOP 10 harbouring pPet12aAhydro and pPet12aASalmo deposited by Danisco A/S of Langebrogade 1, DK-1001 Copenhagen K, Denmark under the Budapest Treaty on the International Recognition of the Deposit of Microorganisms for the purposes of Patent Procedure at the National Collection of Industrial, Marine and Food Bacteria (NCIMB) 23 St. Machar Street, Aberdeen, Scotland, United Kingdom on 22 December 2003 under accession numbers NCIMB 41204 and NCIMB 41205, respectively.

A lipid acyltransferase enzyme for use in any one of the methods and/or uses of the present invention may be a phospholipid glycerol acyl transferase. Phospholipid glycerol acyl transferases include those isolated from *Aeromonas spp.,* preferably *Aeromonas hydrophila* or *A. salmonicida,* most preferably *A. salmonicida* or variants thereof.

Most preferred lipid acyl transferases for use in the present invention are encoded by SEQ ID No.s 1, 3, 4, 15, 16, 34 and 35. It will be recognised by the skilled person that it is preferable that the signal peptides of the acyl transferase has been cleaved during expression of the transferase. The signal peptide of SEQ ID No.s 1, 3, 4, and 15 are amino acids 1-18. Therefore the most preferred regions are amino acids 19-335 for SEQ ID No. 1 and SEQ ID No. 3 (*A. hydrophilia*) and amino acids 19-336 for SEQ ID No. 4, and SEQ ID No. 15 (*A. salmonicida*). When used to determine the homology of identity of the amino acid sequences, it is preferred that the alignments as herein described use the mature sequence.

In one embodiment, suitably the lipid acyl transferase for use in the present invention comprises (or consists of) the amino acid sequence shown in SEQ ID No. 16 or comprises (or consists of) an amino acid sequence which has at least 70%, at least 75%, at least 85%, at least 90%, at least 95%, at least 98% identity to SEQ ID No. 16.

In one embodiment, suitably the lipid acyl transferase for use in the present invention is encoded by a nucleotide sequence comprising (or consisting of) a nucleotide sequence shown in SEQ ID No. 49 or comprises (or consists of) a nucleotide sequence which has at least 70%, at least 75%, at least 85%, at least 90%, at least 95%, at least 98% identity to SEQ ID No. 49.

Therefore the most preferred regions for determining homology (identity) are amino acids 19-335 for SEQ ID No. 1 and 3 (*A. hydrophilia*) and amino acids 19-336 for SEQ ID No.s 4, 15 (*A. salmonicida*). SEQ ID No.s 34 and 35 are mature protein sequences of a lipid acyl transferase from *A. hydrophilia* and A. *salmonicida* respectively which may or may not undergo further post-translational modification.

A lipid acyltransferase enzyme for use any one of the methods and uses of the present invention may be a lipid acyltransferase that may also be isolated from *Thermobifida,* preferably *T. fusca,* most preferably that encoded by SEQ ID No. 28.

Suitable lipid acyltransferases for use in accordance with the present invention and/or in the methods of the present invention may comprise any one of the following amino acid sequences and/or be encoded by the following nucleotide sequences:

a) a nucleic acid which encodes a polypeptide exhibiting lipid acyltransferase activity and is at least 70% identical (preferably at least 80%, more preferably at least 90% identical) with the polypeptide sequence shown in SEQ ID No. 16 or with the polypeptide shown in SEQ ID no. 68 or with the polypeptide shown in SEQ ID no. 121 or with the polypeptide shown in SEQ ID no. 122 or with the polypeptide shown in SEQ ID no. 123;
b) a (isolated) polypeptide comprising (or consisting of) an amino acid sequence as shown in SEQ ID No. 16 or SEQ ID No. 68 or an amino acid sequence which is at least 70% identical (preferably at least 80% identical, more preferably at least 90% identical) with SEQ ID No. 16, SEQ ID No. 68, SEQ ID No. 121, SEQ ID No. 122 or SEQ ID No. 123;
c) a nucleic acid encoding a lipid acyltransferase, which nucleic acid comprises (or consists of) a nucleotide sequence shown as SEQ ID No. 49 or a nucleotide sequence which is at least 70% identical (preferably at least 80%, more preferably at least 90% identical) with the nucleotide sequence shown as SEQ ID No. 49;
d) a nucleic acid which hybridises under medium or high stringency conditions to a nucleic acid probe comprising the nucleotide sequence shown as SEQ ID No. 49 and encodes for a polypeptide exhibiting lipid acyltransferase activity;
e) a nucleic acid which is a fragment of the nucleic acid sequences specified in a), c) or d); or
f) a polypeptide which is a fragment of the polypeptide specified in b).

A lipid acyltransferase enzyme for use any one of the methods and uses of the present invention may be a lipid acyltransferase that may also be isolated from *Streptomyces,* preferable *S. avermitis,* most preferably that encoded by SEQ ID No. 32. Other possible enzymes for use in the present invention from *Streptomyces* include those encoded by SEQ ID No.s 5, 6, 9, 10, 11, 12, 13, 14, 31, and 33.

An enzyme for use in the invention may also be isolated from *Corynebacterium,* preferably C. *efficiens,* most preferably that encoded by SEQ ID No. 29.

Suitably, the lipid acyltransferase enzyme for use any one of the methods and/or uses of the present invention may be a lipid acyltransferase that comprises any one of the amino acid sequences shown as SEQ ID Nos. 37, 38, 40, 41, 43, 45, or 47 or an amino acid sequence which has at least 70%, 75%, 80%, 85%, 90%, 95%, 96%, 97% or 98% identity therewith, or may be encoded by any one of the nucleotide sequences shown as SEQ ID Nos. 36, 39, 42, 44, 46, or 48 or a nucleotide sequence which has at least 70%, 75%, 80%, 85%, 90%, 95%, 96%, 97% or 98% identity therewith.

In one embodiment, the nucleotide sequence encoding a lipid acyltransferase enzyme for use any one of the methods and/or uses of the present invention is selected from the group consisting of:

a) a nucleic acid comprising a nucleotide sequence shown in SEQ ID No. 36;
b) a nucleic acid which is related to the nucleotide sequence of SEQ ID No. 36 by the degeneration of the genetic code; and
c) a nucleic acid comprising a nucleotide sequence which has at least 70% identity with the nucleotide sequence shown in SEQ ID No. 36.

In one embodiment, the lipid acyltransferase enzyme for use any one of the methods and/or uses of the present invention is a lipid acyltransferase that comprises an amino acid sequence as shown in SEQ ID No. 37 or an amino acid sequence which has at least 60% identity thereto.

In a further embodiment the lipid acyltransferase enzyme for use any one of the methods and/or uses of the present invention may be a lipid acyltransferase comprising any one of the amino acid sequences shown as SEQ ID No. 37, 38, 40, 41, 43, 45 or 47 or an amino acid sequence which has at least 70%, 75%, 80%, 85%, 90%, 95%, 96%, 97% or 98% identity therewith, or may be encoded by any one of the nucleotide sequences shown as SEQ ID No. 39, 42, 44, 46 or 48 or a nucleotide sequence which has at least 70%, 75%, 80%, 85%, 90%, 95%, 96%, 97% or 98% identity therewith.

In a further embodiment the lipid acyltransferase enzyme for use any one of the methods and/or uses of the present invention may be a lipid acyltransferase comprising any one of amino sequences shown as SEQ ID No. 38, 40, 41, 45 or 47 or an amino acid sequence which has at least 70%, 75%, 80%, 85%, 90%, 95%, 96%, 97% or 98% identity therewith for the uses described herein.

In a further embodiment the lipid acyltransferase for use in any one of the methods and/or uses of the present invention may be a lipid acyltransferase comprising any one of amino sequences shown as SEQ ID No. 38, 40, or 47 or an amino acid sequence which has at least 70%, 75%, 80%, 85%, 90%, 95%, 96%, 97% or 98% identity therewith for the uses described herein.

More preferably in one embodiment the lipid acyltransferase for use in any one of the methods and/or uses of the present invention may be a lipid acyltransferase comprising the amino acid sequence shown as SEQ ID No. 47 or an amino acid sequence which has at least 70%, 75%, 80%, 85%, 90%, 95%, 96%, 97% or 98% identity therewith.

In another embodiment the lipid acyltransferase for use in any one of the methods and uses of the present invention may be a lipid acyltransferase comprising the amino acid sequence shown as SEQ ID No. 43 or 44 or an amino acid sequence which has at least 80%, 85%, 90%, 95%, 96%, 97% or 98% identity therewith.

In another embodiment the lipid acyltransferase for use in any one of the methods and uses of the present invention may be a lipid acyltransferase comprising the amino acid sequence shown as SEQ ID No. 41 or an amino acid sequence which has at least 70%, 75%, 80%, 85%, 90%, 95%, 96%, 97% or 98% identity therewith.

In one embodiment the lipid acyltransferase for use in any one of the methods and uses of the present invention may be encoded by a nucleic acid selected from the group consisting of:

a) a nucleic acid comprising a nucleotide sequence shown in SEQ ID No. 36;
b) a nucleic acid which is related to the nucleotide sequence of SEQ ID No. 36 by the degeneration of the genetic code; and
c) a nucleic acid comprising a nucleotide sequence which has at least 70% identity with the nucleotide sequence shown in SEQ ID No. 36.

In one embodiment the lipid acyltransferase according to the present invention may be a lipid acyltransferase obtainable, preferably obtained, from the *Streptomyces* strains L130 or L131 deposited by Danisco A/S of Langebrogade

1, DK-1001 Copenhagen K, Denmark under the Budapest Treaty on the International Recognition of the Deposit of Microorganisms for the purposes of Patent Procedure at the National Collection of Industrial, Marine and Food Bacteria (NCIMB) 23 St. Machar Street, Aberdeen, Scotland, United Kingdom on 23 June 2004 under accession numbers NCIMB 41226 and NCIMB 41227, respectively.

Suitable nucleotide sequences encoding a lipid acyltransferase for use in any one of the methods and/or uses of the present invention may encode a polynucleotide encoding a lipid acyltransferase (SEQ ID No. 16); or may encode an amino acid sequence of a lipid acyltransferase (SEQ ID No. 16).

A suitable lipid acyltransferases for use in any one of the methods and/or uses of the present invention may be an amino acid sequence which may be identified by alignment to the L131 (SEQ ID No. 37) sequence using Align X, the Clustal W pairwise alignment algorithm of VectorNTI using default settings.

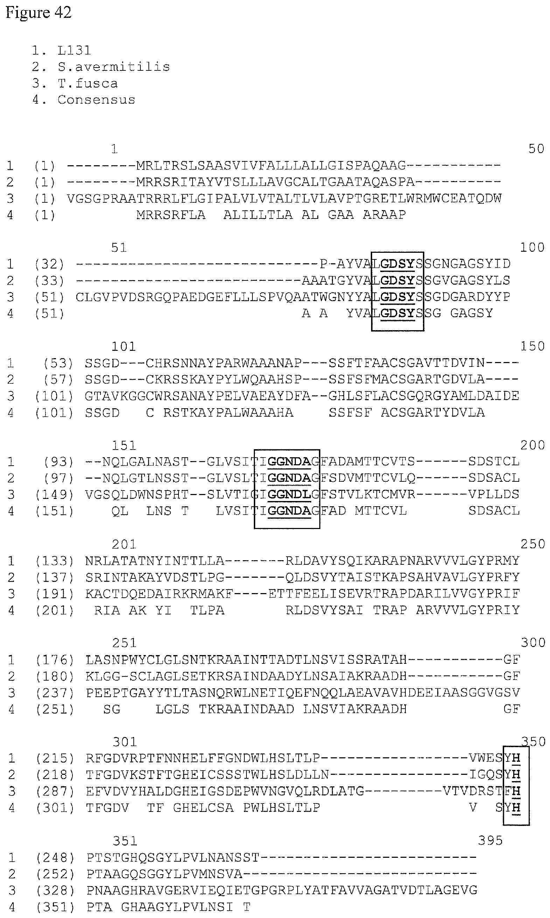

An alignment of the L131 and homologues from S. *avermitilis* and *T. fusca* illustrates that the conservation of the GDSx motif (GDSY in L131 and S. *avermitilis* and *T. fusca*), the GANDY box, which is either GGNDA or GGNDL, and the HPT block (considered to be the conserved catalytic histidine). These three conserved blocks are highlighted in Figure 42.

When aligned to either the pfam Pfam00657 consensus sequence (as described in WO 2004/064987) and/ or the L131 sequence herein disclosed (SEQ ID No 37) it is possible to identify three conserved regions, the GDSx block, the GANDY block and the HTP block (see WO 2004/064987 for further details).

When aligned to either the pfam Pfam00657 consensus sequence (as described in WO 2004/064987) and/ or the L131 sequence herein disclosed (SEQ ID No 37)

i) The lipid acyltransferase for use in any one of the methods and uses of the present invention may be a lipid acyltransferase that has a GDSx motif, more preferably a GDSx motif selected from GDSL or GDSY motif.
and/or
ii) The lipid acyltransferase for use in any one of the methods and uses of the present invention may be a lipid acyltransferase that has a GANDY block, more preferably a GANDY block comprising amino GGNDx, more preferably GGNDA or GGNDL.
and/or
iii) The lipid acyltransferase for use in any one of the methods and uses of the present invention may be a lipid acyltransferase that has preferably an HTP block.
and preferably
iv) the lipid acyltransferase for use in any one of the methods and uses of the present invention may be a lipid acyltransferase that has preferably a GDSx or GDSY motif, and a GANDY block comprising amino GGNDx, preferably GGNDA or GGNDL, and a HTP block (conserved histidine).

The lipid acyltransferase as used herein may be referred to as a glycerophospholipid cholesterol acyltransferase. In other words the lipid acyltransferase for use in the present invention preferably has the ability to "hydrolyse" phospholipids and at the same time esterify cholesterol with the free fatty acid from the hydrolyzation this is effective a tranferase reaction (i.e. an interesterification and/or a transesterification reaction.

The degree of "hydrolysis" can be described as the ratio of phosphatidylcholine (PC) and/or phosphatidylethanolamine (PE) converted into lyso-PC or lyso-PE respectively. By the enzymatic hydrolyzation of PC into lyso-PC, the ratio between the hydrophilic part of the phospholipid molecule (polar head group) and the hydrophobic part (fatty acid chains) is altered. By removing one fatty acid (saturated and/or unsaturated fatty acids) the hydrophobic part is reduced, thus making the entire molecule more hydrophilic. Furthermore the sterical molecule conformation may be changed, which may influence phase structures (e.g. micellation) formed by the molecules in dispersion, as well as interactions with other molecules like e.g. milk proteins.

Lyso-lecithin products are known to possess improved emulsifying properties. With a high degree of interesterification and/or transesterification it is possible to obtain smaller mean oil droplet sizes in a comparative emulsification test.

**Phosphatidylcholine** **Cholesterol**

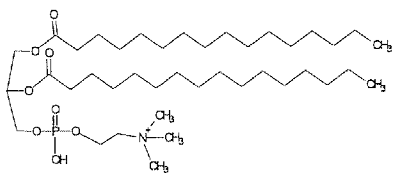

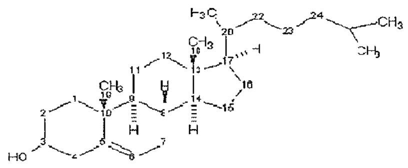

**+ lipid acyltransferase ⇓ enzyme**

**Lysophosphatidylcholine** **Cholesterol ester**

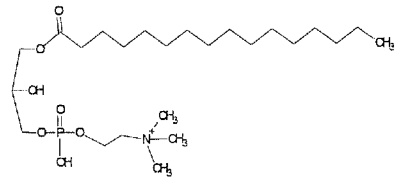

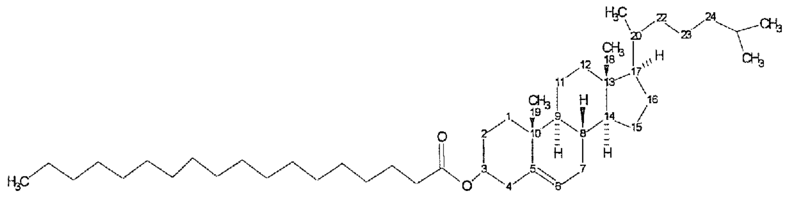

The function of lipid acyltransferase is that cholesterol and phospholipids will be changed into cholesterol-esters and lyso-phospholipids, giving two resulting components with surface-active properties in relation to O/W emulsions. Thus the final products will contain no or significantly reduced cholesterol and have an improved emulsion stability.

The enzyme according to the present invention is preferably not a phospholipase enzyme, such as a phospholipase A1 classified as E.C. 3.1.1.32 or a phospholipase A2 classified as E.C. 3.1.1.4.

**Variant lipid acyl transferase**

In a preferred embodiment the nucleotide sequence encoding a lipid acyltransferase for use in any one of the methods and/or uses of the present invention may encode a lipid acyltransferase that is a variant lipid acyl transferase.

Variants which have an increased activity on phospholipids, such as increased hydrolytic activity and/ or increased transferase activity, preferably increased transferase activity on phospholipids may be used.

Preferably the variant lipid acyltransferase is prepared by one or more amino acid modifications of the lipid acyl transferases as defined hereinabove.

Suitably, the lipid acyltransferase for use in any one of the methods and uses of the present invention may be a lipid acyltransferase that may be a variant lipid acyltransferase, in which case the enzyme may be characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues defined in set 2 or set 4 or set 6 or set 7 (as defined in WO 2005/066347 and hereinbelow).

For instance the variant lipid acyltransferase may be characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues detailed in set 2 or set 4 or set 6 or set 7 (as defined in WO 2005/066347 and hereinbelow) identified by said parent sequence being structurally aligned with the structural model of P10480 defined herein, which is preferably obtained by structural alignment of P10480 crystal structure coordinates with 1IVN.PDB and/or 1DEO.PDB as defined in WO 2005/066347 and hereinbelow.

In a further embodiment a lipid acyltransferase for use in any one of the methods and/or uses of the present invention may be a variant lipid acyltransferase that may be characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues taught in set 2 identified when said parent sequence is aligned to the pfam consensus sequence (SEQ ID No. 2 -Figure 3) and modified according to a structural model of P10480 to ensure best fit overlap as defined in WO 2005/066347 and hereinbelow.

Suitably a lipid acyltransferase for use in any one of the methods and uses of the present invention may be a variant lipid acyltransferase enzyme that may comprise an amino acid sequence, which amino acid sequence is shown as SEQ ID No. 34, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 1, SEQ ID No. 15, SEQ ID No. 25, SEQ ID No. 26, SEQ ID No. 27, SEQ ID No. 28, SEQ ID No. 29, SEQ ID No. 30, , SEQ ID No. 32, SEQ ID No. 33 or SEQ ID No. 35 except for one or more amino acid modifications at any one or more of the amino acid residues defined in set 2 or set 4 or set 6 or set 7 (as defined in WO 2005/066347 and hereinbelow) identified by sequence alignment with SEQ ID No. 34.

Alternatively the lipid acyltransferase may be a variant lipid acyltransferase enzyme comprising an amino acid sequence, which amino acid sequence is shown as SEQ ID No. 34, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 1, SEQ ID No. 15, SEQ ID No. 25, SEQ ID No. 26, SEQ ID No. 27, SEQ ID No. 28, SEQ ID No. 29, SEQ ID No. 30, , SEQ ID No. 32, SEQ ID No. 33 or SEQ ID No. 35 except for one or more amino acid modifications at any one or more of the amino acid residues defined in set 2 or set 4 or set 6 or set 7 as defined in WO 2005/066347 and hereinbelow, identified by said parent sequence being structurally aligned with the structural model of P10480 defined herein, which is preferably obtained by structural alignment of P10480 crystal structure coordinates with 1IVN.PDB and/or 1DEO.PDB as taught within WO 2005/066347 and hereinbelow.

Alternatively, the lipid acyltransferase may be a variant lipid acyltransferase enzyme comprising an amino acid sequence, which amino acid sequence is shown as SEQ ID No. 34, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 1, SEQ ID No. 15, SEQ ID No. 25, SEQ ID No. 26, SEQ ID No. 27, SEQ ID No. 28, SEQ ID No. 29, SEQ ID No. 30, SEQ ID No. 32, SEQ ID No. 33 or SEQ ID No. 35 except for one or more amino acid modifications at any one or more of the amino acid residues taught in set 2 identified when said parent sequence is aligned to the pfam consensus sequence (SEQ ID No. 2) and modified according to a structural model of P10480 to ensure best fit overlap as taught within WO 2005/066347 and hereinbelow.

Preferably, the parent enzyme is an enzyme which comprises, or is homologous to, the amino acid sequence shown as SEQ ID No. 34 and/or SEQ ID No. 15 and/or SEQ ID No. 35.

Preferably, the lipid acyltransferase may be a variant enzyme which comprises an amino acid sequence, which amino acid sequence is shown as SEQ ID No. 34 or SEQ ID No. 35 except for one or more amino acid modifications at any one or more of the amino acid residues defined in set 2 or set 4 or set 6 or set 7 as defined in WO 2005/066347 and hereinbelow.

Other suitable variant lipid acyltransferases for use in the methods/uses of the present invention are those described in PCT/IB2009/054535.

The tertiary structure of the lipid acyltransferases has revealed an unusual and interesting structure which allows lipid acyltransferases to be engineered more successfully. In particular the lipid acyltransferase tertiary structure has revealed a cave and canyon structure the residues forming these structures are defined herein below.

Alterations in the cave region may (for example) alter the enzyme's substrate chain length specificity for example.

Alterations in the canyon (particularly some preferred key modifications) have been found to be important in for example enhancing or changing the enzyme's substrate specificity.

In particular it has been found by the present inventors that there are a number of modifications in the canyon which rank highly and produce interesting variants with improved properties - these can be found at positions 31, 27, 85, 86, 119 and 120. In some embodiments positions 31 and/or 27 are highly preferred.

These variant lipid acyltransferase enzyme may be encoded by a nucleotide sequence which has at least 90% identity with a nucleotide sequence encoding a parent lipid acyltransferase and comprise at least one modification (suitably at least two modifications) at a position(s) which corresponds in the encoded amino acid sequence to an amino acid(s) located in a) the canyon region of the enzyme and/or b) insertion site 1 and/or c) insertion site 2, wherein the canyon region, insertion site 1 and/or insertion site 2 of the enzyme is defined as that region which when aligned based on primary or tertiary structure corresponds to the canyon region, insertion site 1 or insertion site 2 of the enzyme shown herein as SEQ ID No. 16 or SEQ ID No. 68 as described herein below.

In one embodiment preferably the modification(s) at a position located in the canyon and/or insertion site 1 and/or insertion site 2 is combined with at least one modification at a position which corresponds in the encoded amino

acid sequence to an amino acid located outside of the canyon region and/or insertion site 1 and/or insertion site 2.

In one embodiment, the lipid acyltransferase comprises at least one modification (suitably at least two modifications) at a position(s) which corresponds in the encoded amino acid sequence to an amino acid(s) located at position 27, 31, 85, 86, 122, 119, 120, 201, 245, 232, 235 and/or 236 (preferably at position 27, 31, 85, 86, 119 and/or 120, more preferably at position 27 and/or 31), wherein the position numbering is defined as that position which when aligned based on primary or tertiary structure corresponds to the same position of the enzyme shown herein as SEQ ID No. 16.

In a further embodiment, the variant lipid acyltransferase comprises at least one modification at a position(s) which corresponds in the encoded amino acid sequence to an amino acid(s) located at position 27 and/or 31 in combination with at least one further modification, wherein the position numbering is defined as that position which when aligned based on primary or tertiary structure corresponds to the same position of the enzyme shown herein as SEQ ID No. 16.

Suitably, the at least one further modification may be at one or more of the following positions 85, 86, 122, 119, 120, 201, 245, 23, 81, 82, 289, 227, 229, 233, 33, 207, 130, wherein the position numbering is defined as that position which when aligned based on primary or tertiary structure corresponds to the same position of the enzyme shown herein as SEQ ID No. 16.

The lipid acyltransferase amino acid sequence for use in the present invention may comprise a modified backbone such that at least one modification (suitably at least two modifications) is made at a position(s) which corresponds in the encoded amino acid sequence to an amino acid(s) located in a) the canyon region of the enzyme and/or b) insertion site 1 and/or c) insertion site 2, wherein the canyon region, insertion site 1 and/or insertion site 2 enzyme is defined as that region which when aligned based on primary or tertiary structure corresponds to the canyon region, insertion site 1 or insertion site 2, respectively, of the enzyme shown herein as SEQ ID No. 16 or SEQ ID No. 68.

In one embodiment preferably the modification(s) at a position located in the canyon and/or insertion site 1 and/or insertion site 2 is combined with at least one modification at a position which corresponds in the encoded amino acid sequence to an amino acid located outside of the canyon region and/or insertion site 1 and/or insertion site 2.

Preferably, the lipid acyltransferase amino acid sequence backbone is modified such that at least one modification (suitably at least two modifications) is made at a position(s) which corresponds in the encoded amino acid sequence to an amino acid(s) located in position 27, 31, 85, 86, 122, 119, 120, 201, 245, 232, 235 and/or 236 (preferably at position 27, 31, 85, 86 119 and/or 120, more preferably at position 27 and/or 31), wherein the position numbering is defined as that position which when aligned based on primary or tertiary structure corresponds to the same position of the enzyme shown herein as SEQ ID No. 16.

In further preferred embodiments, the lipid acyltransferase amino acid sequence backbone comprises at least one modification (suitably at least two modifications) at a position(s) which corresponds in the encoded amino acid sequence to an amino acid(s) located in position 27, 31 in combination with at least one further modification, wherein the position numbering is defined as that position which when aligned based on primary or tertiary structure corresponds to the same position of the enzyme shown herein as SEQ ID No. 16.

Suitably, the at least one further modification may be at one or more of the following positions 85, 86, 122, 119, 120, 201, 245, 23, 81, 82, 289, 227, 229, 233, 33, 207, 130, wherein the position numbering is defined as that position which when aligned based on primary or tertiary structure corresponds to the same position of the enzyme shown herein as SEQ ID No. 16.

Further provided is an altered or variant lipid acyltransferase for use in the present invention comprising an amino acid sequence that is at least 70% identical to the lipid acyltransferase from *Aeromonas salmonicida* shown herein as SEQ ID No. 16 or 68, wherein a substrate chain length specificity determining segment that lies immediately N-terminal of the Asp residue of the catalytic triad of said altered lipid acyltransferase has an altered length relative to said lipid acyltransferase from *Aeromonas salmonicida* shown herein as SEQ ID No. 16 or 68.

Preferably the alteration comprises an amino acid insertion or deletion in said substrate chain length specificity determining segment, such as substituting said substrate chain length specificity determining segment of said parent enzyme with the substrate chain length specificity determining segment of a different lipid acyltransferase to produce said altered lipid acyltransferase. Preferably, said altering increases the length of acyl chain that can be transferred by said lipid acyltransferase.

Preferably, the altered lipid acyltransferase comprises an amino acid sequence that is at least 90% identical to the lipid acyltransferase from *Aeromonas salmonicida* shown herein as SEQ ID No. 16 or 68.

The nucleotide sequence encoding the variant lipid acyltransferase enzyme before modification is a nucleotide sequence shown herein as SEQ ID No. 120, SEQ ID No. 49, SEQ ID No. 50, SEQ ID No. 51, SEQ ID No. 62, SEQ ID No. 63 or SEQ ID No. 24; or is a nucleotide sequence which has at least 70% identity (preferably at least 80%, more preferably at least 90%, even more preferably at least 95% identity) with a nucleotide sequence shown herein as SEQ ID No. 120, SEQ ID No. 49, SEQ ID No. 50, SEQ ID No. 51, SEQ ID No. 62, SEQ ID No. 63 or SEQ ID No. 24; or is a nucleotide sequence which is related to SEQ ID No. 120, SEQ ID No. 49, SEQ ID No. 50, SEQ ID No. 51, SEQ ID No. 62, SEQ ID No. 63, SEQ ID No. 24 by the degeneration of the genetic code; or is a nucleotide sequence which hybridises under medium stringency or high stringency conditions to a nucleotide sequence shown herein as SEQ ID No. 120,

SEQ ID No. 49, SEQ ID No. 50, SEQ ID No. 51, SEQ ID No. 62, SEQ ID No. 63 or SEQ ID No. 24.

In a preferred embodiment, the variant lipid acyltransferase is encoded by a nucleic acid (preferably an isolated or recombinant nucleic acid) sequence which hybridises under medium or high stringency conditions over substantially the entire length of SEQ ID No. 49 or SEQ ID No. 120 or a compliment of SEQ ID No. 49 or SEQ ID No. 120, wherein the encoded polypeptide comprising one or more amino acid residues selected from Q, H, N, T, F, Y or C at position 31; R, Y, S, V, I, A, T, M, F, C or L at position 86; R, G, H, K, Y, D, N, V, C, Q, L, E, S or F at position 27; H, R, D, E 85; T or I at position 119; K or E at position 120; S, L, A, F, W, Y, R, H, M or C at position 122; R at position 201; S as position 245; A or V at position 235; G or S at position 232; G or E at position 236, wherein the positions are equivalent amino acid positions with respect of SEQ ID No. 16.

The variant lipid acyltransferase may comprise a pro-peptide or a polypeptide which has lipid acyltransferase activity and comprises an amino acid sequence which is at least 90% (preferably at least 95%, more preferably at least 98%, more preferably at least 99%) identical with the amino acid sequence shown as SEQ ID No. 16 or 68 and comprises one or more modifications at one or more of the following positions: 27, 31, 85, 86, 122, 119, 120, 201, 245, 232, 235 and/or 236 (preferably at position 27, 31, 85, 86, 119 and/or 120 more preferably at position 27 and/or 31).

In one embodiment the variant comprises a pro-peptide or a polypeptide which has lipid acyltransferase activity and comprises an amino acid sequence shown as SEQ ID No. 16 or 68 except for one or more modifications at one or more of the following positions: 27, 31, 85, 86, 122, 119, 120, 201, 245, 232, 235 and/or 236 (preferably at position 27, 31, 85, 86, 119 and/or 120 more preferably at position 27 and/or 31).

In another embodiment, the lipid acyltransferase comprises a pro-peptide or a polypeptide which has lipid acyltransferase activity and comprises an amino acid sequence which is at least 90% (preferably at least 95%, more preferably at least 98%, more preferably at least 99%) identical with the amino acid sequence shown as SEQ ID No. 16 or 68 and comprises one or more modifications at positions 27 and/or 31 in combination with at least one further modification, wherein the position numbering is defined as that position which when aligned based on primary or tertiary structure corresponds to the same position of the enzyme shown herein as SEQ ID No. 6.

Suitably, the at least one further modification may be at one or more of the following positions 85, 86, 122, 119, 120, 201, 245, 23, 81, 82, 289, 227, 229, 233, 33, 207, 130, wherein the position numbering is defined as that position which when aligned based on primary or tertiary structure corresponds to the same position of the enzyme shown herein as SEQ ID No. 16.

In a preferred embodiment, the lipid acyltransferase comprises a pro-peptide or a polypeptide which has lipid acyltransferase activity and comprises an amino acid sequence shown as SEQ ID No. 16 or 68 except for one or more modifications at one or more of the following positions: 27 and/or 31 in combination with at least one further modification.

Suitably, the at least one further modification may be at one or more of the following positions 85, 86, 122, 119, 120, 201, 245, 23, 81, 82, 289, 227, 229, 233, 33, 207 and/or 130, wherein the position numbering is defined as that position which when aligned based on primary or tertiary structure corresponds to the same position of the enzyme shown herein as SEQ ID No. 16.

The lipid acyltransferase may be a pro-peptide which undergoes further post-translational modification to a mature peptide, i.e. a polypeptide which has lipid acyltransferase activity. By way of example only SEQ ID No. 68 is the same as SEQ ID No. 16 except that SEQ ID No. 68 has undergone post-translational and/or post-transcriptional modification to remove some amino acids, more specifically 38 amino acids. Therefore the polypeptide shown herein as SEQ ID No. 16 could be considered in some circumstances (i.e. in some host cells) as a pro-peptide - which is further processed to a mature peptide by post-translational and/or post-transcriptional modification. The precise modifications, e.g. cleavage site(s), in respect of the post-translational and/or post-transcriptional modification may vary slightly depending on host species. In some host species there may be no post translational and/or post-transcriptional modification, hence the pro-peptide would then be equivalent to the mature peptide (i.e. a polypeptide which has lipid acyltransferase activity). Without wishing to be bound by theory, the cleavage site(s) may be shifted by a few residues (e.g. 1, 2 or 3 residues) in either direction compared with the cleavage site shown by reference to SEQ ID No. 68 compared with SEQ ID No.16. In other words, rather than cleavage at position 235-ATR to position 273 (RRSAS) for example, the cleavage may commence at residue 232, 233, 234, 235, 236, 237 or 238 for example. In addition or alternatively, the cleavage may end at residue 270, 271, 272, 273, 274, 275 or 276 for example. In addition or alternatively, the cleavage may result in the removal of about 38 amino acids, in some embodiments the cleavage may result in the removal of between 30-45 residues, such as 34-42 residues, such as 36-40 residues, preferably 38 residues.

In some embodiments, in order to establish homology to primary structure, the amino acid sequence of a lipid acyltransferase is directly compared to the lipid acyltransferase enzyme shown herein as SEQ ID No. 16 or 68 primary sequence and particularly to a set of residues known to be invariant in all or most lipid acyltransferases for which sequences are known. After aligning the conserved residues, allowing for necessary insertions and deletions in order to maintain alignment (i.e., avoiding the elimination of conserved residues through arbitrary deletion and insertion), the residues equivalent to particular amino acids in the primary sequence of SEQ ID No. 16 or 68 are defined. In preferred embodiments, alignment of conserved residues conserves 100% of such residues. However, alignment of greater than

75% or as little as 50% of conserved residues are also adequate to define equivalent residues. In preferred embodiments, conservation of the catalytic serine and histidine residues are maintained. Conserved residues are used to define the corresponding equivalent amino acid residues of the lipid acyltransferase shown in SEQ ID No. 16 or 68 in other lipid acyltransferases, such as from other *Aeromonas* species, as well as any other organisms.

In order to align a parent lipid acyltransferase with SEQ ID No. 16 or SEQ ID No. 68 (the reference sequence), sequence alignment such as pairwise alignment can be used (http://www.ebi.ac.uk/emboss/align/index.html). Thereby, the equivalent amino acids in alternative parental lipid acyltransferase polypeptides, which correspond to one or more of the amino acids defined with reference to SEQ ID No. 68 or SEQ ID No. 16 can be determined and modified. As the skilled person will readily appreciate, when using the emboss pairwise alignment, standard settings usually suffice. Corresponding residues can be identified using "needle" in order to make an alignment that covers the whole length of both sequences. However, it is also possible to find the best region of similarity between two sequences, using "water".

Alternatively, particularly in instances where parent lipid acyltransferase shares low primary sequence homology with SEQ ID No. 16 or SEQ ID No. 68, the corresponding amino acids in alternative parent lipid acyltransferase which correspond to one or more of the amino acids defined with reference to SEQ ID No. 16 or SEQ ID No. 68 can be determined by structural alignment to the structural model of SEQ ID No. 68 or SEQ ID No. 16, preferably SEQ ID No. 68.

Thus, equivalent residues may be defined by determining homology at the level of tertiary structure for a lipid acyltransferase whose tertiary structure has been determined by X-ray crystallography. In this context, "equivalent residues" are defined as those for which the atomic coordinates of two or more of the main chain atoms of a particular amino acid residue of the lipid acyltransferase shown herein as SEQ ID No. 16 or 68 (N on N, CA on CA, C on C, and O on O) are within 0.13nm and preferably 0.1 nm after alignment. Alignment is achieved after the best model has been oriented and positioned to give the maximum overlap of atomic coordinates of non-hydrogen protein atoms of the lipid acyltransferase in question to the lipid acyltransferase shown herein as SEQ ID No. 16 or 68. As known in the art, the best model is the crystallographic model giving the lowest R factor for experimental diffraction data at the highest resolution available. Equivalent residues which are functionally and/or structurally analogous to a specific residue of the lipid acyltransferase as shown herein as SEQ ID No. 16 or 68 are defined as those amino acids of the lipid acyltransferase that preferentially adopt a conformation such that they either alter, modify or modulate the protein structure, to effect changes in substrate specification, e.g. substrate binding and/or catalysis in a manner defined and attributed to a specific residue of the lipid acyltransferase shown herein as SEQ ID No. 16 or 68. Further, they are those residues of the lipid acyltransferase (in cases where a tertiary structure has been obtained by x-ray crystallography), which occupy an analogous position to the extent that although the main chain atoms of the given residue may not satisfy the criteria of equivalence on the basis of occupying a homologous position, the atomic coordinates of at least two of the side chain atoms of the residue lie with 0.13 nm of the corresponding side chain atoms of the lipid acyltransferase shown herein as SEQ ID No. 16 or 68.

The coordinates of the three dimensional structure of the lipid acyltransferase shown herein as SEQ ID No. 68 (which is a *Aeromonas salmonicida* lipid acyltransferase comprising an N80D mutation) are described in PCT/IB2009/054535 and find use in determining equivalent residues on the level of tertiary structure.

There is a large insertion in the acyltransferase of *Aeromonas salmonicida* between the last beta strand and the ASP-X-X_HIS motif when compared to structurally similar *E. coli* thioesterase. This insertion creates a large cavity (hereinafter referred to as the "cave" that binds the aliphatic chain of the acyl enzyme intermediate. Modulating the sequence and size of this region results in a smaller or larger "cave" or cavity for the aliphatic chain of the acyl enzyme intermediate, i.e., the acyl chain that is transferred by the enzyme. Thus the enzymes of this family may be engineered to preferentially transfer acyl chains of different lengths.

Four insertions are found in the *Aeromonas salmonicida* lipid acyltransferase relative to the *E. coli* thioesterase (PDB entry 1IVN) that link common secondary structural elements common to both structures.

The amino acids coordinates of these insertions in the lipid acyltransferase shown here as SEQ ID No. 68 are listed in the Table below:

Table: Insertions in lipid acyltransferase:

| Insertion | Residues |
|---|---|
| Insertion 1 | 22-36 |
| Insertion 2 | 74-88 |
| Insertion 3 | 162-168 |
| Insertion 4 | 213-281 |

As described in detail in PCT/IB2009/054535 in the lipid acyltransferase, there is a large surface for substrate to bind that can be divided into two areas that are separated by Ser 16 and His 291, where Ser 16 and His 291 along with Asp288 form the characteristic catalytic triad. These two areas can be characterized as being a deep channel or "canyon" - hereinafter referred to the "canyon" - leading into an enclosed cavity or "cave" running through the molecule.

The residues forming the canyon are listed in the Table below:

Table: CANYON residues:

| | |
|---|---|
| Insertion 1 | M23, M27, Y30, L31 |
| Segment 1 | F42, G67, G68 |
| Insertion 2 | D80, P81, K82, Q84, V85, I86 |
| Segment 2a | Y117, A119, Y120 |
| Insertion 4 | G229, Y230, V231 |

The residues forming the cave are listed in table below.

Table: CAVE residues:

| | |
|---|---|
| Segment 1 | D15, S16, L18 |
| Segment 2 | W111, A114, L115, L118 |
| Segment 3 | P156, D157, L158, Q160, N161 |
| Segment 4 | F206, A207, E208, M209, L210 |
| Segment 5 | M285, F286, V290, H291, P292 V295 |

Segments 3 and 4 precede insertions 3 and 4 respectively, and segment 5 immediately follows insertion 4. Insertions 4 and 5 also contribute to the over enclosure resulting in the cave, thus the cave is different to the canyon in that insertions 1 and 2 form the lining of the canyon while insertions 3 and 4 form the overlaying structure. Insertions 3 and insertion 4 cover the cave.

In one embodiment the lipid acyltransferase for use in the present invention may be altered by modifying the amino acid residues in one or more of the canyon, the cave, the insertion 1, the insertion 2, the insertion 3 or the insertion 4.

In one embodiment the lipid acyltransferase for use in the present invention may be altered by modifying the amino acid residues in one or more of the canyon, insertion 1 or insertion 2.

In one embodiment, the dimensions of the acyl chain binding cavity of a lipid acyltransferase may be altered by making changes to the amino acid residues that form the larger cave. This may be done by modulating the size the regions that link the common features of secondary structure as discussed above. In particular, the size of the cave may be altered by changing the amino acids in the region between the last (fifth) beta strand of the enzyme and the Asp-X-X-His motif that forms part of the catalytic triad.

The substrate chain length specificity determining segment of a lipid acyltransferase is a region of contiguous amino acids that lies between the β5 β -strand of the enzyme and the Asp residue of the catalytic triad of that enzyme (the Asp residue being part of the Asp-Xaa-Xaa-His motif).

The tertiary structures of the *Aeromonas salmonicida* lipid acyltransferase and the E. *coli t*hioesterase (deposited as NCBI's Genbank database as accession number 1IVN_A; GID:33357066) each showing a signature three-layer alpha/beta/alpha structure, where the beta-sheets are composed of five parallel strands allow the substrate chain length specificity determining segments of each of the lipid acyltransferase enzymes to be determined.

The substrate chain length specificity determining segment of the *Aeromonas salmonicida* lipid acyltransferase lies immediately N-terminal to the Asp residue of the catalytic triad of the enzyme. However, the length of the substrate chain length specificity determining segment may vary according to the distance between the Asp residue and the β5 β-stand of the enzyme. For example, the substrate chain length specificity determining segments of the lipid acyltransferase are about 13 amino, 19 amino acids and about 70 amino acids in length, respectively. As such, depending on the lipid acyltransferase, a substrate chain length specificity determining segment may be in the range of 10 to 70 amino acids in length, e.g., in the range of 10 to 30 amino acids in length, 30 to 50 amino acids in length, or 50 to 70 amino acids.

The Table below provides an exemplary sequence for the substrate chain length specificity determining segment of the lipid acyltransferase enzyme.

| *A. salmonicida* lipid acyltransferase (GCAT) | |
|---|---|
| AEMLRDPQNFGLSDVENPCYDGGYVWKPFATRSV STDRQLSASPQERLAIAGNPLLAQAVASPMARRSA SPLNCEGKMF | SEQ ID No. 124 |

In certain embodiments, the amino acid sequence of a substrate chain length specificity determining segment may or may not be the amino acid sequence of a wild-type enzyme. In certain embodiments, the substrate chain length specificity determining segment may have an amino acid sequence that is at least 70%, e.g., at least 80%, at least 90% or at least 95% identical to the substrate chain length specificity determining segment of a wild type lipid acyltransferase.

Suitably the variant enzyme may be prepared using site directed mutagenesis.

Preferred modifications are located at one or more of the following positions L031, I086, M027, V085, A119, Y120, W122, E201, F235, W232, A236, and/or Q245.

In particular key modifications include one or more of the following modifications: L31Q, H, N, T, F, Y or C (preferably L31 Q); M27R, G, H, K, Y, D, N, V, C, Q, L, E, S or F (preferably M27V); V85H, R, D or E; I86R,Y, S, V, I, A, T, M, F, C or L (preferably I86S or A); A119T or I; Y120K or E; W122S, L or A (preferably W122L); E201R; Q245S; F235A or V; W232G or S; and/or A236G or E.

In one embodiment when the at least one modification is made in the canyon the modification(s) are made at one or more of the following positions: 31, 27, 85, 86, 119, 120.

In particular key modifications in the canyon include one or more of the following modifications: L31Q, H, N, T, F, Y or C (preferably L31 Q); M27R, G, H, K, Y, D, N, V, C, Q, L, E, S or F (preferably M27V); V85H, R, D or E; I86R,Y, S, V, I, A, T, M, F, C or L (preferably I86S or A); A119T or I; Y120K or E, which may be in combination with one another and/or in combination with a further modification.

In one embodiment preferably when the modification is made in insertion site 1 the modifications are made at one or more positions 31 and/or 27. Suitably the modifications may be L31 Q, H, N, T, F, Y or C (preferably L31 Q) and/or M27R, G, H, K, Y, D, N, V, C, Q, L, E, S or F (preferably M27V).

In one embodiment preferably when the modification is made in insertion site 2 the modifications are made at positions are 085, 086. Suitably the modifications may be V85H, R, D or E and/or I86R, Y, S, V, I, A, T, M, F, C or L.

In one embodiment preferably when the modification is made in insertion site 4 the modifications are made at position 245. Suitably the modification may be Q245S.

In one embodiment preferably the modification is made in at least insertion site 1.

In another embodiment preferably a modification is made in at least insertion site 1 in combination with a further modification in insertion site 2 and/or 4 and/or at one or more of the following positions 119, 120, 122, 201, 77, 130, 82, 120, 207, 167, 227, 215, 230, 289.

In a further embodiment preferably a modification is made in at least the canyon region in combination with a further modification in insertion site 4 and/or at one or more of the following positions 122, 201, 77, 130, 82, 120, 207, 167, 227, 215, 230, 289.

Preferred modifications are given for particular site:

R130R, V, Q, H, A, D, L, I, K, N, C, Y, G, S, F, T or M;
K82R, N, H, S, L, E, T, M or G;
G121S, R, G, E, K, D, N, V, Q or A;
Y74Y or W;
Y83 F or P;
I77T, M, H, Q, S, C, A, E, L, Y, F, R or V;
A207E;
Q167T, H, I, G, L or M;
D227L, C, S, E, F, V, I, T, Y, P, G, R, D, H or A;
N215G;
Y230A, G, V, R, I, T, S, N, H, E, D, Q, K; or
N289P.

In combination with one or more modifications at positions 31, 27, 85, 86, 119, 120, 122, 201, 245, 235, 232, and/or 236 (for example the modification may be one or more of the following: L31Q, H, N, T, F, Y or C (preferably L31 Q); M27R, G, H, K, Y, D, N, V, C, Q, L, E, S or F (preferably M27V); V85H, R, D or E; I86R,Y, S, V, I, A, T, M, F, C or

L (preferably I86S or A); A119T or I; Y120K or E; W122S, L or A (preferably W122L); E201 R; Q245S; F235A or V; W232G or S; and/or A236G or E) suitably the variant lipid acyltransferase may be additionally modified at one or more of the following positions 130, 82, 121, 74, 83, 77, 207, 167, 227, 215, 230, 289 (for example the additional modification may be one or more of the following: R130R, V, Q, H, A, D, L, I, K, N, C, Y, G, S, F, T or M; K82R, N, H, S, L, E, T, M or G; G121S, R, G, E, K, D, N, V, Q or A; Y74Y or W; Y83 F or P; I77T, M, H, Q, S, C, A, E, L, Y, F, R or V; A207E; Q167T, H, I, G, L or M; D227L, C, S, E, F, V, I, T, Y, P, G, R, D, H or A; N215G; Y230A, G, V, R, I, T, S, N, H, E, D, Q, K; and/or N289P), preferably the variant lipid acyltransferase may be additionally modified at at least one or more of the following positions: 130, 82, 77 or 227.

For the avoidance of doubt the lipid acyltransferase backbone when aligned (on a primary or tertiary basis) with the lipid acyltransferase enzyme shown herein as SEQ ID No. 16 preferably has D in position 80. We have therefore shown in many of the combinations taught herein N80D as a modification. If N80D is not mentioned as a suitable modification and the parent backbone does not comprise D in position 80, then an additional modification of N80D should be incorporated into the variant lipid acyltransferase to ensure that the variant comprises D in position 80.

When the backbone or parent lipid acyltransferase already contains the N80D modification, the other modifications can be expressed without referencing the N80D modification, i.e. L31Q, N80D, W122L could have been expressed as L31Q, W122L for example.

However, it is important to note that the N80D modification is a preferred modification and a backbone enzyme or parent enzyme is preferably used which already possesses amino acid D in position 80. If, however, a backbone is used which does not contain amino acid D in position (such as one more of the lipid acyltransferases shown here as SEQ ID No. 1, 3, 4, 15, 34, or 35 for instance) then preferably an additional modification of N80D is included.

Suitably, the substitution at position 31 identified by alignment of the parent sequence with SEQ ID No. 68 or SEQ ID No. 16 may be a substitution to an amino acid residue selected from the group consisting of: Q, H, Y and F, preferably Q.

Suitably, the variant polypeptide comprises one or more further modification(s) at any one or more of amino acid residue positions: 27, 77, 80, 82, 85, 85, 86, 121, 122, 130, 167, 207, 227, 230 and 289, which position is identified by alignment of the parent sequence with SEQ ID No. 68. Suitably, at least one of the one or more further modification(s) may be at amino acid residue position: 86, 122 or 130, which position is identified by alignment of the parent sequence with SEQ ID No. 68.

Suitably, the variant lipid acyltransferase comprises one or more of the following further substitutions: I86 (A, C, F, L, M, S, T, V, R, I or Y); W122 (S, A, F, W, C, H, L, M, R or Y); R130 A, C, D, G, H, I, K, L, M, N, Q, T, V, R, F or Y); or any combination thereof.

The variant lipid acyltransferase may comprise one of the following combinations of modifications (where the parent back bone already comprises amino acid D in position 80, the modification can be expressed without reference to N80D):

L31Q, N80D, I86S, W122F
L31Q, N80D, W122L
L31 Q, N80D, I86V, W122L
L31Q, N80D, I86I, W122L
L31Q, N80D, I86S, R130R
L31Q, N80D, K82R, I86A
L31Q, N80D, I86S, W122W
L31Q, N80D, I86S, W122Y
M27V, L31Q, N80D
L31Q, N80D, I86A, W122L
L31Q, N80D, W122L
L31Q, N80D, I86S, G121S
L31Q, N80D, I86S
L31Q, N80D, K82R, I86S
L31Q, N80D, I86S, W122L, R130Y
L31Q, N80D, I86S, W122L, R130V
L31Q, N80D, I86S
L31Q, N80D, I86T, W122L
L31Q, N80D, I86S, W122L
L31Q, N80D, W122L, R130Q
L31Q, N80D, I86S, W122L, R130R
L31Q, N80D, I86S
L31Q, N80D, G121R

L31Q, N80D, I86A
M27C, L31Q, N80D
M27Q, L31Q, N80D
L31Q, N80D, G121S
L31Q, N80D, I86S, W122R
L31Q, N80D, R130Q
L31Q, N80D, I86S, W122H
L31Q, N80D, I86M, W122L
L31Q, N80D, R130N
L31Q, N80D, I86S, W122L
L31Q, N80D, K82N
L31Q, N80D, I86S, W122M
L31Q, N80D, W122L
L31Q, N80D, K82H
L31Q, N80D, R130H
L31Q, N80D, R130A
L31Q, N80D, G121S
L31Q, N80D, I86S, W122L, R130D
L31Q, N80D, I86M
L31Q, Y74Y, N80D
L31Q, N80D, R130L
L31Q, N80D, Y83F
L31Q, N80D, K82S
L31Q, I77T, N80D
L31Q, N80D, I86S, W122L, R130I
L31Q, N80D, I86S, W122L
L31Q, N80D, I86F, W122L
M27N, L31Q, N80D
L31Q, N80D, Y83P
L31Q, N80D, R130K
L31Q, N80D, K82R, I86S, W122L
L31Q, N80D, K82L
L31Q, N80D, 186S, G121G
L31Q, N80D, I86A, R130Q
M27H, L31Q, N80D
L31Q. N80D, W122L, A207E
L31Q, N80D, W122L, R130L
L31Q, N80D, K82E
L31Q, N80D, G121E
L31Q, N80D, W122L, R130R
L31Q, I77M, N80D
L31Q, N80D, K82T
L31Q, N80D, W122L
L31Q, N80D, W122H
L31Q, N80D, Q167T
L31Q, I77H, N80D
L31Q, N80D, G121K
L31Q, I77Q, N80D
L31Q, N80D, W122L, R130N
L31Q, N80D, W122L
L31 Q, N80D, G121D
L31Q, N80D, R130T
L31Q, N80D, R130T
L31Q, N80D, K82M
L31Q, N80D, Q167H
L31Q, N80D, I86T
L31Q, N80D, Q167I
L31Q, N80D, I86C

L31Q, N80D, Q167G
M27L, L31Q, N80D
L31Q, N80D, I86S, G121R
L31Q, I77S, N80D
L31Q, I77C, N80D
L31Q, N80D, G121N
L31Q, I77A, N80D
L31Q, N80D, R130M
L31Q, N80D, W122F
M27G, L31Q, N80D
L31Q, N80D, K82G
L31Q, N80D, I86S, W122L, R130K
L31Q, N80D, R130A
L31Q, N80D, I86I
L31Q, I77E, N80D
L31Q, N80D, D227L
L31Q, N80D, V85H, N215G
L31Q, N80D, I86A, W122L, R130N
L31Q, I77R, N80D
L31Q, N80D, I86F
L31Q, N80D, I86Y, W122L
M27K, L31Q, N80D
L31Q, N80D, D227C
L31Q, N80D, R130L
L31Q, N80D, I86C, W122L
L31Q, N80D, Q167L
L31Q, N80D, V85H
L31Q, N80D, Q167M
M27D, L31Q, N80D
L31Q, N80D, I86L
L31 Q, N80D, Y230A
L31 Q, N80D, W122R
L31Q, N80D, Y230G
L31Q, N80D, D227S
L31Q, N80D, W122L, A207E, N289P
L31 Q, N80D, W122Y
L31Q, N80D, I86L, W122L
L31Q, N80D, K82R, I86S, G121S, R130Q
L31Q, Y74W, N80D
L31Q, N80D, R130F
L31Q, N80D, G121V
L31Q, N80D, W122L, R130M
L31Q, N80D, R130V
L31Q, N80D, Y230V
L31Q, N80D, N215G
L31Q, N80D, I86S, W122L, R130N
L31Q, N80D, Y230R
M27E, L31Q, N80D
L31Q, N80D, Y230I
L31Q, N80D, I86S, W122L, R130S
L31Q, N80D, K82R
L31Q, N80D, D227E
L31Q, N80D, K82R, I86A, G121S
L31Q, N80D, R130G
L31Q, I77V, N80D
L31Q, N80D, G121G
L31Q, N80D, Y230T
L31Q, N80D, K82R, I86S, R130N

L31Q, N80D, D227F
L31Q, N80D, I86A, G121R
L31Q, N80D, I86S, R130N
L31Q, N80D, W122C
L31Q, N80D, Y230S
L31Q, N80D, R130Y
L31Q, N80D, R130C
L31Q, I77L, N80D
A119T, N80D
A199A, N80D
G67A, N80D, V85H

wherein said positions are identified by alignment of the parent sequence with SEQ ID No. 68 or SEQ ID No. 16.

Suitably, the variant lipid acyltransferase may be identical to the parent lipid acyltransferase except for a modification at position 31 and, optionally, one or more further modification(s) at any one or more of amino acid residue positions: 27, 77, 80, 82, 85, 85, 86, 121, 122, 130, 167, 207, 227, 230 and 289, which position is identified by alignment of the parent sequence with SEQ ID No. 68 or SEQ ID No. 16.

Suitably, the variant lipid acyltransferase may be identical to the parent lipid acyltransferase except for a modification at position 31 and, optionally, one or more further modification(s) at any one or more of amino acid residue positions: 86, 122 or 130, which position is identified by alignment of the parent sequence with SEQ ID No. 68 or SEQ ID No. 16.

In one embodiment, where the parent sequence is SEQ ID No. 16 or SEQ ID No. 68 or where the parent sequence is encoded by SEQ ID No. 49 or SEQ ID No. 120, the variant polypeptide has any one of the modifications as detailed above, except for a modification at position 80. In this regard, SEQ ID No. 16, SEQ ID No. 68 or a polypeptide encoded by SEQ ID No. 49 or SEQ ID No. 120 will already have aspartic acid at position 80, when said positions are identified by alignment of the parent sequence with SEQ ID No. 16.

Suitably, the variant lipid acyltransferase or the variant lipid acyltransferase obtainable by a method according to the present invention may have at least 75% identity to the parent lipid acyltransferase, suitably the variant lipid acyltransferase may have at least 75% or at least 80% or at least 85% or at least 90% or at least 95% or at least 98% identity to the parent lipid acyltransferase.

The present invention also relates to a variant polypeptide having lipid acyltransferase activity, wherein the variant comprises a modification at at least position 31 compared to a parent lipid acyltransferase, wherein position 31 is identified by alignment with SEQ ID No. 68 or SEQ ID No. 16.

In one embodiment preferably the variant lipid acyltransferase has the following modifications and/or the following modifications are made in the methods of the present invention:

- L31Q, N80D, W122L (which can be expressed as L31Q, W122L where the backbone enzyme already has D in position 80);
- M27V, L31Q, N80D (which can be expressed as N27V, L31Q where the backbone enzyme already has D in position 80);
- L31Q, N80D, K82R, I86A (which can be expressed as L31Q, K82R, I86A where the backbone enzyme already has D in position 80); and/or
- L31Q, N80D, I86S, W122F (which can be expressed as L31Q, I86S, W122F where the backbone enzyme already has D in position 80).

## IMPROVED PROPERTIES

The variant lipid acyltransferase for use in the present invention have at least one improved property compared with a parent (i.e. backbone) or unmodified lipid acyltransferase.

The term "improved property" as used herein may include a) an altered substrate specificity of the lipid acyltransferase, for instance and by way of example only i) an altered ability of the enzymes to use certain compounds as acceptors, for example an improved ability to utilise a carbohydrate as an acceptor molecule thus improving the enzymes ability to produce a carbohydrate ester) or ii) an altering ability to use saturated or unsaturated fatty acids as a substrate or iii) a changed specificity such that the variant lipid acyltransferase preferentially utilises the fatty acid from the Sn1 or Sn2 position of a lipid substrate or iv) an altered substrate chain length specificity of in the variant enzyme; b) altered kinetics of the enzyme; and/or c) lowered ability of the variant lipid acyltransferase to carry out a hydrolysis reaction whilst maintaining or enhancing the enzymes ability to carry out an acyl transferase reaction.

Other improved properties may be for example related to improvements and/or changes in pH and/or temperature stability, and/or detergent and/or oxidative stability. Indeed, it is contemplated that enzymes having various degrees of stability in one or more of these characteristics (pH, temperature, proteolytic stability, detergent stability, and/or oxidative stability) can be prepared in accordance with the present invention.

Characterization of wild-type (e.g. parent lipid acyltransferase) and mutant (e.g. variant lipid acyltransferase) proteins is accomplished via any means suitable and is preferably based on the assessment of properties of interest.

In some embodiments the variant enzyme of the present invention, when compared with the parent enzyme, may have an increased transferase activity and either the same or less hydrolytic activity. In other words, suitably the variant enzyme may have a higher transferase activity to hydrolytic activity (e.g. transferase : hydrolysis activity) compared with the parent enzyme. Suitably, the variant enzyme may preferentially transfer an acyl group from a lipid (including phospholipid, galactolipid or triacylglycerol) to an acyl acceptor rather than simply hydrolysing the lipid.

Suitably, the lipid acyltransferase for use in the invention may be a variant with enhanced enzyme activity on polar lipids, preferably phospholipids and/or glycolipids, when compared to the parent enzyme. Preferably, such variants also have low or no activity on lyso-polar lipids. The enhanced activity on polar lipids, preferably phospholipids and/or glycolipids, may be the result of hydrolysis and/or transferase activity or a combination of both. Preferably the enhanced activity on polar lipids in the result of transferase activity.

Variant lipid acyltransferases for use in the invention may have decreased activity on triglycerides, and/or monoglycerides and/or diglycerides compared with the parent enzyme.

Suitably the variant enzyme may have no activity on triglycerides and/or monoglycerides and/or diglycerides.

DEFINITION OF SETS

Amino acid set 1:

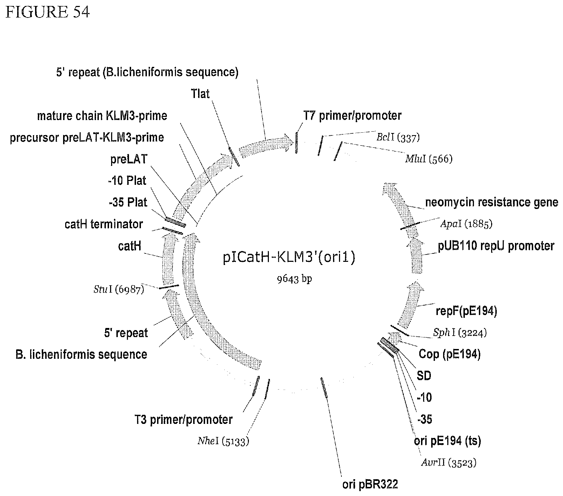

Amino acid set 1 (note that these are amino acids in 1IVN - Figure 53 and Figure 54) Gly8, Asp9, Ser10, Leu11, Ser12, Tyr15, Gly44, Asp45, Thr46, Glu69, Leu70, Gly71, Gly72, Asn73, Asp74, Gly75, Leu76, Gln106, Ile107, Arg108, Leu109, Pro110, Tyr113, Phe121, Phe139, Phe140, Met141, Tyr145, Met151, Asp154, His157, Gly155, Ile156, Pro158

The highly conserved motifs, such as GDSx and catalytic residues, were deselected from set 1 (residues underlined). For the avoidance of doubt, set 1 defines the amino acid residues within 10Å of the central carbon atom of a glycerol in the active site of the 11VN model.

Amino acid set 2:

Amino acid set 2 (note that the numbering of the amino acids refers to the amino acids in the P10480 mature sequence)
Leu17, Lys22, Met23, Gly40, Asn80, Pro81, Lys82, Asn87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Met209, Leu210, Arg211, Asn215, Lys284, Met285, Gln289 and Val290.

Selected residues in Set 1 compared with Set 2 are shown in Table 1.

Table 1

| IVN model | | | P10480 Mature sequence Residue Number |
|---|---|---|---|
| IVN | A.hyd homologue | | |
| | PFAM | Structure | |
| Gly8 | Gly32 | | |
| Asp9 | Asp33 | | |
| Ser10 | Ser34 | | |
| Leu11 | Leu35 | | Leu17 |
| Ser12 | Ser36 | | Ser18 |
| | | | Lys22 |
| | | | Met23 |
| Tyr15 | Gly58 | | Gly40 |
| Gly44 | Asn98 | | Asn80 |
| Asp45 | Pro99 | | Pro81 |
| Thr46 | Lys100 | | Lys82 |

(continued)

| IVN model | | | P10480 Mature sequence Residue Number |
|---|---|---|---|
| IVN | A.hyd homologue | | |
| | PFAM | Structure | |
| | | | Asn87 |
| | | | Asn88 |
| Glu69 | Trp129 | | Trp111 |
| Leu70 | Val130 | | Vall 12 |
| Gly71 | Gly131 | | |
| Gly72 | Ala132 | | Ala114 |
| Asn73 | Asn133 | | |
| Asp74 | Asp134 | | |
| Gly75 | Tyr135 | | Tyr117 |
| Leu76 | Leu136 | | Leu118 |
| Gln106 | | Pro174 | Pro156 |
| Ile107 | | Gly177 | Gly159 |
| Arg108 | | Gln178 | Gln160 |
| Leu109 | | Asn179 | Asn161 |
| Pro110 | | 180 to 190 | Pro162 |
| Tyr113 | | | Ser163 |
| | | | Ala164 |
| | | | Arg165 |
| | | | Ser166 |
| | | | Gln167 |
| | | | Lys168 |
| | | | Val169 |
| | | | Val170 |
| | | | Glu171 |
| | | | Ala172 |
| Phe121 | His198 | Tyr197 | Tyr179 |
| | | His 198 | His180 |
| | | Asn199 | Asn181 |
| Phe139 | Met227 | | Met209 |
| Phe140 | Leu228 | | Leu210 |
| Met141 | Arg229 | | Arg211 |
| Tyr145 | Asn233 | | Asn215 |
| | | | Lys284 |
| Met151 | Met303 | | Met285 |
| Asp154 | Asp306 | | |
| Gly155 | Gln307 | | Gln289 |
| Ile156 | Val308 | | Val290 |
| His157 | His309 | | |
| Pro158 | Pro310 | | |

Amino acid set 3:

Amino acid set 3 is identical to set 2 but refers to the *Aeromonas salmonicida* (SEQ ID No. 35) coding sequence, i.e. the amino acid residue numbers are 18 higher in set 3 as this reflects the difference between the amino acid numbering in the mature protein (SEQ ID No. 35) compared with the protein including a signal sequence (SEQ ID No. 4).

The mature proteins of *Aeromonas salmonicida* GDSX (SEQ ID No. 35) and *Aeromonas hydrophila* GDSX

(SEQ ID No. 34) differ in five amino acids. These are Thr3Ser, LYS182Gln, Glu309Ala, Thr310Asn, and Gly318-, where the *salmonicida* residue is listed first and the *hydrophila* residue is listed last. The *hydrophila* protein is only 317 amino acids long and lacks a residue in position 318. The *Aeromonas salmonicida* GDSX has considerably high activity on polar lipids such as galactolipid substrates than the *Aeromonas hydrophila* protein. Site scanning was performed on all five amino acid positions.

Amino acid set 4:

Amino acid set 4 is S3, Q182, E309, S310, and -318.

Amino acid set 5:

F13S, D15N, S18G, S18V, Y30F, D116N, D116E, D157 N, Y226F, D228N Y230F.

Amino acid set 6:

Amino acid set 6 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318.

The numbering of the amino acids in set 6 refers to the amino acids residues in P10480 (SEQ ID No. 3) - corresponding amino acids in other sequence backbones can be determined by homology alignment and/or structural alignment to P10480 and/or 1IVN.

Amino acid set 7:

Amino acid set 7 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318, Y30X (where X is selected from A, C, D, E, G, H, I, K, L, M, N, P, Q, R, S, T, V, or W), Y226X (where X is selected from A, C, D, E, G, H, I, K, L, M, N, P, Q, R, S, T, V, or W), Y230X (where X is selected from A, C, D, E, G, H, I, K, L, M, N, P, Q, R, S, T, V, or W), S18X (where X is selected from A, C, D, E, F, H, I, K, L, M, N, P, Q, R, T, W or Y), D157X (where X is selected from A, C, E, F, G, H, I, K, L, M, P, Q, R, S, T, V, W or Y).

The numbering of the amino acids in set 7 refers to the amino acids residues in P10480 (SEQ ID No. 3) - corresponding amino acids in other sequence backbones can be determined by homology alignment and/or structural alignment to P10480 and/or 1IVN).

Suitably, the variant enzyme comprises one or more of the following amino acid modifications compared with the parent enzyme:

S3E, A, G, K, M, Y, R, P, N, T or G
E309Q, R or A, preferably Q or R
-318Y, H, S or Y, preferably Y.

Preferably, X of the GDSX motif is L. Thus, preferably the parent enzyme comprises the amino acid motif GDSL.

Suitably, said first parent lipid acyltransferase may comprise any one of the following amino acid sequences: SEQ ID No. 34, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 1, SEQ ID No. 15, SEQ ID No. 25, SEQ ID No. 26, SEQ ID No. 27, SEQ ID No. 28, SEQ ID No. 29, SEQ ID No. 30, SEQ ID No. 32, SEQ ID No. 33 or SEQ ID No. 35.

Suitably, said second related lipid acyltransferase may comprise any one of the following amino acid sequences: SEQ ID No. 3, SEQ ID No. 34, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 1, SEQ ID No. 15, SEQ ID No. 25, SEQ ID No. 26, SEQ ID No. 27, SEQ ID No. 28, SEQ ID No. 29, SEQ ID No. 30, SEQ ID No. 32, SEQ ID No. 33 or SEQ ID No. 35.

The variant enzyme must comprise at least one amino acid modification compared with the parent enzyme. In some embodiments, the variant enzyme may comprise at least 2, preferably at least 3, preferably at least 4, preferably at least 5, preferably at least 6, preferably at least 7, preferably at least 8, preferably at least 9, preferably at least 10 amino acid modifications compared with the parent enzyme.

When referring to specific amino acid residues herein the numbering is that obtained from alignment of the variant sequence with the reference sequence shown as SEQ ID No. 34 or SEQ ID No. 35.

In one aspect preferably the variant enzyme comprises one or more of the following amino acid substitutions:

S3A, C, D, E, F, G, H, I, K, L, M, N, P, Q, R, T, V, W, or Y; and/or
L17A, C, D, E, F, G, H, I, K, M, N, P, Q, R, S, T, V, W, or Y; and/or
S18A, C, D, E, F, H, I, K, L, M, N, P, Q, R, T, W, or Y; and/or
K22A, C, D, E, F, G, H, I, L, M, N, P, Q, R, S, T, V, W, or Y; and/or
M23A, C, D, E, F, G, H, I, K, L, N, P, Q, R, S, T, V, W, or Y; and/or
Y30A, C, D, E, G, H, I, K, L, M, N, P, Q, R, S, T, V, orW; and/or
G40A, C, D, E, F, H, I, K, L, M, N, P, Q, R, S, T, V, W, or Y; and/or
N80A, C, D, E, F, G, H, I, K, L, M, P, Q, R, S, T, V, W, or Y; and/or
P81A, C, D, E, F, G, H, I, K, L, M, N, Q, R, S, T, V, W, or Y; and/or
K82A, C, D, E, F, G, H, I, L, M, N, P, Q, R, S, T, V, W, or Y; and/or
N87A, C, D, E, F, G, H, I, K, L, M, P, Q, R, S, T, V, W, or Y; and/or
N88A, C, D, E, F, G, H, I, K, L, M, P, Q, R, S, T, V, W, or Y; and/or
W111A, C, D, E, F, G, H, I, K, L, M, N, P, Q, R, S, T, V, W or Y; and/or
V112A, C, D, E, F, G, H, I, K, L, M, N, P, Q, R, S, T, W, or Y; and/or
A114C, D, E, F, G, H, I, K, L, M, N, P, Q, R, S, T, V, W, or Y; and/or
Y117A, C, D, E, F, G, H, I, K, L, M, N, P, Q, R, S, T, V, orW; and/or
L118A, C, D, E, F, G, H, I, K, M, N, P, Q, R, S, T, V, W, or Y; and/or
P156A, C, D, E, F, G, H, I, K, L, M, N, Q, R, S, T, V, W, or Y; and/or
D157A, C, E, F, G, H, I, K, L, M, P, Q, R, S, T, V, W, or Y; and/or
G159A, C, D, E, F, H, I, K, L, M, N, P, Q, R, S, T, V, W, or Y; and/or
Q160A, C, D, E, F, G, H, I, K, L, M, N, P, R, S, T, V, W, or Y; and/or
N161A, C, D, E, F, G, H, I, K, L, M P, Q, R, S, T, V, W, or Y; and/or
P162A, C, D, E, F, G, H, I, K, L, M, N, Q, R, S, T, V, W, or Y; and/or
S163A, C, D, E, F, G, H, I, K, L, M, N, P, Q, R, T, V, W, or Y; and/or
A164C, D, E, F, G, H, I, K, L, M, N, P, Q, R, S, T, V, W, or Y; and/or
R165A, C, D, E, F, G, H, I, K, L, M, N, P, Q, S, T, V, W, or Y; and/or
S166A, C, D, E, F, G, H, I, K, L, M, N, P, Q, R, T, V, W, or Y; and/or
Q167A, C, D, E, F, G, H, I, K, L, M, N, P, R, S, T, V, W, or Y; and/or
K168A, C, D, E, F, G, H, I, L, M, N, P, Q, R, S, T, V, W, or Y; and/or
V169A, C, D, E, F, G, H, I, K, L, M, N, P, Q, R, S, T, W, or Y; and/or
V170A, C, D, E, F, G, H, I, K, L, M, N, P, Q, R, S, T, W, or Y; and/or
E171A, C, D, F, G, H, I, K, L, M, N, P, Q, R, S, T, V, W, or Y; and/or
A172C, D, E, F, G, H, I, K, L, M, N, P, Q, R, S, T, V, W, or Y; and/or
Y179A, C, D, E, F, G, H, I, K, L, M, N, P, Q, R, S, T, V, orW; and/or
H180A, C, D, E, F, G, I, K, L, M, P, Q, R, S, T, V, W, or Y; and/or
N181A, C, D, E, F, G, H, I, K, L, M, P, Q, R, S, T, V, W, orY; and/or
Q182A, C, D, E, F, G, H, I, K, L, M, N, P, R, S, T, V, W, or Y, preferably K; and/or
M209A, C, D, E, F, G, H, I, K, L, N, P, Q, R, S, T, V, W, or Y; and/or
L210 A, C, D, E, F, G, H, I, K, M, N, P, Q, R, S, T, V, W, or Y; and/or
R211 A, C, D, E, F, G, H, I, K, L, M, N, P, Q, R, S, T, V, W, or Y; and/or
N215 A, C, D, E, F, G, H, I, K, L, M, N, P, Q, R, S, T, V, W, or Y; and/or
Y226A, C, D, E, G, H, I, K, L, M, N, P, Q, R, S, T, V, or W; and/or
Y230A, C, D, E, G, H, I, K, L, M, N, P, Q, R, S, T, V or W; and/or
K284A, C, D, E, F, G, H, I, L, M, N, P, Q, R, S, T, V, W, or Y; and/or
M285A, C, D, E, F, G, H, I, K, L, N, P, Q, R, S, T, V, W, or Y; and/or
Q289A, C, D, E, F, G, H, I, K, L, M, N, P, R, S, T, V, W, or Y; and/or
V290A, C, D, E, F, G, H, I, K, L, M, N, P, Q, R, S, T, W, or Y; and/or
E309A, C, D, F, G, H, I, K, L, M, N, P, Q, R, S, T, V, W, or Y; and/or
S310A, C, D, E, F, G, H, I, K, L, M, N, P, Q, R, T, V, W, or Y.

In addition or alternatively thereto there may be one or more C-terminal extensions. Preferably the additional C-terminal extension is comprised of one or more aliphatic amino acids, preferably a non-polar amino acid, more preferably of I, L, V or G. Thus, the present invention further provides for a variant enzyme comprising one or more of the following C-terminal extensions: 3181, 318L, 318V, 318G.

Preferred variant enzymes may have a decreased hydrolytic activity against a phospholipid, such as phosphatidylcholine (PC), may also have an increased transferase activity from a phospholipid.
Preferred variant enzymes may have an increased transferase activity from a phospholipid, such as phosphatidylcholine (PC), these may also have an increased hydrolytic activity against a phospholipid.
Modification of one or more of the following residues may result in a variant enzyme having an increased absolute transferase activity against phospholipid:

S3, D157, S310, E309, Y179, N215, K22, Q289, M23, H180, M209, L210, R211, P81, V112, N80, L82, N88; N87

Specific preferred modifications which may provide a variant enzyme having an improved transferase activity from a phospholipid may be selected from one or more of the following:

S3A, C, D, E, F, G, H, I, K, L, M, N, P, Q, R, T, V, W or Y; preferably N, E, K, R, A, P or M, most preferably S3A
D157A, C, E, F, G, H, I, K, L, M, N, P, Q, R, S, T, V, W or Y; preferably D157S, R, E, N, G, T, V, Q, KorC
S310A, C, D, E, F, G, H, I, K, L, M, N, P, Q, R, T, V, W or Y; preferably S310T -318 E
E309A, C, D, E, F, G, H, I, K, L, M, N, P, Q, R, T, V, W or Y; preferably E309 R, E, L, R or A
Y179A, C, D, E, F, G, H, I, K, L, M, N, P, Q, R, S, T, V or W; preferably Y179 D, T, E, R, N, V, K, Q or S, more preferably E, R, N, V, K or Q
N215A, C, D, E, F, G, H, I, K, L, M, P, Q, R, S, T, V, W or Y; preferably N215 S, L, R orY
K22A, C, D, E, F, G, H, I, L, M, N, P, Q, R, S, T, V, W or Y; preferably K22 E, R, C or A
Q289A, C, D, E, F, G, H, I, K, L, M, N, P, R, S, T, V, W or Y; preferably Q289 R, E, G, PorN
M23A, C, D, E, F, G, H, I, K, L N, P, Q, R, S, T, V, W or Y; preferably M23 K, Q, L, G, TorS
H180A, C, D, E, F, G, I, K, L, M, P, Q, R, S, T, V, W or Y; preferably H180 Q, R or K
M209 A, C, D, E, F, G, H, I, K, L, N, P, Q, R, S, T, V, W or Y; preferably M209 Q, S, R, A, N, Y, E, V or L
L210A, C, D, E, F, G, H, I, K, M, N, P, Q, R, S, T, V, W or Y; preferably L210 R, A, V, S, T, I, W or M
R211A, C, D, E, F, G, H, I, K, L, M, N, P, Q, S, T, V, W or Y; preferably R211T
P81A, C, D, E, F, G, H, I, K, L, M, N, Q, R, S, T, V, W or Y; preferably P81G
V112A, C, D, E, F, G, H, I, K, L, M, N, P, Q, R, S, T, W or Y; preferably V112C
N80A, C, D, E, F, G, H, I, K, L, M, P, Q, R, S, T, V, W or Y; preferably N80 R, G, N, D, P, T, E, V, A or G
L82A, C, D, E, F, G, H, I, M, N, P, Q, R, S, T, V, W or Y; preferably L82N, S or E
N88A, C, D, E, F, G, H, I, K, L, M, P, Q, R, S, T, V, W or Y; preferably N88C
N87A, C, D, E, F, G, H, I, K, L, M, P, Q, R, S, T, V, W or Y; preferably N87M or G

Preferred modification of one or more of the following residues results in a variant enzyme having an increased absolute transferase activity against phospholipid:

S3 N, R, A, G
M23 K, Q, L, G, T, S
H180 R
L82 G
Y179 E, R, N, V, K or Q
E309 R, S, L or A

One preferred modification is N80D. This is particularly the case when using the reference sequence SEQ ID No. 35 as the backbone. Thus, the reference sequence may be SEQ ID No. 16. This modification may be in combination with one or more further modifications. Therefore in a preferred embodiment of the present invention the nucleotide sequence encoding a lipid acyltransferase for use in any one of the methods and uses of the present invention may encode a lipid acyltransferase that comprises SEQ ID No. 35 or an amino acid sequence which has 75% or more, preferably 85% or more, more preferably 90% or more, even more preferably 95% or more, even more preferably 98% or more, or even more preferably 99% or more identity to SEQ ID No. 35.
As noted above, when referring to specific amino acid residues herein the numbering is that obtained from alignment of the variant sequence with the reference sequence shown as SEQ ID No. 34 or SEQ ID No. 35.
Much by preference, the nucleotide sequence encoding a lipid acyltransferase for use in any one of the methods and uses of the present invention may encode a lipid comprising the amino acid sequence shown as SEQ ID No. 16 or the amino acid sequence shown as SEQ ID No. 68, or an amino acid sequence which has 70% or more, preferably 75% or more, preferably 85% or more, more preferably 90% or more, even more preferably 95% or more, even more preferably 98% or more, or even more preferably 99% or more identity to SEQ ID No. 16 or SEQ ID No. 68. This enzyme may be considered a variant enzyme.

In a preferred embodiment, the variant enzyme comprises one of SEQ ID No. 121, SEQ ID No. 122 or SEQ ID No. 123.

For the purposes of the present invention, the degree of identity is based on the number of sequence elements which are the same. The degree of identity in accordance with the present invention for amino acid sequences may be suitably determined by means of computer programs known in the art, such as Vector NTI 10 (Invitrogen Corp.). For pairwise alignment the score used is preferably BLOSUM62 with Gap opening penalty of 10.0 and Gap extension penalty of 0.1.

Suitably, the degree of identity with regard to an amino acid sequence is determined over at least 20 contiguous amino acids, preferably over at least 30 contiguous amino acids, preferably over at least 40 contiguous amino acids, preferably over at least 50 contiguous amino acids, preferably over at least 60 contiguous amino acids.

Suitably, the degree of identity with regard to an amino acid sequence may be determined over the whole sequence.

Suitably, the nucleotide sequence encoding a lipid acyltransferase or the lipid acyl transferase enzyme for use in the present invention may be obtainable, preferably obtained, from organisms from one or more of the following genera: *Aeromonas*, *Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas, Candida, Thermobifida* and *Corynebacterium.*

Suitably, the nucleotide sequence encoding a lipid acyltransferase or the lipid acyl transferase enzyme for use in the present invention may be obtainable, preferably obtained, from one or more of the following organisms: *Aeromonas hydrophila, Aeromonas salmonicida, Streptomyces coelicolor, Streptomyces rimosus, Mycobacterium, Streptococcus pyogenes, Lactococcus lactis, Streptococcus pyogenes, Streptococcus thermophilus, Streptomyces thermosacchari, Streptomyces avermitilis Lactobacillus helveticus, Desulfitobacterium dehalogenans, Bacillus sp, Campylobacter jejuni, Vibrionaceae, Xylella fastidiosa, Sulfolobus solfataricus, Saccharomyces cerevisiae, Aspergillus terreus, Schizosaccharomyces pombe, Listeria innocua, Listeria monocytogenes, Neisseria meningitidis, Mesorhizobium loti, Ralstonia solanacearum, Xanthomonas campestris, Xanthomonas axonopodis, Candida parapsilosis, Thermobifida fusca* and *Corynebacterium efficiens.*

In one aspect, preferably the nucleotide sequence encoding a lipid acyltransferase for use in any one of the methods and/or uses of the present invention encodes a lipid acyl transferase enzyme according to the present invention is obtainable, preferably obtained or derived, from one or more of *Aeromonas spp., Aeromonas hydrophila* or *Aeromonas salmonicida.*

In one aspect, preferably the lipid acyltransferase for use in any one of the methods and/or uses of the present invention is a lipid acyl transferase enzyme obtainable, preferably obtained or derived, from one or more of *Aeromonas spp., Aeromonas hydrophila* or *Aeromonas salmonicida.*

Enzymes which function as lipid acyltransferases in accordance with the present invention can be routinely identified using the assay taught herein below:

ASSAY FOR TRANSFERASE ACTIVITY

The transferase activity is preferably measured by the molar amount of cholesterol ester formed by acyl transfer from phospholipids and/or lipids in milk to cholesterol relative to the amount of cholesterol originally available.

Milk is incubated with enzyme or water (as control) for 30 minutes at 40°C. Milk lipids are isolated by solvent extraction and the isolated lipids are analysed by GLC.

Based on GLC analysis the amount of cholesterol (CHL), cholesterol ester (CHLE) and free fatty acids (FFA) are calculated:

$$\% \text{ Transferase} = \frac{CHLE(t) - CHLE(0)}{CHLE(t) - CHLE(0) + FFA(t) - FFA(0)} \times 100$$

Where

CHLE(O) = mol/l cholesterol ester (control)
CHLE(t) = mol/l cholesterol ester (enzyme treatment)
FFA(O) = mol/l free fatty acids (cControl)
FFA(t) = mol/l free fatty acids (eEnzyme treatment)

GLC analysis may be carried out according to Example 5 below. Using this assay, lipid acyltransferases/lipid acyl transferase in accordance with the present invention are those which have at least 5% transferase activity, preferably

at least 10% transferase activity, preferably at least 15%, 20%, 25% 26%, 28%, 30%, 40%, 50%, 60% or 70% transferase activity.

The term "transferase" as used herein is interchangeable with the term "lipid acyltransferase".

Suitably, the lipid acyltransferase as defined herein catalyses one or more of the following reactions: interesterification, transesterification, alcoholysis, hydrolysis.

The term "interesterification" refers to the enzymatic catalysed transfer of acyl groups between a lipid donor and lipid acceptor, wherein the lipid donor is not a free acyl group.

The term "transesterification" as used herein means the enzymatic catalysed transfer of an acyl group from a lipid donor (other than a free fatty acid) to an acyl acceptor (other than water).

As used herein, the term "alcoholysis" refers to the enzymatic cleavage of a covalent bond of an acid derivative by reaction with an alcohol ROH so that one of the products combines with the H of the alcohol and the other product combines with the OR group of the alcohol.

As used herein, the term "alcohol" refers to an alkyl compound containing a hydroxyl group.

As used herein, the term "hydrolysis" refers to the enzymatic catalysed transfer of an acyl group from a lipid to the OH group of a water molecule.

The term "without increasing or without substantially increasing the free fatty acids" as used herein means that preferably the lipid acyl transferase according to the present invention has 100% transferase activity (i.e. transfers 100% of the acyl groups from an acyl donor onto the acyl acceptor, with no hydrolytic activity); however, the enzyme may transfer less than 100% of the acyl groups present in the lipid acyl donor to the acyl acceptor. In which case, preferably the acyltransferase activity accounts for at least 5%, more preferably at least 10%, more preferably at least 20%, more preferably at least 30%, more preferably at least 40%, more preferably 50%, more preferably at least 60%, more preferably at least 70%, more preferably at least 80%, more preferably at least 90% and more preferably at least 98% of the total enzyme activity. The % transferase activity (i.e. the transferase activity as a percentage of the total enzymatic activity) may be determined by the following the "Assay for Transferase Activity" given above.

In some aspects of the present invention, the term "without substantially increasing free fatty acids" as used herein means that the amount of free fatty acid in a edible oil treated with an lipid acyltransferase according to the present invention is less than the amount of free fatty acid produced in the edible oil when an enzyme other than a lipid acyltransferase according to the present invention had been used, such as for example as compared with the amount of free fatty acid produced when a conventional phospholipase enzyme, e.g. Lecitase Ultra™ (Novozymes A/S, Denmark), had been used.

The enzyme according to the present invention may be used with one or more other suitable food grade enzymes. Thus, it is within the scope of the present invention that, in addition to the enzyme of the invention, at least one further enzyme is added to the foodstuff. Such further enzymes include starch degrading enzymes such as endo- or exoamylases, pullulanases, debranching enzymes, hemicellulases including xylanases, cellulases, oxidoreductases, e.g. peroxidases, phenol oxidases, glucose oxidase, pyranose oxidase, sulfhydryl oxidase, or a carbohydrate oxidase such as one which oxidises maltose, for example hexose oxidase (HOX), lipases, phospholipases, glycolipases, galactolipases and proteases.

In one embodiment the enzyme may be Dairy HOX™, which acts as an oxygen scavenger to prolong shelf life of cheese while providing browning control in pizza ovens. Therefore in a one aspect the present invention relates to the use of an enzyme capable of reducing the Maillard reaction in a foodstuff (see WO 02/39828), such as a dairy product, for example cheese,
wherein the enzyme is preferably a maltose oxidising enzyme such as carbohydrate oxidae, glucose oxidase and/or hexose oxidase, in the process or preparing a food material and/or foodstuff according to the present invention.

In one preferred embodiment the lipid acyltransferase is used in combination with a lipase having one or more of the following lipase activities: glycolipase activity (E.C. 3.1.1.26, triacylglycerol lipase activity (E.C. 3.1.1.3), phospholipase A2 activity (E.C. 3.1.1.4) or phospholipase A1 activity (E.C. 3.1.1.32). Suitable, lipolytic enzymes are well known in the art and include by way of example the following lipolytic enzymes: LIPOPAN® F, LIPOPAN® XTRA and/or LECITASE® ULTRA (Novozymes A/S, Denmark), phospholipase A2 (e.g. phospholipase A2 from LIPOMOD™ 22L from Biocatalysts, LIPOMAX™ from Genencor), LIPOLASE® (Novozymes A/S, Denmark), YIELDMAX™ (Chr. Hansen, Denmark), PANAMORE™ (DSM), the lipases taught in WO 03/97835, EP 0 977 869 or EP 1 193 314. This combination of a lipid acyl transferase as defined herein and a lipase may be particularly preferred in dough or baked products or in fine food products such as cakes and confectionary.

In some embodiments, it may also be beneficial to combine the use of lipid acyltransferase with a lipolytic enzymes such as rennet paste prepared from calf, lamb, kid stomachs, or Palatase A750L (Novo), Palatase M200L (Novo), Palatase M1000 (Novo), or Piccantase A (DSM), also Piccantase from animal sources from DSM (K, KL, L & C) or Lipomod 187, Lipomod 338 (Biocatalysts). These lipases are used conventionally in the production of cheese to produce cheese flavours. These lipases may also be used to produce an enzymatically-modified foodstuff, for example a dairy product (e.g. cheese), particularly where said dairy product consists of, is produced from or comprises butterfat.

A combination of the lipid acyltransferase with one or more of these lipases may have a beneficial effect on flavour in the dairy product (e.g. cheese for instance).

The use of lipases in combination with the enzyme of the invention may be particularly advantageous in instances where some accumulation of free fatty acids maybe desirable, for example in cheese where the free fatty acids can impart a desirable flavour, or in the preparation of fine foods. The person skilled in the art will be able to combine proportions of lipolytic enzymes, for example LIPOPAN® F, LIPOPAN® XTRA and/or LECITASE® ULTRA (Novozymes A/S, Denmark), phospholipase A2 (e.g. phospholipase A2 from LIPOMOD™ 22L from Biocatalysts, LIPOMAX™ from Genencor), LIPOLASE® (Novozymes A/S, Denmark), YIELDMAX™ (Chr. Hansen, Denmark), PANAMORE™ (DSM), the lipases taught in WO 03/97835, EP 0 977 869 or EP 1 193 314 and the lipid acyltransferase of the present invention to provide the desired ratio of hydrolytic to transferase activity which results in a preferred technical effect or combination of technical effects in the foodstuff (such as those listed herein under 'Technical Effects').

It may also be beneficial to combine the use of lipid acyltransferase with a phospholipase, such as phospholipase A1, phospholipase A2, phospholipase B, Phospholipase C and/or phospholipase D.

The combined use may be performed sequentially or concurrently, e.g. the lipid acyl transferase treatment may occur prior to or during the further enzyme treatment. Alternatively, the further enzyme treatment may occur prior to or during the lipid acyl transferase treatment.

In the case of sequential enzyme treatments, in some embodiments it may be advantageous to remove the first enzyme used, e.g. by heat deactivation or by use of an immobilised enzyme, prior to treatment with the second (and/or third etc.) enzyme.

## POST-TRANSCRIPTION AND POST-TRANSLATIONAL MODIFICATIONS

Suitably the lipid acyltransferase in accordance with the present invention may be encoded by any one of the nucleotide sequences taught herein.

Depending upon the host cell used post-transcriptional and/or post-translational modifications may be made. It is envisaged that the lipid acyltransferase for use in the present methods and/or uses encompasses lipid acyltransferases which have undergone post-transcriptional and/or post-translational modification.

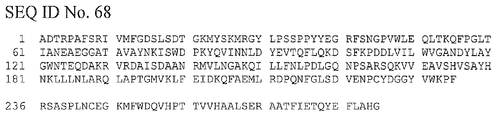

By way of example only, the expression of the nucleotide sequence shown herein as SEQ ID No. 49 (see Figure 57) in a host cell (such as *Bacillus licheniformis* for example) results in post-transcriptional and/or post-translational modifications which lead to the amino acid sequence shown herein as SEQ ID No. 68 (see Figure 73).

SEQ ID No. 68 is the same as SEQ ID No. 16 (shown herein in Figure 1) except that SEQ ID No. 68 has undergone post-translational and/or post-transcriptional modification to remove 38 amino acids.

SEQ ID NO. 16 may also be post transcriptionally and/or post translationally modified to remove 39, 40 or 41 amino as shown in SEQ ID NOs. 121, 122 and 123 respectively.

## ISOLATED

In one aspect, the lipid acyltransferase is a recovered/isolated lipid acyltransferase. Thus, the lipid acyltransferase produced may be in an isolated form.

In another aspect, the nucleotide sequence encoding a lipid acyltransferase for use in the present invention may be in an isolated form.

The term "isolated" means that the sequence or protein is at least substantially free from at least one other component with which the sequence or protein is naturally associated in nature and as found in nature.

## PURIFIED

In one aspect, the lipid acyltransferase may be in a purified form.

In another aspect, the nucleotide sequence encoding a lipid acyltransferase for use in the present invention may be in a purified form.

The term "purified" means that the sequence is in a relatively pure state - e.g. at least about 51% pure, or at least about 75%, or at least about 80%, or at least about 90% pure, or at least about 95% pure or at least about 98% pure.

## CLONING A NUCLEOTIDE SEQUENCE ENCODING A POLYPEPTIDE ACCORDING TO THE PRESENT INVENTION

A nucleotide sequence encoding either a polypeptide which has the specific properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger

RNA from the organism producing the polypeptide. If the amino acid sequence of the polypeptide is known, labeled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

NUCLEOTIDE SEQUENCES

The present invention also encompasses nucleotide sequences encoding polypeptides having the specific properties as defined herein. The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or antisense strand.

The term "nucleotide sequence" in relation to the present invention includes genomic DNA, cDNA, synthetic DNA, and RNA. Preferably it means DNA, more preferably cDNA for the coding sequence.

In a preferred embodiment, the nucleotide sequence *per* se encoding a polypeptide having the specific properties as defined herein does not cover the native nucleotide sequence in its natural environment when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. Thus, the polypeptide of the present invention can be expressed by a nucleotide sequence in its native organism but wherein the nucleotide sequence is not under the control of the promoter with which it is naturally associated within that organism.

Preferably the polypeptide is not a native polypeptide. In this regard, the term "native polypeptide" means an entire polypeptide that is in its native environment and when it has been expressed by its native nucleotide sequence.

Typically, the nucleotide sequence encoding polypeptides having the specific properties as defined herein is prepared using recombinant DNA techniques (i.e. recombinant DNA). However, in an alternative embodiment of the invention, the nucleotide sequence could be synthesised, in whole or in part, using chemical methods well known in the art (see Caruthers MH et al (1980) Nuc Acids Res Symp Ser 215-23 and Horn T et al (1980) Nuc Acids Res Symp Ser 225-232).

MOLECULAR EVOLUTION

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme in accordance with the present invention.

Mutations may be introduced using synthetic oligonucleotides. These oligonucleotides contain nucleotide sequences flanking the desired mutation sites.

A suitable method is disclosed in Morinaga et al (Biotechnology (1984) 2, p646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which

can also be combined with the use of mutagenic DNA analogues such as those described in EP 0 866 796. Error prone PCR technologies are suitable for the production of variants of lipid acyl transferases with preferred characteristics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins. Alternatively one can use one or multiple non-identical nucleotide sequences and introduce mutations during the reassembly of the full nucleotide sequence. DNA shuffling and family shuffling technologies are suitable for the production of variants of lipid acyl transferases with preferred characteristics. Suitable methods for performing 'shuffling' can be found in EP0 752 008, EP1 138 763, EP1 103 606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded polypeptide by various means. Using in silico and exo mediated recombination methods (see WO 00/58517, US 6,344,328, US 6,361,974), for example, molecular evolution can be performed where the variant produced retains very low homology to known enzymes or proteins. Such variants thereby obtained may have significant structural analogy to known transferase enzymes, but have very low amino acid sequence homology.

As a non-limiting example, In addition, mutations or natural variants of a polynucleotide sequence can be recombined with either the wild type or other mutations or natural variants to produce new variants. Such new variants can also be screened for improved functionality of the encoded polypeptide.

The application of the above-mentioned and similar molecular evolution methods allows the identification and selection of variants of the enzymes of the present invention which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate.

As will be apparent to a person skilled in the art, using molecular evolution tools an enzyme may be altered to improve the functionality of the enzyme.

Suitably, the nucleotide sequence encoding a lipid acyltransferase used in the invention may encode a variant lipid acyltransferase, i.e. the lipid acyltransferase may contain at least one amino acid substitution, deletion or addition, when compared to a parental enzyme. Variant enzymes retain at least 1%, 2%, 3%, 5%, 10%, 15%, 20%, 30%, 40%, 50%, 60%, 70%, 80%, 90%, 95%, 97%, 99% homology with the parent enzyme. Suitable parent enzymes may include any enzyme with esterase or lipase activity. Preferably, the parent enzyme aligns to the pfam00657 consensus sequence.

In a preferable embodiment a variant lipid acyltransferase enzyme retains or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a lipid acyltransferase enzyme with significant transferase activity suitable for use in the compositions and methods of the present invention.

Suitably, the nucleotide sequence encoding a lipid acyltransferase for use in any one of the methods and/or uses of the present invention may encode a lipid acyltransferase that may be a variant with enhanced enzyme activity on polar lipids, preferably phospholipids and/or glycolipids when compared to the parent enzyme. Preferably, such variants also have low or no activity on lyso polar lipids. The enhanced activity on polar lipids, phospholipids and/or glycolipids may be the result of hydrolysis and/or transferase activity or a combination of both.

Variant lipid acyltransferases may have decreased activity on triglycerides, and/or monoglycerides and/or diglycerides compared with the parent enzyme.

Suitably the variant enzyme may have no activity on triglycerides and/or monoglycerides and/or diglycerides.

Alternatively, the variant enzyme may have increased activity on triglycerides, and/or may also have increased activity on one or more of the following, polar lipids, phospholipids, lecithin, phosphatidylcholine, glycolipids, digalactosyl monoglyceride, monogalactosyl monoglyceride.

Variants of lipid acyltransferases are known, and one or more of such variants may be suitable for use in the methods and uses according to the present invention and/or in the enzyme compositions according to the present invention. By way of example only, variants of lipid acyltransferases are described in the following references may be used in accordance with the present invention: Hilton & Buckley J. Biol. Chem. 1991 Jan 15: 266 (2): 997-1000; Robertson et al J. Biol. Chem. 1994 Jan 21; 269(3):2146-50; Brumlik et al J. Bacteriol. 1996 Apr; 178 (7): 2060-4; Peelman et al Protein Sci. 1998 Mar; 7(3):587-99.

AMINO ACID SEQUENCES

The present invention also encompasses the use of amino acid sequences encoded by a nucleotide sequence which encodes a lipid acyltransferase for use in any one of the methods and/or uses of the present invention.
As used herein, the term "amino acid sequence" is synonymous with the term "polypeptide" and/or the term "protein". In some instances, the term "amino acid sequence" is synonymous with the term "peptide".
The amino acid sequence may be prepared/isolated from a suitable source, or it may be made synthetically or it may be prepared by use of recombinant DNA techniques.
Suitably, the amino acid sequences may be obtained from the isolated polypeptides taught herein by standard techniques.
One suitable method for determining amino acid sequences from isolated polypeptides is as follows:

Purified polypeptide may be freeze-dried and 100 μg of the freeze-dried material may be dissolved in 50 μl of a mixture of 8 M urea and 0.4 M ammonium hydrogen carbonate, pH 8.4. The dissolved protein may be denatured and reduced for 15 minutes at 50°C following overlay with nitrogen and addition of 5 μl of 45 mM dithiothreitol. After cooling to room temperature, 5 μl of 100 mM iodoacetamide may be added for the cysteine residues to be derivatized for 15 minutes at room temperature in the dark under nitrogen.

135 μl of water and 5 μg of endoproteinase Lys-C in 5 μl of water may be added to the above reaction mixture and the digestion may be carried out at 37°C under nitrogen for 24 hours.
The resulting peptides may be separated by reverse phase HPLC on a VYDAC C18 column (0.46x15cm;1 10μm; The Separation Group, California, USA) using solvent A: 0.1% TFA in water and solvent B: 0.1 % TFA in acetonitrile. Selected peptides may be re-chromatographed on a Develosil C18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

SEQUENCE IDENTITY OR SEQUENCE HOMOLOGY

Here, the term "homologue" means an entity having a certain homology with the subject amino acid sequences and the subject nucleotide sequences. Here, the term "homology" can be equated with "identity".
The homologous amino acid sequence and/or nucleotide sequence should provide and/or encode a polypeptide which retains the functional activity and/or enhances the activity of the enzyme.
In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.
In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.
Homology comparisons can be conducted by eye, or more usually, with the aid of readily available sequence comparison programs. These commercially available computer programs can calculate % homology between two or more sequences.
% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.
Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.
However, these more complex methods assign "gap penalties" to each gap that occurs in the alignment so that, for the same number of identical amino acids, a sequence alignment with as few gaps as possible - reflecting higher

relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons.

Calculation of maximum % homology therefore firstly requires the production of an optimal alignment, taking into consideration gap penalties. A suitable computer program for carrying out such an alignment is the Vector NTI (Invitrogen Corp.). Examples of other software that can perform sequence comparisons include, but are not limited to, the BLAST package (see Ausubel et al 1999 Short Protocols in Molecular Biology, 4th Ed - Chapter 18), and FASTA (Altschul et al 1990 J. Mol. Biol. 403-410). Both BLAST and FASTA are available for offline and online searching (see Ausubel *et al* 1999, pages 7-58 to 7-60). However, for some applications, it is preferred to use the Vector NTI program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

Should Gap Penalties be used when determining sequence identity, then preferably the following parameters are used for pairwise alignment:

| **FOR BLAST** | |
|---|---|
| GAP OPEN | 0 |
| GAP EXTENSION | 0 |

| **FOR CLUSTAL** | DNA | PROTEIN | |
|---|---|---|---|
| WORD SIZE | 2 | 1 | K triple |
| GAP PENALTY | 15 | 10 | |
| GAP EXTENSION | 6.66 | 0.1 | |

In one embodiment, preferably the sequence identity for the nucleotide sequences is determined using CLUSTAL with the gap penalty and gap extension set as defined above.

Suitably, the degree of identity with regard to a nucleotide sequence is determined over at least 20 contiguous nucleotides, preferably over at least 30 contiguous nucleotides, preferably over at least 40 contiguous nucleotides, preferably over at least 50 contiguous nucleotides, preferably over at least 60 contiguous nucleotides, preferably over at least 100 contiguous nucleotides.

Suitably, the degree of identity with regard to a nucleotide sequence may be determined over the whole sequence.

In one embodiment the degree of amino acid sequence identity in accordance with the present invention may be suitably determined by means of computer programs known in the art, such as Vector NTI 10 (Invitrogen Corp.). For pairwise alignment the matrix used is preferably BLOSUM62 with Gap opening penalty of 10.0 and Gap extension penalty of 0.1.

Suitably, the degree of identity with regard to an amino acid sequence is determined over at least 20 contiguous amino acids, preferably over at least 30 contiguous amino acids, preferably over at least 40 contiguous amino acids, preferably over at least 50 contiguous amino acids, preferably over at least 60 contiguous amino acids.

Suitably, the degree of identity with regard to an amino acid sequence may be determined over the whole sequence.

The sequences may also have deletions, insertions or substitutions of amino acid residues which produce a

silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to Table 2 below. Amino acids in the same block in the second column and preferably in the same line in the third column may be substituted for each other:

Table 2

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

Nucleotide sequences for use in the present invention or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences.

The present invention also encompasses the use of nucleotide sequences that are complementary to the sequences discussed herein, or any derivative, fragment or derivative thereof. If the sequence is complementary to a fragment thereof then that sequence can be used as a probe to identify similar coding sequences in other organisms etc.

Polynucleotides which are not 100% homologous to the sequences of the present invention but fall within the scope of the invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino

acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction polypeptide recognition sites, or to alter the property or function of the polypeptides encoded by the polynucleotides. Polynucleotides (nucleotide sequences) of the invention may be used to produce a primer, e.g. a PCR primer, a primer for an alternative amplification reaction, a probe e.g. labelled with a revealing label by conventional means using radioactive or nonradioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

HYBRIDISATION

The present invention also encompasses the use of sequences that are complementary to the sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the subject sequences discussed herein, or any derivative, fragment or derivative thereof.

The present invention also encompasses sequences that are complementary to sequences that are capable of hybridising to the nucleotide sequences discussed herein.

Hybridisation conditions are based on the melting temperature (Tm) of the nucleotide binding complex, as taught in Berger and Kimmel (1987, Guide to Molecular Cloning Techniques, Methods in Enzymology, Vol. 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical nucleotide sequences while an intermediate (or low) stringency hybridisation can be used to identify or detect similar or related polynucleotide sequences.

Preferably, the present invention encompasses the use of sequences that are complementary to sequences that are capable of hybridising under high stringency conditions or intermediate stringency conditions to nucleotide sequences encoding polypeptides having the specific properties as defined herein.

More preferably, the present invention encompasses the use of sequences that are complementary to sequences that are capable of hybridising under high stringency conditions (e.g. 65°C and 0.1xSSC {1xSSC = 0.15 M NaCl, 0.015 M Na-citrate pH 7.0}) to nucleotide sequences encoding polypeptides having the specific properties as defined herein.

The present invention also relates to the use of nucleotide sequences that can hybridise to the nucleotide sequences discussed herein (including complementary sequences of those discussed herein).

The present invention also relates to the use of nucleotide sequences that are complementary to sequences

that can hybridise to the nucleotide sequences discussed herein (including complementary sequences of those discussed herein).

Also included within the scope of the present invention are the use of polynucleotide sequences that are capable of hybridising to the nucleotide sequences discussed herein under conditions of intermediate to maximal stringency.

In a preferred aspect, the present invention covers the use of nucleotide sequences that can hybridise to the nucleotide sequences discussed herein, or the complement thereof, under stringent conditions (e.g. 50°C and 0.2xSSC).

In a more preferred aspect, the present invention covers the use of nucleotide sequences that can hybridise to the nucleotide sequences discussed herein, or the complement thereof, under high stringency conditions (e.g. 65°C and 0.1xSSC).

## EXPRESSION OF POLYPEPTIDES

A nucleotide sequence for use in the present invention or for encoding a polypeptide having the specific properties as defined herein can be incorporated into a recombinant replicable vector. The vector may be used to replicate and express the nucleotide sequence, in polypeptide form, in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The polypeptide produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

## CONSTRUCTS

The term "construct" - which is synonymous with terms such as "conjugate", "cassette" and "hybrid" - includes a nucleotide sequence encoding a polypeptide having the specific properties as defined herein for use according to the present invention directly or indirectly attached to a promoter. An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker which allows for the selection of the genetic construct.

For some applications, preferably the construct comprises at least a nucleotide sequence of the present invention or a nucleotide sequence encoding a polypeptide having the specific properties as defined herein operably linked to a promoter.

## ORGANISM

The term "organism" in relation to the present invention includes any organism that could comprise a nucleotide sequence according to the present invention or a nucleotide sequence encoding for a polypeptide having the specific properties as defined herein and/or products obtained therefrom.

The term "transgenic organism" in relation to the present invention includes any organism that comprises a nucleotide sequence coding for a polypeptide having the specific properties as defined herein and/or the products obtained therefrom, and/or wherein a promoter can allow expression of the nucleotide sequence coding for a polypeptide having the specific properties as defined herein within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism of the present invention includes an organism comprising any one of, or combinations of, a nucleotide sequence coding for a polypeptide having the specific properties as defined herein, constructs as defined herein, vectors as defined herein, plasmids as defined herein, cells as defined herein, or the products thereof. For example the transgenic organism can also comprise a nucleotide sequence coding for a polypeptide having the specific properties as defined herein under the control of a promoter not associated with a sequence encoding a lipid acyltransferase in nature.

TRANSFORMATION OF HOST CELLS/ORGANISM

The host organism can be a prokaryotic or a eukaryotic organism.

Examples of suitable prokaryotic hosts include bacteria such as *E. coli* and *Bacillus licheniformis,* preferably *B. licheniformis.*

Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

In another embodiment the transgenic organism can be a yeast.

Filamentous fungi cells may be transformed using various methods known in the art - such as a process involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

TRANSFORMED FUNGUS

A host organism may be a fungus - such as a filamentous fungus. Examples of suitable such hosts include any member belonging to the genera Thermomyces, Acremonium, Aspergillus, Penicillium, Mucor, Neurospora, Trichoderma and the like.

Teachings on transforming filamentous fungi are reviewed in US-A-5741665 which states that standard techniques for transformation of filamentous fungi and culturing the fungi are well known in the art. An extensive review of techniques as applied to N. *crassa* is found, for example in Davis and de Serres, Methods Enzymol (1971) 17A: 79-143.

Further teachings on transforming filamentous fungi are reviewed in US-A-5674707.

In one aspect, the host organism can be of the genus *Aspergillus,* such as *Aspergillus niger.*

A transgenic *Aspergillus* according to the present invention can also be prepared by following, for example, the teachings of Turner G. 1994 (Vectors for genetic manipulation. In: Martinelli S.D., Kinghorn J.R. (Editors) Aspergillus: 50 years on. Progress in industrial microbiology vol 29. Elsevier Amsterdam 1994. pp. 641-666).

Gene expression in filamentous fungi has been reviewed in Punt et al. (2002) Trends Biotechnol 2002 May;20(5):200-6, Archer & Peberdy Crit Rev Biotechnol (1997) 17(4):273-306.

TRANSFORMED YEAST

In another embodiment, the transgenic organism can be a yeast.

A review of the principles of heterologous gene expression in yeast are provided in, for example, Methods Mol Biol (1995), 49:341-54, and Curr Opin Biotechnol (1997) Oct;8(5):554-60

In this regard, yeast - such as the species *Saccharomyces cerevisi or Pichia pastoris* (see FEMS Microbiol Rev (2000, 24(1):45-66), may be used as a vehicle for heterologous gene expression.

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic *Saccharomyces* according to the present invention can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells may be selected using various selective markers - such as auxotrophic markers dominant antibiotic resistance markers.

A suitable yeast host organism can be selected from the biotechnologically relevant yeasts species such as, but not limited to, yeast species selected from *Pichia* spp., *Hansenula* spp., *Kluyveromyces, Yarrowinia* spp., *Saccharomyces* spp., including S. *cerevisiae,* or *Schizosaccharomyce* spp. including *Schizosaccharomyce pombe.*

A strain of the methylotrophic yeast species *Pichia pastoris* may be used as the host organism.

In one embodiment, the host organism may be a *Hansenula* species, such as H. *polymorpha* (as described in WO01/39544).

TRANSFORMED PLANTS/PLANT CELLS

A host organism suitable for the present invention may be a plant. A review of the general techniques may be found in articles by Potrykus (Annu Rev Plant Physiol Plant MolBiol [1991] 42:205-225) and Christou (Agro-Food-Industry Hi-Tech March/April 1994 17-27), or in WO 01/16308. The transgenic plant may produce enhanced levels of phytosterol esters and phytostanol esters, for example.

Therefore the present invention also relates to a method for the production of a transgenic plant with enhanced levels of phytosterol esters and phytostanol esters, comprising the steps of transforming a plant cell with a lipid acyltransferase as defined herein (in particular with an expression vector or construct comprising a lipid acyltransferase as defined herein), and growing a plant from the transformed plant cell.

SECRETION

Often, it is desirable for the polypeptide to be secreted from the expression host into the culture medium from where the enzyme may be more easily recovered. According to the present invention, the secretion leader sequence may be selected on the basis of the desired expression host. Hybrid signal sequences may also be used with the context of the present invention.

Typical examples of secretion leader sequences not associated with a nucleotide sequence encoding a lipid acyltransferase in nature are those originating from the fungal amyloglucosidase (AG) gene (*glaA* - both 18 and 24 amino acid versions e.g. from *Aspergillus*), the a-factor gene (yeasts e.g. *Saccharomyces, Kluyveromyces* and *Hansenula)* or the α-amylase gene (*Bacillus*).

DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies such as Pharmacia Biotech (Piscataway, NJ), Promega (Madison, WI), and US Biochemical Corp (Cleveland, OH) supply commercial kits and protocols for these procedures.

Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US 3,817,837; US 3,850,752; US 3,939,350; US 3,996,345; US 4,277,437; US 4,275,149 and US 4,366,241.

Also, recombinant immunoglobulins may be produced as shown in US-A-4,816,567.

FUSION PROTEINS

The lipid acyltransferase for use in the present invention may be produced as a fusion protein, for example to aid in extraction and purification thereof. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the activity of the protein sequence.

Gene fusion expression systems in E. coli have been reviewed in Curr. Opin. Biotechnol. (1995) 6(5):501-6.

The amino acid sequence of a polypeptide having the specific properties as defined herein may be ligated to a non-native sequence to encode a fusion protein. For example, for screening of peptide libraries for agents capable of affecting the substance activity, it may be useful to encode a chimeric substance expressing a non-native epitope that is recognised by a commercially available antibody.

The invention will now be described, by way of example only, with reference to the following Figures and Examples.

Figure 1 shows the amino acid sequence of a mutant *Aeromonas salmonicida* mature lipid acyltransferase (GCAT) with a mutation of Asn80Asp (notably, amino acid 80 is in the mature sequence) (SEQ ID 16);

Figure 2 shows an amino acid sequence (SEQ ID No. 1) of a lipid acyl transferase from *Aeromonas hydrophila* (ATCC #7965);

Figure 3 shows a pfam00657 consensus sequence from database version 6 (SEQ ID No. 2);

Figure 4 shows an amino acid sequence (SEQ ID No. 3) obtained from the organism *Aeromonas hydrophila* (P10480; GI: 121051);

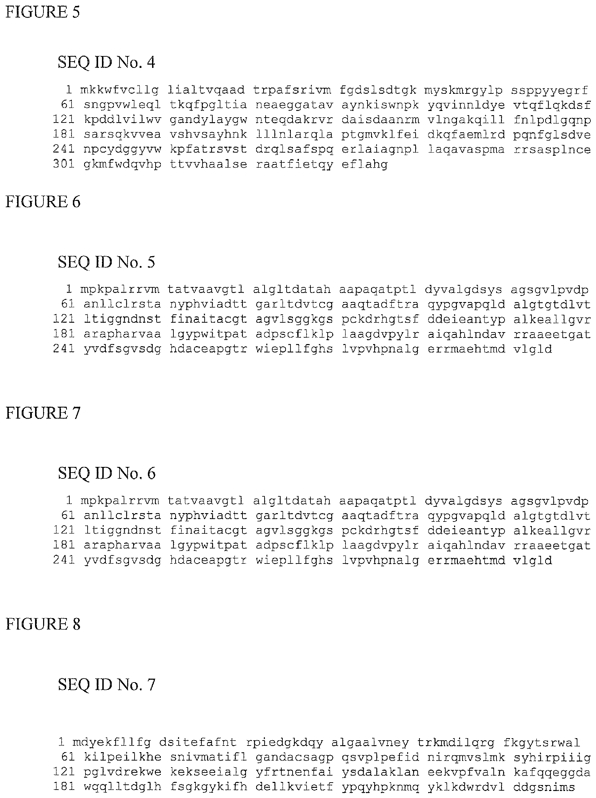

Figure 5 shows an amino acid sequence (SEQ ID No. 4) obtained from the organism *Aeromonas salmonicida* (AAG098404; GI:9964017);

Figure 6 shows an amino acid sequence (SEQ ID No. 5) obtained from the organism *Streptomyces coelicolor* A3(2) (Genbank accession number NP_631558);

Figure 7 shows an amino acid sequence (SEQ ID No. 6) obtained from the organism *Streptomyces coelicolor* A3(2) (Genbank accession number: CAC42140);

Figure 8 shows an amino acid sequence (SEQ ID No. 7) obtained from the organism *Saccharomyces cerevisiae* (Genbank accession number P41734);

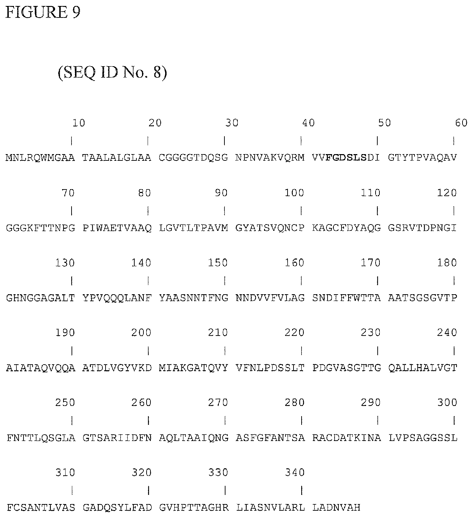

Figure 9 shows an amino acid sequence (SEQ ID No. 8) obtained from the organism *Ralstonia* (Genbank accession number: AL646052);

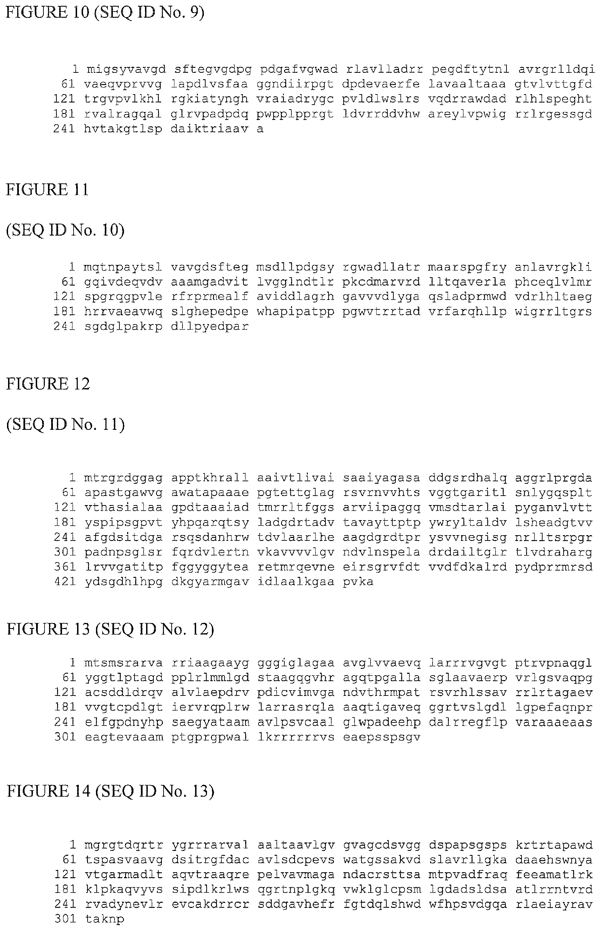

Figure 10 shows SEQ ID No. 9. Scoe1 NCBI protein accession code CAB39707.1 GI:4539178 conserved hypothetical protein *[Streptomyces coelicolor* A3(2)];

Figure 11 shows an amino acid shown as SEQ ID No. 10. Scoe2 NCBI protein accession code CAC01477.1 GI:9716139 conserved hypothetical protein *[Streptomyces coelicolor* A3(2)];

Figure 12 shows an amino acid sequence (SEQ ID No. 11) Scoe3 NCBI protein accession code CAB88833.1 GI:7635996 putative secreted protein *[Streptomyces coelicolor* A3(2)];

Figure 13 shows an amino acid sequence (SEQ ID No. 12) Scoe4 NCBI protein accession code CAB89450.1 GI:7672261 putative secreted protein *[Streptomyces coelicolor* A3(2)];

Figure 14 shows an amino acid sequence (SEQ ID No. 13) Scoe5 NCBI protein accession code CAB62724.1 GI:6562793 putative lipoprotein *[Streptomyces coelicolor* A3(2)];

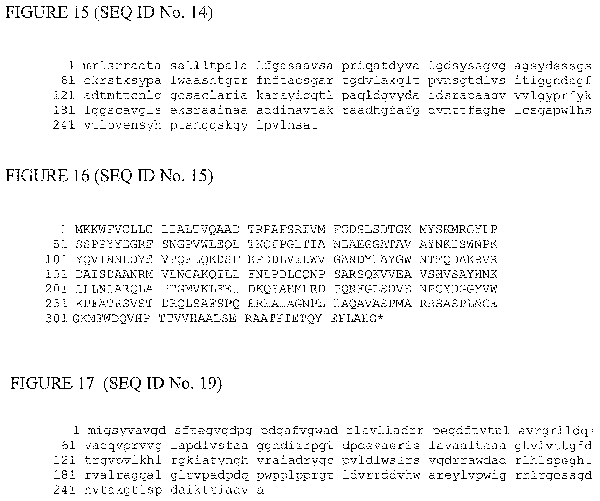

Figure 15 shows an amino acid sequence (SEQ ID No. 14) Srim1 NCBI protein accession code AAK84028.1 G1:15082088 GDSL-lipase *[Streptomyces rimosus*];

Figure 16 shows an amino acid sequence (SEQ ID No. 15) of a lipid acyltransferase from *Aeromonas salmonicida* subsp. *Salmonicida* (ATCC#14174);

Figure 17 shows SEQ ID No. 19. Scoe1 NCBI protein accession code CAB39707.1 GI:4539178 conserved hypothetical protein [*Streptomyces coelicolor* A3(2)];

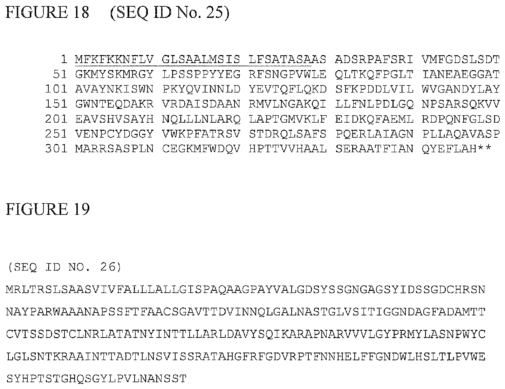

Figure 18 shows an amino acid sequence (SEQ ID No. 25) of the fusion construct used for mutagenesis of the *Aeromonas hydrophila* lipid acyltransferase gene. The underlined amino acids is a xylanase signal peptide;

Figure 19 shows a polypeptide sequence of a lipid acyltransferase enzyme from *Streptomyces* (SEQ ID No. 26);

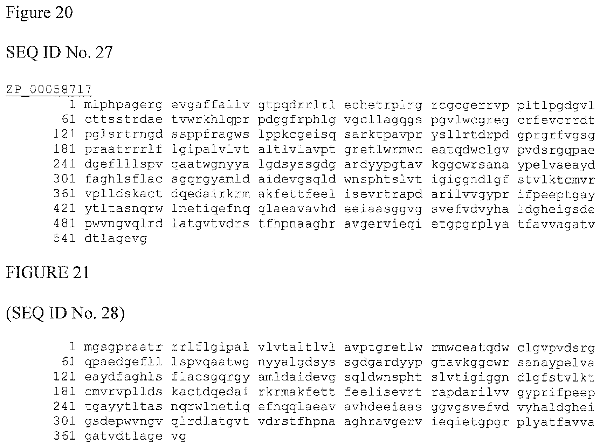

Figure 20 shows a polypeptide sequence of a lipid acyltransferase enzyme from *Thermobifida* (SEQ ID No. 27);

Figure 21 shows a polypeptide sequence of a lipid acyltransferase enzyme from *Thermobifida* (SEQ ID No. 28);

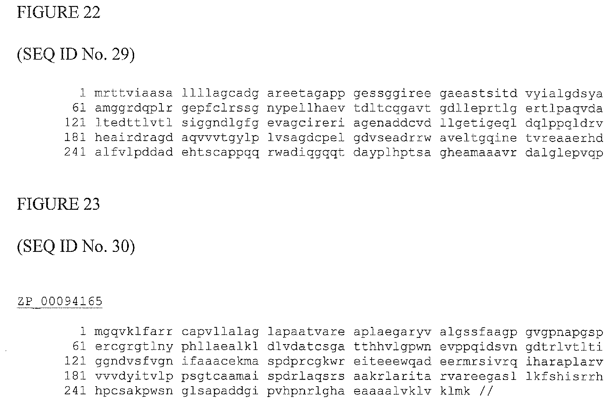

Figure 22 shows a polypeptide of a lipid acyltransferase enzyme from *Corynebacterium efficiens* GDSx 300 amino acid (SEQ ID No. 29);

Figure 23 shows a polypeptide of a lipid acyltransferase enzyme from *Novosphingobium aromaticivorans* GDSx 284 amino acid (SEQ ID No. 30);

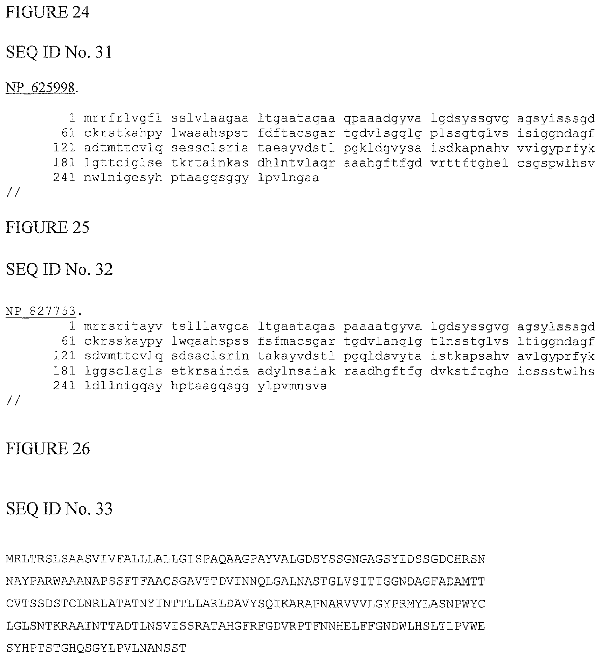

Figure 24 shows a polypeptide of a lipid acyltransferase enzyme from *Streptomyces coelicolor* GDSx 269 aa (SEQ ID No. 31);

Figure 25 shows a polypeptide of a lipid acyltransferase enzyme from *Streptomyces avermitilis* \ GDSx 269 amino acid (SEQ ID No. 32);

Figure 26 shows a polypeptide of a lipid acyltransferase enzyme from *Streptomyces* (SEQ ID No. 33);

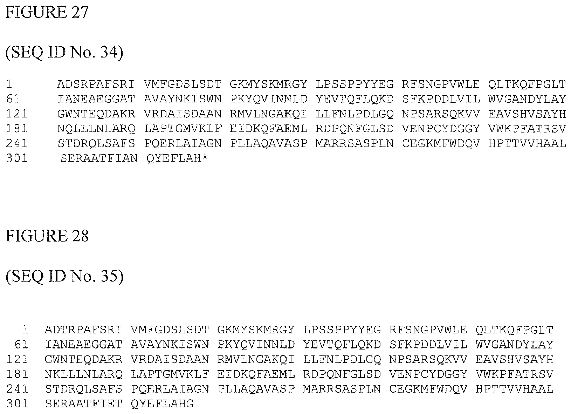

Figure 27 shows an amino acid sequence (SEQ ID No. 34) obtained from the organism *Aeromonas hydrophila* (P10480; GI:121051) (notably, this is the mature sequence);

Figure 28 shows the amino acid sequence (SEQ ID No. 35) of *Aeromonas salmonicida* mature lipid acyltransferase (GCAT) (notably, this is the mature sequence);

Figure 29 shows a nucleotide sequence (SEQ ID No. 36) from *Streptomyces thermosacchari;*

Figure 30 shows an amino acid sequence (SEQ ID No. 37) from *Streptomyces thermosacchari;*

Figure 31 shows an amino acid sequence (SEQ ID No. 38) from *Thermobifida fusca*/GDSx 548 amino acid;

Figure 32 shows a nucleotide sequence (SEQ ID No. 39) from *Thermobifida fusca;*

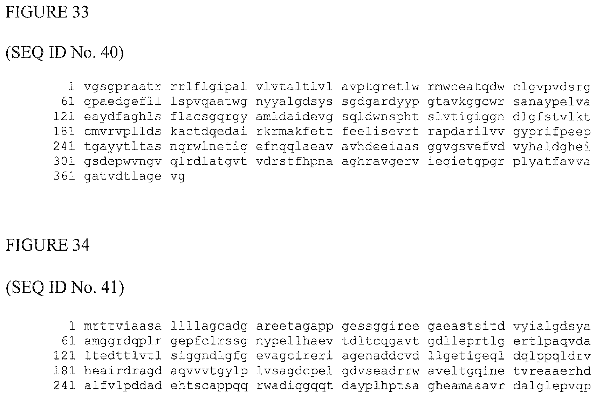

Figure 33 shows an amino acid sequence (SEQ ID No. 40) from *Thermobifida fusca*/GDSx;

Figure 34 shows an amino acid sequence (SEQ ID No. 41) from *Corynebacterium efficiens*/GDSx 300 amino acid;

Figure 35 shows a nucleotide sequence (SEQ ID No. 42) from *Corynebacterium efficiens;*

Figure 36 shows an amino acid sequence (SEQ ID No. 43) from S. *coelicolor*/ GDSx 268 amino acid;

Figure 37 shows a nucleotide sequence (SEQ ID No. 44) from S. *coelicolor;*

Figure 38 shows an amino acid sequence (SEQ ID No. 45) from S. *avermitilis;*

Figure 39 shows a nucleotide sequence (SEQ ID No. 46) from S. *avermitilis;*

Figure 40 shows an amino acid sequence (SEQ ID No. 47) from *Thermobifida fusca*/*GDSx;*

Figure 41 shows a nucleotide sequence (SEQ ID No. 48) from *Thermobifida fusca*/*GDSx;*

Figure 42 shows an alignment of the L131 and homologues from *S. avermitilis* and *T. fusca* illustrates that the conservation of the GDSx motif (GDSY in L131 and *S. avermitilis* and *T. fusca*), the GANDY box, which is either GGNDA or GGNDL, and the HPT block (considered to be the conserved catalytic histidine). These three conserved blocks are highlighted;

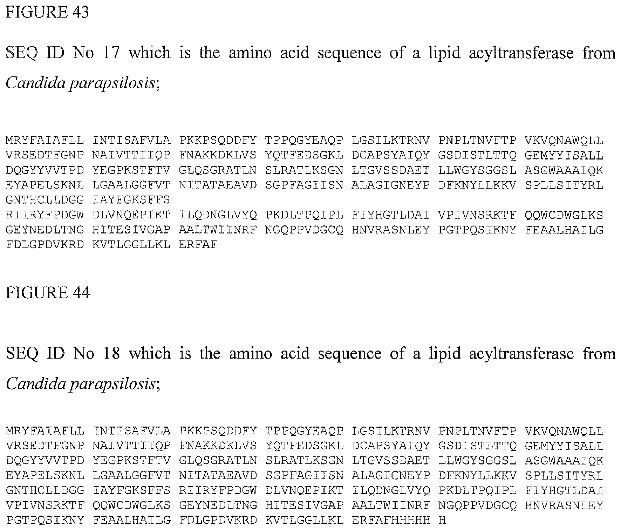

Figure 43 shows SEQ ID No 17 which is the amino acid sequence of a lipid acyltransferase from *Candida parapsilosis;*

Figure 44 shows SEQ ID No 18 which is the amino acid sequence of a lipid acyltransferase from *Candida parapsilosis;*

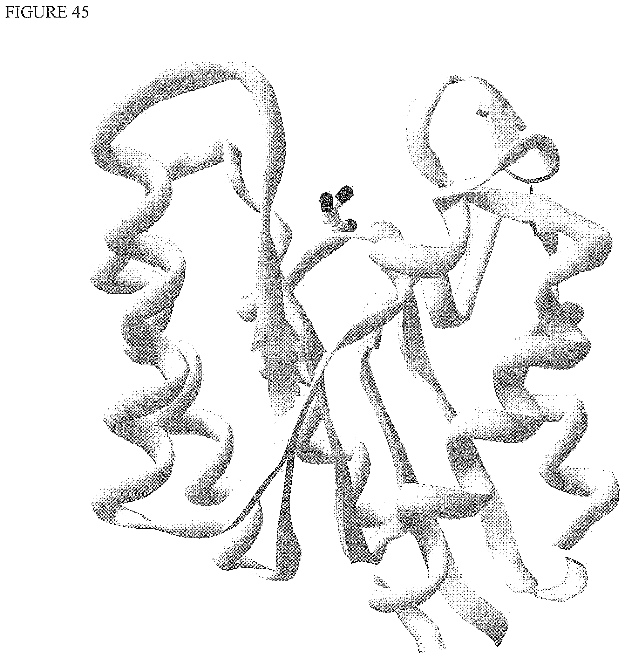

Figure 45 shows a ribbon representation of the 1IVN.PDB crystal structure which has glycerol in the active site. The Figure was made using the Deep View Swiss-PDB viewer;

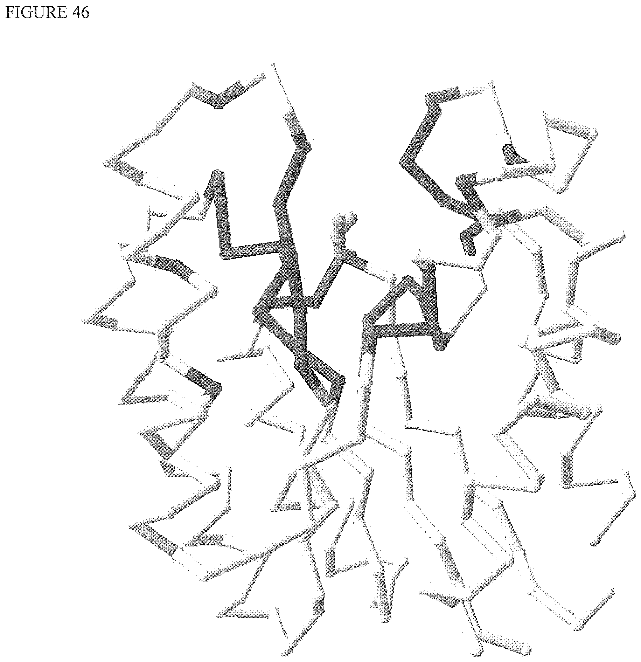

Figure 46 shows 1IVN.PDB Crystal Structure - Side View using Deep View Swiss-PDB viewer, with glycerol in active site - residues within 10Å of active site glycerol are coloured black;

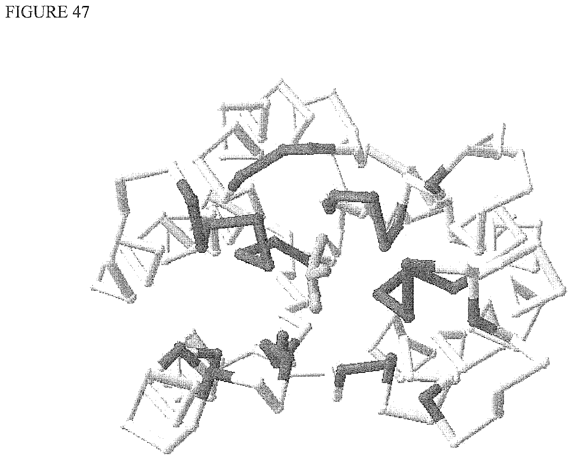

Figure 47 shows 1IVN.PDB Crystal Structure - Top View using Deep View Swiss-PDB viewer, with glycerol in active site - residues within 10Å of active site glycerol are coloured black;

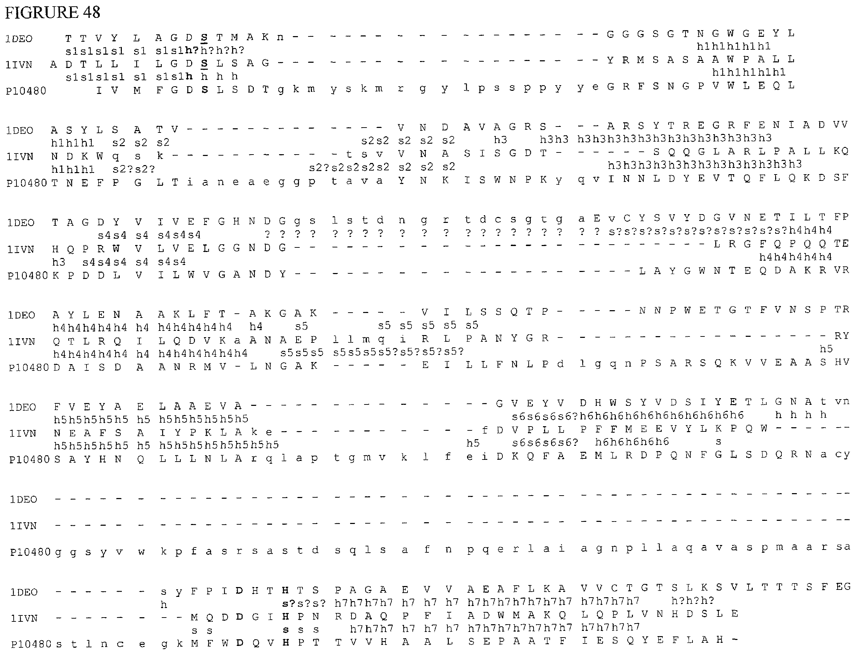

Figure 48 shows alignment 1;

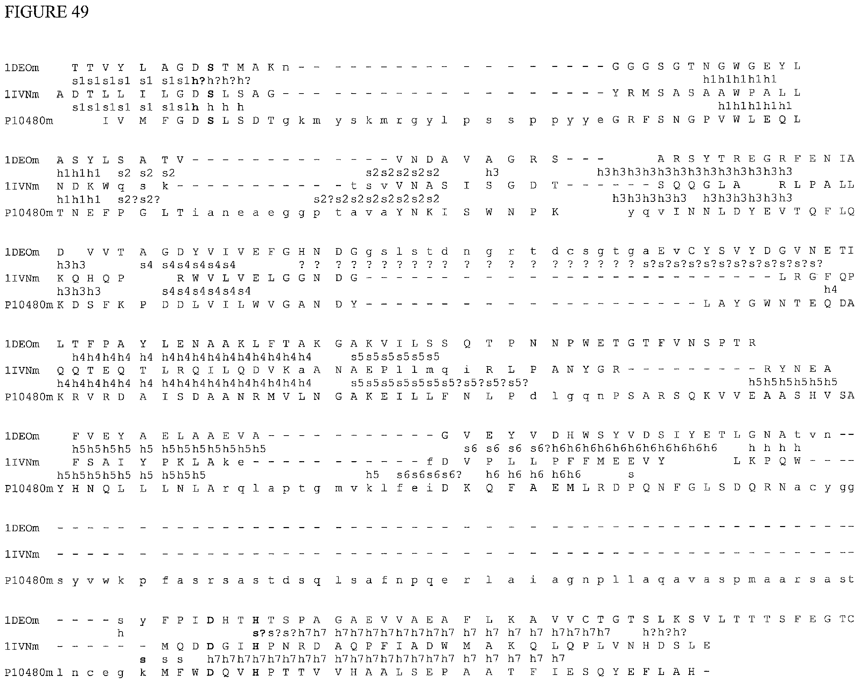

Figure 49 shows alignment 2;

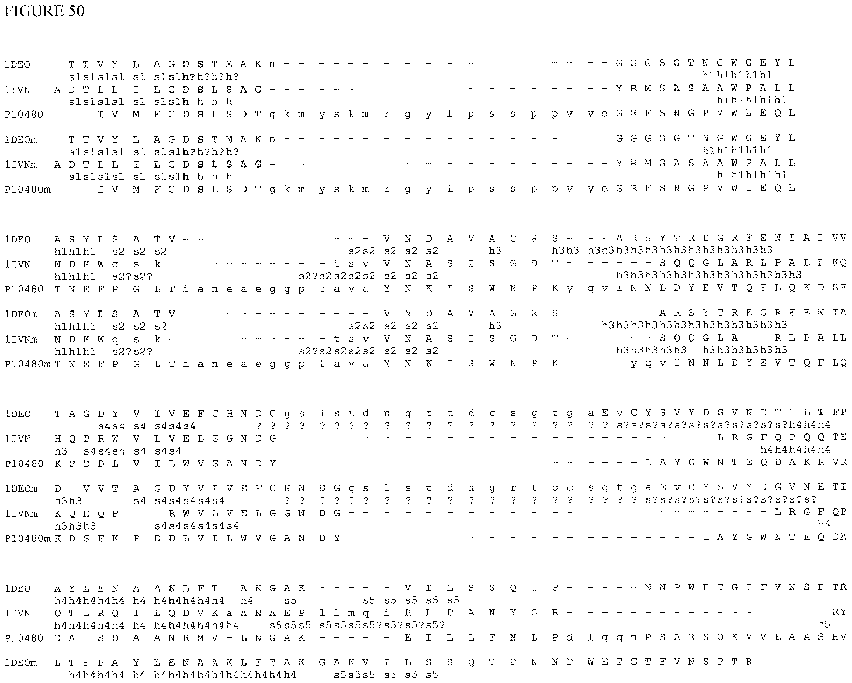

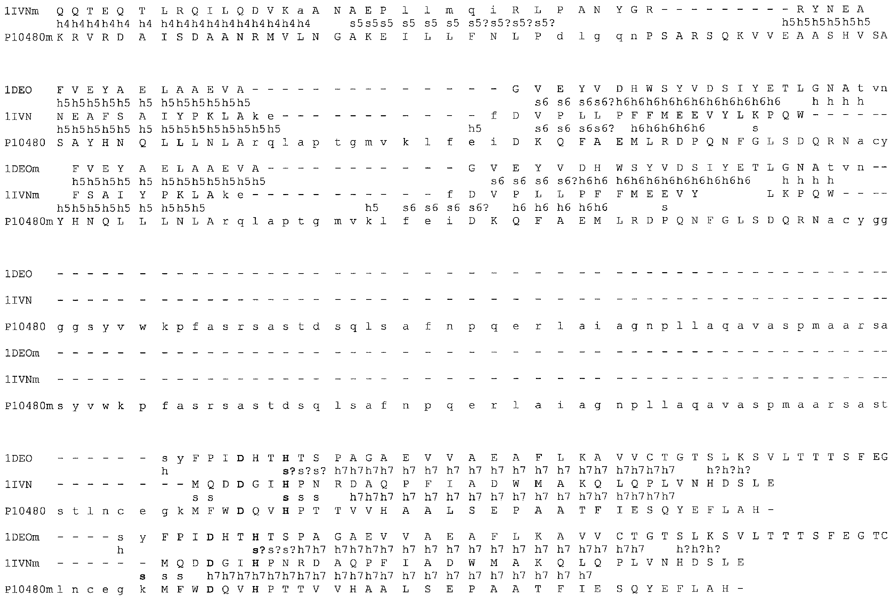

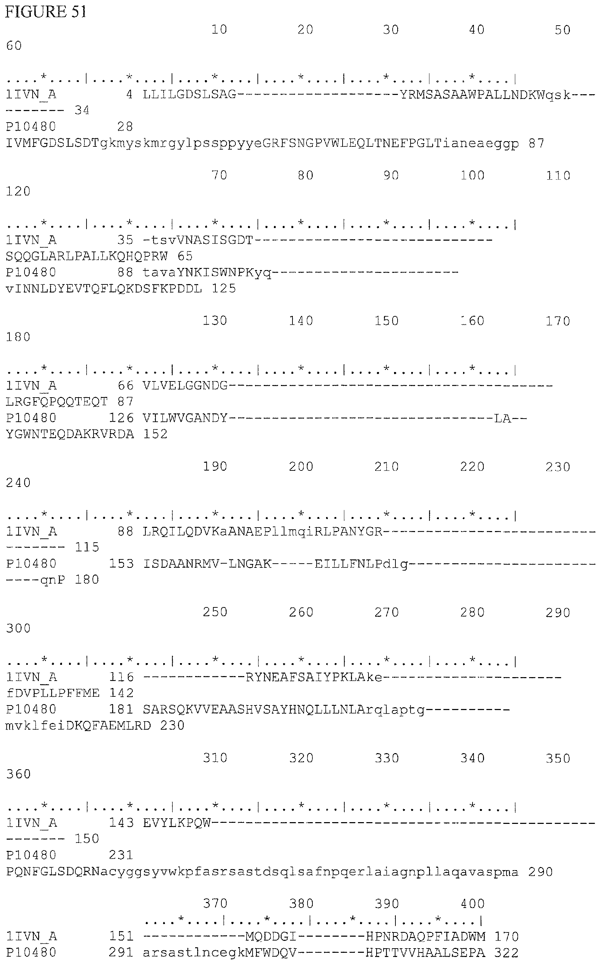

Figures 50 and 51 show an alignment of 1IVN to P10480 (P10480 is the database sequence for *A. hydrophila* enzyme), this alignment was obtained from the PFAM database and used in the model building process; and

Figure 52 shows an alignment where P10480 is the database sequence for *Aeromonas hydrophila.* This sequence is used for the model construction and the site selection (note that the full protein (SEQ ID No. 25) is depicted, the mature protein (equivalent to SEQ ID No. 34) starts at residue 19. A. sal is *Aeromonas salmonicida* (SEQ ID No. 4) GDSX lipase, A. hyd is *Aeromonas hydrophila* (SEQ ID No. 34) GDSX lipase; the consensus sequence contains a * at the position of a difference between the listed sequences);

Figure 53 shows a gene construct used in Example 1;

Figure 54 shows a codon optimised gene construct (no. 052907) used in Example 1; and

Figure 55 shows the sequence of the XhoI insert containing the LAT-KLM3' precursor gene, the -35 and -10 boxes are underlined;

Figure 56 shows BML780-KLM3'CAP50 (comprising SEQ ID No. 16 - upper colony) and BML780 (the empty host strain - lower colony) after 48h growth at 37°C on 1% tributyrin agar;

Figure 57 shows a nucleotide sequence from *Aeromonas salmonicida* (SEQ ID No. 49) including the signal sequence (preLAT - positions 1 to 87);

Figure 58 shows a nucleotide sequence (SEQ ID No. 50) encoding a lipid acyl transferase according to the present invention obtained from the organism *Aeromonas hydrophila;*

Figure 59 shows a nucleotide sequence (SEQ ID No. 51) encoding a lipid acyl transferase according to the present invention obtained from the organism *Aeromonas salmonicida;*

Figure 60 shows a nucleotide sequence (SEQ ID No. 52) encoding a lipid acyl transferase according to the present invention obtained from the organism *Streptomyces coelicolor* A3(2) (Genbank accession number NC_003888.1:8327480..8328367);

Figure 61 shows a nucleotide sequence (SEQ ID No. 53) encoding a lipid acyl transferase according to the present invention obtained from the organism *Streptomyces coelicolor* A3(2) (Genbank accession number AL939131.1:265480..266367);

Figure 62 shows a nucleotide sequence (SEQ ID No. 54) encoding a lipid acyl transferase according to the present invention obtained from the organism *Saccharomyces cerevisiae* (Genbank accession number Z75034);

Figure 63 shows a nucleotide sequence (SEQ ID No. 55) encoding a lipid acyl transferase according to the present invention obtained from the organism *Ralstonia*;

Figure 64 shows a nucleotide sequence shown as SEQ ID No. 56 encoding NCBI protein accession code CAB39707.1 GI:4539178 conserved hypothetical protein [*Streptomyces coelicolor* A3(2)];

Figure 65 shows a nucleotide sequence shown as SEQ ID No. 57 encoding Scoe2 NCBI protein accession code CAC01477.1 GI:9716139 conserved hypothetical protein [*Streptomyces coelicolor* A3(2)];

Figure 66 shows a nucleotide sequence shown as SEQ ID No. 58 encoding Scoe3 NCBI protein accession code CAB88833.1 GI:7635996 putative secreted protein. [*Streptomyces coelicolor* A3(2)];

Figure 67 shows a nucleotide sequence shown as SEQ ID No. 59 encoding Scoe4 NCBI protein accession code CAB89450.1 GI:7672261 putative secreted protein. [*Streptomyces coelicolor* A3(2)];

Figure 68 shows a nucleotide sequence shown as SEQ ID No. 60, encoding Scoe5 NCBI protein accession code CAB62724.1 GI:6562793 putative lipoprotein *[Streptomyces coelicolor* A3(2)];

Figure 69 shows a nucleotide sequence shown as SEQ ID No. 61 encoding Srim1 NCBI protein accession code AAK84028.1 GI:15082088 GDSL-lipase [*Streptomyces rimosus*];

Figure 70 shows a nucleotide sequence (SEQ ID No. 62) encoding a lipid acyltransferase from *Aeromonas hydrophila* (ATCC #7965);

Figure 71 shows a nucleotide sequence (SEQ ID No 63) encoding a lipid acyltransferase from *Aeromonas salmonicida* subsp. *Salmonicida* (ATCC#14174);

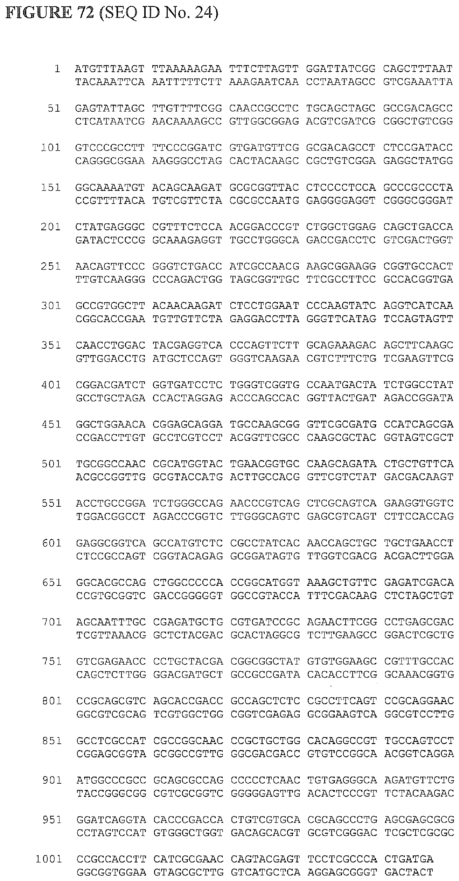

Figure 72 shows a nucleotide sequence (SEQ ID No. 24) encoding an enzyme from *Aeromonas hydrophila* including a xylanase signal peptide;

Figure 73 shows the amino acid sequence of a mutant *Aeromonas salmonicida* mature lipid acyltransferase (GCAT) with a mutation of Asn80Asp (notably, amino acid 80 is in the mature sequence) - shown herein as SEQ ID No. 16 - and after undergoing post-translational modification as SEQ ID No. 68 - amino acid residues 235 and 236 of SEQ ID No. 68 are not covalently linked following post-translational modification. The two peptides formed are held together by one or more S-S bridges. Amino acid 236 in SEQ ID No. 68 corresponds with the amino acid residue number 274 in SEQ ID No. 16 shown herein;

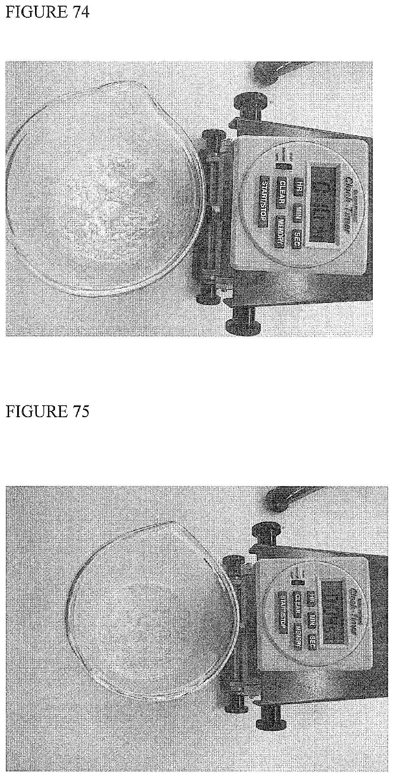

Figure 74 shows milk powder made from standard, untreated whole milk;

Figure 75 shows milk powder made from standard whole milk treated with a solution of KLM3 enzyme, (KTP08015, 1300 TIPU/g milk, corresponding to 12.4 mg enzyme/g milk); the activity of the enzyme in TIPU units being measured as described below;

Figure 76 illustrates the apparatus used to carry out the wettability test of Example 3;

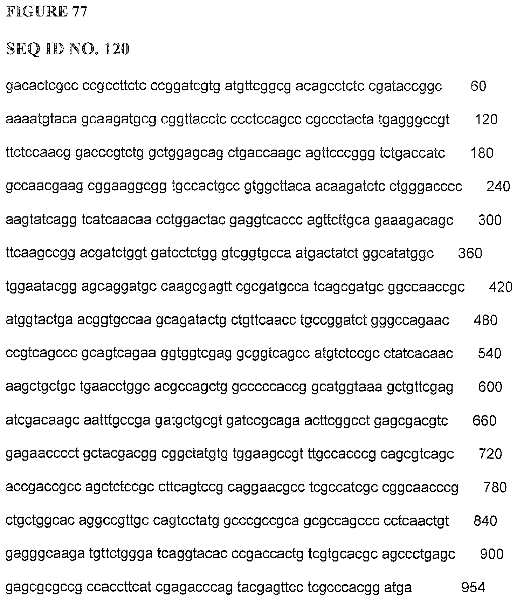

Figure 77 shows a nucleotide sequence (SEQ ID NO. 120) which encodes a lipid acyltransferase from *A. salmonicida;*

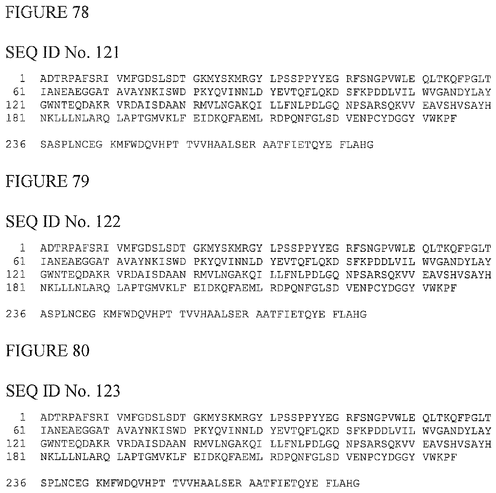

Figure 78 shows the amino acid sequence of a mutant *Aeromonas salmonicida* mature lipid acyltransferase (GCAT) with a mutation of Asn80Asp (notably, amino acid 80 is in the mature sequence) - shown herein as SEQ ID No. 16 - and after undergoing post-translational modification as SEQ ID No. 121 - amino acid residues 235 and 236 of SEQ ID No. 121 are not covalently linked following post-translational modification; the two peptides formed are held together by one or more S-S bridges; amino acid 236 in SEQ ID No. 121 corresponds with the amino acid residue number 275 in SEQ ID No. 16 shown herein;

Figure 79 shows the amino acid sequence of a mutant *Aeromonas salmonicida* mature lipid acyltransferase (GCAT) with a mutation of Asn80Asp (notably, amino acid 80 is in the mature sequence) - shown herein as SEQ ID No. 16 - and after undergoing post-translational modification as SEQ ID No. 122 - amino acid residues 235 and 236 of SEQ ID No. 122 are not covalently linked following post-translational modification; the two peptides formed are held together by one or more S-S bridges; amino acid 236 in SEQ ID No. 122 corresponds with the amino acid residue number 276 in SEQ ID No. 16 shown herein; and

Figure 80 shows the amino acid sequence of a mutant *Aeromonas salmonicida* mature lipid acyltransferase (GCAT) with a mutation of Asn80Asp (notably, amino acid 80 is in the mature sequence) - shown herein as SEQ ID No. 16 - and after undergoing post-translational modification as SEQ ID No. 123 - amino acid residues 235 and 236 of SEQ ID No. 123 are not covalently linked following post-translational modification; the two peptides formed are held together by one or more S-S bridges; amino acid 236 in SEQ ID No. 123 corresponds with the amino acid residue number 277 in SEQ ID No. 16 shown herein.

<u>Determination of phospholipase activity (TIPU-K Assay):</u>

Substrate:

0.6% L-$\alpha$ phosphatidylcholine 95% Plant (Avanti #441601), 0.4% Triton-X 100 (Sigma X-100), and 5 mM $CaCl_2$

were dissolved in 0.05M 4-(2-hydroxyethyl)-1-piperazineethanesulfonic acid (HEPES) buffer pH 7.

Assay procedure:

34 μl substrate was added to a cuvette, using a KoneLab automatic analyzer. At time T= 0 min, 4μl enzyme solution was added. Also a blank with water instead of enzyme was analyzed. The sample was mixed and incubated at 30°C for 10 minutes.
The free fatty acid content of sample was analyzed by using the NEFA C kit from WAKO GmbH.

Enzyme activity TIPU pH 7 was calculated as micromole fatty acid produced per minute under assay conditions.

Example 1: Expression of KLM3' in *Bacillus licheniformis*

A nucleotide sequence (SEQ ID No. 49) encoding a lipid acyltransferase (SEQ. ID No. 16, hereinafter KLM3') was expressed in *Bacillus licheniformis* as a fusion protein with the signal peptide of B. *licheniformis* α-amylase (LAT) (see FIGS. 53 and 54). For optimal expression in Bacillus, a codon optimized gene construct (no. 052907) was ordered at Geneart (Geneart AG, Regensburg, Germany).

Construct no. 052907 contains an incomplete LAT promoter (only the -10 sequence) in front of the LAT-KLM3' precursor gene and the LAT transcription (Tlat) downstream of the LAT-KLM3' precursor gene (see FIGS 53 and 55). To create a *Xho*I fragment that contains the LAT-KLM3' precursor gene flanked by the complete LAT promoter at the 5' end and the LAT terminator at the 3' end, a PCR (polymerase chain reaction) amplification was performed with the primers Plat5XhoI_FW and EBS2XhoI_RV and gene construct 052907 as template.

Plat5XhoI_FW:

ccccgctcgaggcttttcttttggaagaaaatatagggaaaatggtacttgttaaaaattc
ggaatatttatacaatatcatatgtttcacattgaaagggg

EBS2XhoI_RV: tggaatctcgaggttttatcctttaccttgtctcc

PCR was performed on a thermocycler with Phusion High Fidelity DNA polymerase (Finnzymes OY, Espoo, Finland) according to the instructions of the manufacturer (annealing temperature of 55°C).

The resulting PCR fragment was digested with restriction enzyme *Xho*I and ligated with T4 DNA ligase into *Xho*I digested pICatH according to the instructions of the supplier (Invitrogen, Carlsbad, California, USA).

The ligation mixture was transformed into *B. subtilis* strain SC6.1 as described in US 2002-0182734 (WO 02/14490). The sequence of the *Xho*I insert containing the LAT-KLM3' precursor gene was confirmed by DNA sequencing (BaseClear, Leiden, The Netherlands) and one of the correct plasmid clones was designated pICatH-KLM3'(ori1) (Figure 53). pICatH-KLM3'(ori1) was transformed into B. *licheniformis* strain BML780 (a derivative of BRA7 and BML612, see WO2005111203) at the permissive temperature (37°C).

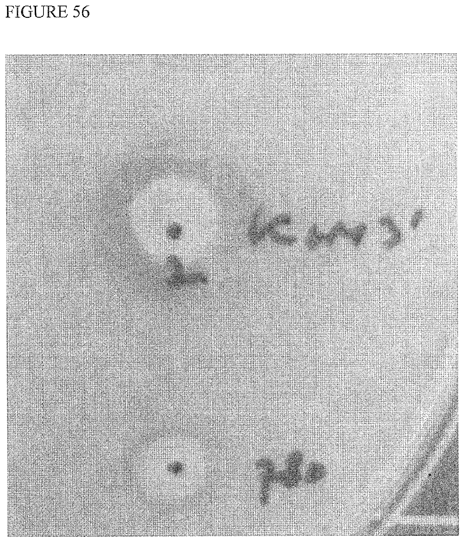

One neomycin resistant (neoR) and chloramphenicol resistant (CmR) transformant was selected and designated BML780(pICatH-KLM3'(ori1)). The plasmid in BML780(pICatH-KLM3'(ori1)) was integrated into the catH region on the B. *licheniformis* genome by growing the strain at a non-permissive temperature (50°C) in medium with 5 μg/ml chloramphenicol. One CmR resistant clone was selected and designated BML780-pICatH-KLM3'(ori1). BML780-pICatH-KLM3'(ori1) was grown again at the permissive temperature for several generations without antibiotics to loop-out vector sequences and then one neomycin sensitive (neoS), CmR clone was selected. In this clone, vector sequences of pICatH on the chromosome are excised (including the neomycin resistance gene) and only the catH - LATKLM3' cassette is left. Next, the catH - LATKLM3' cassette on the chromosome was amplified by growing the strain in/on media with increasing concentrations of chloramphenicol. After various rounds of amplification, one clone (resistant against 50 μg/ml chloramphenicol) was selected and designated BML780-KLM3'CAP50. To verify KLM3'expression, BML780-KLM3'CAP50 and BML780 (the empty host strain) were grown for 48h at 37°C on a Heart Infusion (Bacto) agar plate with 1% tributyrin. A clearing zone, indicative for lipid acyltransferase activity, was clearly visible around the colony of BML780-KLM3'CAP50 but not around the host strain BML780 (see Figure 56). This result shows that a substantial amount of KLM3' is expressed in B. *licheniformis* strain BML780-KLM3'CAP50 and that these KLM3' molecules are functional.

Comparative Example 1

Vector construct

The plasmid construct is pCS32new N80D, which is a pCCmini derivative carrying the sequence encoding the mature form of the native *Aeromonas salmonicida* glycerophospholipid-cholesterol acyltransferase with a Asn to Asp substitution at position 80 (KLM3'), under control of the p32 promoter and with a CGTase signal sequence.

The host strain used for the expression is in the *Bacillus subtilis* OS21ΔAprE strain.

The expression level is measured as transferase activity, expressed as % cholesterol esterified, calculated from the difference in free cholesterol in the reference sample and free cholesterol in the enzyme sample in reactions with PC ($T_{PC}$) as donor and cholesterol as acceptor molecule.

Culture conditions

5 ml of LB broth (casein enzymatic digest, 10 g/l; low-sodium yeast extract, 5 g/l; sodium chloride, 5 g/l; inert tableting aids, 2 g/l) supplemented with 50 mg/l kanamycin, was inoculated with a single colony and incubated at 30°C for 6 hours at 205 rpm. 0.7 ml of this culture was used to inoculate 50 ml of SAS media ($K_2HPO_4$, 10 g/l; MOPS (3-morpholinopropanesulfonic acid), 40 g/l; sodium Chloride, 5 g/l; Antifoam (Sin 260), 5 drops/l; Soy flour degreased, 20 g/l; Biospringer 106 (100 % dw YE), 20 g/l) supplemented with 50 mg/l kanamycin and a solution of high maltose starch hydrolysates (60 g/l). Incubation was continued for 40 hours at 30°C and 180 rpm before the culture supernatant was separated by centrifugation at 19000 rpm for 30 min. The supernatant was transferred into a clean tube and directly used for transferase activity measurement.

Preparation of substrates and enzymatic reaction

PC (Avanti Polar Lipids #441601) and cholesterol (Sigma C8503) was scaled in the ratio 9:1, dissolved in chloroform, and evaporated to dryness.

The substrate was prepared by dispersion of 3% PC:Cholesterol 9:1 in 50 mM HEPES buffer pH 7.

0.250 ml substrate solution was transferred into a 3 ml glass tube with screw lid. 0.025 ml culture supernatant was added and the mixture was incubated at 40°C for 2 hours. A reference sample with water instead of enzyme was also prepared. Heating the reaction mixture in a boiling water bath for 10 minutes stopped the enzyme reaction. 2 ml of 99% ethanol was added to the reaction mixture before submitted to cholesterol assay analysis.

Cholesterol assay

100 µl substrate containing 1.4 U/ml Cholesterol oxidase (SERVA Electrophoresis GmbH cat. No 17109), 0.4 mg/ml ABTS (Sigma A-1888), 6 U/ml Peroxidase (Sigma 6782) in 0.1 M Tris-HCl, pH 6.6 and 0.5 % Triton X-100 (Sigma X-100) was incubated at 37°C for 5 minutes before 5 µl enzyme reaction sample was added and mixed. The reaction mixture was incubated for further 5 minutes and $OD_{405}$ was measured. The content of cholesterol was calculated from the analyses of standard solutions of cholesterol containing 0.4 mg/ml, 0.3 mg/ml, 0.20 mg/ml, 0.1 mg/ml, 0.05 mg/ml, and 0 mg/ml cholesterol in 99 % ethanol.

Results

Table 3 below shows the average of 8 separate expression cultures:

Table 3

| **Strain** | **$T_{PC}$[a]** |
|---|---|
| **OS21ΔAprE[pCS3 2new]** | 74.2 ±10.1[b] |

[a] $T_{PC}$ is the transferase activity, expressed as % cholesterol esterified, calculated from the difference in free cholesterol in the reference sample and free cholesterol in the enzyme sample in reactions with PC as donor molecule and cholesterol as acceptor molecule.

[b] Average of 8 separate expression cultures

Example 2: Enzymation Test

In the trials described below, the moisture content, wetting time and cholesterol and cholesterol ester levels of milk powder formed by spray drying 25 litres of standard whole milk which had been treated with an enzyme for 30 minutes and 4 hours (as described below) were compared with milk powder formed by feeding 25 litres of standard whole milk directly to the spray drying tower (referred to below as the control sample).

Enzyme treatment of whole milk from ARLA

20 litres of whole milk was heated to 40°C and 76 μl of a solution solution of the enzyme of SEQ ID No. 16 (hereinafter KLM3'), (KTP08015, 1300 TIPU/g milk, corresponding to 12.4 mg enzyme/g milk) was added.
Mixing was continued for 38 minutes to ensure homogeneity, and the treated milk was then divided into 2 lots. Lot 1 was pumped to the spray tower immediately; lot 2 was pumped to the spray tower 4 hours after adding the enzyme.
The parameters used for operation of the pilot plant spray dryer during trials were as follows:

Control sample (ARLA whole milk)

Spray tower: NIRO DRYER model P 6.3; 400 $m^3$; air inlet at 220°C; power 54 kW.
Outlet temperatures: 105 / 40.5°C (air/product).
Feed temperature 40°C. Rannie feed pump 17 rpm.
Atomizing nozzle pressure 18 MPa (180 bar) (ata). Nozzle type KMFP SKYM M76.

Enzyme treated lot 1

400 $m^3$; air inlet at 195°C; power 48 kW.
Outlet temperatures: 100-103 / 43°C (air/product).
Feed temperature 40°C. Rannie feed pump 15rpm.
Atomizing nozzle pressure 16 MPa (160 bar) (ata). Nozzle type KMFP SKYM M76.

Enzyme treated lot 2

400 $m^3$; air inlet at 195°C; power 48 kW.
Outlet temperatures: 100-103 / 43°C (air/product).
Feed temperature 42°C. Rannie feed pump 16rpm.
Atomizing nozzle pressure: 17.5 MPa (175 bar) (ata). Nozzle type KMFP SKYM M76.

Example 3: Wettability Test

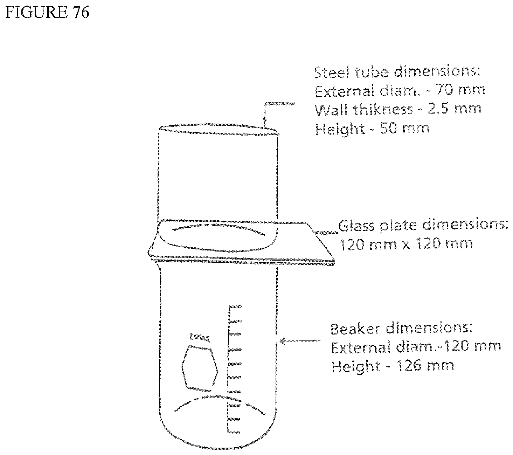

The milk powders derived from Example 2 were tested for wettability in accordance with IDF method 87:1979 with due consideration to the fact that method is intended for testing instantized milk powders, whereas the powders made from the pilot plant dryer is a non-instantized and non agglomerated powder. The apparatus used is illustrated in Figure 76.
The results are shown in Table 4 below.
Powder characteristics show that the powder made from enzymated milk is more free-flowing and has slightly lower tendency for lumping.

Table 4

| Sample | Wetting time |
|---|---|
| Control | > 10 minutes in all 3 repeat tests |
| Enzyme treated lot 1 | 1 st repeat test 403 s; |

(continued)

| Sample | Wetting time |
|---|---|
| | 2nd repeat test 394 s;<br>average wetting time 399 s. |
| Enzyme treated lot 2 | 1 st repeat test 320 s;<br>2nd repeat test 309 s;<br>average wetting time 315 s. |

EXAMPLE 4: Analysts of residual moisture content in powder samples after storage

The samples prepared according to Example 2 above were analysed for residual moisture content in powder samples after storage for one week at 5°C using a Moisture Analyser ML-50 from A&D Company, Limited. The moisture analyses were conducted after drying at 120°C until constant weight and at 140°C until constant weight. The results are shown in Table 5 below.

Table 5

| Sample | Drying temp. 120°C | Drying temp. 140°C | Average, % water |
|---|---|---|---|
| Control | 2.5 | 2.6 | 2.6 |
| Enzyme treated lot 1 | 2.0 | 2.1 | 2.1 |
| Enzyme treated lot 2 | 1.7 | 1.8 | 1.8 |

Example 5: Determination of cholesterol and cholesterol ester levels

The milk powder samples prepared in Example 2 above were analysed by GLC for the content of cholesterol and cholesterol ester. The method used is described below.

100 mg milk powder was scaled in a 15 ml centrifuge tube with lid. 5 ml choleroform:methanol 2:1 was added, and the sample was extracted for 30 minutes by rotation on a RotaMix® at 40 rpm. The sample was centrifuged. A scaled aliquot of the solvent was transferred to a 10 ml Dramglass and the solvent was evaporated under a steam of Nitrogen at 50°C. The isolated sample was analysed by GLC.

Gas Chromatography:

Perkin Elmer Autosystem 9000 Capillary Gas Chromatograph equipped with WCOT fused silica column 12.5 m x 0.25 mm ID x 0.1 μ film thickness 5% phenyl-methylsilicone (CP Sil 8 CB from Chrompack).
Carrier gas: Helium.
Injector. PSSI cold split injection (initial temp 50°C heated to 385°C), volume 1.0μl.
Detector FID: 395°C

| | | | |
|---|---|---|---|
| Oven program (used since 30.10.2003): | 1 | 2 | 3 |
| Oven temperature, °C. | 90 | 280 | 350 |
| Isothermal time, min. | 1 | 0 | 10 |
| Temperature rate, °C/min. | 15 | 4 | |

Preparation of milk samples for GC analysis:

The lipid fraction is redissolved in heptane/pyridine (2:1) containing heptadecane as internal standard and cholesterol is measured by GC.

500μl sample solution is then transferred to a crimp vial, 100 μl MSTFA:TMCS - 99:1 (N-Methyl-N-trimethylsilyl-trifluoroacetamide) is added and reacted for 20 minutes at 60°C.

Calculation: Response factors for cholesterol and cholesterol esters are determined from pure reference material (weighing for pure material 8-10mg in 12 ml pyridine, containing internal standard heptadecane, 0.5 mg/ml).

The results are shown in Table 6 below.

Table 6

| Sample | Cholesterol, % | Cholesterol ester, % | Esterified cholesterol, % |
|---|---|---|---|
| Control | 0.084 | 0 | 0 |
| Enzyme treated lot 1 | 0.021 | 0.080 | 69.2 |
| Enzyme treated lot 2 | 0.007 | 0.094 | 89.0 |

Conclusions

Enzyme treatment of whole milk with lipid acyltransferase KLM3 has a strong impact on the wettability of the milk powder produced from the milk. The enzyme treatment also has an impact on the drying temperature, as it is shown that the enzyme treated samples has a lower water content than control.

Free cholesterol in milk produced from milk treated with acyltransferase was significantly reduced compared to a control without enzyme treatment.

# DEPOSIT RECEIPTS

BUDAPEST TREATY ON THE INTERNATIONAL RECOGNITION OF THE DEPOSIT OF MICROORGANISMS FOR THE PURPOSES OF PATENT PROCEDURE

| | |
|---|---|
| Danisco A/S<br>Langebrogade 1<br>DK-1001 Copenhagen<br>Denmark | INTERNATIONAL FORM<br><br>RECEIPT IN THE CASE OF AN ORIGINAL DEPOSIT issued pursuant to Rule 7.1 by the INTERNATIONAL DEPOSITARY AUTHORITY identified at the bottom of this page |

NAME AND ADDRESS OF DEPOSITOR

**I. IDENTIFICATION OF THE MICROORGANISM**

| | |
|---|---|
| Identification reference given by the DEPOSITOR: | Accession number given by the INTERNATIONAL DEPOSITARY AUTHORITY: |
| *Escherichia coli* TOP10pPet12aAhydro | NCIMB 41204 |

**II. SCIENTIFIC DESCRIPTION AND/OR PROPOSED TAXONOMIC DESIGNATION**

The microorganism identified under I above was accompanied by:

[ ] a scientific description

[X] a proposed taxonomic designation

(Mark with a cross where applicable)

**III. RECEIPT AND ACCEPTANCE**

This International Depositary Authority accepts the microorganism identified under I above, which was received by it on 22 December 2003 (date of the original deposit)[1]

**IV. RECEIPT OF REQUEST FOR CONVERSION**

The microorganism identified under I above was received by this International Depositary Authority on (date of the original deposit) and a request to convert the original deposit to a deposit under the Budapest Treaty was received by it on (date of receipt of request for conversion)

**V. INTERNATIONAL DEPOSITARY AUTHORITY**

| | |
|---|---|
| Name: NCIMB Ltd.,<br><br>Address: 23 St Machar Drive<br>Aberdeen<br>AB24 3RY<br>Scotland, UK. | Signature(s) of person(s) having the power to represent the International Depositary Authority or of authorised official(s):<br><br>Date: 9 January 2004 |

[1] Where Rule 6/4(d) applies, such date is the date on which the status of International Depositary Authority was acquired.

Form BP/4 (sole page)

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

Danisco A/S
Langebrogade 1
DK-1001 Copenhagen
Denmark

NAME AND ADDRESS OF THE PARTY TO WHOM THE VIABILITY STATEMENT IS ISSUED

VIABILITY STATEMENT
issued pursuant to Rule 10.2 by the
INTERNATIONAL DEPOSITARY AUTHORITY
identified on the following page

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY: NCIMB 41204<br>Date of the deposit or of the transfer[1]:<br>22 December 2003 |

III. VIABILITY STATEMENT

The viability of the microorganism identified under II above was tested on 22 December 2003 [2]. On that date, the said microorganism was:

[X] [3] viable

[ ] [3] no longer viable

[1] Indicate the date of the original deposit or, where a new deposit or a transfer has been made, the most recent relevant date (date of the new deposit or date of the transfer).

[2] In the cases referred to in Rule 10.2(a)(ii) and (iii), refer to the most recent viability test.

[3] Mark with a cross the applicable box.

Form BP/9 (first page)

| IV. CONDITIONS UNDER WHICH THE VIABILITY TEST HAS BEEN PERFORMED[4] |
|---|
| |

| V. INTERNATIONAL DEPOSITARY AUTHORITY | |
|---|---|
| Name: NCIMB Ltd.,<br>Address: 23 St Machar Drive<br>Aberdeen<br>AB24 3RY<br>Scotland | Signature(s) of person(s) having the power to represent the International Depositary Authority or of authorised official(s):<br>Date: 9 January 2004 |

4 Fill in if the information has been requested and if the results of the test were negative.

Form BP/9 (second and last page)

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

Danisco A/S
Langebrogade 1
DK-1001 Copenhagen
Denmark

NAME AND ADDRESS OF DEPOSITOR

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

| Identification reference given by the DEPOSITOR: | Accession number given by the INTERNATIONAL DEPOSITARY AUTHORITY: |
|---|---|
| *Escherichia coli* TOP10pPet12aAsalmo | NCIMB 41205 |

II. SCIENTIFIC DESCRIPTION AND/OR PROPOSED TAXONOMIC DESIGNATION

The microorganism identified under I above was accompanied by:

[ ] a scientific description

[X] a proposed taxonomic designation

(Mark with a cross where applicable)

III. RECEIPT AND ACCEPTANCE

This International Depositary Authority accepts the microorganism identified under I above, which was received by it on 22 December 2003 (date of the original deposit)[1]

IV. RECEIPT OF REQUEST FOR CONVERSION

The microorganism identified under I above was received by this International Depositary Authority on (date of the original deposit) and a request to convert the original deposit to a deposit under the Budapest Treaty was received by it on

(date of receipt of request for conversion)

V. INTERNATIONAL DEPOSITARY AUTHORITY

Name: NCIMB Ltd.,

Address: 23 St Machar Drive
Aberdeen
AB24 3RY
Scotland, UK.

Signature(s) of person(s) having the power to represent the International Depositary Authority or of authorised official(s):

Date: 9 January 2004

[1] Where Rule 6/4(d) applies, such date is the date on which the status of International Depositary Authority was acquired.

Form BP/4 (sole page)

BUDAPEST TREATY ON THE INTERNATIONAL RECOGNITION OF THE DEPOSIT OF MICROORGANISMS FOR THE PURPOSES OF PATENT PROCEDURE

Danisco A/S
Langebrogade 1
DK-1001 Copenhagen
Denmark

INTERNATIONAL FORM

VIABILITY STATEMENT
issued pursuant to Rule 10.2 by the
INTERNATIONAL DEPOSITARY AUTHORITY
identified on the following page

NAME AND ADDRESS OF THE PARTY TO WHOM THE VIABILITY STATEMENT IS ISSUED

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY: NCIMB 41205<br>Date of the deposit or of the transfer[1]:<br>22 December 2003 |

III. VIABILITY STATEMENT

The viability of the microorganism identified under II above was tested on 22 December 2003 [2]. On that date, the said microorganism was:

[X][3] viable

[ ][3] no longer viable

[1] Indicate the date of the original deposit or, where a new deposit or a transfer has been made, the most recent relevant date (date of the new deposit or date of the transfer).

[2] In the cases referred to in Rule 10.2(a)(ii) and (iii), refer to the most recent viability test.

[3] Mark with a cross the applicable box.

Form BP/9 (first page)

| | |
|---|---|
| IV. CONDITIONS UNDER WHICH THE VIABILITY TEST HAS BEEN PERFORMED[4] | |
| | |
| V. INTERNATIONAL DEPOSITARY AUTHORITY | |
| Name: NCIMB Ltd.,<br>Address: 23 St Machar Drive<br>Aberdeen<br>AB24 3RY<br>Scotland | Signature(s) of person(s) having the power to represent the International Depositary Authority or of authorised official(s):<br>Date: 9 January 2004 |

[4] Fill in if the information has been requested and if the results of the test were negative.

Form BP/9 (second and last page)

BUDAPEST TREATY ON THE INTERNATIONAL RECOGNITION OF THE DEPOSIT OF MICROORGANISMS FOR THE PURPOSES OF PATENT PROCEDURE

Danisco Intellectual Assets
Danisco A/S
Langebrogade 1
DK-1001 Copenhagen
Denmark

NAME AND ADDRESS OF DEPOSITOR

INTERNATIONAL FORM

RECEIPT IN THE CASE OF AN ORIGINAL DEPOSIT issued pursuant to Rule 7.1 by the INTERNATIONAL DEPOSITARY AUTHORITY identified at the bottom of this page

I. IDENTIFICATION OF THE MICROORGANISM

| Identification reference given by the DEPOSITOR: | Accession number given by the INTERNATIONAL DEPOSITARY AUTHORITY: |
|---|---|
| *Streptomyces* sp. L130 | NCIMB 41226 |

II. SCIENTIFIC DESCRIPTION AND/OR PROPOSED TAXONOMIC DESIGNATION

The microorganism identified under I above was accompanied by:

[ ] a scientific description

[X] a proposed taxonomic designation

(Mark with a cross where applicable)

III. RECEIPT AND ACCEPTANCE

This International Depositary Authority accepts the microorganism identified under I above, which was received by it on 23 June 2004 (date of the original deposit)[1]

IV. RECEIPT OF REQUEST FOR CONVERSION

The microorganism identified under I above was received by this International Depositary Authority on (date of the original deposit) and a request to convert the original deposit to a deposit under the Budapest Treaty was received by it on (date of receipt of request for conversion)

V. INTERNATIONAL DEPOSITARY AUTHORITY

| | |
|---|---|
| Name: NCIMB Ltd., | Signature(s) of person(s) having the power to represent the International Depositary Authority or of authorised official(s): |
| Address: 23 St Machar Drive<br>Aberdeen<br>AB24 3RY<br>Scotland, UK. | Date: 28 June 2004 |

[1] Where Rule 6/4(d) applies, such date is the date on which the status of International Depositary Authority was acquired.

Form BP/4 (sole page)

BUDAPEST TREATY ON THE INTERNATIONAL RECOGNITION OF THE DEPOSIT OF MICROORGANISMS FOR THE PURPOSES OF PATENT PROCEDURE

Danisco Intellectual Assets
Danisco A/S
Langebrogade 1
DK-1001 Copenhagen
Denmark

NAME AND ADDRESS OF THE PARTY TO WHOM THE VIABILITY STATEMENT IS ISSUED

INTERNATIONAL FORM

VIABILITY STATEMENT
issued pursuant to Rule 10.2 by the INTERNATIONAL DEPOSITARY AUTHORITY identified on the following page

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY:<br>NCIMB 41226<br>Date of the deposit or of the transfer[1]:<br>23 June 2004 |

III. VIABILITY STATEMENT

The viability of the microorganism identified under II above was tested on 25 June 2004 [2]. On that date, the said microorganism was:

[3] [X] viable

[ ] no longer viable

[1] Indicate the date of the original deposit or, where a new deposit or a transfer has been made, the most recent relevant date (date of the new deposit or date of the transfer).

[2] In the cases referred to in Rule 10.2(a)(ii) and (iii), refer to the most recent viability test.

[3] Mark with a cross the applicable box.

Form BP/9 (first page)

| IV. CONDITIONS UNDER WHICH THE VIABILITY TEST HAS BEEN PERFORMED[4] | |
|---|---|
| | |
| **V. INTERNATIONAL DEPOSITARY AUTHORITY** | |
| Name: NCIMB Ltd.,<br>Address: 23 St Machar Drive<br>Aberdeen<br>AB24 3RY<br>Scotland | Signature(s) of person(s) having the power to represent the International Depositary Authority or of authorised official(s):<br>[signature]<br>Date: 28 June 2004 |

[4] Fill in if the information has been requested and if the results of the test were negative.

Form BP/9 (second and last page)

BUDAPEST TREATY ON THE INTERNATIONAL RECOGNITION OF THE DEPOSIT OF MICROORGANISMS FOR THE PURPOSES OF PATENT PROCEDURE

Danisco Intellectual Assets
Danisco A/S
Langebrogade 1
DK-1001 Copenhagen
Denmark

NAME AND ADDRESS OF DEPOSITOR

INTERNATIONAL FORM

RECEIPT IN THE CASE OF AN ORIGINAL DEPOSIT issued pursuant to Rule 7.1 by the INTERNATIONAL DEPOSITARY AUTHORITY identified at the bottom of this page

I. IDENTIFICATION OF THE MICROORGANISM

| Identification reference given by the DEPOSITOR: | Accession number given by the INTERNATIONAL DEPOSITARY AUTHORITY: |
|---|---|
| *Streptomyces* sp. L131 | NCIMB 41227 |

II. SCIENTIFIC DESCRIPTION AND/OR PROPOSED TAXONOMIC DESIGNATION

The microorganism identified under I above was accompanied by:

[ ] a scientific description

[X] a proposed taxonomic designation

(Mark with a cross where applicable)

III. RECEIPT AND ACCEPTANCE

This International Depositary Authority accepts the microorganism identified under I above, which was received by it on 23 June 2004 (date of the original deposit)[1]

IV. RECEIPT OF REQUEST FOR CONVERSION

The microorganism identified under I above was received by this International Depositary Authority on (date of the original deposit) and a request to convert the original deposit to a deposit under the Budapest Treaty was received by it on (date of receipt of request for conversion)

V. INTERNATIONAL DEPOSITARY AUTHORITY

Name: NCIMB Ltd.,

Address: 23 St Machar Drive
Aberdeen
AB24 3RY
Scotland, UK.

Signature(s) of person(s) having the power to represent the International Depositary Authority or of authorised official(s):

Terence Dando

Date: 28 June 2004

[1] Where Rule 6/4(d) applies, such date is the date on which the status of International Depositary Authority was acquired.

Form BP/4 (sole page)

BUDAPEST TREATY ON THE INTERNATIONAL RECOGNITION OF THE DEPOSIT OF MICROORGANISMS FOR THE PURPOSES OF PATENT PROCEDURE

| Danisco Intellectual Assets<br>Danisco A/S<br>Langebrogade 1<br>DK-1001 Copenhagen<br>Denmark |
|---|

NAME AND ADDRESS OF THE PARTY TO WHOM THE VIABILITY STATEMENT IS ISSUED

INTERNATIONAL FORM

VIABILITY STATEMENT
issued pursuant to Rule 10.2 by the INTERNATIONAL DEPOSITARY AUTHORITY identified on the following page

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY: NCIMB 41227<br>Date of the deposit or of the transfer[1]:<br>23 June 2003 |
| III. VIABILITY STATEMENT | |
| The viability of the microorganism identified under II above was tested on 25 June 2004 [2]. On that date, the said microorganism was:<br>[3] X viable<br>[3] ☐ no longer viable | |

1 Indicate the date of the original deposit or, where a new deposit or a transfer has been made, the most recent relevant date (date of the new deposit or date of the transfer).

2 In the cases referred to in Rule 10.2(a)(ii) and (iii), refer to the most recent viability test.

3 Mark with a cross the applicable box.

Form BP/9 (first page)

| | |
|---|---|
| IV. CONDITIONS UNDER WHICH THE VIABILITY TEST HAS BEEN PERFORMED[4] | |
| | |
| V. INTERNATIONAL DEPOSITARY AUTHORITY | |
| Name: NCIMB Ltd., <br> Address: 23 St Machar Drive <br> Aberdeen <br> AB24 3RY <br> Scotland | Signature(s) of person(s) having the power to represent the International Depositary Authority or of authorised official(s): <br> Date: 28 June 2004 |

Fill in if the information has been requested and if the results of the test were negative.

Form BP/9 (second and last page)

## Claims

**1.** A method of producing powder milk, said method comprising:

(a) contacting milk or a fraction thereof with a lipid acyltransferase enzyme; and
(b) drying the enzyme treated milk to produce powder milk.

**2.** A method according to claim 1, wherein the lipid acyltransferase is selected from lipid acyltransferases in enzyme class (E.C.) 2.3.1.x or (E.C.) 2.3.1.43.

**3.** A method according to claim 1 or 2 wherein the lipid acyl transferase is one which when tested using the Transferase Assay defined herein has at least 10% acyltransferase activity.

**4.** A method according to any one of claims 1 to 3, wherein the lipid acyltransferase is a lipid acyltransferase that is capable of esterifying at least about 10% of the cholesterol present in the starting milk.

**5.** A method according to any one of the preceding claims wherein the lipid acyltransferase enzyme is characterised as an enzyme which possesses acyltransferase activity and which comprises the amino acid sequence motif GDSX, wherein X is one or more of the following amino acid residues L, A, V, I, F, Y, H, Q, T, N, M or S.

**6.** A method according to any one of the preceding claims wherein said lipid acyltransferase is a polypeptide having lipid acyltransferase activity which polypeptide is obtained by expression of any one of the nucleotide sequences shown as SEQ ID No. 36, SEQ ID No. 38, SEQ ID No. 39, SEQ ID No. 42, SEQ ID No. 44, SEQ ID No. 46, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62 or SEQ ID No. 63 or a nucleotide sequence which as has 75% or more identity therewith.

**7.** A method according to any one of the preceding claims wherein said lipid acyltransferase is a polypeptide having lipid acyltransferase activity which polypeptide is obtained by expression of:

(i) the nucleotide sequence shown as SEQ ID No. 49 or a nucleotide sequence which as has 75% or more identity therewith;
(ii) a nucleic acid which encodes said polypeptide wherein said polypeptide is at least 70% identical with the polypeptide sequence shown in SEQ ID No. 16 or with the polypeptide sequence shown in SEQ ID No. 68; or
(iii) a nucleic acid which hybridises under medium stringency conditions to a nucleic probe comprising the nucleotide sequence shown as SEQ ID No. 49.

**8.** A method according to any one of the preceding claims wherein said lipid acyltransferase is a polypeptide having lipid acyltransferase activity which polypeptide comprises any one of the amino acid sequences shown as SEQ ID No. 1, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 34, SEQ ID No. 35, SEQ ID No. 68 or an amino acid sequence which as has 75% or more identity therewith.

**9.** A method according to any one of the preceding claims wherein said lipid acyltransferase is a polypeptide having lipid acyltransferase activity which polypeptide comprises the amino acid sequence shown as SEQ ID No. 68 or an amino acid sequence which has 75% or more identity therewith.

**10.** A method according to any one of the preceding claims wherein the enzyme treated milk is dried by spray drying to produce powder milk.

**11.** A method according to claim 10, wherein the enzyme treated milk is fed into the spray dryer at a temperature ranging from about 20°C to about 95°C.

**12.** A method according to claim 10 or 11, wherein the outlet air temperature of the spray dryer ranges from about 50°C to about 150°C.

**13.** A method according to any one of claims 10 to 12, wherein the product outlet temperature of the spray dryer ranges from about 20°C to about 60°C.

**14.** A method according to any one of the preceding claims wherein the enzyme treated milk is dried by drum drying to produce powder milk.

**15.** A method according to claim 14, wherein the temperature of the drum ranges from about 90°C to about 150°C.

**16.** Powder milk obtainable or obtained by the process of any one of claims 1-15.

**17.** A milk product produced by rehydrating the powder milk of claim 16.

**18.** Use of a lipid acyltransferase in the manufacture of powder milk for:

a) improving the rehydration properties of the powder milk or
b) reducing the cholesterol content of the powder milk or
c) reducing the free fatty acid content of the powder milk compared with powder milk which during its manufacture has been treated with a phospholipase or
d) improving the flowability of the powder milk or
e) reducing fouling of the equipment used in the manufacture of the powder milk;

wherein milk or a fraction thereof is contacted with the lipid acyltransferase enzyme and the enzyme treated milk is dried.

**19.** Use according to claim 18, wherein the improved rehydration properties comprise a reduction in the wettability of the powder milk.

**20.** Use according to claim 19, wherein the lipid acyltransferase is a lipid acyltransferase as defined in any one of claims 2 to 9.

**Patentansprüche**

**1.** Verfahren zur Herstellung von Milchpulver, wobei das Verfahren Folgendes umfasst:

(a) das Kontaktieren von Milch oder eines Teils davon mit einem Lipidacyltransferaseenzym; und
(b) das Trocknen der mit Enzym behandelten Milch, um Milchpulver herzustellen.

**2.** Verfahren nach Anspruch 1, wobei die Lipidacyltransferase unter Lipidacyltransferasen in der Enzymklasse (E.C.) 2.3.1.x oder (E.C.) 2.3.1.43 ausgewählt wird.

**3.** Verfahren nach Anspruch 1 oder 2, wobei die Lipidacyltransferase eine ist, die beim Testen unter Anwendung des hier definierten Transferase-Assays eine Acyltransferaseaktivität von mindestens 10 % aufweist.

**4.** Verfahren nach einem der Ansprüche 1 bis 3, wobei die Lipidacyltransferase eine Lipidacyltransferase ist, die in der Lage ist, mindestens etwa 10 % des in der Ausgangsmilch vorliegenden Cholesterins zu verestern.

**5.** Verfahren nach einem der vorhergehenden Ansprüche, wobei das Lipidacyltransferaseenzym als Enzym gekennzeichnet ist, das Acyltransferaseaktivität aufweist und das das Aminosäuresequenz-Motiv GDSX umfasst, wobei X eines oder mehrere der folgenden Aminosäurereste L, A, V, I, F, Y, H, Q, T, N, M oder S ist.

**6.** Verfahren nach einem der vorhergehenden Ansprüche, wobei das Lipidacyltransferaseenzym ein Polypeptid ist, das Lipidacyltransferaseaktivität aufweist, welches Polypeptid durch die Expression irgendeiner der Nucleotidsequenzen, die als SEQ ID NO: 36, SEQ ID NO: 38, SEQ ID NO: 39, SEQ ID NO: 42, SEQ ID NO: 44, SEQ ID NO: 46, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62 oder SEQ ID NO: 63 gezeigt sind, oder einer Nucleotidsequenz, die 75 % oder mehr Identität damit aufweist, erhalten wird.

**7.** Verfahren nach einem der vorhergehenden Ansprüche, wobei die Lipidacyltransferase ein Polypeptid ist, das Lipidacyltransferaseaktivität aufweist, welches Polypeptid erhalten wird durch die Expression von:

(i) der Nucleotidsequenz, die als SEQ ID NO: 49 gezeigt ist oder einer Nucleotidsequenz, die 75 % oder mehr Identität damit aufweist;

(ii) einer Nucleinsäure, die für das Polypeptid codiert, wobei das Polypeptid mindestens 70 % mit der Polypeptidsequenz, die in SEQ ID NO: 16 gezeigt ist, oder mit der Polypeptidsequenz, die in SEQ ID NO: 68 gezeigt ist, identisch ist; oder
(iii) einer Nucleinsäure, die unter mittleren Stringenzbedingungen an eine Nucleinsonde hybridisiert, die die Nucleotidsequenz umfasst, die als SEQ ID NO: 49 gezeigt ist.

**8.** Verfahren nach einem der vorhergehenden Ansprüche, wobei die Lipidacyltransferase ein Polypeptid ist, das Acyltransferaseaktivität aufweist, welches Polypeptid irgendeine der Aminosäuresequenzen umfasst, die als SEQ ID NO: 1, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO:10, SEQ ID NO:11, SEQ ID NO:12, SEQ ID NO:13, SEQ ID NO:14, SEQ ID NO: 15, SEQ ID NO: 16, SEQ ID NO: 17, SEQ ID NO: 18, SEQ ID NO: 34, SEQ ID NO: 35, SEQ ID NO: 68 gezeigt sind, oder eine Aminosäuresequenz, die 75 % oder mehr Identität damit aufweist.

**9.** Verfahren nach einem der vorhergehenden Ansprüche, wobei die Lipidacyltransferase ein Polypeptid ist, das Lipidacyltransferaseaktivität aufweist, welches Polypeptid die Aminosäuresequenz, die als SEQ ID NO: 68 gezeigt ist, oder eine Aminosäuresequenz, die 75 % oder mehr Identität damit aufweist, umfasst.

**10.** Verfahren nach einem der vorhergehenden Ansprüche, wobei die mit Enzym behandelte Milch durch Sprühtrocknen getrocknet wird, um Milchpulver herzustellen.

**11.** Verfahren nach Anspruch 10, wobei die mit Enzym behandelte Milch in die Sprühtrocknungsvorrichtung bei einer Temperatur im Bereich von etwa 20 °C bis etwa 95 °C eingeführt wird.

**12.** Verfahren nach Anspruch 10 oder 11, wobei die Auslasslufttemperatur der Sprühtrocknungsvorrichtung im Bereich von etwa 50 °C bis etwa 150 °C liegt.

**13.** Verfahren nach einem der Ansprüche 10 bis 12, wobei die Produktauslasstemperatur der Sprühtrocknungsvorrichtung im Bereich von etwa 20 °C bis etwa 60 °C liegt.

**14.** Verfahren nach einem der vorhergehenden Ansprüche, wobei die mit Enzym behandelte Milch durch Trommeltrocknen getrocknet wird, um Milchpulver herzustellen.

**15.** Verfahren nach Anspruch 14, wobei die Temperatur der Trommel im Bereich von etwa 90 °C bis etwa 150 °C liegt.

**16.** Milchpulver, das durch das Verfahren nach einem der Ansprüche 1 bis 15 erhältlich oder ist oder erhalten wird.

**17.** Milchprodukt, das durch Rehydratisierung des Milchpulvers nach Anspruch 16 hergestellt wird.

**18.** Verwendung einer Lipidacyltransferase bei der Herstellung von Milchpulver, zum:

a) Verbessern der Rehydratisierungseigenschaften des Milchpulvers oder
b) Reduzieren des Cholesteringehalts des Milchpulvers oder
c) Reduzieren des Gehalts an freier Fettsäure des Milchpulvers im Vergleich mit Milchpulver, das während seiner Herstellung mit einer Phospholipase behandelt worden ist, oder
d) Verbessern der Fließfähigkeit des Milchpulvers oder
e) Reduzieren der Verschmutzung der Vorrichtung, die zur Herstellung des Milchpulvers verwendet wird;

wobei Milch oder ein Teil davon mit dem Lipidacyltransferaseenzym in Kontakt gebracht wird und die mit Enzym behandelte Milch getrocknet wird.

**19.** Verwendung nach Anspruch 18, wobei die verbesserten Rehydratisierungseigenschaften eine Reduktion der Benetzbarkeit des Milchpulvers umfassen.

**20.** Verwendung nach Anspruch 19, wobei die Lipidacyltransferase eine Lipidacyltransferase wie in einem der Ansprüche 2 bis 9 definiert, ist.

## Revendications

1. Procédé de production de lait en poudre, ledit procédé comprenant:

   (a) la mise en contact de lait ou d'une fraction de celui-ci avec une enzyme lipide acyltransférase; et
   (b) le séchage du lait traité à l'enzyme pour produire du lait en poudre.

2. Procédé selon la revendication 1, la lipide acyltransférase étant sélectionnée parmi les lipides acyltransférases de la classe d'enzyme (E.C.) 2.3.1.x ou (E.C.) 2.3.1.43.

3. Procédé selon la revendication 1 ou 2 dans lequel la lipide acyltransférase est l'une qui lorsque testée en utilisant l'essai transférase défini ci-inclus présente au moins 10 % d'activité acyltransférase.

4. Procédé selon l'une quelconque des revendications 1 à 3, la lipide acyltransférase étant une lipide acyltransférase capable d'estérification d'au moins environ 10 % du cholestérol présent dans le lait de départ.

5. Procédé selon l'une quelconque des revendications précédentes dans lequel l'enzyme lipide acyltransférase est **caractérisée** comme étant une enzyme qui possède une activité acyltransférase et qui comprend le motif de séquence d'acides aminés GDSX, où X est l'un ou plusieurs des résidus d'acides aminés suivants L, A, V, I, F, Y, H, Q, T, N, MouS.

6. Procédé selon l'une quelconque des revendications précédentes dans lequel ladite lipide acyltransférase est un polypeptide présentant une activité lipide acyltransférase lequel polypeptide est obtenu par l'expression de n'importe laquelle des séquences nucléotidiques présentées comme SEQ ID n°: 36, SEQ ID n°: 38, SEQ ID n°: 39, SEQ ID n° : 42, SEQ ID n°:44, SEQ ID n°: 46, SEQ ID n°: 48, SEQ ID n°:49, SEQ ID n°: 50, SEQ ID n° : 51, SEQ ID n° : 52, SEQ ID n°: 53, SEQ ID n°: 54, SEQ ID n°: 55, SEQ ID n°: 56, SEQ ID n°: 57, SEQ ID n°: 58, SEQ ID n°: 59, SEQ ID n°: 60, SEQ ID n°: 61, SEQ ID n°: 62 ou SEQ ID n°: 63 ou une séquence nucléotidique qui présente 75 % d'identité ou plus avec elle.

7. Procédé selon l'une quelconque des revendications précédentes dans lequel ladite lipide acyltransférase est un polypeptide ayant une activité lipide acyltransférase lequel polypeptide est obtenu par l'expression de:

   (i) la séquence nucléotidique présentée comme SEQ ID n°: 49 ou une séquence nucléotidique qui possède 75 % d'identité ou plus avec elle;
   (ii) un acide nucléique qui code pour ledit polypeptide où ledit polypeptide est identique au moins à 70 % avec la séquence polypeptidique présentée dans SEQ ID n°: 16 ou avec la séquence polypeptidique présentée dans SEQ ID n°: 68; ou
   (iii) un acide nucléique qui s'hybride sous des conditions de rigueur modérées à une sonde nucléique comprenant la séquence nucléotidique présentée comme SEQ ID n°: 49.

8. Procédé selon l'une quelconque des revendications précédentes dans lequel ladite lipide acyltransférase est un polypeptide ayant une activité lipide acyltransférase lequel polypeptide comprend n'importe laquelle des séquences d'acides aminés présentées comme SEQ ID n°: 1, SEQ ID n°: 3, SEQ ID n°: 4, SEQ ID n°: 5, SEQ ID n°: 6, SEQ ID n°: 7, SEQ ID n°: 8, SEQ ID n°: 9, SEQ ID n°: 10, SEQ ID n°: 11, SEQ ID n°: 12, SEQ ID n°: 13, SEQ ID n°: 14, SEQ ID n°: 15, SEQ ID n°: 16, SEQ ID n°: 17, SEQ ID n°: 18, SEQ ID n°: 34, SEQ ID n°: 35, SEQ ID n°: 68 ou une séquence d'acides aminés qui présente 75 % d'identité ou plus avec elle.

9. Procédé selon l'une quelconque des revendications précédentes dans lequel ladite lipide acyltransférase est un polypeptide ayant une activité lipide acyltransférase lequel polypeptide comprend la séquence d'acides aminés présentée comme SEQ ID n°: 68 ou une séquence d'acides aminés qui présente 75 % d'identité ou plus avec elle.

10. Procédé selon l'une quelconque des revendications précédentes dans lequel le lait traité avec une enzyme est séché par lyophilisation pour produire du lait en poudre.

11. Procédé selon la revendication 10, dans lequel le lait traité avec une enzyme est alimenté dans le lyophiliseur à une température s'étendant d'environ 20°C à environ 95°C.

12. Procédé selon les revendications 10 ou 11, dans lequel la température de l'air de l'orifice de sortie du lyophiliseur

s'étend d'environ 50°C à environ 150°C.

**13.** Procédé selon l'une quelconque des revendications 10 à 12, dans lequel la température de l'orifice de sortie de produit du lyophiliseur s'étend d'environ 20°C à environ 60°C.

**14.** Procédé selon l'une quelconque des revendications précédentes dans lequel le lait traité avec une enzyme est séché avec un sécheur à tambour pour produire du lait en poudre.

**15.** Procédé selon la revendication 14, dans lequel la température du tambour s'étend d'environ 90°C à environ 150°C.

**16.** Lait en poudre pouvant être obtenu ou étant obtenu grâce au procédé selon l'une quelconque des revendications 1 à 15.

**17.** Produit laitier produit par réhydratation du lait en poudre selon la revendication 16.

**18.** Utilisation d'une lipide acyltransférase dans la fabrication de lait en poudre pour:

a) améliorer les propriétés de réhydratation du lait en poudre ou
b) réduire la teneur en cholestérol du lait en poudre ou
c) réduire la teneur en acide gras libre du lait en poudre comparé au lait en poudre qui durant sa fabrication a été traité avec une phospholipase ou
d) améliorer l'aptitude à l'écoulement du lait en poudre ou
e) réduire l'encrassage de l'équipement utilisé dans la fabrication du lait en poudre;

où le lait ou une fraction de celui-ci est mis(e) en contact avec l'enzyme lipide acyltransférase et le lait traité avec une enzyme est séché.

**19.** Utilisation selon la revendication 18, les propriétés améliorées de réhydratation comprenant une réduction de l'aptitude à l'humidification du lait en poudre.

**20.** Utilisation selon la revendication 19, la lipide acyltransférase étant une lipide acyltransférase telle que définie selon l'une quelconque des revendications 2 à 9.

FIGURE 1

SEQ ID No. 16

```
  1  ADTRPAFSRI VMFGDSLSDT GKMYSKMRGY LPSSPPYYEG RFSNGPVWLE QLTKQFPGLT
 61  IANEAEGGAT AVAYNKISWD PKYQVINNLD YEVTQFLQKD SFKPDDLVIL WVGANDYLAY
121  GWNTEQDAKR VRDAISDAAN RMVLNGAKQI LLFNLPDLGQ NPSARSQKVV EAVSHVSAYH
181  NKLLLNLARQ LAPTGMVKLF EIDKQFAEML RDPQNFGLSD VENPCYDGGY VWKPFATRSV
241  STDRQLSAFS PQERLAIAGN PLLAQAVASP MARRSASPLN CEGKMFWDQV HPTTVVHAAL
301  SERAATFIET QYEFLAHG
```

FIGURE 2

(SEQ ID No. 1)

```
  1 MKKWFVCLLG LVALTVQAAD SRPAFSRIVM FGDSLSDTGK MYSKMRGYLP
 51 SSPPYYEGRF SNGPVWLEQL TKQFPGLTIA NEAEGGATAV AYNKISWNPK
101 YQVINNLDYE VTQFLQKDSF KPDDLVILWV GANDYLAYGW NTEQDAKRVR
151 DAISDAANRM VLNGAKQILL FNLPDLGQNP SARSQKVVEA VSHVSAYHNQ
201 LLLNLARQLA PTGMVKLFEI DKQFAEMLRD PQNFGLSDVE NPCYDGGYVW
251 KPFATRSVST DRQLSAFSPQ ERLAIAGNPL LAQAVASPMA RRSASPLNCE
301 GKMFWDQVHP TTVVHAALSE RAATFIANQY EFLAH*
```

FIGURE 3

(SEQ ID No. 2)

```
  1 ivafGDSlTd geayygdsdg ggwgagladr Ltallrlrar prgvdvfnrg isGrtsdGrl
 61 ivDalvallF laqslglpnL pPYLsgdflr GANFAsagAt Ilptsgpfli QvqFkdfksq
121 vlelrqalgl lqellrllpv ldakspdlvt imiGtNDlit saffgpkste sdrnvsvpef
181 kdnlrqlikr Lrsnngarii vlitlvilnl gplGClPlkl alalassknv dasgclerln
241 eavadfneal relaiskled qlrkdglpdv kgadvpyvDl ysifqdldgi qnpsayvyGF
301 ettkaCCGyG gryNynrvCG naglcnvtak aCnpssylls flfwDgfHps ekGykavAea
361 l
```

FIGURE 4

(SEQ ID No. 3)

```
  1 mkkwfvcllg lvaltvqaad srpafsrivm fgdslsdtgk myskmrgylp ssppyyegrf
 61 sngpvwleql tnefpgltia neaeggptav aynkiswnpk yqvinnldye vtqflqkdsf
121 kpddlvilwv gandylaygw nteqdakrvr daisdaanrm vlngakeill fnlpdlgqnp
181 sarsqkvvea ashvsayhnq lllnlarqla ptgmvklfei dkqfaemlrd pqnfglsdqr
241 nacyggsyvw kpfasrsast dsqlsafnpq erlaiagnpl laqavaspma arsastlnce
301 gkmfwdqvhp ttvvhaalse paatfiesqy eflah
```

FIGURE 5

SEQ ID No. 4

```
  1 mkkwfvcllg lialtvqaad trpafsrivm fgdslsdtgk myskmrgylp ssppyyegrf
 61 sngpvwleql tkqfpgltia neaeggatav aynkiswnpk yqvinnldye vtqflqkdsf
121 kpddlvilwv gandylaygw nteqdakrvr daisdaanrm vlngakqill fnlpdlgqnp
181 sarsqkvvea vshvsayhnk lllnlarqla ptgmvklfei dkqfaemlrd pqnfglsdve
241 npcydggyvw kpfatrsvst drqlsafspq erlaiagnpl laqavaspma rrsasplnce
301 gkmfwdqvhp ttvvhaalse raatfietqy eflahg
```

FIGURE 6

SEQ ID No. 5

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

FIGURE 7

SEQ ID No. 6

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

FIGURE 8

SEQ ID No. 7

```
  1 mdyekfllfg dsitefafnt rpiedgkdqy algaalvney trkmdilqrg fkgytsrwal
 61 kilpeilkhe snivmatifl gandacsagp qsvplpefid nirqmvslmk syhirpiiig
121 pglvdrekwe kekseeialg yfrtnenfai ysdalaklan eekvpfvaln kafqqeggda
181 wqqlltdglh fsgkgykifh dellkvietf ypqyhpknmq yklkdwrdvl ddgsnims
```

FIGURE 9

(SEQ ID No. 8)

```
        10         20         30         40         50         60
         |          |          |          |          |          |
MNLRQWMGAA TAALALGLAA CGGGGTDQSG NPNVAKVQRM VVFGDSLSDI GTYTPVAQAV

        70         80         90        100        110        120
         |          |          |          |          |          |
GGGKFTTNPG PIWAETVAAQ LGVTLTPAVM GYATSVQNCP KAGCFDYAQG GSRVTDPNGI

       130        140        150        160        170        180
         |          |          |          |          |          |
GHNGGAGALT YPVQQQLANF YAASNNTFNG NNDVVFVLAG SNDIFFWTTA AATSGSGVTP

       190        200        210        220        230        240
         |          |          |          |          |          |
AIATAQVQQA ATDLVGYVKD MIAKGATQVY VFNLPDSSLT PDGVASGTTG QALLHALVGT

       250        260        270        280        290        300
         |          |          |          |          |          |
FNTTLQSGLA GTSARIIDFN AQLTAAIQNG ASFGFANTSA RACDATKINA LVPSAGGSSL

       310        320        330        340
         |          |          |          |
FCSANTLVAS GADQSYLFAD GVHPTTAGHR LIASNVLARL LADNVAH
```

FIGURE 10 (SEQ ID No. 9)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalragqal glrvpadpdq pwpplpprgt ldvrrddvhw areylvpwig rrlrgessgd
241 hvtakgtlsp daiktriaav a
```

FIGURE 11

(SEQ ID No. 10)

```
  1 mqtnpaytsl vavgdsfteg msdllpdgsy rgwadllatr maarspgfry anlavrgkli
 61 gqivdeqvdv aaamgadvit lvgglndtlr pkcdmarvrd lltqaverla phceqlvlmr
121 spgrqgpvle rfrprmealf aviddlagrh gavvvdlyga qsladprmwd vdrlhltaeg
181 hrrvaeavwq slghepedpe whapipatpp pgwvtrrtad vrfarqhllp wigrrltgrs
241 sgdglpakrp dllpyedpar
```

FIGURE 12

(SEQ ID No. 11)

```
  1 mtrgrdggag apptkhrall aaivtlivai saaiyagasa ddgsrdhalq aggrlprgda
 61 apastgawvg awatapaaae pgtettglag rsvrnvvhts vggtgaritl snlygqsplt
121 vthasialaa gpdtaaaiad tmrrltfggs arviipaggq vmsdtarlai pyganvlvtt
181 yspipsgpvt yhpqarqtsy ladgdrtadv tavayttptp ywryltaldv lsheadgtvv
241 afgdsitdga rsqsdanhrw tdvlaarlhe aagdgrdtpr ysvvnegisg nrlltsrpgr
301 padnpsglsr fqrdvlertn vkavvvvlgv ndvlnspela drdailtglr tlvdraharg
361 lrvvgatitp fggyggytea retmrqevne eirsgrvfdt vvdfdkalrd pydprrmrsd
421 ydsgdhlhpg dkgyarmgav idlaalkgaa pvka
```

FIGURE 13 (SEQ ID No. 12)

```
  1 mtsmsrarva rriaagaayg gggiglagaa avglvvaevq larrrvgvgt ptrvpnaqgl
 61 yggtlptagd pplrlmmlgd staagqgvhr agqtpgalla sglaavaerp vrlgsvaqpg
121 acsddldrqv alvlaepdrv pdicvimvga ndvthrmpat rsvrhlssav rrlrtagaev
181 vvgtcpdlgt iervrqplrw larrasrqla aaqtigaveq ggrtvslgdl lgpefaqnpr
241 elfgpdnyhp saegyataam avlpsvcaal glwpadeehp dalrregflp varaaaeaas
301 eagtevaaam ptgprgpwal lkrrrrrrvs eaepsspsgv
```

FIGURE 14 (SEQ ID No. 13)

```
  1 mgrgtdqrtr ygrrrarval aaltaavlgv gvagcdsvgg dspapsgsps krtrtapawd
 61 tspasvaavg dsitrgfdac avlsdcpevs watgssakvd slavrllgka daaehswnya
121 vtgarmadlt aqvtraaqre pelvavmaga ndacrsttsa mtpvadfraq feeamatlrk
181 klpkaqvyvs sipdlkrlws qgrtnplgkq vwklglcpsm lgdadsldsa atlrrntvrd
241 rvadynevlr evcakdrrcr sddgavhefr fgtdqlshwd wfhpsvdgqa rlaeiayrav
301 taknp
```

FIGURE 15 (SEQ ID No. 14)

```
  1 mrlsrraata sallltpala lfgasaavsa priqatdyva lgdsyssgvg agsydsssgs
 61 ckrstksypa lwaashtgtr fnftacsgar tgdvlakqlt pvnsgtdlvs itiggndagf
121 adtmttcnlq gesaclaria karayiqqtl paqldqvyda idsrapaaqv vvlgyprfyk
181 lggscavgls eksraainaa addinavtak raadhgfafg dvnttfaghe lcsgapwlhs
241 vtlpvensyh ptangqskgy lpvlnsat
```

FIGURE 16 (SEQ ID No. 15)

```
  1 MKKWFVCLLG LIALTVQAAD TRPAFSRIVM FGDSLSDTGK MYSKMRGYLP
 51 SSPPYYEGRF SNGPVWLEQL TKQFPGLTIA NEAEGGATAV AYNKISWNPK
101 YQVINNLDYE VTQFLQKDSF KPDDLVILWV GANDYLAYGW NTEQDAKRVR
151 DAISDAANRM VLNGAKQILL FNLPDLGQNP SARSQKVVEA VSHVSAYHNK
201 LLLNLARQLA PTGMVKLFEI DKQFAEMLRD PQNFGLSDVE NPCYDGGYVW
251 KPFATRSVST DRQLSAFSPQ ERLAIAGNPL LAQAVASPMA RRSASPLNCE
301 GKMFWDQVHP TTVVHAALSE RAATFIETQY EFLAHG*
```

FIGURE 17 (SEQ ID No. 19)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalragqal glrvpadpdq pwpplpprgt ldvrrddvhw areylvpwig rrlrgessgd
241 hvtakgtlsp daiktriaav a
```

FIGURE 18 (SEQ ID No. 25)

```
  1 MFKFKKNFLV GLSAALMSIS LFSATASAAS ADSRPAFSRI VMFGDSLSDT
 51 GKMYSKMRGY LPSSPPYYEG RFSNGPVWLE QLTKQFPGLT IANEAEGGAT
101 AVAYNKISWN PKYQVINNLD YEVTQFLQKD SFKPDDLVIL WVGANDYLAY
151 GWNTEQDAKR VRDAISDAAN RMVLNGAKQI LLFNLPDLGQ NPSARSQKVV
201 EAVSHVSAYH NQLLLNLARQ LAPTGMVKLF EIDKQFAEML RDPQNFGLSD
251 VENPCYDGGY VWKPFATRSV STDRQLSAFS PQERLAIAGN PLLAQAVASP
301 MARRSASPLN CEGKMFWDQV HPTTVVHAAL SERAATFIAN QYEFLAH**
```

FIGURE 19

(SEQ ID NO. 26)

```
MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST
```

Figure 20

SEQ ID No. 27

ZP_00058717

```
  1 mlphpagerg evgaffallv gtpqdrrlrl echetrplrg rcgcgerrvp pltlpgdgvl
 61 cttsstrdae tvwrkhlqpr pdggfrphlg vgcllagqgs pgvlwcgreg crfevcrrdt
121 pglsrtrngd ssppfragws lppkcgeisq sarktpavpr ysllrtdrpd gprgrfvgsg
181 praatrrrlf lgipalvlvt altlvlavpt gretlwrmwc eatqdwclgv pvdsrgqpae
241 dgeflllspv qaatwgnyya lgdsyssgdg ardyypgtav kggcwrsana ypelvaeayd
301 faghlsflac sgqrgyamld aidevgsqld wnsphtslvt igiggndlgf stvlktcmvr
361 vplldskact dqedairkrm akfettfeel isevrtrapd arilvvgypr ifpeeptgay
421 ytltasnqrw lnetiqefnq qlaeavavhd eeiaasggvg svefvdvyha ldgheigsde
481 pwvngvqlrd latgvtvdrs tfhpnaaghr avgervieqi etgpgrplya tfavvagatv
541 dtlagevg
```

FIGURE 21

(SEQ ID No. 28)

```
  1 mgsgpraatr rrlflgipal vlvtaltlvl avptgretlw rmwceatqdw clgvpvdsrg
 61 qpaedgefll lspvqaatwg nyyalgdsys sgdgardyyp gtavkggcwr sanaypelva
121 eaydfaghls flacsgqrgy amldaidevg sqldwnspht slvtigiggn dlgfstvlkt
181 cmvrvpllds kactdqedai rkrmakfett feelisevrt rapdarilvv gyprifpeep
241 tgayytltas nqrwlnetiq efnqqlaeav avhdeeiaas ggvgsvefvd vyhaldghei
301 gsdepwvngv qlrdlatgvt vdrstfhpna aghravgerv ieqietgpgr plyatfavva
361 gatvdtlage vg
```

FIGURE 22

(SEQ ID No. 29)

```
  1 mrttviaasa llllagcadg areetagapp gessggiree gaeastsitd vyialgdsya
 61 amggrdqplr gepfclrssg nypellhaev tdltcqgavt gdlleprtlg ertlpaqvda
121 ltedttlvtl siggndlgfg evagcireri agenaddcvd llgetigeql dqlppqldrv
181 heairdragd aqvvvtgylp lvsagdcpel gdvseadrrw aveltgqine tvreaaerhd
241 alfvlpddad ehtscappqq rwadiqgqqt dayplhptsa gheamaaavr dalglepvqp
```

FIGURE 23

(SEQ ID No. 30)

ZP_00094165

```
  1 mgqvklfarr capvllalag lapaatvare aplaegaryv algssfaagp gvgpnapgsp
 61 ercgrgtlny phllaealkl dlvdatcsga tthhvlgpwn evppqidsvn gdtrlvtlti
121 ggndvsfvgn ifaaacekma spdprcgkwr eiteeewqad eermrsivrq iharaplarv
181 vvvdyitvlp psgtcaamai spdrlaqsrs aakrlarita rvareegasl lkfshisrrh
241 hpcsakpwsn glsapaddgi pvhpnrlgha eaaaalvklv klmk //
```

FIGURE 24

SEQ ID No. 31

NP_625998.

```
      1 mrrfrlvgfl sslvlaagaa ltgaataqaa qpaaadgyva lgdsyssgvg agsyisssgd
     61 ckrstkahpy lwaaahspst fdftacsgar tgdvlsgqlg plssgtglvs isiggndagf
    121 adtmttcvlq sessclsria taeayvdstl pgkldgvysa isdkapnahv vvigyprfyk
    181 lgttciglse tkrtainkas dhlntvlaqr aaahgftfgd vrttftghel csgspwlhsv
    241 nwlnigesyh ptaagqsggy lpvlngaa
//
```

FIGURE 25

SEQ ID No. 32

NP_827753.

```
      1 mrrsritayv tslllavgca ltgaataqas paaaatgyva lgdsyssgvg agsylsssgd
     61 ckrsskaypy lwqaahspss fsfmacsgar tgdvlanqlg tlnsstglvs ltiggndagf
    121 sdvmttcvlq sdsaclsrin takayvdstl pgqldsvyta istkapsahv avlgyprfyk
    181 lggsclagls etkrsainda adylnsaiak raadhgftfg dvkstftghe icssstwlhs
    241 ldllnigqsy hptaagqsgg ylpvmnsva
//
```

FIGURE 26

SEQ ID No. 33

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

FIGURE 27

(SEQ ID No. 34)

```
1       ADSRPAFSRI VMFGDSLSDT GKMYSKMRGY LPSSPPYYEG RFSNGPVWLE QLTKQFPGLT
61      IANEAEGGAT AVAYNKISWN PKYQVINNLD YEVTQFLQKD SFKPDDLVIL WVGANDYLAY
121     GWNTEQDAKR VRDAISDAAN RMVLNGAKQI LLFNLPDLGQ NPSARSQKVV EAVSHVSAYH
181     NQLLLNLARQ LAPTGMVKLF EIDKQFAEML RDPQNFGLSD VENPCYDGGY VWKPFATRSV
241     STDRQLSAFS PQERLAIAGN PLLAQAVASP MARRSASPLN CEGKMFWDQV HPTTVVHAAL
301     SERAATFIAN QYEFLAH*
```

FIGURE 28

(SEQ ID No. 35)

```
  1 ADTRPAFSRI VMFGDSLSDT GKMYSKMRGY LPSSPPYYEG RFSNGPVWLE QLTKQFPGLT
 61 IANEAEGGAT AVAYNKISWN PKYQVINNLD YEVTQFLQKD SFKPDDLVIL WVGANDYLAY
121 GWNTEQDAKR VRDAISDAAN RMVLNGAKQI LLFNLPDLGQ NPSARSQKVV EAVSHVSAYH
181 NKLLLNLARQ LAPTGMVKLF EIDKQFAEML RDPQNFGLSD VENPCYDGGY VWKPFATRSV
241 STDRQLSAFS PQERLAIAGN PLLAQAVASP MARRSASPLN CEGKMFWDQV HPTTVVHAAL
301 SERAATFIET QYEFLAHG
```

FIGURE 29

(SEQ ID No. 36)

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

FIGURE 30

(SEQ ID NO. 37):

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

FIGURE 31

SEQ ID No. 38

```
  1 mlphpagerg evgaffallv gtpqdrrlrl echetrplrg rcgcgerrvp pltlpgdgvl
 61 cttsstrdae tvwrkhlqpr pdggfrphlg vgcllagqgs pgvlwcgreg crfevcrrdt
121 pglsrtrngd ssppfragws lppkcgeisq sarktpavpr ysllrtdrpd gprgrfvgsg
181 praatrrrlf lgipalvlvt altlvlavpt gretlwrmwc eatqdwclgv pvdsrgqpae
241 dgeflllspv qaatwgnyya lgdsyssgdg ardyypgtav kggcwrsana ypelvaeayd
301 faghlsflac sgqrgyamld aidevgsqld wnsphtslvt igiggndlgf stvlktcmvr
361 vplldskact dqedairkrm akfettfeel isevrtrapd arilvvgypr ifpeeptgay
421 ytltasnqrw lnetiqefnq qlaeavavhd eeiaasggvg svefvdvyha ldgheigsde
481 pwvngvqlrd latgvtvdrs tfhpnaaghr avgervieqi etgpgrplya tfavvagatv
541 dtlagevg
```

FIGURE 32

(SEQ ID No. 39)

```
   1 ggtggtgaac cagaacaccc ggtcgtcggc gtgggcgtcc aggtgcaggt gcaggttctt
  61 caactgctcc agcaggatgc cgccgtggcc gtgcacgatg gccttgggca ggcctgtggt
 121 ccccgacgag tacagcaccc atagcggatg gtcgaacggc agcggggtga actccagttc
 181 cgcgccttcg cccgcggctt cgaactccgc ccaggacagg gtgtcggcga cagggccgca
 241 gcccaggtac ggcaggacga cggtgtgctg caggctgggc atgccgtcgc gcagggcttt
 301 gagcacgtca cggcggtcga agtccttacc gccgtagcgg tagccgtcca cggccagcag
 361 cactttcggt tcgatctgcg cgaaccggtc gaggacgctg cgcaccccga agtcggggga
 421 acaggacgac caggtcgcac cgatcgcggc gcaggcgagg aatgcggccg tcgcctcggc
 481 gatgttcggc aggtaggcca cgacccggtc gccggggccc accccgaggc tgcggagggc
 541 cgcagcgatc gcggcggtgc gggtccgcag ttctccccag gtccactcgg tcaacggccg
 601 gagttcggac gcgtgccgga tcgccacggc tgatgggtca cggtcgcgga agatgtgctc
 661 ggcgtagttg agggtggcgc cggggaacca gacggcgccg ggcatggcgt cggaggcgag
 721 cactgtggtg tacggggtgg cggcgcgcac ccggtagtac tcccagatcg cggaccagaa
 781 tccttcgagg tcggttaccg accagcgcca cagtgcctcg tagtccggtg cgtccacacc
 841 gcggtgctcc cgcacccagc gggtgaacgc ggtgaggttg gcgcgttctt tgcgctcctc
 901 gtcgggactc cacaggatcg gcggctgcgg cttgagtgtc atgaaacgcg acccccttcgt
 961 ggacggtgcg gatgcggtga gcgtcgggtg cctcccctaa cgctccccgg tgacggagtg
1021 ttgtgcacca catctagcac gcgggacgcg gaaaccgtat ggagaaaaca cctacaaccc
1081 cggccggacg gtgggtttcg gccacactta ggggtcgggt gcctgcttgc cgggcagggc
1141 agtcccgggg tgctgtggtg cgggcgggag ggctgtcgct tcgaggtgtg ccggcgggac
1201 actccgggcc tcagccgtac ccgcaacggg gacagttctc ctcccttccg ggctggatgg
1261 tcccttcccc cgaaatgcgg cgagatctcc cagtcagccc ggaaaacacc cgctgtgccc
1321 aggtactctt tgcttcgaac agacaggccg gacggtccac gggggaggtt tgtgggcagc
1381 ggaccacgtg cggcgaccag acgacggttg ttcctcggta tccccgctct tgtacttgtg
1441 acagcgctca cgctggtctt ggctgtcccg acggggcgcg agacgctgtg gcgcatgtgg
1501 tgtgaggcca cccaggactg gtgcctgggg gtgccggtcg actcccgcgg acagcctgcg
1561 gaggacggcg agtttctgct gctttctccg gtccaggcag cgacctgggg gaactattac
1621 gcgctcgggg attcgtactc ttcgggggac ggggcccgcg actactatcc cggcaccgcg
1681 gtgaagggcg gttgctggcg gtccgctaac gcctatccgg agctggtcgc cgaagcctac
1741 gacttcgccg gacacttgtc gttcctggcc tgcagcggcc agcgcggcta cgccatgctt
1801 gacgctatcg acgaggtcgg ctcgcagctg gactggaact cccctcacac gtcgctggtg
1861 acgatcggga tcggcggcaa cgatctgggg ttctccacgg ttttgaagac ctgcatggtg
1921 cgggtgccgc tgctggacag caaggcgtgc acggaccagg aggacgctat ccgcaagcgg
1981 atggcgaaat tcgagacgac gtttgaagag ctcatcagcg aagtgcgcac ccgcgcgccg
2041 gacgcccgga tccttgtcgt gggctacccc cggatttttc cggaggaacc gaccggcgcc
2101 tactacacgc tgaccgcgag caaccagcgg tggctcaacg aaaccattca ggagttcaac
2161 cagcagctcg ccgaggctgt cgcggtccac gacgaggaga ttgccgcgtc gggcggggtg
2221 ggcagcgtgg agttcgtgga cgtctaccac gcgttggacg gccacgagat cggctcggac
2281 gagccgtggg tgaacggggt gcagttgcgg gacctcgcca ccggggtgac tgtggaccgc
2341 agtaccttcc accccaacgc cgctgggcac cgggcggtcg gtgagcgggt catcgagcag
2401 atcgaaaccg gcccgggccg tccgctctat gccactttcg cggtggtggc gggggcgacc
2461 gtggacactc tcgcgggcga ggtggggtga cccggcttac cgtccggccc gcaggtctgc
2521 gagcactgcg gcgatctggt ccactgccca gtgcagttcg tcttcggtga tgaccagcgg
2581 cggggagagc cggatcgttg agccgtgcgt gtctttgacg agcacacccc gctgcaggag
2641 ccgttcgcac agttctcttc cggtggccag agtcgggtcg acgtcgatcc cagcccacag
2701 gccgatgctg cgggccgcga ccacgccgtt gccgaccagt tggtcgaggc gggcgcgcag
2761 cacgggggcg agggcgcgga catggtccag gtaagggccg tcgcggacga ggctcaccac
2821 ggcagtgccg accgcgcagg cgagggcgtt gccgccgaag gtgctgccgt gctggccggg
2881 gcggatcacg tcgaagactt ccgcgtcgcc taccgccgcc gccacgggca ggatgccgcc
2941 gcccagcgct ttgccgaaca ggtagatatc ggcgtcgact ccgctgtggt cgcaggcccg
```

FIGURE 33

(SEQ ID No. 40)

```
  1 vgsgpraatr rrlflgipal vlvtaltlvl avptgretlw rmwceatqdw clgvpvdsrg
 61 qpaedgefll lspvqaatwg nyyalgdsys sgdgardyyp gtavkggcwr sanaypelva
121 eaydfaghls flacsgqrgy amldaidevg sqldwnspht slvtigiggn dlgfstvlkt
181 cmvrvpllds kactdqedai rkrmakfett feelisevrt rapdarilvv gyprifpeep
241 tgayytltas nqrwlnetiq efnqqlaeav avhdeeiaas ggvgsvefvd vyhaldghei
301 gsdepwvngv qlrdlatgvt vdrstfhpna aghravgerv ieqietgpgr plyatfavva
361 gatvdtlage vg
```

FIGURE 34

(SEQ ID No. 41)

```
  1 mrttviaasa llllagcadg areetagapp gessggiree gaeastsitd vyialgdsya
 61 amggrdqplr gepfclrssg nypellhaev tdltcqgavt gdllleprtlg ertlpaqvda
121 ltedttlvtl siggndlgfg evagcireri agenaddcvd llgetigeql dqlppqldrv
181 heairdragd aqvvvtgylp lvsagdcpel gdvseadrrw aveltgqine tvreaaerhd
241 alfvlpddad ehtscappqq rwadiqgqqt dayplhptsa gheamaaavr dalglepvqp
```

FIGURE 35

(SEQ ID No. 42)

```
   1 ttctggggtg ttatggggtt gttatcggct cgtcctgggt ggatcccgcc aggtggggta
  61 ttcacggggg acttttgtgt ccaacagccg agaatgagtg ccctgagcgg tgggaatgag
 121 gtgggcgggg ctgtgtcgcc atgagggggc ggcgggctct gtggtgcccc gcgacccccg
 181 gccccggtga gcggtgaatg aaatccggct gtaatcagca tcccgtgccc accccgtcgg
 241 ggaggtcagc gcccggagtg tctacgcagt cggatcctct cggactcggc catgctgtcg
 301 gcagcatcgc gctcccgggt cttggcgtcc ctcggctgtt ctgcctgctg tccctggaag
 361 gcgaaatgat caccggggag tgatacaccg gtggtctcat cccggatgcc cacttcggcg
 421 ccatccggca attcgggcag ctccgggtgg aagtaggtgg catccgatgc gtcggtgacg
 481 ccatagtggg cgaagatctc atcctgctcg agggtgctca ggccactctc cggatcgata
 541 tcgggggcgt ccttgatggc gtccttgctg aaaccgaggt gcagcttgtg ggcttccaat
 601 ttcgcaccac ggagcgggac gaggctggaa tgacggccga agagcccgtg gtggacctca
 661 acgaaggtgg gtagtcccgt gtcatcattg aggaacacgc cctccaccgc acccagcttg
 721 tggccggagt tgtcgtaggc gctggcatcc agaagggaaa cgatctcata tttgtcggtg
 781 tgctcagaca tgatcttcct ttgctgtcgg tgtctggtac taccacggta gggctgaatg
 841 caactgttat ttttctgtta ttttaggaat tggtccatat cccacaggct ggctgtggtc
 901 aaatcgtcat caagtaatcc ctgtcacaca aaatgggtgg tgggagccct ggtcgcggtt
 961 ccgtgggagg cgccgtgccc cgcaggatcg tcggcatcgg cggatctggc cggtaccccg
1021 cggtgaataa aatcattctg taaccttcat cacggttggt tttaggtatc cgcccctttc
1081 gtcctgaccc cgtccccggc gcgcgggagc ccgcgggttg cggtagacag ggagacgtg
1141 gacaccatga ggacaacggt catcgcagca agcgcattac tccttctcgc cggatgcgcg
1201 gatggggccc gggaggagac cgccggtgca ccgccgggtg agtcctccgg gggcatccgg
1261 gaggaggggg cggaggcgtc gacaagcatc accgacgtct acatcgccct cggggattcc
1321 tatgcggcga tgggcggcgg ggatcagccg ttacggggtg agccgttctg cctgcgctcg
1381 tccggtaatt acccggaact cctccacgca gaggtcaccg atctcacctg ccagggggcg
1441 gtgaccgggg atctgctcga acccaggacg ctgggggagc gcacgctgcc ggcgcaggtg
1501 gatgcgctga cggaggacac caccctggtc accctctcca tcgggggcaa tgacctcgga
1561 ttcggggagg tggcgggatg catccgggaa cggatcgccg gggagaacgc tgatgattgc
1621 gtggacctgc tgggggaaac catcggggag cagctcgatc agcttccccc gcagctggac
1681 cgcgtgcacg aggctatccg ggaccgcgcc ggggacgcgc aggttgtggt caccggttac
1741 ctgccgctcg tgtctgccgg ggactgcccc gaactggggg atgtctccga ggcggatcgt
1801 cgttgggcgg ttgagctgac cgggcagatc aacgagaccg tgcgcgaggc ggccgaacga
1861 cacgatgccc tctttgtcct gcccgacgat gccgatgagc acaccagttg tgcacccccca
1921 cagcagcgct gggcggatat ccagggccaa cagaccgatg cctatccgct gcacccgacc
1981 tccgccggcc atgaggcgat ggccgccgcc gtccgggacg cgctgggcct ggaaccggtc
2041 cagccgtagc gccgggcgcg cgcttgtcga cgaccaaccc atgccaggct gcagtcacat
2101 ccgcacatag cgcgcgcggg cgatggagta cgcaccatag aggatgagcc cgatgccgac
2161 gatgatgagc agcacactgc cgaagggttg ttccccgagg gtgcgcagag ccgagtccag
2221 acctgcggcc tgctccggat catgggccca accggcgatg acgatcaaca cccccaggat
2281 cccgaaggcg ataccacggg cgacataacc ggctgttccg gtgatgatga tcgcggtccc
2341 gacctgccct gaccccgcac ccgcctccag atcctcccgg aaatcccggg tggccccctt
2401 ccagaggttg tagacacccg cccccagtac caccagcccg gcgaccacaa ccagcaccac
2461 accccagggt tgggatagga cggtggcggt gacatcggtg gcggtctccc catcggaggt
2521 gctgccgccc cgggcgaagg tggaggtggt caccgccagg gagaagtaga ccatggccat
2581 gaccgccccc ttggcccttt ccttgaggtc ctcgcccgcc agcagctggc tcaattgcca
2641 gagtcccagg gccgccaggg cgatgacggc aacccacagg aggaactgcc cacccggagc
2701 ctccgcgatg gtggccaggg cacctgaatt cgaggcctca tcacccgaac cgccggatcc
2761 agtggcgatg cgcaccgcga tccacccgat gaggatgtgc agtatgccca ggacaatgaa
2821 accacctctg gccagggtgg tcagcgcggg gtggtcctcg gcctggtcgg cagcccgttc
2881 gatcgtccgt ttcgcggatc tggtgtcgcc cttatccata gctcccattg aaccgccttg
2941 aggggtgggc ggccactgtc agggcggatt gtgatctgaa ctgtgatgtt ccatcaaccc
```

FIGURE 36

(SEQ ID No. 43)

```
  1 mrrfrlvgfl sslvlaagaa ltgaataqaa qpaaadgyva lgdsyssgvg agsyisssgd
 61 ckrstkahpy lwaaahspst fdftacsgar tgdvlsgqlg plssgtglvs isiggndagf
121 adtmttcvlq sessclsria taeayvdstl pgkldgvysa isdkapnahv vvigyprfyk
181 lgttciglse tkrtainkas dhlntvlaqr aaahgftfgd vrttftghel csgspwlhsv
241 nwlnigesyh ptaagqsggy lpvlngaa
```

Figure 37

(SEQ ID No. 44)

```
   1 cccggcggcc cgtgcaggag cagcagccgg cccgcgatgt cctcgggcgt cgtcttcatc
  61 aggccgtcca tcgcgtcggc gaccggcgcc gtgtagttgg cccggacctc gtcccaggtg
 121 cccgcggcga tctggcgggt ggtgcggtgc gggccgcgcc gaggggagac gtaccagaag
 181 cccatcgtca cgttctccgg ctgcggttcg ggctcgtccg ccgctccgtc cgtcgcctcg
 241 ccgagcacct tctcggcgag gtcggcgctg gtcgccgtca ccgtgacgtc ggcgccccgg
 301 ctccagcgcg agatcagcag cgtccagccg tcgccctccg ccagcgtcgc gctgcggtcg
 361 tcgtcgcggg cgatccgcag cacgcgcgcg ccgggcggca gcagcgtggc gccggaccgt
 421 acgcggtcga tgttcgccgc gtgcgagtac ggctgctcac ccgtggcgaa acggccgagg
 481 aacagcgcgt cgacgacgtc ggacggggag tcgctgtcgt ccacgttgag ccggatcggc
 541 agggcttcgt gcgggttcac ggacatgtcg ccatgatcgg gcacccggcc gccgcgtgca
 601 cccgctttcc cgggcacgca cgacaggggc tttctcgccg tcttccgtcc gaacttgaac
 661 gagtgtcagc catttcttgg catggacact tccagtcaac gcgcgtagct gctaccacgg
 721 ttgtggcagc aatcctgcta agggaggttc catgagacgt ttccgacttg tcggcttcct
 781 gagttcgctc gtcctcgccg ccggcgccgc cctcaccggg gcagcgaccg cccaggcggc
 841 ccaacccgcc gccgccgacg gctatgtggc cctcggcgac tcctactcct ccggggtcgg
 901 agcgggcagc tacatcagct cgagcggcga ctgcaagcgc agcacgaagg cccatcccta
 961 cctgtgggcg gccgcccact cgccctccac gttcgacttc accgcctgtt ccggcgcccg
1021 tacgggtgat gttctctccg gacagctcgg cccgctcagc tccggcaccg gcctcgtctc
1081 gatcagcatc ggcggcaacg acgccggttt cgccgacacc atgacgacct gtgtgctcca
1141 gtccgagagc tcctgcctgt cgcggatcgc caccgccgag gcgtacgtcg actcgacgct
1201 gcccggcaag ctcgacggcg tctactcggc aatcagcgac aaggcgccga acgcccacgt
1261 cgtcgtcatc ggctacccgc gcttctacaa gctcggcacc acctgcatcg gcctgtccga
1321 gaccaagcgg acggcgatca acaaggcctc cgaccacctc aacaccgtcc tcgcccagcg
1381 cgccgccgcc cacggcttca ccttcggcga cgtacgcacc accttcaccg gccacgagct
1441 gtgctccggc agcccctggc tgcacagcgt caactggctg aacatcggcg agtcgtacca
1501 ccccaccgcg gccggccagt ccggtggcta cctgccggtc ctcaacggcg ccgcctgacc
1561 tcaggcggaa ggagaagaag aaggagcgga gggagacgag gagtgggagg ccccgcccga
1621 cggggtcccc gtccccgtct ccgtctccgt cccggtcccg caagtcaccg agaacgccac
1681 cgcgtcggac gtggcccgca ccggactccg cacctccacg cgcacggcac tctcgaacgc
1741 gccggtgtcg tcgtgcgtcg tcaccaccac gccgtcctgg cgcgagcgct cgccgcccga
1801 cgggaaggac agcgtccgcc accccggatc ggagaccgac ccgtccgcgg tcacccaccg
1861 gtagccgacc tccgcgggca gccgcccgac cgtgaacgtc gccgtgaacg cgggtgcccg
1921 gtcgtgcggc ggcggacagg cccccgagta gtgggtgcgc gagcccacca cggtcacctc
1981 caccgactgc gctgcggggc
```

FIGURE 38

(SEQ ID No. 45)

```
  1 mrrsritayv tslllavgca ltgaataqas paaaatgyva lgdsyssgvg agsylsssgd
 61 ckrsskaypy lwqaahspss fsfmacsgar tgdvlanqlg tlnsstglvs ltiggndagf
121 sdvmttcvlq sdsaclsrin takayvdstl pgqldsvyta istkapsahv avlgyprfyk
181 lggsclagls etkrsainda adylnsaiak raadhgftfg dvkstftghe icssstwlhs
241 ldllnigqsy hptaagqsgg ylpvmnsva
```

FIGURE 39

SEQ ID No. 46

```
   1 ccaccgccgg gtcggcggcg agtctcctgg cctcggtcgc ggagaggttg gccgtgtagc
  61 cgttcagcgc ggcgccgaac gtcttcttca ccgtgccgcc gtactcgttg atcaggccct
 121 tgcccttgct cgacgcggcc ttgaagccgg tgcccttctt gagcgtgacg atgtagctgc
 181 ccttgatcgc ggtgggggag ccggcggcga gcaccgtgcc ctcggccggg gtggcctggg
 241 cgggcagtgc ggtgaatccg cccacgaggg cgccggtcgc cacggcggtt atcgcggcga
 301 tccggatctt cttgctacgc agctgtgcca tacgagggag tcctcctctg ggcagcggcg
 361 cgcctgggtg gggcgcacgg ctgtgggggg tgcgcgcgtc atcacgcaca cggccctgga
 421 gcgtcgtgtt ccgccctggg ttgagtaaag cctcggccat ctacgggggt ggctcaaggg
 481 agttgagacc ctgtcatgag tctgacatga gcacgcaatc aacggggccg tgagcacccc
 541 ggggcgaccc cggaaagtgc cgagaagtct tggcatggac acttcctgtc aacacgcgta
 601 gctggtacga cggttacggc agagatcctg ctaaagggag gttccatgag acgttcccga
 661 attacggcat acgtgacctc actcctcctc gccgtcggct gcgccctcac cggggcagcg
 721 acggcgcagg cgtccccagc cgccgcggcc acgggctatg tggccctcgg cgactcgtac
 781 tcgtccggtg tcggcgccgg cagctacctc agctccagcg gcgactgcaa gcgcagttcg
 841 aaggcctatc cgtacctctg gcaggccgcg cattcaccct cgtcgttcag tttcatggct
 901 tgctcgggcg ctcgtacggg tgatgtcctg gccaatcagc tcggcaccct gaactcgtcc
 961 accggcctgg tctccctcac catcggaggc aacgacgcgg gcttctccga cgtcatgacg
1021 acctgtgtgc tccagtccga cagcgcctgc ctctcccgca tcaacacggc gaaggcgtac
1081 gtcgactcca ccctgcccgg ccaactcgac agcgtgtaca cggcgatcag cacgaaggcc
1141 ccgtcggccc atgtggccgt gctgggctac ccccgcttct acaaactggg cggctcctgc
1201 ctcgcgggcc tctcggagac caagcggtcc gccatcaacg acgcggccga ctatctgaac
1261 agcgccatcg ccaagcgcgc cgccgaccac ggcttcacct tcggcgacgt caagagcacc
1321 ttcaccggcc atgagatctg ctccagcagc acctggctgc acagtctcga cctgctgaac
1381 atcggccagt cctaccaccc gaccgcggcc ggccagtccg gcggctatct gccggtcatg
1441 aacagcgtgg cctgagctcc cacggcctga atttttaagg cctgaatttt taaggcgaag
1501 gtgaaccgga agcggaggcc ccgtccgtcg ggtctccgt cgcacaggtc accgagaacg
1561 gcacggagtt ggacgtcgtg cgcaccgggt cgcgcacctc gacggcgatc tcgttcgaga
1621 tcgttccgct cgtgtcgtac gtggtgacga acacctgctt ctgctgggtc tttccgccgc
1681 tcgccgggaa ggacagcgtc ttccagcccg gatccgggac ctcgcccttc ttggtcaccc
1741 agcggtactc cacctcgacc ggcacccggc ccaccgtgaa ggtcgccgtg aacgtgggcg
1801 cctgggcggt gggcggcggg caggcaccgg agtagtcggt gtgcacgccg gtgaccgtca
1861 ccttcacgga ctgggccggc ggggtcgtcg taccgccgcc gccaccgccg cctcccggag
1921 tggagcccga gctgtggtcg cccccgccgt cggcgttgtc gtcctcgggg gttttcgaac
```

FIGURE 40

SEQ ID No. 47

```
  1 mgsgpraatr rrlflgipal vlvtaltlvl avptgretlw rmwceatqdw clgvpvdsrg
 61 qpaedgefll lspvqaatwg nyyalgdsys sgdgardyyp gtavkggcwr sanaypelva
121 eaydfaghls flacsgqrgy amldaidevg sqldwnspht slvtigiggn dlgfstvlkt
181 cmvrvpllds kactdqedai rkrmakfett feelisevrt rapdarilvv gyprifpeep
241 tgayytltas nqrwlnetiq efnqqlaeav avhdeeiaas ggvgsvefvd vyhaldghei
301 gsdepwvngv qlrdlatgvt vdrstfhpna aghravgerv ieqietgpgr plyatfavva
361 gatvdtlage vg
```

FIGURE 41

SEQ ID No. 48

```
1      ctgcagacac ccgccccgcc ttctcccgga tcgtcatgtt cggcgactcc ctcagcgaca
61     ccggcaagat gtactccaag atgcgcggct acctgccgtc ctccccgccg tactacgagg
121    gccgcttctc gaacggcccg gtctggctgg agcagctgac gaagcagttc cccggcctga
181    cgatcgccaa cgaggccgag gggggcgcga ccgcagtcgc ctacaacaag atctcctgga
241    acccgaagta ccaggtcatt aacaacctcg actacgaggt cacccagttc ttgcagaagg
301    actcgttcaa gcccgacgac ctggtcatcc tgtgggtggg cgccaacgac tacctggcct
361    acggttggaa cacggagcag gacgccaagc gggtgcgcga cgccatctcg gacgcggcaa
421    accgcatggt cctgaacggc gcgaagcaga tcctgctgtt caacctgccc gacctgggcc
481    agaacccgtc cgcccgctcc cagaaggtcg tcgaggccgt ctcgcacgtg tccgcctacc
541    acaacaagct gctcctcaac ctcgcccggc agctcgcccc gacgggcatg gtcaagctgt
601    tcgagatcga caagcagttc gcggagatgc tgcgcgaccc ccagaacttc ggcctgagcg
661    acgtggagaa cccgtgctac gacggcggct acgtgtggaa gccgttcgcc acccggtccg
721    tctcgaccga ccggcagctg tcggccttct cgccccagga gcgcctggcg atcgctggca
781    acccgctcct ggcacaggcg gtagcttcgc cgatggcccg ccgctcggcc tcgcccctca
841    actgcgaggg caagatgttc tgggaccagg tccaccccac caccgtggtc cacgccgccc
901    tctcggagcg cgccgccacc ttcatcgaga cccagtacga gttcctcgcc cactagtcta
961    gaggatcc
```

Figure 42

1. L131
2. S.avermitilis
3. T.fusca
4. Consensus

```
                1                                                  50
1  (1)  --------MRLTRSLSAASVIVFALLLALLGISPAQAAG-----------
2  (1)  --------MRRSRITAYVTSLLLAVGCALTGAATAQASPA----------
3  (1)  VGSGPRAATRRRLFLGIPALVLVTALTLVLAVPTGRETLWRMWCEATQDW
4  (1)          MRRSRFLA  ALILLTLA AL GAA ARAAP

                51                                                 100
1  (32)  -----------------------------P-AYVALGDSYSSGNGAGSYID
2  (33)  ---------------------------AAATGYVALGDSYSSGVGAGSYLS
3  (51)  CLGVPVDSRGQPAEDGEFLLLSPVQAATWGNYYALGDSYSSGDGARDYYP
4  (51)                            A A  YVALGDSYSSG GAGSY

                101                                                150
1   (53)  SSGD---CHRSNNAYPARWAAANAP---SSFTFAACSGAVTTDVIN----
2   (57)  SSGD---CKRSSKAYPYLWQAAHSP---SSFSFMACSGARTGDVLA----
3  (101)  GTAVKGGCWRSANAYPELVAEAYDFA--GHLSFLACSGQRGYAMLDAIDE
4  (101)  SSGD   C RSTKAYPALWAAAHA    SSFSF ACSGARTYDVLA

                151                                                200
1   (93)  --NQLGALNAST--GLVSITIGGNDAGFADAMTTCVTS------SDSTCL
2   (97)  --NQLGTLNSST--GLVSLTIGGNDAGFSDVMTTCVLQ------SDSACL
3  (149)  VGSQLDWNSPHT--SLVTIGIGGNDLGFSTVLKTCMVR------VPLLDS
4  (151)    QL  LNS T   LVSITIGGNDAGFAD MTTCVL        SDSACL

                201                                                250
1  (133)  NRLATATNYINTTLLA-------RLDAVYSQIKARAPNARVVVLGYPRMY
2  (137)  SRINTAKAYVDSTLPG-------QLDSVYTAISTKAPSAHVAVLGYPRFY
3  (191)  KACTDQEDAIRKRMAKF----ETTFEELISEVRTRAPDARILVVGYPRIF
4  (201)   RIA AK YI  TLPA       RLDSVYSAI TRAP ARVVVLGYPRIY

                251                                                300
1  (176)  LASNPWYCLGLSNTKRAAINTTADTLNSVISSRATAH-----------GF
2  (180)  KLGG-SCLAGLSETKRSAINDAADYLNSAIAKRAADH-----------GF
3  (237)  PEEPTGAYYTLTASNQRWLNETIQEFNQQLAEAVAVHDEEIAASGGVGSV
4  (251)    SG    LGLS TKRAAINDAAD LNSVIAKRAADH           GF

                301                                                350
1  (215)  RFGDVRPTFNNHELFFGNDWLHSLTLP----------------VWESYH
2  (218)  TFGDVKSTFTGHEICSSSTWLHSLDLLN----------------IGQSYH
3  (287)  EFVDVYHALDGHEIGSDEPWVNGVQLRDLATG---------VTVDRSTFH
4  (301)  TFGDV  TF GHELCSA PWLHSLTLP                V  SYH

                351                                    395
1  (248)  PTSTGHQSGYLPVLNANSST-------------------------
2  (252)  PTAAGQSGGYLPVMNSVA---------------------------
3  (328)  PNAAGHRAVGERVIEQIETGPGRPLYATFAVVAGATVDTLAGEVG
4  (351)  PTA GHAAGYLPVLNSI T
```

FIGURE 43

SEQ ID No 17 which is the amino acid sequence of a lipid acyltransferase from *Candida parapsilosis*;

```
MRYFAIAFLL INTISAFVLA PKKPSQDDFY TPPQGYEAQP LGSILKTRNV PNPLTNVFTP VKVQNAWQLL
VRSEDTFGNP NAIVTTIIQP FNAKKDKLVS YQTFEDSGKL DCAPSYAIQY GSDISTLTTQ GEMYYISALL
DQGYYVVTPD YEGPKSTFTV GLQSGRATLN SLRATLKSGN LTGVSSDAET LLWGYSGGSL ASGWAAAIQK
EYAPELSKNL LGAALGGFVT NITATAEAVD SGPFAGIISN ALAGIGNEYP DFKNYLLKKV SPLLSITYRL
GNTHCLLDGG IAYFGKSFFS
RIIRYFPDGW DLVNQEPIKT ILQDNGLVYQ PKDLTPQIPL FIYHGTLDAI VPIVNSRKTF QQWCDWGLKS
GEYNEDLTNG HITESIVGAP AALTWIINRF NGQPPVDGCQ HNVRASNLEY PGTPQSIKNY FEAALHAILG
FDLGPDVKRD KVTLGGLLKL ERFAF
```

FIGURE 44

SEQ ID No 18 which is the amino acid sequence of a lipid acyltransferase from *Candida parapsilosis*;

```
MRYFAIAFLL INTISAFVLA PKKPSQDDFY TPPQGYEAQP LGSILKTRNV PNPLTNVFTP VKVQNAWQLL
VRSEDTFGNP NAIVTTIIQP FNAKKDKLVS YQTFEDSGKL DCAPSYAIQY GSDISTLTTQ GEMYYISALL
DQGYYVVTPD YEGPKSTFTV GLQSGRATLN SLRATLKSGN LTGVSSDAET LLWGYSGGSL ASGWAAAIQK
EYAPELSKNL LGAALGGFVT NITATAEAVD SGPFAGIISN ALAGIGNEYP DFKNYLLKKV SPLLSITYRL
GNTHCLLDGG IAYFGKSFFS RIIRYFPDGW DLVNQEPIKT ILQDNGLVYQ PKDLTPQIPL FIYHGTLDAI
VPIVNSRKTF QQWCDWGLKS GEYNEDLTNG HITESIVGAP AALTWIINRF NGQPPVDGCQ HNVRASNLEY
PGTPQSIKNY FEAALHAILG FDLGPDVKRD KVTLGGLLKL ERFAFHHHHH H
```

FIGURE 45

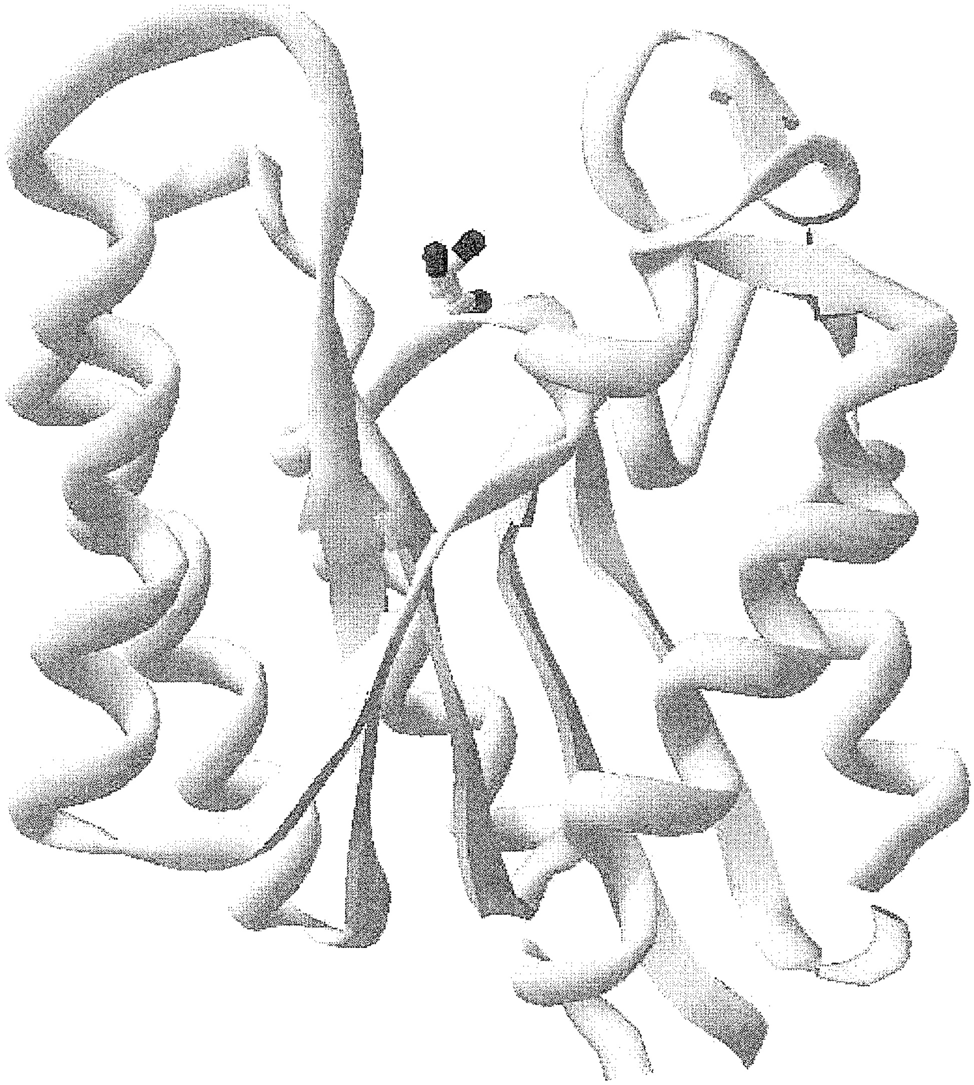

FIGURE 46

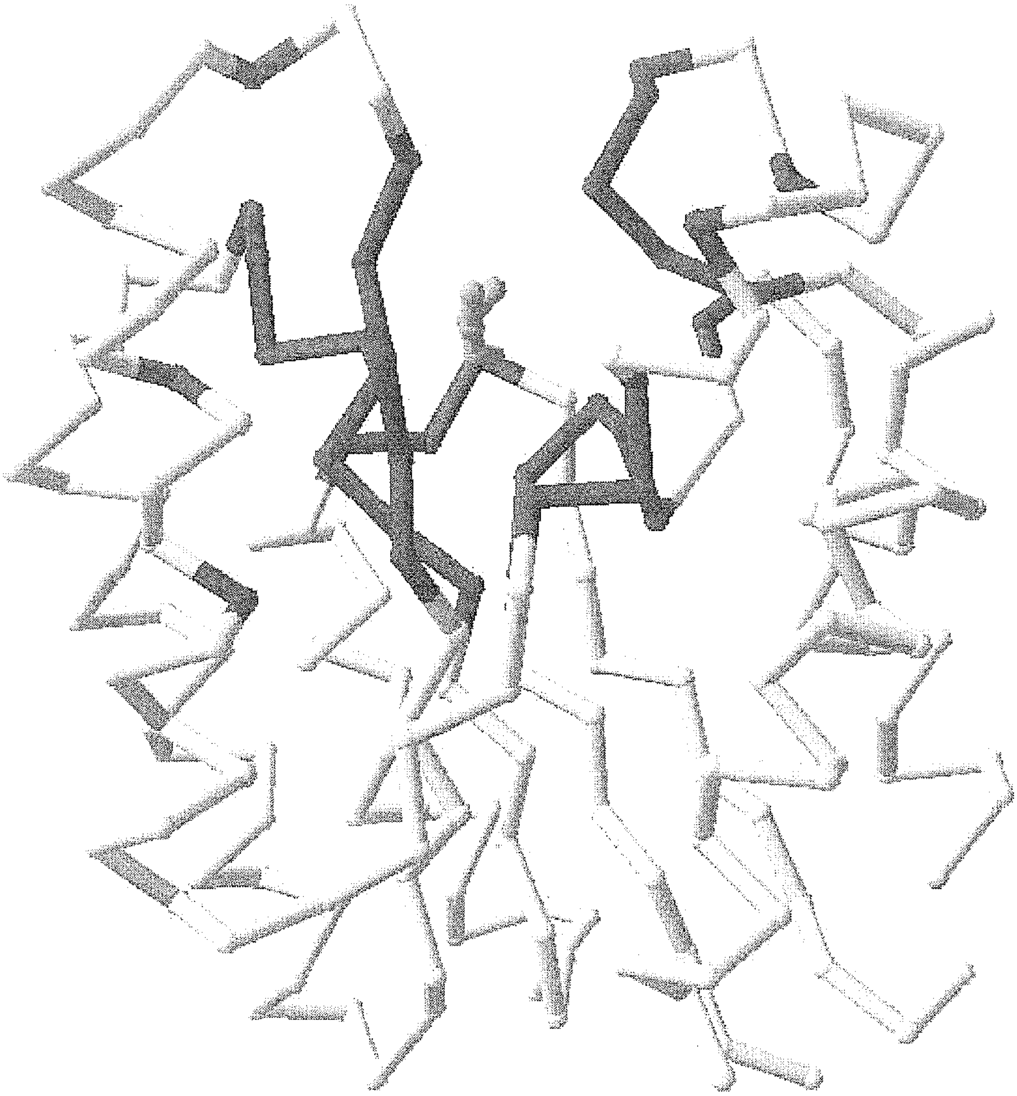

FIGURE 47

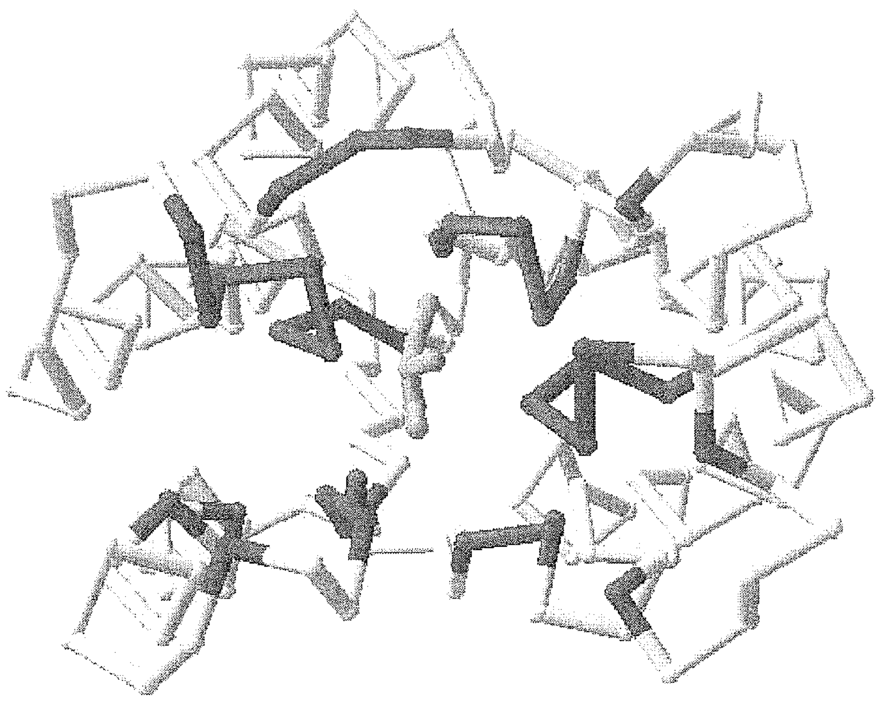

FIGRURE 48

```
1DEO     T T V Y  L  A G D S T M A K n - - - - - - - - - - - - - - - - - - - - - - G G G S G T N G W G E Y L
          s1s1s1s1 s1 s1s1h?h?h?h?                                                   h1h1h1h1h1
1IVN   A D T L L  I  L G D S L S A G - - - - - - - - - - - - - - - - - - - - - - - Y R M S A S A A W P A L L
          s1s1s1s1 s1 s1s1h h h h                                                       h1h1h1h1h1
P10480      I V  M  F G D S L S D T g k m  y s k m  r  g  y  l p s s p p y  y e G R F S N G P V W L E Q L

1DEO     A S Y L S  A  T V - - - - - - - - - - - - - - - V  N  D  A V A G R S -  - - A R S Y T R E G R F E N I A D VV
         h1h1h1  s2 s2 s2                     s2s2 s2 s2 s2    h3    h3h3 h3h3h3h3h3h3h3h3h3h3h3h3h3
1IVN     N D K W q  s  k - - - - - - - - - - - t s v  V  N  A  S I S G D T -  - - - - - S Q Q G L A R L P A L L KQ
         h1h1h1  s2?s2?               s2?s2s2s2s2 s2 s2 s2                    h3h3h3h3h3h3h3h3h3h3h3h3
P10480 T N E F P  G  L T i a n e a e g g p  t a v a  Y  N  K  I S W N P K y  q v I N N L D Y E V T Q F L Q K D SF

1DEO     T A G D Y  V  I V E F G H N D G g s  l s t d  n  g  r  t d c s g t g  a E v C Y S V Y D G V N E T I L T FP
              s4s4 s4 s4s4s4      ? ? ? ?  ? ? ? ?  ?  ?  ?  ? ? ? ? ? ? ?  ? ? s?s?s?s?s?s?s?s?s?s?s?s?h4h4h4
1IVN     H Q P R W  V  L V E L G G N D G - - - - - - - - - - - - - - - - - - - - - - - - - - - - L R G F Q P Q Q TE
         h3  s4s4s4 s4 s4s4                                                                     h4h4h4h4h4
P10480 K P D D L  V  I L W V G A N D Y - - - - - - - - - - - - - - - - - - - - - - - - L A Y G W N T E Q D A K R VR

1DEO     A Y L E N  A  A K L F T - A K G A K - - - - -  V  I  L S S Q T P -  - - - - N N P W E T G T F V N S P TR
         h4h4h4h4h4 h4 h4h4h4h4h4  h4    s5         s5 s5 s5 s5 s5
1IVN     Q T L R Q  I  L Q D V K a A N A E P  l l m q  i  R  L  P A N Y G R -  - - - - - - - - - - - - - - - - - - RY
         h4h4h4h4h4 h4 h4h4h4h4h4h4    s5s5s5  s5s5s5s5?s5?s5?s5?                                          h5
P10480 D A I S D  A  A N R M V - L N G A K - - - - -  E  I  L L F N L P d  l g q n P S A R S Q K V V E A A S HV

1DEO     F V E Y A  E  L A A E V A - - - - - - - - - - - - - - - - - G V E Y V  D H W S Y V D S I Y E T L G N A t vn
         h5h5h5h5h5 h5 h5h5h5h5h5h5                                  s6s6s6s6?h6h6h6h6h6h6h6h6h6h6    h h h h
1IVN     N E A F S  A  I Y P K L A k e - - - - - - - - - - - - - - f D V P L L  P F F M E E V Y L K P Q W - - - - - --
         h5h5h5h5h5 h5 h5h5h5h5h5h5h5h5                          h5    s6s6s6s6?  h6h6h6h6h6      s
P10480 S A Y H N  Q  L L L N L A r q l a p  t g m v  k  l  f  e i D K Q F A  E M L R D P Q N F G L S D Q R N a cy

1DEO     - - - - -  -  - - - - - - - - - - -  - - - -  -  -  -  - - - - - - - -  - - - - - - - - - - - - - - - - - - --

1IVN     - - - - -  -  - - - - - - - - - - -  - - - -  -  -  -  - - - - - - - -  - - - - - - - - - - - - - - - - - - --

P10480 g g s y v  w  k p f a s r s a s t d  s q l s  a  f  n  p q e r l a i  a g n p l l a q a v a s p m a a r sa

1DEO     - - - - -  -  s y F P I D H T H T S  P A G A  E  V  V  A E A F L K A  V V C T G T S L K S V L T T T S F EG
                       h               s?s?s?  h7h7h7h7 h7 h7 h7 h7h7h7h7h7h7h7 h7h7h7h7     h?h?h?
1IVN     - - - - -  -  - - M Q D D G I H P N  R D A Q  P  F  I  A D W M A K Q  L Q P L V N H D S L E
                           s s         s s s    h7h7h7 h7 h7 h7 h7h7h7h7h7h7h7 h7h7h7h7
P10480 s t l n c  e  g k M F W D Q V H P T  T V V H  A  A  L  S E P A A T F  I E S Q Y E F L A H -
```

FIGURE 49

```
1DEOm     T T V Y  L  A G D S T M A K n - -  - - - - - - - - - -  -  -  -  -  - - - - G G G S G T N G W G E Y L
            s1s1s1s1 s1 s1s1h?h?h?h?                                                          h1h1h1h1h1
1IVNm   A D T L L  I  L G D S L S A G - - -  - - - - - - - - - -  -  -  -  -  - - - - Y R M S A S A A W P A L L
            s1s1s1s1 s1 s1s1h h h h                                                              h1h1h1h1h1
P10480m       I V  M  F G D S L S D T g k m  y s k m r g y l  p  s  s  p  p y y e G R F S N G P V W L E Q L

1DEOm   A S Y L S  A  T V - - - - - - - - - -  - - - - V N D A  V  A  G  R  S - - -       A R S Y T R E G R F E N IA
        h1h1h1  s2 s2 s2                       s2s2s2s2s2     h3             h3h3h3h3h3h3h3h3h3h3h3h3
1IVNm   N D K W q  s  k - - - - - - - - - - -  - t s v V N A S  I  S  G  D  T - - - - - - S Q Q G L A     R L P A LL
        h1h1h1  s2?s2?                   s2?s2s2s2s2s2s2s2                    h3h3h3h3h3  h3h3h3h3h3
P10480m T N E F P  G  L T i a n e a e g g p  t a v a Y N K I  S  W  N  P  K         y q v I N N L D Y E V T Q F LQ

1DEOm   D   V V T  A  G D Y V I V E F G H N  D G g s l s t d  n  g  r  t  d c s g t g a E v C Y S V Y D G V N E TI
        h3h3       s4 s4s4s4s4s4          ? ?  ? ? ? ? ? ? ? ?  ?  ?  ?  ?  ? ? ? ? ? ? s?s?s?s?s?s?s?s?s?s?s?s?
1IVNm   K Q H Q P       R W V L V E L G G N  D G - - - - - - -  -  -  -  -  - - - - - - - - - - - - - - - - L R G F QP
        h3h3h3        s4s4s4s4s4s4                                                                          h4
P10480m K D S F K  P  D D L V I L W V G A N  D Y - - - - - - -  -  -  -  -  - - - - - - - - - - - - L A Y G W N T E Q DA

1DEOm   L T F P A  Y  L E N A A K L F T A K  G A K V I L S S  Q  T  P  N  N P W E T G T F V N S P T R
          h4h4h4h4 h4 h4h4h4h4h4h4h4h4h4h4     s5s5s5s5s5s5s5
1IVNm   Q Q T E Q  T  L R Q I L Q D V K a A  N A E P l l m q  i  R  L  P  A N Y G R - - - - - - - - - R Y N E A
        h4h4h4h4h4 h4 h4h4h4h4h4h4h4h4h4h4     s5s5s5s5s5s5s5s5?s5?s5?s5?                         h5h5h5h5h5h5
P10480m K R V R D  A  I S D A A N R M V L N  G A K E I L L F  N  L  P  d  l g q n P S A R S Q K V V E A A S H V SA

1DEOm     F V E Y  A  E L A A E V A - - - -  - - - - - - - - G  V  E  Y  V  D H W S Y V D S I Y E T L G N A t v n --
          h5h5h5h5 h5 h5h5h5h5h5h5h5                          s6 s6 s6 s6?h6h6h6h6h6h6h6h6h6h6    h h h h
1IVNm     F S A I  Y  P K L A k e - - - - -  - - - - - - f D  V  P  L  L  P F F M E E V Y          L K P Q W - ---
        h5h5h5h5h5 h5 h5h5h5                 h5  s6s6s6s6?   h6 h6 h6 h6h6      s
P10480m Y H N Q L  L  L N L A r q l a p t g  m v k l f e i D  K  Q  F  A  E M L R D P Q N F G L S D Q R N a c y gg

1DEOm   - - - - -  -  - - - - - - - - - - -  - - - - - - - -  -  -  -  -  - - - - - - - - - - - - - - - - - - - --

1IVNm   - - - - -  -  - - - - - - - - - - -  - - - - - - - -  -  -  -  -  - - - - - - - - - - - - - - - - - - - --

P10480m s y v w k  p  f a s r s a s t d s q  l s a f n p q e  r  l  a  i  a g n p l l a q a v a s p m a a r s a st

1DEOm   - - - - s  y  F P I D H T H T S P A  G A E V V A E A  F  L  K  A  V V C T G T S L K S V L T T T S F E G TC
                h           s?s?s?h7h7  h7h7h7h7h7h7h7h7h7 h7 h7 h7 h7 h7h7h7h7     h?h?h?
1IVNm   - - - - -  -  M Q D D G I H P N R D  A Q P F I A D W  M  A  K  Q  L Q P L V N H D S L E
                   s  s s  h7h7h7h7h7h7h7h7  h7h7h7h7h7h7h7h7  h7 h7 h7 h7 h7
P10480m l n c e g  k  M F W D Q V H P T T V  V H A A L S E P  A  A  T  F  I E S Q Y E F L A H -
```

FIGURE 50

```
1DEO     T T V Y  L  A G D S T M A K n - - - - - - - - - - - - - - - - - - - - - - - - - - G G G S G T N G W G E Y L
         s1s1s1s1 s1 s1s1h?h?h?h?                                                                 h1h1h1h1h1
1IVN   A D T L L  I  L G D S L S A G - - - - - - - - - - - - - - - - - - - - - - - - - - Y R M S A S A A W P A L L
         s1s1s1s1 s1 s1s1h h h h                                                                    h1h1h1h1h1
P10480       I V  M  F G D S L S D T g k m  y s k m  r  g  y  l  p  s  s  p  p y  y e G R F S N G P V W L E Q L

1DEOm    T T V Y  L  A G D S T M A K n - - - - - - - - - - - - - - - - - - - - - - - - - - G G G S G T N G W G E Y L
         s1s1s1s1 s1 s1s1h?h?h?h?                                                                 h1h1h1h1h1
1IVNm  A D T L L  I  L G D S L S A G - - - - - - - - - - - - - - - - - - - - - - - - - - Y R M S A S A A W P A L L
         s1s1s1s1 s1 s1s1h h h h                                                                    h1h1h1h1h1
P10480m      I V  M  F G D S L S D T g k m  y s k m  r  g  y  l  p  s  s  p  p y  y e G R F S N G P V W L E Q L


1DEO    A S Y L S  A  T V - - - - - - - - - - - - - - -  V  N  D  A  V  A  G  R  S -  - - A R S Y T R E G R F E N I A D VV
        h1h1h1  s2 s2 s2                           s2s2 s2 s2 s2       h3       h3h3 h3h3h3h3h3h3h3h3h3h3h3h3h3
1IVN    N D K W q  s  k - - - - - - - - - - - - t s v  V  N  A  S  I  S  G  D  T -  - - - - - S Q Q G L A R L P A L L KQ
        h1h1h1  s2?s2?                    s2?s2s2s2s2 s2 s2 s2                       h3h3h3h3h3h3h3h3h3h3h3h3h3
P10480  T N E F P  G  L T i a n e a e g g p  t a v a  Y  N  K  I  S  W  N  P  K y  q v I N N L D Y E V T Q F L Q K D SF

1DEOm   A S Y L S  A  T V - - - - - - - - - - - - - - -  V  N  D  A  V  A  G  R  S -  - -       A R S Y T R E G R F E N IA
        h1h1h1  s2 s2 s2                           s2s2 s2 s2 s2       h3                 h3h3h3h3h3h3h3h3h3h3h3h3h3
1IVNm   N D K W q  s  k - - - - - - - - - - - - t s v  V  N  A  S  I  S  G  D  T -  - - - - - S Q Q G L A     R L P A LL
        h1h1h1  s2?s2?                    s2?s2s2s2s2 s2 s2 s2                       h3h3h3h3h3  h3h3h3h3h3h3
P10480m T N E F P  G  L T i a n e a e g g p  t a v a  Y  N  K  I  S  W  N  P  K          y q v I N N L D Y E V T Q F LQ


1DEO    T A G D Y  V  I V E F G H N D G g s  l s t d  n  g  r  t  d  c  s  g  t g  a E v C Y S V Y D G V N E T I L T FP
              s4s4 s4 s4s4s4         ? ? ? ?  ? ? ? ?  ?  ?  ?  ?  ?  ?  ?  ?  ? ?  ? ? s?s?s?s?s?s?s?s?s?s?s?h4h4h4
1IVN    H Q P R W  V  L V E L G G N D G - - - - - - - - - - - - - - - - - - - - - - - - - - - - - - L R G F Q P Q Q TE
        h3  s4s4s4 s4 s4s4                                                                              h4h4h4h4h4
P10480  K P D D L  V  I L W V G A N D Y - - - - - - - - - - - - - - - - - - - - - - - - - L A Y G W N T E Q D A K R VR

1DEOm   D   V V T  A  G D Y V I V E F G H N  D G g s  l  s  t  d  n  g  r  t  d c  s g t g a E v C Y S V Y D G V N E TI
        h3h3       s4 s4s4s4s4s4         ? ?  ? ? ? ?  ?  ?  ?  ?  ?  ?  ?  ?  ? ?  ? ? ? ? s?s?s?s?s?s?s?s?s?s?s?
1IVNm   K Q H Q P       R W V L V E L G G N  D G - - - - - - - - - - - - - - - - - - - - - - - - - - - - - L R G F QP
        h3h3h3          s4s4s4s4s4s4                                                                                h4
P10480m K D S F K  P  D D L V I L W V G A N  D Y - - - - - - - - - - - - - - - - - - - - - - - - - L A Y G W N T E Q DA


1DEO    A Y L E N  A  A K L F T - A K G A K  - - - -  -  V  I  L  S  S  Q  T  P -  - - - - N N P W E T G T F V N S P TR
        h4h4h4h4h4 h4 h4h4h4h4h4  h4    s5            s5 s5 s5 s5 s5
1IVN    Q T L R Q  I  L Q D V K a A N A E P  l l m q  i  R  L  P  A  N  Y  G  R -  - - - - - - - - - - - - - - - - - - RY
        h4h4h4h4h4 h4 h4h4h4h4h4h4     s5s5s5 s5s5s5s5?s5?s5?s5?                                                   h5
P10480  D A I S D  A  A N R M V - L N G A K  - - - -  -  E  I  L  L  F  N  L  P d  l g q n P S A R S Q K V V E A A S HV

1DEOm   L T F P A  Y  L E N A A K L F T A K  G A K V  I  L  S  S  Q  T  P  N  N P  W E T G T F V N S P T R
          h4h4h4h4 h4 h4h4h4h4h4h4h4h4h4h4     s5s5s5 s5 s5 s5
```

```
1IVNm     Q Q T E Q  T  L R Q I L Q D V K a A  N A E P  l  l  m  q  i  R  L  P  A N  Y G R - - - - - - - - - - R Y N E A
          h4h4h4h4h4 h4 h4h4h4h4h4h4h4h4h4h4     s5s5s5 s5 s5 s5 s5?s5?s5?s5?                            h5h5h5h5h5h5
P10480m K R V R D  A  I S D A A N R M V L N  G A K E  I  L  L  F  N  L  P  d  l g  q n P S A R S Q K V V E A A S H V SA

1DEO      F V E Y A  E  L A A E V A - - - - - - - - - - - - - - - - - - -  G  V  E  Y V  D H W S Y V D S I Y E T L G N A t vn
          h5h5h5h5h5 h5 h5h5h5h5h5h5                                          s6 s6 s6s6?h6h6h6h6h6h6h6h6h6h6h6  h h h h
1IVN      N E A F S  A  I Y P K L A k e - - - - - - - - - - - - - - - -  f  D  V  P  L L  P F F M E E V Y L K P Q W - - - - - -
          h5h5h5h5h5 h5 h5h5h5h5h5h5h5h5                     h5       s6 s6 s6s6?  h6h6h6h6h6    s
P10480    S A Y H N  Q  L L L N L A r q l a p  t g m v  k  l  f  e  i  D  K  Q  F A  E M L R D P Q N F G L S D Q R N a cy

1DEOm       F V E Y  A  E L A A E V A - - - - - - - - - - - - - - -  G  V  E  Y  V  D H  W S Y V D S I Y E T L G N A t v n --
            h5h5h5h5 h5 h5h5h5h5h5h5h5                         s6 s6 s6 s6?h6h6 h6h6h6h6h6h6h6h6h6  h h h h
1IVNm       F S A I  Y  P K L A k e - - - - - - - - - - - - - -  f  D  V  P  L  L  P F  F M E E V Y      L K P Q W - - - -
          h5h5h5h5h5 h5 h5h5h5                        h5    s6 s6 s6 s6?  h6 h6 h6 h6h6      s
P10480m Y H N Q L  L  L N L A r q l a p t g  m v k l  f  e  i  D  K  Q  F  A  E M  L R D P Q N F G L S D Q R N a c y gg

1DEO      - - - - -  -  - - - - - - - - - - - - - - - - - - - - - - - - - - - - - - - - - - - - - - - - - - - - - - - - - -

1IVN      - - - - -  -  - - - - - - - - - - - - - - - - - - - - - - - - - - - - - - - - - - - - - - - - - - - - - - - - - -

P10480    g g s y v  w  k p f a s r s a s t d  s q l s  a  f  n  p  q  e  r  l  a i  a g n p l l a q a v a s p m a a r sa

1DEOm     - - - - -  -  - - - - - - - - - - - - - - - - - - - - - - - - - - - - - - - - - - - - - - - - - - - - - - - - - -

1IVNm     - - - - -  -  - - - - - - - - - - - - - - - - - - - - - - - - - - - - - - - - - - - - - - - - - - - - - - - - - -

P10480m s y v w k  p  f a s r s a s t d s q  l s a f  n  p  q  e  r  l  a  i  a g  n p l l a q a v a s p m a a r s a st

1DEO      - - - - -  -  s y F P I D H T H T S  P A G A  E  V  V  A  E  A  F  L  KA  V V C T G T S L K S V L T T T S F EG
                        h             s?s?s?  h7h7h7h7 h7 h7 h7 h7 h7 h7 h7 h7 h7h7 h7h7h7h7   h?h?h?
1IVN      - - - - -  -  - - M Q D D G I H P N  R D A Q  P  F  I  A  D  W  M  A  K Q  L Q P L V N H D S L E
                            s s       s s s     h7h7h7 h7 h7 h7 h7 h7 h7 h7 h7 h7h7 h7h7h7h7
P10480    s t l n c  e  g k M F W D Q V H P T  T V V H  A  A  L  S  E  P  A  A  T F  I E S Q Y E F L A H -

1DEOm     - - - - s  y  F P I D H T H T S P A  G A E V  V  A  E  A  F  L  K  A  V V  C T G T S L K S V L T T T S F E G TC
                  h               s?s?s?h7h7  h7h7h7h7 h7 h7 h7 h7 h7 h7 h7 h7 h7h7 h7h7   h?h?h?
1IVNm     - - - - -  -  M Q D D G I H P N R D  A Q P F  I  A  D  W  M  A  K  Q  L Q  P L V N H D S L E
                     s  s s  h7h7h7h7h7h7h7h7h7 h7h7h7h7 h7 h7 h7 h7 h7 h7 h7 h7 h7
P10480m l n c e g  k  M F W D Q V H P T T V  V H A A  L  S  E  P  A  A  T  F  I E  S Q Y E F L A H -
```

FIGURE 51

```
                  10        20        30        40        50
60

....*....|....*....|....*....|....*....|....*....|....*....|
1IVN_A        4 LLILGDSLSAG-------------------YRMSASAAWPALLNDKWqsk---
------- 34
P10480       28
IVMFGDSLSDTgkmyskmrgylpssppyyeGRFSNGPVWLEQLTNEFPGLTianeaeggp 87

                  70        80        90       100       110
120

....*....|....*....|....*....|....*....|....*....|....*....|
1IVN_A       35 -tsvVNASISGDT----------------------------
SQQGLARLPALLKQHQPRW 65
P10480       88 tavaYNKISWNPKyq----------------------
vINNLDYEVTQFLQKDSFKPDDL 125

                 130       140       150       160       170
180

....*....|....*....|....*....|....*....|....*....|....*....|
1IVN_A       66 VLVELGGNDG--------------------------------------
LRGFQPQQTEQT 87
P10480      126 VILWVGANDY-------------------------------LA--
YGWNTEQDAKRVRDA 152

                 190       200       210       220       230
240

....*....|....*....|....*....|....*....|....*....|....*....|
1IVN_A       88 LRQILQDVKaANAEPllmqiRLPANYGR-------------------------
------- 115
P10480      153 ISDAANRMV-LNGAK-----EILLFNLPdlg----------------------
----qnP 180

                 250       260       270       280       290
300

....*....|....*....|....*....|....*....|....*....|....*....|
1IVN_A      116 ------------RYNEAFSAIYPKLAke----------------------
fDVPLLPFFME 142
P10480      181 SARSQKVVEAASHVSAYHNQLLLNLArqlaptg----------
mvklfeiDKQFAEMLRD 230

                 310       320       330       340       350
360

....*....|....*....|....*....|....*....|....*....|....*....|
1IVN_A      143 EVYLKPQW---------------------------------------------
------- 150
P10480      231
PQNFGLSDQRNacyggsyvwkpfasrsastdsqlsafnpqerlaiagnpllaqavaspma 290

                 370       380       390       400
                ....*....|....*....|....*....|....*....|
1IVN_A      151 ------------MQDDGI--------HPNRDAQPFIADWM 170
P10480      291 arsastlncegkMFWDQV--------HPTTVVHAALSEPA 322
```

FIGURE 52

```
                      1                                                 50
  P10480        (1)   MKKWFVCLLGLVALTVQAADSRPAFSRIVMFGDSLSDTGKMYSKMRGYLP
  A. sal        (1)   ------------------ADTRPAFSRIVMFGDSLSDTGKMYSKMRGYLP
  A. hyd        (1)   ------------------ADSRPAFSRIVMFGDSLSDTGKMYSKMRGYLP
Consensus       (1)                     AD*RPAFSRIVMFGDSLSDTGKMYSKMRGYLP
                      51                                               100
  P10480       (51)   SSPPYYEGRFSNGPVWLEQLTNEFPGLTIANEAEGGPTAVAYNKISWNPK
  A. sal       (33)   SSPPYYEGRFSNGPVWLEQLTKQFPGLTIANEAEGGATAVAYNKISWNPK
  A. hyd       (33)   SSPPYYEGRFSNGPVWLEQLTKQFPGLTIANEAEGGATAVAYNKISWNPK
Consensus      (51)   SSPPYYEGRFSNGPVWLEQLT**FPGLTIANEAEGG*TAVAYNKISWNPK
                      101                                              150
  P10480      (101)   YQVINNLDYEVTQFLQKDSFKPDDLVILWVGANDYLAYGWNTEQDAKRVR
  A. sal       (83)   YQVINNLDYEVTQFLQKDSFKPDDLVILWVGANDYLAYGWNTEQDAKRVR
  A. hyd       (83)   YQVINNLDYEVTQFLQKDSFKPDDLVILWVGANDYLAYGWNTEQDAKRVR
Consensus     (101)   YQVINNLDYEVTQFLQKDSFKPDDLVILWVGANDYLAYGWNTEQDAKRVR
                      151                                              200
  P10480      (151)   DAISDAANRMVLNGAKEILLFNLPDLGQNPSARSQKVVEAASHVSAYHNQ
  A. sal      (133)   DAISDAANRMVLNGAKQILLFNLPDLGQNPSARSQKVVEAVSHVSAYHNK
  A. hyd      (133)   DAISDAANRMVLNGAKQILLFNLPDLGQNPSARSQKVVEAVSHVSAYHNQ
Consensus     (151)   DAISDAANRMVLNGAK*ILLFNLPDLGQNPSARSQKVVEA*SHVSAYHN*
                      201                                              250
  P10480      (201)   LLLNLARQLAPTGMVKLFEIDKQFAEMLRDPQNFGLSDQRNACYGGSYVW
  A. sal      (183)   LLLNLARQLAPTGMVKLFEIDKQFAEMLRDPQNFGLSDVENPCYDGGYVW
  A. hyd      (183)   LLLNLARQLAPTGMVKLFEIDKQFAEMLRDPQNFGLSDVENPCYDGGYVW
Consensus     (201)   LLLNLARQLAPTGMVKLFEIDKQFAEMLRDPQNFGLSD**N*CY*G*YVW
                      251                                              300
  P10480      (251)   KPFASRSASTDSQLSAFNPQERLAIAGNPLLAQAVASPMAARSASTLNCE
  A. sal      (233)   KPFATRSVSTDRQLSAFSPQERLAIAGNPLLAQAVASPMARRSASPLNCE
  A. hyd      (233)   KPFATRSVSTDRQLSAFSPQERLAIAGNPLLAQAVASPMARRSASPLNCE
Consensus     (251)   KPFA*RS*STD*QLSAF*PQERLAIAGNPLLAQAVASPMA*RSAS*LNCE
                      301                                 336
  P10480      (301)   GKMFWDQVHPTTVVHAALSEPAATFIESQYEFLAH-
  A. sal      (283)   GKMFWDQVHPTTVVHAALSERAATFIETQYEFLAHG
  A. hyd      (283)   GKMFWDQVHPTTVVHAALSERAATFIANQYEFLAH-
Consensus     (301)   GKMFWDQVHPTTVVHAALSE*AATFI**QYEFLAH*
```

FIGURE 53

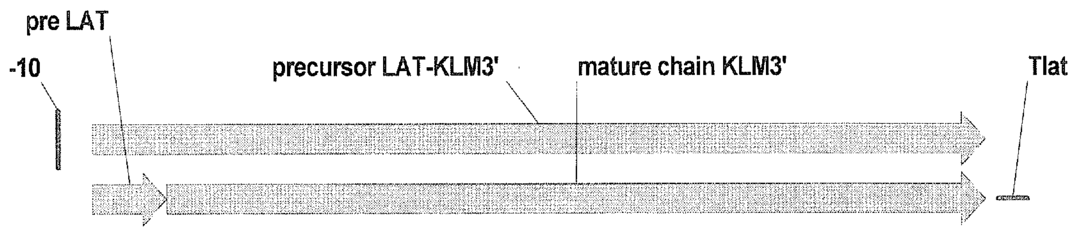


Gene construct for KLM3' expression

1182 bp

FIGURE 54

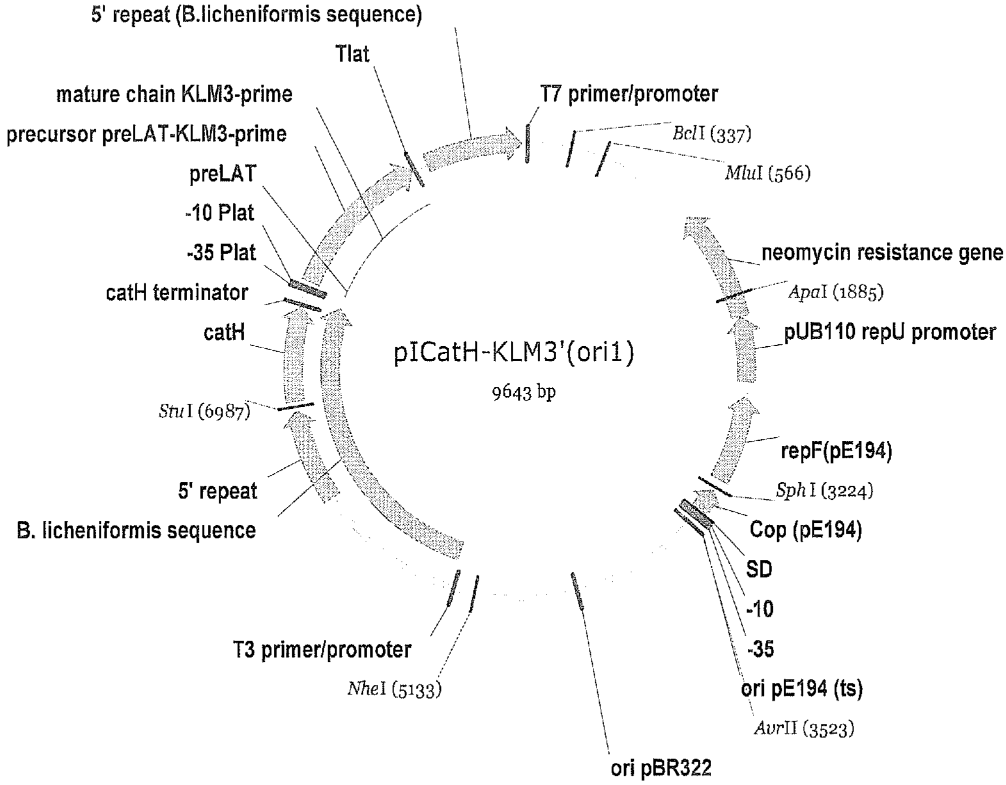
5' repeat (B.licheniformis sequence)
Tlat
mature chain KLM3-prime
precursor preLAT-KLM3-prime
preLAT
-10 Plat
-35 Plat
catH terminator
catH
StuI (6987)
5' repeat
B. licheniformis sequence
T3 primer/promoter
NheI (5133)
ori pBR322
T7 primer/promoter
BclI (337)
MluI (566)
neomycin resistance gene
ApaI (1885)
pUB110 repU promoter
pICatH-KLM3'(ori1)
9643 bp
repF(pE194)
SphI (3224)
Cop (pE194)
SD
-10
-35
ori pE194 (ts)
AvrII (3523)

# FIGURE 55

```
                                                    -35
   1  GCTTTTCTTT TGGAAGAAAA TATAGGGAAA ATGGTACTTG TTAAAAATTC GGAATATTTA
      CGAAAAGAAA ACCTTCTTTT ATATCCCTTT TACCATGAAC AATTTTTAAG CCTTATAAAT
        -10                                       M  K  Q  Q   K  R  L ·
  61  TACAATATCA TATGTTTCAC ATTGAAAGGG GAGGAGAATC ATGAAACAAC AAAAACGGCT
      ATGTTATAGT ATACAAAGTG TAACTTTCCC CTCCTCTTAG TACTTTGTTG TTTTTGCCGA
      · Y  A  R   L  L  T  L   L  F  A   L  I  F   L  L  P  H   S  A  A ·
 121  TTACGCCCGA TTGCTGACGC TGTTATTTGC GCTCATCTTC TTGCTGCCTC ATTCTGCAGC
      AATGCGGGCT AACGACTGCG ACAATAAACG CGAGTAGAAG AACGACGGAG TAAGACGTCG
      · S  A  A   D  T  R  P   A  F  S   R  I  V   M  F  G  D   S  L  S ·
 181  TTCAGCAGCA GATACAAGAC CGGCGTTTAG CCGGATCGTC ATGTTTGGAG ATAGCCTGAG
      AAGTCGTCGT CTATGTTCTG GCCGCAAATC GGCCTAGCAG TACAAACCTC TATCGGACTC
      · D  T  G   K  M  Y  S   K  M  R   G  Y  L   P  S  S  P   P  Y  Y ·
 241  CGATACGGGC AAAATGTATA GCAAAATGAG AGGCTATCTT CCGTCAAGCC CGCCGTATTA
      GCTATGCCCG TTTTACATAT CGTTTTACTC TCCGATAGAA GGCAGTTCGG GCGGCATAAT
      · E  G  R   F  S  N  G   P  V  W   L  E  Q   L  T  K  Q   F  P  G ·
 301  TGAAGGCCGC TTTAGCAATG GACCGGTCTG GCTGGAACAA CTGACGAAAC AATTTCCGGG
      ACTTCCGGCG AAATCGTTAC CTGGCCAGAC CGACCTTGTT GACTGCTTTG TTAAAGGCCC
      · L  T  I   A  N  E  A   E  G  G   A  T  A   V  A  Y  N   K  I  S ·
 361  ACTGACGATC GCTAATGAAG CAGAAGGAGG AGCAACAGCG GTCGCCTATA ACAAAATCAG
      TGACTGCTAG CGATTACTTC GTCTTCCTCC TCGTTGTCGC CAGCGGATAT TGTTTTAGTC
      · W  D  P   K  Y  Q  V   I  N  N   L  D  Y   E  V  T  Q   F  L  Q ·
 421  CTGGGACCCG AAATATCAGG TCATCAACAA CCTGGACTAT GAAGTCACAC AGTTTCTTCA
      GACCCTGGGC TTTATAGTCC AGTAGTTGTT GGACCTGATA CTTCAGTGTG TCAAAGAAGT
      · K  D  S   F  K  P  D   D  L  V   I  L  W   V  G  A  N   D  Y  L ·
 481  GAAAGACAGC TTTAAACCGG ATGATCTGGT CATCCTTTGG GTCGGCGCCA ATGATTATCT
      CTTTCTGTCG AAATTTGGCC TACTAGACCA GTAGGAAACC CAGCCGCGGT TACTAATAGA
      · A  Y  G   W  N  T  E   Q  D  A   K  R  V   R  D  A  I   S  D  A ·
 541  GGCGTATGGC TGGAACACAG AACAAGATGC CAAAAGAGTC AGAGATGCCA TCAGCGATGC
      CCGCATACCG ACCTTGTGTC TTGTTCTACG GTTTTCTCAG TCTCTACGGT AGTCGCTACG
      · A  N  R   M  V  L  N   G  A  K   Q  I  L   L  F  N  L   P  D  L ·
 601  CGCTAATAGA ATGGTCCTGA ACGGCGCCAA ACAAATCCTG CTGTTTAACC TGCCGGATCT
      GCGATTATCT TACCAGGACT TGCCGCGGTT TGTTTAGGAC GACAAATTGG ACGGCCTAGA
      · G  Q  N   P  S  A  R   S  Q  K   V  V  E   A  V  S  H   V  S  A ·
 661  GGGACAAAAT CCGAGCGCCA GAAGCCAAAA AGTCGTCGAA GCAGTCAGCC ATGTCAGCGC
      CCCTGTTTTA GGCTCGCGGT CTTCGGTTTT TCAGCAGCTT CGTCAGTCGG TACAGTCGCG
      · Y  H  N   K  L  L  L   N  L  A   R  Q  L   A  P  T  G   M  V  K ·
 721  CTATCATAAC AAACTGCTGC TGAACCTGGC AAGACAATTG GCACCGACGG GAATGGTTAA
      GATAGTATTG TTTGACGACG ACTTGGACCG TTCTGTTAAC CGTGGCTGCC CTTACCAATT
      · L  F  E   I  D  K  Q   F  A  E   M  L  R   D  P  Q  N   F  G  L ·
 781  ATTGTTTGAA ATTGACAAAC AGTTTGCCGA AATGCTGAGA GATCCGCAAA ATTTTGGCCT
      TAACAAACTT TAACTGTTTG TCAAACGGCT TTACGACTCT CTAGGCGTTT TAAAACCGGA
      · S  D  V   E  N  P  C   Y  D  G   G  Y  V   W  K  P  F   A  T  R ·
 841  GAGCGATGTC GAAAACCCGT GCTATGATGG CGGATATGTC TGGAAACCGT TTGCCACAAG
      CTCGCTACAG CTTTTGGGCA CGATACTACC GCCTATACAG ACCTTTGGCA AACGGTGTTC
      · S  V  S   T  D  R  Q   L  S  A   F  S  P   Q  E  R  L   A  I  A ·
 901  AAGCGTCAGC ACGGATAGAC AACTGTCAGC GTTTAGCCCG CAAGAAAGAC TGGCAATCGC
      TTCGCAGTCG TGCCTATCTG TTGACAGTCG CAAATCGGGC GTTCTTTCTG ACCGTTAGCG
      · G  N  P   L  L  A  Q   A  V  A   S  P  M   A  R  R  S   A  S  P ·
 961  CGGAAATCCG CTTTTGGCAC AAGCAGTTGC TTCACCGATG GCAAGAAGAT CAGCAAGCCC
      GCCTTTAGGC GAAAACCGTG TTCGTCAACG AAGTGGCTAC CGTTCTTCTA GTCGTTCGGG
      · L  N  C   E  G  K  M   F  W  D   Q  V  H   P  T  T  V   V  H  A ·
1021  GCTGAATTGC GAAGGCAAAA TGTTTTGGGA TCAGGTCCAT CCGACAACAG TTGTCCATGC
      CGACTTAACG CTTCCGTTTT ACAAAACCCT AGTCCAGGTA GGCTGTTGTC AACAGGTACG
      · A  L  S   E  R  A  A   T  F  I   E  T  Q   Y  E  F  L   A  H  G ·
1081  TGCCCTTTCA GAAAGAGCGG CGACGTTTAT CGAAACACAG TATGAATTTC TGGCCCATGG
      ACGGGAAAGT CTTTCTCGCC GCTGCAAATA GCTTTGTGTC ATACTTAAAG ACCGGGTACC
      ·stop
1141  CTGAGTTAAC AGAGGACGGA TTTCCTGAAG GAAATCCGTT TTTTTATTTT AAGCTTGGAG
      GACTCAATTG TCTCCTGCCT AAAGGACTTC CTTTAGGCAA AAAAATAAAA TTCGAACCTC
1201  ACAAGGTAAA GGATAAAACC TCGAG
      TGTTCCATTT CCTATTTTGG AGCTC
```

FIGURE 56

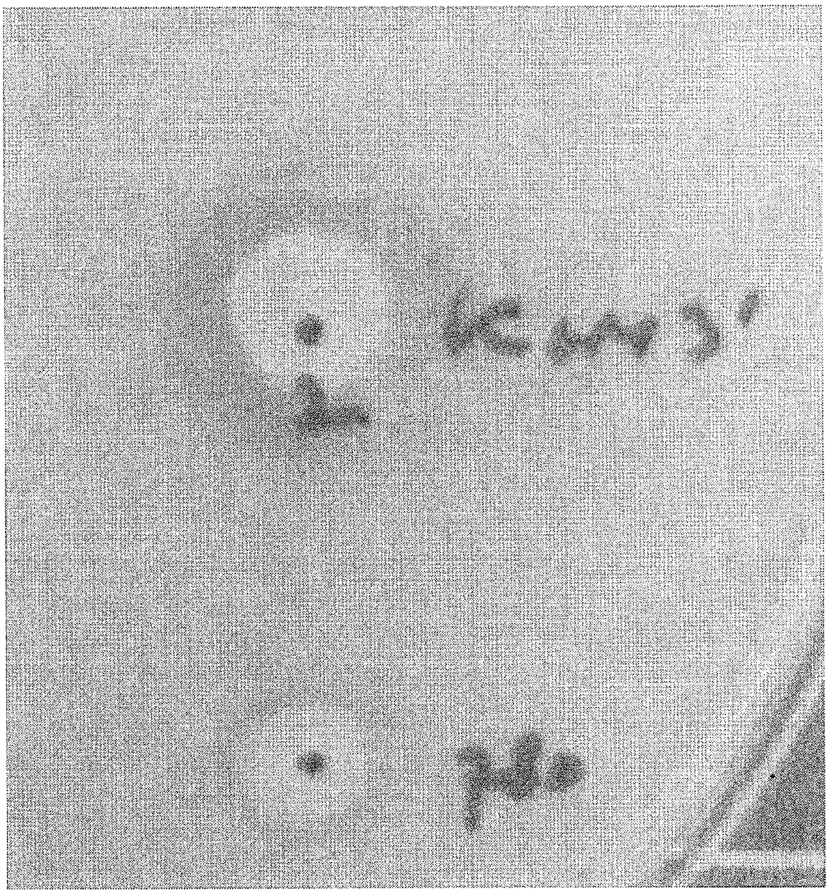

**FIGURE 57** (SEQ ID No 49)

```
   1  ATGAAACAAC AAAAACGGCT TTACGCCCGA TTGCTGACGC TGTTATTTGC
      TACTTTGTTG TTTTTGCCGA AATGCGGGCT AACGACTGCG ACAATAAACG

  51  GCTCATCTTC TTGCTGCCTC ATTCTGCAGC TTCAGCAGCA GATACAAGAC
      CGAGTAGAAG AACGACGGAG TAAGACGTCG AAGTCGTCGT CTATGTTCTG

 101  CGGCGTTTAG CCGGATCGTC ATGTTTGGAG ATAGCCTGAG CGATACGGGC
      GCCGCAAATC GGCCTAGCAG TACAAACCTC TATCGGACTC GCTATGCCCG

 151  AAAATGTATA GCAAAATGAG AGGCTATCTT CCGTCAAGCC CGCCGTATTA
      TTTTACATAT CGTTTTACTC TCCGATAGAA GGCAGTTCGG GCGGCATAAT

 201  TGAAGGCCGC TTTAGCAATG GACCGGTCTG GCTGGAACAA CTGACGAAAC
      ACTTCCGGCG AAATCGTTAC CTGGCCAGAC CGACCTTGTT GACTGCTTTG

 251  AATTTCCGGG ACTGACGATC GCTAATGAAG CAGAAGGAGG AGCAACAGCG
      TTAAAGGCCC TGACTGCTAG CGATTACTTC GTCTTCCTCC TCGTTGTCGC

 301  GTCGCCTATA ACAAAATCAG CTGGGACCCG AAATATCAGG TCATCAACAA
      CAGCGGATAT TGTTTTAGTC GACCCTGGGC TTTATAGTCC AGTAGTTGTT

 351  CCTGGACTAT GAAGTCACAC AGTTTCTTCA GAAAGACAGC TTTAAACCGG
      GGACCTGATA CTTCAGTGTG TCAAAGAAGT CTTTCTGTCG AAATTTGGCC

 401  ATGATCTGGT CATCCTTTGG GTCGGCGCCA ATGATTATCT GGCGTATGGC
      TACTAGACCA GTAGGAAACC CAGCCGCGGT TACTAATAGA CCGCATACCG

 451  TGGAACACAG AACAAGATGC CAAAAGAGTC AGAGATGCCA TCAGCGATGC
      ACCTTGTGTC TTGTTCTACG GTTTTCTCAG TCTCTACGGT AGTCGCTACG

 501  CGCTAATAGA ATGGTCCTGA ACGGCGCCAA ACAAATCCTG CTGTTTAACC
      GCGATTATCT TACCAGGACT TGCCGCGGTT TGTTTAGGAC GACAAATTGG

 551  TGCCGGATCT GGGACAAAAT CCGAGCGCCA GAAGCCAAAA AGTCGTCGAA
      ACGGCCTAGA CCCTGTTTTA GGCTCGCGGT CTTCGGTTTT TCAGCAGCTT

 601  GCAGTCAGCC ATGTCAGCGC CTATCATAAC AAACTGCTGC TGAACCTGGC
      CGTCAGTCGG TACAGTCGCG GATAGTATTG TTTGACGACG ACTTGGACCG

 651  AAGACAATTG GCACCGACGG GAATGGTTAA ATTGTTTGAA ATTGACAAAC
      TTCTGTTAAC CGTGGCTGCC CTTACCAATT TAACAAACTT TAACTGTTTG

 701  AGTTTGCCGA AATGCTGAGA GATCCGCAAA ATTTTGGCCT GAGCGATGTC
      TCAAACGGCT TTACGACTCT CTAGGCGTTT TAAAACCGGA CTCGCTACAG

 751  GAAAACCCGT GCTATGATGG CGGATATGTC TGGAAACCGT TTGCCACAAG
      CTTTTGGGCA CGATACTACC GCCTATACAG ACCTTTGGCA AACGGTGTTC

 801  AAGCGTCAGC ACGGATAGAC AACTGTCAGC GTTTAGCCCG CAAGAAAGAC
      TTCGCAGTCG TGCCTATCTG TTGACAGTCG CAAATCGGGC GTTCTTTCTG

 851  TGGCAATCGC CGGAAATCCG CTTTTGGCAC AAGCAGTTGC TTCACCGATG
      ACCGTTAGCG GCCTTTAGGC GAAAACCGTG TTCGTCAACG AAGTGGCTAC

 901  GCAAGAAGAT CAGCAAGCCC GCTGAATTGC GAAGGCAAAA TGTTTTGGGA
      CGTTCTTCTA GTCGTTCGGG CGACTTAACG CTTCCGTTTT ACAAAACCCT

 951  TCAGGTCCAT CCGACAACAG TTGTCCATGC TGCCCTTTCA GAAAGAGCGG
      AGTCCAGGTA GGCTGTTGTC AACAGGTACG ACGGGAAAGT CTTTCTCGCC

1001  CGACGTTTAT CGAAACACAG TATGAATTTC TGGCCCATGG CTGA
      GCTGCAAATA GCTTTGTGTC ATACTTAAAG ACCGGGTACC GACT
```

## FIGURE 58 (SEQ ID No. 50)

```
  1  ATGAAAAAAT GGTTTGTGTG TTTATTGGGA TTGGTCGCGC TGACAGTTCA GGCAGCCGAC
 61  AGCCGTCCCG CCTTCTCCCG GATCGTGATG TTTGGCGACA GCCTCTCCGA TACCGGCAAG
121  ATGTACAGCA AGATGCGCGG TTACCTCCCC TCCAGCCCCC CCTACTATGA GGGCCGCTTC
181  TCCAACGGGC CCGTCTGGCT GGAGCAGCTG ACCAACGAGT TCCCGGGCCT GACCATAGCC
241  AACGAGGCGG AAGGCGGACC GACCGCCGTG GCTTACAACA AGATCTCCTG GAATCCCAAG
301  TATCAGGTCA TCAACAACCT GGACTACGAG GTCACCCAGT TCCTGCAAAA AGACAGCTTC
361  AAGCCGGACG ATCTGGTGAT CCTCTGGGTC GGCGCCAACG ACTATCTGGC CTATGGCTGG
421  AACACAGAGC AGGATGCCAA GCGGGTGCGC GACGCCATCA GCGATGCGGC CAACCGCATG
481  GTGCTGAACG GCGCCAAGGA GATACTGCTG TTCAACCTGC CGGATCTGGG CCAGAACCCC
541  TCGGCCCGCA GCCAGAAGGT GGTCGAGGCG GCCAGCCATG TCTCCGCCTA CCACAACCAG
601  CTGCTGCTGA ACCTGGCACG CCAGCTGGCT CCCACCGGCA TGGTGAAGCT GTTCGAGATC
661  GACAAGCAGT TTGCCGAGAT GCTGCGTGAT CCGCAGAACT TCGGCCTGAG CGACCAGAGG
721  AACGCCTGCT ACGGTGGCAG CTATGTATGG AAGCCGTTTG CCTCCCGCAG CGCCAGCACC
781  GACAGCCAGC TCTCCGCCTT CAACCCGCAG GAGCGCCTCG CCATCGCCGG CAACCCGCTG
841  CTGGCCCAGG CCGTCGCCAG CCCCATGGCT GCCCGCAGCG CCAGCACCCT CAACTGTGAG
901  GGCAAGATGT TCTGGGATCA GGTCCACCCC ACCACTGTCG TGCACGCCGC CCTGAGCGAG
961  CCCGCCGCCA CCTTCATCGA GAGCCAGTAC GAGTTCCTCG CCCAC
```

## FIGURE 59 (SEQ ID No. 51)

```
  1  ATGAAAAAAT GGTTTGTTTG TTTATTGGGG TTGATCGCGC TGACAGTTCA GGCAGCCGAC
 61  ACTCGCCCCG CCTTCTCCCG GATCGTGATG TTCGGCGACA GCCTCTCCGA TACCGGCAAA
121  ATGTACAGCA AGATGCGCGG TTACCTCCCC TCCAGCCCGC CCTACTATGA GGGCCGTTTC
181  TCCAACGGAC CCGTCTGGCT GGAGCAGCTG ACCAAGCAGT TCCCGGGTCT GACCATCGCC
241  AACGAAGCGG AAGGCGGTGC CACTGCCGTG GCTTACAACA AGATCTCCTG GAATCCCAAG
301  TATCAGGTCT ACAACAACCT GGACTACGAG GTCACCCAGT TCTTGCAGAA AGACAGCTTC
361  AAGCCGGACG ATCTGGTGAT CCTCTGGGTC GGTGCCAATG ACTATCTGGC ATATGGCTGG
421  AATACGGAGC AGGATGCCAA GCGAGTTCGC GATGCCATCA GCGATGCGGC CAACCGCATG
481  GTACTGAACG GTGCCAAGCA GATACTGCTG TTCAACCTGC CGGATCTGGG CCAGAACCCG
541  TCAGCCCGCA GTCAGAAGGT GGTCGAGGCG GTCAGCCATG TCTCCGCCTA TCACAACAAG
601  CTGCTGCTGA ACCTGGCACG CCAGCTGGCC CCCACCGGCA TGGTAAAGCT GTTCGAGATC
661  GACAAGCAAT TTGCCGAGAT GCTGCGTGAT CCGCAGAACT TCGGCCTGAG CGACGTCGAG
721  AACCCCTGCT ACGACGGCGG CTATGTGTGG AAGCCGTTTG CCACCCGCAG CGTCAGCACC
781  GACCGCCAGC TCTCCGCCTT CAGTCCGCAG GAACGCCTCG CCATCGCCGG CAACCCGCTG
841  CTGGCACAGG CCGTTGCCAG TCCTATGGCC CGCCGCAGCG CCAGCCCCCT CAACTGTGAG
901  GGCAAGATGT TCTGGGATCA GGTACACCCG ACCACTGTCG TGCACGCAGC CCTGAGCGAG
961  CGCGCCGCCA CCTTCATCGA GACCCAGTAC GAGTTCCTCG CCCACGGATG A
```

## FIGURE 60 (SEQ ID No. 52)

```
  1  ATGCCGAAGC CTGCCCTTCG CCGTGTCATG ACCGCGACAG TCGCCGCCGT CGGCACGCTC
 61  GCCCTCGGCC TCACCGACGC CACCGCCCAC GCCGCGCCCG CCCAGGCCAC TCCGACCCTG
121  GACTACGTCG CCCTCGGCGA CAGCTACAGC GCCGGCTCCG GCGTCCTGCC CGTCGACCCC
181  GCCAACCTGC TCTGTCTGCG CTCGACGGCC AACTACCCCC ACGTCATCGC GGACACGACG
241  GGCGCCCGCC TCACGGACGT CACCTGCGGC GCCGCGCAGA CCGCCGACTT CACGCGGGCC
301  CAGTACCCGG GCGTCGCACC CCAGTTGGAC GCGCTCGGCA CCGGCACGGA CCTGGTCACG
361  CTCACCATCG GCGGCAACGA CAACAGCACC TTCATCAACG CCATCACGGC CTGCGGCACG
421  GCGGGTGTCC TCAGCGGCGG CAAGGGCAGC CCCTGCAAGG ACAGGCACGG CACCTCCTTC
481  GACGACGAGA TCGAGGCCAA CACGTACCCC GCGCTCAAGG AGGCGCTGCT CGGCGTCCGC
541  GCCAGGGCTC CCCACGCCAG GGTGGCGGCT CTCGGCTACC CGTGGATCAC CCCGGCCACC
601  GCCGACCCGT CCTGCTTCCT GAAGCTCCCC CTCGCCGCCG GTGACGTGCC CTACCTGCGG
661  GCCATCCAGG CACACCTCAA CGACGCGGTC CGGCGGGCCG CCGAGGAGAC CGGAGCCACC
721  TACGTGGACT TCTCCGGGGT GTCCGACGGC CACGACGCCT GCGAGGCCCC CGGCACCCGC
781  TGGATCGAAC CGCTGCTCTT CGGGCACAGC CTCGTTCCCG TCCACCCCAA CGCCCTGGGC
841  GAGCGGCGCA TGGCCGAGCA CACGATGGAC GTCCTCGGCC TGGACTGA
```

## FIGURE 61 (SEQ ID No. 53)

```
  1  TCAGTCCAGG CCGAGGACGT CCATCGTGTG CTCGGCCATG CGCCGCTCGC CCAGGGCGTT
 61  GGGGTGGACG GGAACGAGGC TGTGCCCGAA GAGCAGCGGT TCGATCCAGC GGGTGCCGGG
121  GGCCTCGCAG GCGTCGTGGC CGTCGGACAC CCCGGAGAAG TCCACGTAGG TGGCTCCGGT
181  CTCCTCGGCG GCCCGCCGGA CCGCGTCGTT GAGGTGTGCC TGGATGGCCC GCAGGTAGGG
241  CACGTCACCG GCGGCGAGGG GGAGCTTCAG GAAGCAGGAC GGGTCGGCGG TGGCCGGGGT
301  GATCCACGGG TAGCCGAGAG CCGCCACCCT GGCGTGGGGA GCCCTGGCGC GGACGCCGAG
361  CAGCGCCTCC TTGAGCGCGG GGTACGTGTT GGCCTCGATC TCGTCGTCGA AGGAGGTGCC
421  GTGCCTGTCC TTGCAGGGGC TGCCCTTGCC GCCGCTGAGG ACACCCGCCG TGCCGCAGGC
481  CGTGATGGCG TTGATGAAGG TGCTGTTGTC GTTGCCGCCG ATGGTGAGCG TGACCAGGTC
541  CGTGCCGGTG CCGAGCGCGT CCAACTGGGG TGCGACGCCC GGGTACTGGG CCCGCGTGAA
601  GTCGGCGGTC TGCGCGGCGC CGCAGGTGAC GTCCGTGAGG CGGGCGCCCG TCGTGTCCGC
661  GATGACGTGG GGGTAGTTGG CCGTCGAGCG CAGACAGAGC AGGTTGGCGG GGTCGACGGG
721  CAGGACGCCG GAGCCGGCGC TGTAGCTGTC GCCGAGGGCG ACGTAGTCCA GGGTCGGAGT
781  GGCCTGGGCG GGCGCGGCGT GGGCGGTGGC GTCGGTGAGG CCGAGGGCGA GCGTGCCGAC
841  GGCGGCGACT GTCGCGGTCA TGACACGGCG AAGGGCAGGC TTCGGCAT
```

## FIGURE 62 (SEQ ID No. 54)

```
  1  ATGGATTACG AGAAGTTTCT GTTATTTGGG GATTCCATTA CTGAATTTGC TTTTAATACT
 61  AGGCCCATTG AAGATGGCAA AGATCAGTAT GCTCTTGGAG CCGCATTAGT CAACGAATAT
121  ACGAGAAAAA TGGATATTCT TCAAAGAGGG TTCAAAGGGT ACACTTCTAG ATGGGCGTTG
181  AAAATACTTC CTGAGATTTT AAAGCATGAA TCCAATATTG TCATGGCCAC AATATTTTTG
241  GGTGCCAACG ATGCATGCTC AGCAGGTCCC CAAAGTGTCC CCCTCCCCGA ATTTATCGAT
301  AATATTCGTC AAATGGTATC TTTGATGAAG TCTTACCATA TCCGTCCTAT TATAATAGGA
361  CCGGGGCTAG TAGATAGAGA GAAGTGGGAA AAAGAAAAAT CTGAAGAAAT AGCTCTCGGA
421  TACTTCCGTA CCAACGAGAA CTTTGCCATT TATTCCGATG CCTTAGCAAA ACTAGCCAAT
481  GAGGAAAAAG TTCCCTTCGT GGCTTTGAAT AAGGCGTTTC AACAGGAAGG TGGTGATGCT
541  TGGCAACAAC TGCTAACAGA TGGACTGCAC TTTTCCGGAA AAGGGTACAA AATTTTTCAT
601  GACGAATTAT TGAAGGTCAT TGAGACATTC TACCCCCAAT ATCATCCCAA AAACATGCAG
661  TACAAACTGA AAGATTGGAG AGATGTGCTA GATGATGGAT CTAACATAAT GTCTTGA
```

## FIGURE 63 (SEQ ID No. 55)

```
atgaacctgc gtcaatggat gggcgccgcc acggctgccc ttgccttggg cttggccgcg     60
tgcgggggcg gtgggaccga ccagagcggc aatcccaatg tcgccaaggt gcagcgcatg    120
gtggtgttcg gcgacagcct gagcgatatc ggcacctaca cccccgtcgc gcaggcggtg    180
ggcggcggca agttcaccac caacccgggc ccgatctggg ccgagaccgt ggccgcgcaa    240
ctgggcgtga cgctcacgcc ggcggtgatg ggctacgcca cctccgtgca gaattgcccc    300
aaggccggct gcttcgacta tgcgcagggc ggctcgcgcg tgaccgatcc gaacggcatc    360
ggccacaacg gcggcgcggg ggcgctgacc tacccggttc agcagcagct cgccaacttc    420
tacgcggcca gcaacaacac attcaacggc aataacgatg tcgtcttcgt gctggccggc    480
agcaacgaca ttttcttctg gaccactgcg gcggccacca gcggctccgg cgtgacgccc    540
gccattgcca cggcccaggt gcagcaggcc gcgacggacc tggtcggcta tgtcaaggac    600
atgatcgcca agggtgcgac gcaggtctac gtgttcaacc tgcccgacag cagcctgacg    660
ccggacggcg tggcaagcgg cacgaccggc caggcgctgc tgcacgcgct ggtgggcacg    720
ttcaacacga cgctgcaaag cgggctggcc ggcacctcgg cgcgcatcat cgacttcaac    780
gcacaactga ccgcggcgat ccagaatggc gcctcgttcg gcttcgccaa caccagcgcc    840
cgggcctgcg acgccaccaa gatcaatgcc ctggtgccga gcgccggcgg cagctcgctg    900
ttctgctcgg ccaacacgct ggtggcttcc ggtgcggacc agagctacct gttcgccgac    960
ggcgtgcacc cgaccacggc cggccatcgc ctgatcgcca gcaacgtgct ggcgcgcctg   1020
ctggcggata acgtcgcgca ctga                                          1044
```

## FIGURE 64 (SEQ ID No. 56)

```
  1 gtgatcgggt cgtacgtggc ggtgggggac agcttcaccg agggcgtcgg cgaccccggc
 61 cccgacgggg cgttcgtcgg ctgggccgac cggctcgccg tactgctcgc ggaccggcgc
121 cccgagggcg acttcacgta cacgaacctc gccgtgcgcg gcaggctcct cgaccagatc
181 gtggcggaac aggtcccgcg ggtcgtcgga ctcgcgcccg acctcgtctc gttcgcggcg
241 ggcggcaacg acatcatccg gcccggcacc gatcccgacg aggtcgccga gcggttcgag
301 ctggcggtgg ccgcgctgac cgccgcggcc ggaaccgtcc tggtgaccac cgggttcgac
361 acccgggggg tgcccgtcct caagcacctg cgcggcaaga tcgccacgta caacgggcac
421 gtccgcgcca tcgccgaccg ctacggctgc ccggtgctcg acctgtggtc gctgcggagc
481 gtccaggacc gcagggcgtg ggacgccgac cggctgcacc tgtcgccgga ggggcacacc
541 cgggtggcgc tgcgcgcggg gcaggccctg ggcctgcgcg tcccggccga ccctgaccag
601 ccctggccgc ccctgccgcc gcgcggcacg ctcgacgtcc ggcgcgacga cgtgcactgg
661 gcgcgcgagt acctggtgcc gtggatcggg cgccggctgc ggggcgagtc gtcgggcgac
721 cacgtgacgg ccaaggggac gctgtcgccg gacgccatca agacgcggat cgccgcggtg
781 gcctga
```

## FIGURE 65 (SEQ ID No. 57)

```
  1 atgcagacga accccgcgta caccagtctc gtcgccgtcg gcgactcctt caccgagggc
 61 atgtcggacc tgctgcccga cggctcctac cgtggctggg ccgacctcct cgccacccgg
121 atggcggccc gctcccccgg cttccggtac gccaacctgg cggtgcgcgg gaagctgatc
181 ggacagatcg tcgacgagca ggtggacgtg gccgccgcca tgggagccga cgtgatcacg
241 ctggtcggcg ggctcaacga cacgctgcgg cccaagtgcg acatggcccg ggtgcgggac
301 ctgctgaccc aggccgtgga acggctcgcc ccgcactgcg agcagctggt gctgatgcgc
361 agtcccggtc gccagggtcc ggtgctggag cgcttccggc cccgcatgga ggccctgttc
421 gccgtgatcg acgacctggc cgggcggcac ggcgccgtgg tcgtcgacct gtacggggcc
481 cagtcgctgg ccgaccctcg gatgtgggac gtggaccggc tgcacctgac cgccgagggc
541 caccgccggg tcgcggaggc ggtgtggcag tcgctcggcc acgagcccga ggaccccgag
601 tggcacgcgc cgatcccggc gacgccgccg ccggggtggg tgacgcgcag gaccgcggac
661 gtccggttcg cccggcagca cctgctgccc tggataggcc gcaggctgac cggcgctcg
721 tccggggacg gcctgccggc caagcgcccg gacctgctgc cctacgagga ccccgcacgg
781 tga
```

## FIGURE 66 (SEQ ID No. 58)

```
   1 atgacccggg gtcgtgacgg gggtgcgggg gcgcccccca ccaagcaccg tgccctgctc
  61 gcggcgatcg tcaccctgat agtggcgatc tccgcggcca tatacgccgg agcgtccgcg
 121 gacgacggca gcagggacca cgcgctgcag gccggaggcc gtctcccacg aggagacgcc
 181 gcccccgcgt ccaccggtgc ctgggtgggc gcctgggcca ccgcaccggc cgcggccgag
 241 ccgggcaccg agacgaccgg cctggcgggc cgctccgtgc gcaacgtcgt gcacacctcg
 301 gtcggcggca ccggcgcgcg gatcaccctc tcgaacctgt acgggcagtc gccgctgacc
 361 gtcacacacg cctcgatcgc cctggccgcc gggcccgaca ccgccgccgc gatcgccgac
 421 accatgcgcc ggctcacctt cggcggcagc gcccgggtga tcatcccggc gggcggccag
 481 gtgatgagcg acaccgcccg cctcgccatc ccctacgggg cgaacgtcct ggtcaccacg
 541 tactccccca tcccgtccgg gccggtgacc taccatccgc aggcccggca gaccagctac
 601 ctggccgacg gcgaccgcac ggcggacgtc accgccgtcg cgtacaccac ccccacgccc
 661 tactggcgct acctgaccgc cctcgacgtg ctgagccacg aggccgacgg cacggtcgtg
 721 gcgttcggcg actccatcac cgacggcgcc cgctcgcaga gcgacgccaa ccaccgctgg
 781 accgacgtcc tcgccgcacg cctgcacgag gcggcgggcg acggccggga cacgccccgc
 841 tacagcgtcg tcaacgaggg catcagcggc aaccggctcc tgaccagcag gccggggcgg
 901 ccggccgaca acccgagcgg actgagccgg ttccagcggg acgtgctgga acgcaccaac
 961 gtcaaggccg tcgtcgtcgt cctcggcgtc aacgacgtcc tgaacagccc ggaactcgcc
1021 gaccgcgacg ccatcctgac cggcctgcgc accctcgtcg accgggcgca cgcccgggga
1081 ctgcgggtcg tcggcgccac gatcacgccg ttcggcggct acggcggcta caccgaggcc
1141 cgcgagacga tgcggcagga ggtcaacgag gagatccgct ccggccgggt cttcgacacg
1201 gtcgtcgact tcgacaaggc cctgcgcgac ccgtacgacc cgcgccggat gcgctccgac
1261 tacgacagcg gcgaccacct gcaccccggc gacaaggggt acgcgcgcat gggcgcggtc
1321 atcgacctgg ccgcgctgaa gggcgcggcg ccggtcaagg cgtag
```

## FIGURE 67 (SEQ ID No. 59)

```
   1 atgacgagca tgtcgagggc gagggtggcg cggcggatcg cggccggcgc ggcgtacggc
  61 ggcggcggca tcggcctggc gggagcggcg gcggtcggtc tggtggtggc cgaggtgcag
 121 ctggccagac gcagggtggg ggtgggcacg ccgacccggg tgccgaacgc gcagggactg
 181 tacggcggca ccctgcccac ggccggcgac ccgccgctgc ggctgatgat gctgggcgac
 241 tccacggccg ccgggcaggg cgtgcaccgg gccgggcaga cgccgggcgc gctgctggcg
 301 tccgggctcg cggcggtggc ggagcggccg gtgcggctgg ggtcggtcgc ccagccgggg
 361 gcgtgctcgg acgacctgga ccggcaggtg gcgctggtgc tcgccgagcc ggaccgggtg
 421 cccgacatct gcgtgatcat ggtcggcgcc aacgacgtca cccaccggat gccggcgacc
 481 cgctcggtgc ggcacctgtc ctcggcggta cggcggctgc gcacggccgg tgcggaggtg
 541 gtggtcggca cctgtccgga cctgggcacg atcgagcggg tgcggcagcc gctgcgctgg
 601 ctggcccggc gggcctcacg gcagctcgcg gcggcacaga ccatcggcgc cgtcgagcag
 661 ggcgggcgca cggtgtcgct gggcgacctg ctgggtccgg agttcgcgca gaacccgcgg
 721 gagctcttcg gccccgacaa ctaccacccc tccgccgagg ggtacgccac ggccgcgatg
 781 gcggtactgc cctcggtgtg cgccgcgctc ggcctgtggc cggccgacga ggagcacccg
 841 gacgcgctgc gccgcgaggg cttcctgccg gtggcgcgcg cggcggcgga ggcggcgtcc
 901 gaggcgggta cggaggtcgc cgccgccatg cctacggggc ctcgggggcc ctgggcgctg
 961 ctgaagcgcc ggagacggcg tcgggtgtcg gaggcggaac cgtccagccc gtccggcgtt
1021 tga
```

## FIGURE 68 (SEQ ID No. 60)

```
   1 atgggtcgag ggacggacca gcggacgcgg tacggccgtc gccgggcgcg tgtcgcgctc
  61 gccgccctga ccgccgccgt cctgggcgtg ggcgtggcgg gctgcgactc cgtgggcggc
 121 gactcacccg ctccttccgg cagcccgtcg aagcggacga ggacggcgcc cgcctgggac
 181 accagcccgg cgtccgtcgc cgccgtgggc gactccatca cgcgcggctt cgacgcctgt
 241 gcggtgctgt cggactgccc ggaggtgtcg tgggcgaccg gcagcagcgc gaaggtcgac
 301 tcgctggccg tacggctgct ggggaaggcg gacgcggccg agcacagctg gaactacgcg
 361 gtcaccgggg cccggatggc ggacctgacc gctcaggtga cgcgggcggc gcagcgcgag
 421 ccggagctgg tggcggtgat ggccggggcg aacgacgcgt gccggtccac gacctcggcg
 481 atgacgccgg tggcggactt ccgggcgcag ttcgaggagg cgatggccac cctgcgcaag
 541 aagctcccca aggcgcaggt gtacgtgtcg agcatcccgg acctcaagcg gctctggtcc
 601 cagggccgca ccaacccgct gggcaagcag gtgtggaagc tcggcctgtg cccgtcgatg
 661 ctgggcgacg cggactccct ggactcggcg gcgaccctgc ggcgcaacac ggtgcgcgac
 721 cgggtggcgg actacaacga ggtgctgcgg gaggtctgcg cgaaggaccg gcggtgccgc
 781 agcgacgacg gcgcggtgca cgagttccgg ttcggcacgg accagttgag ccactgggac
 841 tggttccacc cgagtgtgga cggccaggcc cggctggcgg agatcgccta ccgcgcggtc
 901 accgcgaaga atccctga
```

## FIGURE 69 (SEQ ID No. 61)

```
   1 ttcatcacaa cgatgtcaca acaccggcca tccgggtcat ccctgatcgt gggaatgggt
  61 gacaagcctt cccgtgacga aagggtcctg ctacatcaga aatgacagaa atcctgctca
 121 gggaggttcc atgagactgt cccgacgcgc ggccacggcg tccgcgctcc tcctcacccc
 181 ggcgctcgcg ctcttcggcg cgagcgccgc cgtgtccgcg ccgcgaatcc aggccaccga
 241 ctacgtggcc ctcggcgact cctactcctc gggggtcggc gcgggcagct acgacagcag
 301 cagtggctcc tgtaagcgca gcaccaagtc ctacccggcc ctgtgggccg cctcgcacac
 361 cggtacgcgg ttcaacttca ccgcctgttc gggcgcccgc acaggagacg tgctggccaa
 421 gcagctgacc ccggtcaact ccggcaccga cctggtcagc attaccatcg gcggcaacga
 481 cgcgggcttc gccgacacca tgaccacctg caacctccag ggcgagagcg cgtgcctggc
 541 gcggatcgcc aaggcgcgcg cctacatcca gcagacgctg cccgcccagc tggaccaggt
 601 ctacgacgcc atcgacagcc gggcccccgc agcccaggtc gtcgtcctgg gctacccgcg
 661 cttctacaag ctgggcggca gctgcgccgt cggtctctcg gagaagtccc gcgcggccat
 721 caacgccgcc gccgacgaca tcaacgccgt caccgccaag cgcgccgccg accacggctt
 781 cgccttcggg gacgtcaaca cgaccttcgc cgggcacgag ctgtgctccg gcgcccccctg
 841 gctgcacagc gtcacccttc ccgtggagaa ctcctaccac cccacggcca acggacagtc
 901 caagggctac ctgcccgtcc tgaactccgc cacctgatct cgcggctact ccgcccctga
 961 cgaagtcccg cccccgggcg gggcttcgcc gtaggtgcgc gtaccgccgt cgcccgtcgc
1021 gccggtggcc ccgccgtacg tgccgccgcc cccggacgcg gtcggttc
```

# FIGURE 70 (SEQ ID No. 62)

```
   1  ATGAAAAAAT GGTTTGTGTG TTTATTGGGA TTGGTCGCGC TGACAGTTCA
      TACTTTTTTA CCAAACACAC AAATAACCCT AACCAGCGCG ACTGTCAAGT

  51  GGCAGCCGAC AGTCGCCCCG CCTTTTCCCG GATCGTGATG TTCGGCGACA
      CCGTCGGCTG TCAGCGGGGC GGAAAAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAACAGT TCCCGGGTCT GACCATCGCC AACGAAGCGG
      CCTCGTCGAC TGGTTTGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC CTATGGCTGG AACACGGAGC AGGATGCCAA GCGGGTTCGC
      TGATAGACCG GATACCGACC TTGTGCCTCG TCCTACGGTT CGCCCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCTCGCA
      CTATGACGAC AAGTTGGACG GCCTAGACCC GGTCTTGGGC AGTCGAGCGT

 551  GTCAGAAGGT GGTCGAGGCG GTCAGCCATG TCTCCGCCTA TCACAACCAG
      CAGTCTTCCA CCAGCTCCGC CAGTCGGTAC AGAGGCGGAT AGTGTTGGTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGC GAACCAGTAC GAGTTCCTCG
      GGACTCGCTC GCGCGGCGGT GGAAGTAGCG CTTGGTCATG CTCAAGGAGC

1001  CCCAC TGA
      GGGTG ACT
```

# FIGURE 71 (SEQ ID No. 63)

```
   1  ATGAAAAAAT GGTTTGTTTG TTTATTGGGG TTGATCGCGC TGACAGTTCA
      TACTTTTTTA CCAAACAAAC AAATAACCCC AACTAGCGCG ACTGTCAAGT

  51  GGCAGCCGAC ACTCGCCCCG CCTTCTCCCG GATCGTGATG TTCGGCGACA
      CCGTCGGCTG TGAGCGGGGC GGAAGAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAGCAGT TCCCGGGTCT GACCATCGCC AACGAAGCGG
      CCTCGTCGAC TGGTTCGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC ATATGGCTGG AATACGGAGC AGGATGCCAA GCGAGTTCGC
      TGATAGACCG TATACCGACC TTATGCCTCG TCCTACGGTT CGCTCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCCCGCA
      CTATGACGAC AAGTTGGACG GCCTAGACCC GGTCTTGGGC AGTCGGGCGT

 551  GTCAGAAGGT GGTCGAGGCG GTCAGCCATG TCTCCGCCTA TCACAACAAG
      CAGTCTTCCA CCAGCTCCGC CAGTCGGTAC AGAGGCGGAT AGTGTTGTTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGA GACCCAGTAC GAGTTCCTCG
      GGACTCGCTC GCGCGGCGGT GGAAGTAGCT CTGGGTCATG CTCAAGGAGC

1001  CCCACGGATG A
      GGGTGCCTAC T
```

# FIGURE 72 (SEQ ID No. 24)

```
   1 ATGTTTAAGT TTAAAAAGAA TTTCTTAGTT GGATTATCGG CAGCTTTAAT
     TACAAATTCA AATTTTTCTT AAAGAATCAA CCTAATAGCC GTCGAAATTA

  51 GAGTATTAGC TTGTTTTCGG CAACCGCCTC TGCAGCTAGC GCCGACAGCC
     CTCATAATCG AACAAAAGCC GTTGGCGGAG ACGTCGATCG CGGCTGTCGG

 101 GTCCCGCCTT TTCCCGGATC GTGATGTTCG GCGACAGCCT CTCCGATACC
     CAGGGCGGAA AAGGGCCTAG CACTACAAGC CGCTGTCGGA GAGGCTATGG

 151 GGCAAAATGT ACAGCAAGAT GCGCGGTTAC CTCCCCTCCA GCCCGCCCTA
     CCGTTTTACA TGTCGTTCTA CGCGCCAATG GAGGGGAGGT CGGGCGGGAT

 201 CTATGAGGGC CGTTTCTCCA ACGGACCCGT CTGGCTGGAG CAGCTGACCA
     GATACTCCCG GCAAAGAGGT TGCCTGGGCA GACCGACCTC GTCGACTGGT

 251 AACAGTTCCC GGGTCTGACC ATCGCCAACG AAGCGGAAGG CGGTGCCACT
     TTGTCAAGGG CCCAGACTGG TAGCGGTTGC TTCGCCTTCC GCCACGGTGA

 301 GCCGTGGCTT ACAACAAGAT CTCCTGGAAT CCCAAGTATC AGGTCATCAA
     CGGCACCGAA TGTTGTTCTA GAGGACCTTA GGGTTCATAG TCCAGTAGTT

 351 CAACCTGGAC TACGAGGTCA CCCAGTTCTT GCAGAAAGAC AGCTTCAAGC
     GTTGGACCTG ATGCTCCAGT GGGTCAAGAA CGTCTTTCTG TCGAAGTTCG

 401 CGGACGATCT GGTGATCCTC TGGGTCGGTG CCAATGACTA TCTGGCCTAT
     GCCTGCTAGA CCACTAGGAG ACCCAGCCAC GGTTACTGAT AGACCGGATA

 451 GGCTGGAACA CGGAGCAGGA TGCCAAGCGG GTTCGCGATG CCATCAGCGA
     CCGACCTTGT GCCTCGTCCT ACGGTTCGCC CAAGCGCTAC GGTAGTCGCT

 501 TGCGGCCAAC CGCATGGTAC TGAACGGTGC CAAGCAGATA CTGCTGTTCA
     ACGCCGGTTG GCGTACCATG ACTTGCCACG GTTCGTCTAT GACGACAAGT

 551 ACCTGCCGGA TCTGGGCCAG AACCCGTCAG CTCGCAGTCA GAAGGTGGTC
     TGGACGGCCT AGACCCGGTC TTGGGCAGTC GAGCGTCAGT CTTCCACCAG

 601 GAGGCGGTCA GCCATGTCTC CGCCTATCAC AACCAGCTGC TGCTGAACCT
     CTCCGCCAGT CGGTACAGAG GCGGATAGTG TTGGTCGACG ACGACTTGGA

 651 GGCACGCCAG CTGGCCCCCA CCGGCATGGT AAAGCTGTTC GAGATCGACA
     CCGTGCGGTC GACCGGGGGT GGCCGTACCA TTTCGACAAG CTCTAGCTGT

 701 AGCAATTTGC CGAGATGCTG CGTGATCCGC AGAACTTCGG CCTGAGCGAC
     TCGTTAAACG GCTCTACGAC GCACTAGGCG TCTTGAAGCC GGACTCGCTG

 751 GTCGAGAACC CCTGCTACGA CGGCGGCTAT GTGTGGAAGC CGTTTGCCAC
     CAGCTCTTGG GGACGATGCT GCCGCCGATA CACACCTTCG GCAAACGGTG

 801 CCGCAGCGTC AGCACCGACC GCCAGCTCTC CGCCTTCAGT CCGCAGGAAC
     GGCGTCGCAG TCGTGGCTGG CGGTCGAGAG GCGGAAGTCA GGCGTCCTTG

 851 GCCTCGCCAT CGCCGGCAAC CCGCTGCTGG CACAGGCCGT TGCCAGTCCT
     CGGAGCGGTA GCGGCCGTTG GGCGACGACC GTGTCCGGCA ACGGTCAGGA

 901 ATGGCCCGCC GCAGCGCCAG CCCCCTCAAC TGTGAGGGCA AGATGTTCTG
     TACCGGGCGG CGTCGCGGTC GGGGGAGTTG ACACTCCCGT TCTACAAGAC

 951 GGATCAGGTA CACCCGACCA CTGTCGTGCA CGCAGCCCTG AGCGAGCGCG
     CCTAGTCCAT GTGGGCTGGT GACAGCACGT GCGTCGGGAC TCGCTCGCGC

1001 CCGCCACCTT CATCGCGAAC CAGTACGAGT TCCTCGCCCA CTGATGA
     GGCGGTGGAA GTAGCGCTTG GTCATGCTCA AGGAGCGGGT GACTACT
```

SEQ ID No. 68

```
  1  ADTRPAFSRI VMFGDSLSDT GKMYSKMRGY LPSSPPYYEG RFSNGPVWLE QLTKQFPGLT
 61  IANEAEGGAT AVAYNKISWD PKYQVINNLD YEVTQFLQKD SFKPDDLVIL WVGANDYLAY
121  GWNTEQDAKR VRDAISDAAN RMVLNGAKQI LLFNLPDLGQ NPSARSQKVV EAVSHVSAYH
181  NKLLLNLARQ LAPTGMVKLF EIDKQFAEML RDPQNFGLSD VENPCYDGGY VWKPF

236  RSASPLNCEG KMFWDQVHPT TVVHAALSER AATFIETQYE FLAHG
```

FIGURE 74

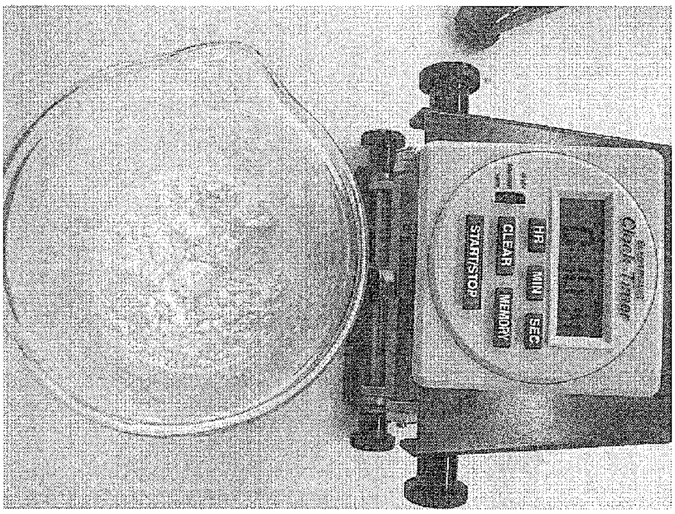

FIGURE 75

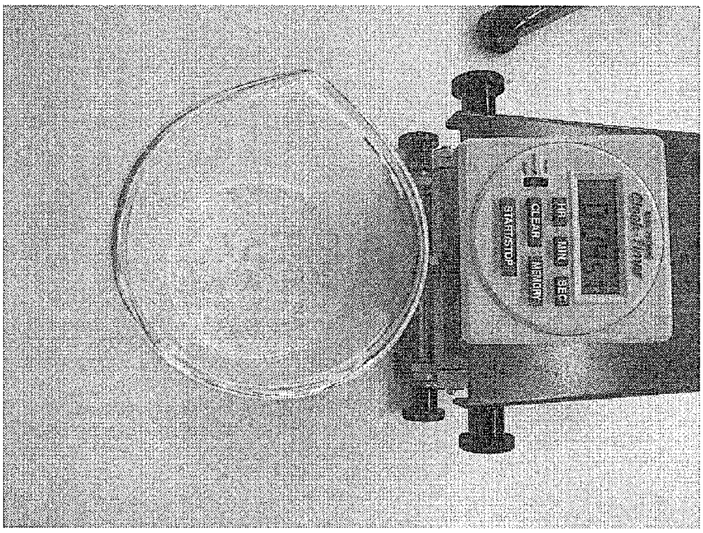

FIGURE 76

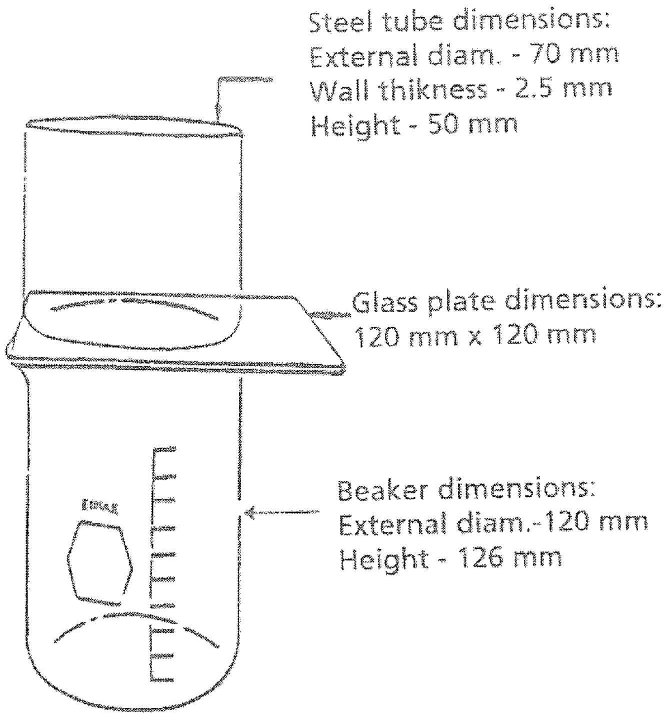
Steel tube dimensions:
External diam. - 70 mm
Wall thikness - 2.5 mm
Height - 50 mm
Glass plate dimensions:
120 mm x 120 mm
Beaker dimensions:
External diam.-120 mm
Height - 126 mm

FIGURE 77

SEQ ID NO. 120

gacactcgcc ccgccttctc ccggatcgtg atgttcggcg acagcctctc cgataccggc 60

aaaatgtaca gcaagatgcg cggttacctc ccctccagcc cgccctacta tgagggccgt 120

ttctccaacg gacccgtctg gctggagcag ctgaccaagc agttcccggg tctgaccatc 180

gccaacgaag cggaaggcgg tgccactgcc gtggcttaca acaagatctc ctgggacccc 240

aagtatcagg tcatcaacaa cctggactac gaggtcaccc agttcttgca gaaagacagc 300

ttcaagccgg acgatctggt gatcctctgg gtcggtgcca atgactatct ggcatatggc 360

tggaatacgg agcaggatgc caagcgagtt cgcgatgcca tcagcgatgc ggccaaccgc 420

atggtactga acggtgccaa gcagatactg ctgttcaacc tgccggatct gggccagaac 480

ccgtcagccc gcagtcagaa ggtggtcgag gcggtcagcc atgtctccgc ctatcacaac 540

aagctgctgc tgaacctggc acgccagctg gcccccaccg gcatggtaaa gctgttcgag 600

atcgacaagc aatttgccga gatgctgcgt gatccgcaga acttcggcct gagcgacgtc 660

gagaacccct gctacgacgg cggctatgtg tggaagccgt ttgccacccg cagcgtcagc 720

accgaccgcc agctctccgc cttcagtccg caggaacgcc tcgccatcgc cggcaacccg 780

ctgctggcac aggccgttgc cagtcctatg gcccgccgca gcgccagccc cctcaactgt 840

gagggcaaga tgttctggga tcaggtacac ccgaccactg tcgtgcacgc agccctgagc 900

gagcgcgccg ccaccttcat cgagacccag tacgagttcc tcgcccacgg atga 954

FIGURE 78

SEQ ID No. 121

```
  1  ADTRPAFSRI VMFGDSLSDT GKMYSKMRGY LPSSPPYYEG RFSNGPVWLE QLTKQFPGLT
 61  IANEAEGGAT AVAYNKISWD PKYQVINNLD YEVTQFLQKD SFKPDDLVIL WVGANDYLAY
121  GWNTEQDAKR VRDAISDAAN RMVLNGAKQI LLFNLPDLGQ NPSARSQKVV EAVSHVSAYH
181  NKLLLNLARQ LAPTGMVKLF EIDKQFAEML RDPQNFGLSD VENPCYDGGY VWKPF

236  SASPLNCEG KMFWDQVHPT TVVHAALSER AATFIETQYE FLAHG
```

FIGURE 79

SEQ ID No. 122

```
  1  ADTRPAFSRI VMFGDSLSDT GKMYSKMRGY LPSSPPYYEG RFSNGPVWLE QLTKQFPGLT
 61  IANEAEGGAT AVAYNKISWD PKYQVINNLD YEVTQFLQKD SFKPDDLVIL WVGANDYLAY
121  GWNTEQDAKR VRDAISDAAN RMVLNGAKQI LLFNLPDLGQ NPSARSQKVV EAVSHVSAYH
181  NKLLLNLARQ LAPTGMVKLF EIDKQFAEML RDPQNFGLSD VENPCYDGGY VWKPF

236  ASPLNCEG KMFWDQVHPT TVVHAALSER AATFIETQYE FLAHG
```

FIGURE 80

SEQ ID No. 123

```
  1  ADTRPAFSRI VMFGDSLSDT GKMYSKMRGY LPSSPPYYEG RFSNGPVWLE QLTKQFPGLT
 61  IANEAEGGAT AVAYNKISWD PKYQVINNLD YEVTQFLQKD SFKPDDLVIL WVGANDYLAY
121  GWNTEQDAKR VRDAISDAAN RMVLNGAKQI LLFNLPDLGQ NPSARSQKVV EAVSHVSAYH
181  NKLLLNLARQ LAPTGMVKLF EIDKQFAEML RDPQNFGLSD VENPCYDGGY VWKPF

236  SPLNCEG KMFWDQVHPT TVVHAALSER AATFIETQYE FLAHG
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006066590 A
- WO 2004064537 A
- WO 2008090395 A
- WO 9117243 A
- WO 2004064987 A
- WO 2005066347 A
- WO 2006008508 A
- WO 2009024862 A
- WO IB2009054535 W
- EP 1275711 A
- WO 0239828 A
- WO 0397835 A
- EP 0977869 A
- EP 1193314 A
- US 4683202 A
- EP 0583265 A
- EP 0866796 A
- WO 0206457 A
- EP 0752008 A
- EP 1138763 A
- EP 1103606 A
- US 6180406 B
- WO 0134835 A
- WO 0058517 A
- US 6344328 B
- US 6361974 B
- EP 0238023 A
- EP 0449375 A
- US 5741665 A
- US 5674707 A
- WO 0139544 A
- WO 0116308 A
- US 3817837 A
- US 3850752 A
- US 3939350 A
- US 3996345 A
- US 4277437 A
- US 4275149 A
- US 4366241 A
- US 4816567 A
- US 20020182734 A
- WO 0214490 A
- WO 2005111203 A
- GB 0920089 A
- US 61262285 B


### Non-patent literature cited in the description


- **R. JOST.** Milk and Dairy Products. Wiley-VCH, 2007
- Food Chemical Codex. National Academy Press, 1996, 803
- **BRUMLIK ; BUCKLEY.** *Journal of Bacteriology,* April 1996, vol. 178 (7), 2060-2064
- **BEUCAGE S.L. et al.** *Tetrahedron Letters,* 1981, vol. 22, 1859-1869
- **MATTHES et al.** *EMBO J.,* 1984, vol. 3, 801-805
- **SAIKI R K et al.** *Science,* 1988, vol. 239, 487-491
- **CARUTHERS MH et al.** *Nuc Acids Res Symp Ser,* 1980, 215-23
- **HORN T et al.** *Nuc Acids Res Symp Ser,* 1980, 225-232
- **MORINAGA et al.** *Biotechnology,* 1984, vol. 2, 646-649
- **NELSON ; LONG.** *Analytical Biochemistry,* 1989, vol. 180, 147-151
- **HILTON ; BUCKLEY.** *J. Biol. Chem.,* 15 January 1991, vol. 266 (2), 997-1000
- **ROBERTSON et al.** *J. Biol. Chem.,* 21 January 1994, vol. 269 (3), 2146-50
- **BRUMLIK et al.** *J. Bacteriol.,* April 1996, vol. 178 (7), 2060-4
- **PEELMAN et al.** *Protein Sci.,* March 1998, vol. 7 (3), 587-99
- **AUSUBEL et al.** Short Protocols in Molecular Biology. 1999
- **ALTSCHUL et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8
- **HIGGINS DG ; SHARP PM.** *Gene,* 1988, vol. 73 (1), 237-244
- **SIMON RJ et al.** *PNAS,* 1992, vol. 89 (20), 9367-9371
- **HORWELL DC.** *Trends Biotechnol,* 1995, vol. 13 (4), 132-134

- **BERGER ; KIMMEL.** Guide to Molecular Cloning Techniques, Methods in Enzymology. Academic Press, 1987, vol. 152
- **SAMBROOK et al.** Molecular Cloning: A Laboratory Manual. Cold Spring Harbor Laboratory Press, 1989
- **POTRYKUS.** *Annu Rev Plant Physiol Plant Mol Biol,* 1991, vol. 42, 205-225
- **CHRISTOU.** *Agro-Food-Industry Hi-Tech,* March 1994, 17-27
- **DAVIS ; DE SERRES.** *Methods Enzymol,* 1971, vol. 17A, 79-143
- Vectors for genetic manipulation. **TURNER G.** Aspergillus: 50 years on. Progress in industrial microbiology. Elsevier, 1994, vol. 29, 641-666
- **PUNT et al.** *Trends Biotechnol,* May 2002, vol. 20 (5), 200-6
- **ARCHER ; PEBERDY.** *Crit Rev Biotechnol,* 1997, vol. 17 (4), 273-306
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression of heterologous genes. **E HINCHCLIFFE ; E KENNY.** Yeasts. Academic Press Ltd, 1993, vol. 5
- **HINNEN et al.** *Proceedings of the National Academy of Sciences of the USA,* 1978, vol. 75
- **BEGGS, J D.** *Nature,* 1978, vol. 275, 104
- **ITO, H et al.** *J Bacteriology,* 1983, vol. 153, 163-168
- **POTRYKUS.** *Annu Rev Plant Physiol Plant MolBiol,* 1991, vol. 42, 205-225
- **E. COLI.** *Curr. Opin. Biotechnol.,* 1995, vol. 6 (5), 501-6

SEQUENCE LISTING

<110> DANISCO A/S

<120> METHOD

<130> P038564WO

<150> GB 0920089.0
<151> 2009-11-17

<150> US 61/262285
<151> 2009-11-18

<160> 124

<170> PatentIn version 3.5

<210> 1
<211> 335
<212> PRT
<213> Aeromonas hydrophila

<400> 1

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160
```

Val Leu Asn Gly Ala Lys Gln Ile Leu Leu Phe Asn Leu Pro Asp Leu
165 170 175

Gly Gln Asn Pro Ser Ala Arg Ser Gln Lys Val Val Glu Ala Val Ser
180 185 190

His Val Ser Ala Tyr His Asn Gln Leu Leu Leu Asn Leu Ala Arg Gln
195 200 205

Leu Ala Pro Thr Gly Met Val Lys Leu Phe Glu Ile Asp Lys Gln Phe
210 215 220

Ala Glu Met Leu Arg Asp Pro Gln Asn Phe Gly Leu Ser Asp Val Glu
225 230 235 240

Asn Pro Cys Tyr Asp Gly Gly Tyr Val Trp Lys Pro Phe Ala Thr Arg
245 250 255

Ser Val Ser Thr Asp Arg Gln Leu Ser Ala Phe Ser Pro Gln Glu Arg
260 265 270

Leu Ala Ile Ala Gly Asn Pro Leu Leu Ala Gln Ala Val Ala Ser Pro
275 280 285

Met Ala Arg Arg Ser Ala Ser Pro Leu Asn Cys Glu Gly Lys Met Phe
290 295 300

Trp Asp Gln Val His Pro Thr Thr Val Val His Ala Ala Leu Ser Glu
305 310 315 320

Arg Ala Ala Thr Phe Ile Ala Asn Gln Tyr Glu Phe Leu Ala His
325 330 335

<210> 2
<211> 361
<212> PRT
<213> Artificial Sequence

<220>
<223> pfam00657 consensus sequence

<400> 2

```
Ile Val Ala Phe Gly Asp Ser Leu Thr Asp Gly Glu Ala Tyr Tyr Gly
1               5                   10                  15

Asp Ser Asp Gly Gly Gly Trp Gly Ala Gly Leu Ala Asp Arg Leu Thr
            20                  25                  30

Ala Leu Leu Arg Leu Arg Ala Arg Pro Arg Gly Val Asp Val Phe Asn
        35                  40                  45
```

```
Arg Gly Ile Ser Gly Arg Thr Ser Asp Gly Arg Leu Ile Val Asp Ala
    50                  55                  60

Leu Val Ala Leu Leu Phe Leu Ala Gln Ser Leu Gly Leu Pro Asn Leu
65                  70                  75                  80

Pro Pro Tyr Leu Ser Gly Asp Phe Leu Arg Gly Ala Asn Phe Ala Ser
                85                  90                  95

Ala Gly Ala Thr Ile Leu Pro Thr Ser Gly Pro Phe Leu Ile Gln Val
            100                 105                 110

Gln Phe Lys Asp Phe Lys Ser Gln Val Leu Glu Leu Arg Gln Ala Leu
        115                 120                 125

Gly Leu Leu Gln Glu Leu Leu Arg Leu Leu Pro Val Leu Asp Ala Lys
    130                 135                 140

Ser Pro Asp Leu Val Thr Ile Met Ile Gly Thr Asn Asp Leu Ile Thr
145                 150                 155                 160

Ser Ala Phe Phe Gly Pro Lys Ser Thr Glu Ser Asp Arg Asn Val Ser
                165                 170                 175

Val Pro Glu Phe Lys Asp Asn Leu Arg Gln Leu Ile Lys Arg Leu Arg
            180                 185                 190

Ser Asn Asn Gly Ala Arg Ile Ile Val Leu Ile Thr Leu Val Ile Leu
        195                 200                 205

Asn Leu Gly Pro Leu Gly Cys Leu Pro Leu Lys Leu Ala Leu Ala Leu
    210                 215                 220

Ala Ser Ser Lys Asn Val Asp Ala Ser Gly Cys Leu Glu Arg Leu Asn
225                 230                 235                 240

Glu Ala Val Ala Asp Phe Asn Glu Ala Leu Arg Glu Leu Ala Ile Ser
                245                 250                 255

Lys Leu Glu Asp Gln Leu Arg Lys Asp Gly Leu Pro Asp Val Lys Gly
            260                 265                 270

Ala Asp Val Pro Tyr Val Asp Leu Tyr Ser Ile Phe Gln Asp Leu Asp
        275                 280                 285

Gly Ile Gln Asn Pro Ser Ala Tyr Val Tyr Gly Phe Glu Thr Thr Lys
    290                 295                 300
```

Ala Cys Cys Gly Tyr Gly Gly Arg Tyr Asn Tyr Asn Arg Val Cys Gly
305 310 315 320

Asn Ala Gly Leu Cys Asn Val Thr Ala Lys Ala Cys Asn Pro Ser Ser
325 330 335

Tyr Leu Leu Ser Phe Leu Phe Trp Asp Gly Phe His Pro Ser Glu Lys
340 345 350

Gly Tyr Lys Ala Val Ala Glu Ala Leu
355 360

<210> 3
<211> 335
<212> PRT
<213> Aeromonas hydrophila

<400> 3

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Asn Glu Phe Pro Gly Leu Thr Ile Ala
65                  70                  75                  80

Asn Glu Ala Glu Gly Gly Pro Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Glu Ile Leu Leu Phe Asn Leu Pro Asp Leu
                165                 170                 175
```

```
Gly Gln Asn Pro Ser Ala Arg Ser Gln Lys Val Val Glu Ala Ala Ser
            180                 185                 190

His Val Ser Ala Tyr His Asn Gln Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Gln Arg
225                 230                 235                 240

Asn Ala Cys Tyr Gly Gly Ser Tyr Val Trp Lys Pro Phe Ala Ser Arg
                245                 250                 255

Ser Ala Ser Thr Asp Ser Gln Leu Ser Ala Phe Asn Pro Gln Glu Arg
            260                 265                 270

Leu Ala Ile Ala Gly Asn Pro Leu Leu Ala Gln Ala Val Ala Ser Pro
        275                 280                 285

Met Ala Ala Arg Ser Ala Ser Thr Leu Asn Cys Glu Gly Lys Met Phe
    290                 295                 300

Trp Asp Gln Val His Pro Thr Thr Val Val His Ala Ala Leu Ser Glu
305                 310                 315                 320

Pro Ala Ala Thr Phe Ile Glu Ser Gln Tyr Glu Phe Leu Ala His
                325                 330                 335
```

<210> 4
<211> 336
<212> PRT
<213> Aeromonas salmonicida

<400> 4

```
Met Lys Lys Trp Phe Val Cys Leu Leu Gly Leu Ile Ala Leu Thr Val
1               5                   10                  15

Gln Ala Ala Asp Thr Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60
```

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Glu Thr Gln Tyr Glu Phe Leu Ala His Gly

325 330 335

<210> 5
<211> 295
<212> PRT
<213> Streptomyces coelicolor

<400> 5

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
            20                  25                  30

Pro Ala Gln Ala Thr Pro Thr Leu Asp Tyr Val Ala Leu Gly Asp Ser
        35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
145                 150                 155                 160

Asp Asp Glu Ile Glu Ala Asn Thr Tyr Pro Ala Leu Lys Glu Ala Leu
                165                 170                 175

Leu Gly Val Arg Ala Arg Ala Pro His Ala Arg Val Ala Ala Leu Gly
            180                 185                 190

Tyr Pro Trp Ile Thr Pro Ala Thr Ala Asp Pro Ser Cys Phe Leu Lys
        195                 200                 205

Leu Pro Leu Ala Ala Gly Asp Val Pro Tyr Leu Arg Ala Ile Gln Ala
    210                 215                 220
```

```
His Leu Asn Asp Ala Val Arg Arg Ala Ala Glu Glu Thr Gly Ala Thr
225                 230                 235                 240

Tyr Val Asp Phe Ser Gly Val Ser Asp Gly His Asp Ala Cys Glu Ala
                245                 250                 255

Pro Gly Thr Arg Trp Ile Glu Pro Leu Leu Phe Gly His Ser Leu Val
            260                 265                 270

Pro Val His Pro Asn Ala Leu Gly Glu Arg Arg Met Ala Glu His Thr
        275                 280                 285

Met Asp Val Leu Gly Leu Asp
    290                 295
```

<210> 6
<211> 295
<212> PRT
<213> Streptomyces coelicolor

<400> 6

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
            20                  25                  30

Pro Ala Gln Ala Thr Pro Thr Leu Asp Tyr Val Ala Leu Gly Asp Ser
        35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
```

145 150 155 160

Asp Asp Glu Ile Glu Ala Asn Thr Tyr Pro Ala Leu Lys Glu Ala Leu
165 170 175

Leu Gly Val Arg Ala Arg Ala Pro His Ala Arg Val Ala Ala Leu Gly
180 185 190

Tyr Pro Trp Ile Thr Pro Ala Thr Ala Asp Pro Ser Cys Phe Leu Lys
195 200 205

Leu Pro Leu Ala Ala Gly Asp Val Pro Tyr Leu Arg Ala Ile Gln Ala
210 215 220

His Leu Asn Asp Ala Val Arg Arg Ala Ala Glu Glu Thr Gly Ala Thr
225 230 235 240

Tyr Val Asp Phe Ser Gly Val Ser Asp Gly His Asp Ala Cys Glu Ala
245 250 255

Pro Gly Thr Arg Trp Ile Glu Pro Leu Leu Phe Gly His Ser Leu Val
260 265 270

Pro Val His Pro Asn Ala Leu Gly Glu Arg Arg Met Ala Glu His Thr
275 280 285

Met Asp Val Leu Gly Leu Asp
290 295

<210> 7
<211> 238
<212> PRT
<213> Saccharomyces cerevisiae

<400> 7

Met Asp Tyr Glu Lys Phe Leu Leu Phe Gly Asp Ser Ile Thr Glu Phe
1               5                   10                  15

Ala Phe Asn Thr Arg Pro Ile Glu Asp Gly Lys Asp Gln Tyr Ala Leu
            20                  25                  30

Gly Ala Ala Leu Val Asn Glu Tyr Thr Arg Lys Met Asp Ile Leu Gln
        35                  40                  45

Arg Gly Phe Lys Gly Tyr Thr Ser Arg Trp Ala Leu Lys Ile Leu Pro
    50                  55                  60

Glu Ile Leu Lys His Glu Ser Asn Ile Val Met Ala Thr Ile Phe Leu
65                  70                  75                  80

Gly Ala Asn Asp Ala Cys Ser Ala Gly Pro Gln Ser Val Pro Leu Pro
                85                  90                  95

Glu Phe Ile Asp Asn Ile Arg Gln Met Val Ser Leu Met Lys Ser Tyr
            100                 105                 110

His Ile Arg Pro Ile Ile Ile Gly Pro Gly Leu Val Asp Arg Glu Lys
        115                 120                 125

Trp Glu Lys Glu Lys Ser Glu Glu Ile Ala Leu Gly Tyr Phe Arg Thr
    130                 135                 140

Asn Glu Asn Phe Ala Ile Tyr Ser Asp Ala Leu Ala Lys Leu Ala Asn
145                 150                 155                 160

Glu Glu Lys Val Pro Phe Val Ala Leu Asn Lys Ala Phe Gln Gln Glu
                165                 170                 175

Gly Gly Asp Ala Trp Gln Gln Leu Leu Thr Asp Gly Leu His Phe Ser
            180                 185                 190

Gly Lys Gly Tyr Lys Ile Phe His Asp Glu Leu Leu Lys Val Ile Glu
        195                 200                 205

Thr Phe Tyr Pro Gln Tyr His Pro Lys Asn Met Gln Tyr Lys Leu Lys
    210                 215                 220

Asp Trp Arg Asp Val Leu Asp Asp Gly Ser Asn Ile Met Ser
225                 230                 235

<210> 8
<211> 347
<212> PRT

<213> Ralstonia sp.

<400> 8

```
Met Asn Leu Arg Gln Trp Met Gly Ala Ala Thr Ala Ala Leu Ala Leu
1               5                   10                  15

Gly Leu Ala Ala Cys Gly Gly Gly Gly Thr Asp Gln Ser Gly Asn Pro
            20                  25                  30

Asn Val Ala Lys Val Gln Arg Met Val Val Phe Gly Asp Ser Leu Ser
        35                  40                  45

Asp Ile Gly Thr Tyr Thr Pro Val Ala Gln Ala Val Gly Gly Gly Lys
    50                  55                  60

Phe Thr Thr Asn Pro Gly Pro Ile Trp Ala Glu Thr Val Ala Ala Gln
```

```
65                  70                  75                  80

Leu Gly Val Thr Leu Thr Pro Ala Val Met Gly Tyr Ala Thr Ser Val
                85                  90                  95

Gln Asn Cys Pro Lys Ala Gly Cys Phe Asp Tyr Ala Gln Gly Gly Ser
            100                 105                 110

Arg Val Thr Asp Pro Asn Gly Ile Gly His Asn Gly Gly Ala Gly Ala
        115                 120                 125

Leu Thr Tyr Pro Val Gln Gln Gln Leu Ala Asn Phe Tyr Ala Ala Ser
    130                 135                 140

Asn Asn Thr Phe Asn Gly Asn Asn Asp Val Val Phe Val Leu Ala Gly
145                 150                 155                 160

Ser Asn Asp Ile Phe Phe Trp Thr Thr Ala Ala Ala Thr Ser Gly Ser
                165                 170                 175

Gly Val Thr Pro Ala Ile Ala Thr Ala Gln Val Gln Gln Ala Ala Thr
            180                 185                 190

Asp Leu Val Gly Tyr Val Lys Asp Met Ile Ala Lys Gly Ala Thr Gln
        195                 200                 205

Val Tyr Val Phe Asn Leu Pro Asp Ser Ser Leu Thr Pro Asp Gly Val
    210                 215                 220

Ala Ser Gly Thr Thr Gly Gln Ala Leu Leu His Ala Leu Val Gly Thr
225                 230                 235                 240

Phe Asn Thr Thr Leu Gln Ser Gly Leu Ala Gly Thr Ser Ala Arg Ile
                245                 250                 255

Ile Asp Phe Asn Ala Gln Leu Thr Ala Ala Ile Gln Asn Gly Ala Ser
            260                 265                 270

Phe Gly Phe Ala Asn Thr Ser Ala Arg Ala Cys Asp Ala Thr Lys Ile
        275                 280                 285

Asn Ala Leu Val Pro Ser Ala Gly Gly Ser Ser Leu Phe Cys Ser Ala
    290                 295                 300

Asn Thr Leu Val Ala Ser Gly Ala Asp Gln Ser Tyr Leu Phe Ala Asp
305                 310                 315                 320

Gly Val His Pro Thr Thr Ala Gly His Arg Leu Ile Ala Ser Asn Val
                325                 330                 335
```

```
Leu Ala Arg Leu Leu Ala Asp Asn Val Ala His
            340                 345
```

<210> 9
<211> 261
<212> PRT
<213> Streptomyces coelicolor

<400> 9

```
Met Ile Gly Ser Tyr Val Ala Val Gly Asp Ser Phe Thr Glu Gly Val
1               5                   10                  15

Gly Asp Pro Gly Pro Asp Gly Ala Phe Val Gly Trp Ala Asp Arg Leu
            20                  25                  30

Ala Val Leu Leu Ala Asp Arg Arg Pro Glu Gly Asp Phe Thr Tyr Thr
        35                  40                  45

Asn Leu Ala Val Arg Gly Arg Leu Leu Asp Gln Ile Val Ala Glu Gln
    50                  55                  60

Val Pro Arg Val Val Gly Leu Ala Pro Asp Leu Val Ser Phe Ala Ala
65                  70                  75                  80

Gly Gly Asn Asp Ile Ile Arg Pro Gly Thr Asp Pro Asp Glu Val Ala
                85                  90                  95

Glu Arg Phe Glu Leu Ala Val Ala Ala Leu Thr Ala Ala Ala Gly Thr
            100                 105                 110

Val Leu Val Thr Thr Gly Phe Asp Thr Arg Gly Val Pro Val Leu Lys
        115                 120                 125

His Leu Arg Gly Lys Ile Ala Thr Tyr Asn Gly His Val Arg Ala Ile
    130                 135                 140

Ala Asp Arg Tyr Gly Cys Pro Val Leu Asp Leu Trp Ser Leu Arg Ser
145                 150                 155                 160

Val Gln Asp Arg Arg Ala Trp Asp Ala Asp Arg Leu His Leu Ser Pro
                165                 170                 175

Glu Gly His Thr Arg Val Ala Leu Arg Ala Gly Gln Ala Leu Gly Leu
            180                 185                 190

Arg Val Pro Ala Asp Pro Asp Gln Pro Trp Pro Pro Leu Pro Pro Arg
        195                 200                 205
```

```
Gly Thr Leu Asp Val Arg Arg Asp Asp Val His Trp Ala Arg Glu Tyr
    210                 215                 220

Leu Val Pro Trp Ile Gly Arg Arg Leu Arg Gly Glu Ser Ser Gly Asp
225                 230                 235                 240

His Val Thr Ala Lys Gly Thr Leu Ser Pro Asp Ala Ile Lys Thr Arg
                245                 250                 255

Ile Ala Ala Val Ala
            260
```

<210> 10
<211> 260
<212> PRT
<213> Streptomyces coelicolor

<400> 10

Met Gln Thr Asn Pro Ala Tyr Thr Ser Leu Val Ala Val Gly Asp Ser
1               5                   10                  15

Phe Thr Glu Gly Met Ser Asp Leu Leu Pro Asp Gly Ser Tyr Arg Gly
            20                  25                  30

Trp Ala Asp Leu Leu Ala Thr Arg Met Ala Ala Arg Ser Pro Gly Phe
        35                  40                  45

Arg Tyr Ala Asn Leu Ala Val Arg Gly Lys Leu Ile Gly Gln Ile Val
    50                  55                  60

Asp Glu Gln Val Asp Val Ala Ala Ala Met Gly Ala Asp Val Ile Thr
65                  70                  75                  80

Leu Val Gly Gly Leu Asn Asp Thr Leu Arg Pro Lys Cys Asp Met Ala
                85                  90                  95

Arg Val Arg Asp Leu Leu Thr Gln Ala Val Glu Arg Leu Ala Pro His
            100                 105                 110

Cys Glu Gln Leu Val Leu Met Arg Ser Pro Gly Arg Gln Gly Pro Val
        115                 120                 125

Leu Glu Arg Phe Arg Pro Arg Met Glu Ala Leu Phe Ala Val Ile Asp
    130                 135                 140

Asp Leu Ala Gly Arg His Gly Ala Val Val Val Asp Leu Tyr Gly Ala
145                 150                 155                 160

Gln Ser Leu Ala Asp Pro Arg Met Trp Asp Val Asp Arg Leu His Leu
                165                 170                 175

Thr Ala Glu Gly His Arg Arg Val Ala Glu Ala Val Trp Gln Ser Leu
180 185 190

Gly His Glu Pro Glu Asp Pro Glu Trp His Ala Pro Ile Pro Ala Thr
195 200 205

Pro Pro Pro Gly Trp Val Thr Arg Arg Thr Ala Asp Val Arg Phe Ala
210 215 220

Arg Gln His Leu Leu Pro Trp Ile Gly Arg Arg Leu Thr Gly Arg Ser
225 230 235 240

Ser Gly Asp Gly Leu Pro Ala Lys Arg Pro Asp Leu Leu Pro Tyr Glu
245 250 255

Asp Pro Ala Arg
260

<210> 11
<211> 454
<212> PRT
<213> Streptomyces coelicolor

<400> 11

Met Thr Arg Gly Arg Asp Gly Gly Ala Gly Ala Pro Pro Thr Lys His
1 5 10 15

Arg Ala Leu Leu Ala Ala Ile Val Thr Leu Ile Val Ala Ile Ser Ala
20 25 30

Ala Ile Tyr Ala Gly Ala Ser Ala Asp Asp Gly Ser Arg Asp His Ala
35 40 45

Leu Gln Ala Gly Gly Arg Leu Pro Arg Gly Asp Ala Ala Pro Ala Ser
50 55 60

Thr Gly Ala Trp Val Gly Ala Trp Ala Thr Ala Pro Ala Ala Ala Glu
65 70 75 80

Pro Gly Thr Glu Thr Thr Gly Leu Ala Gly Arg Ser Val Arg Asn Val
85 90 95

Val His Thr Ser Val Gly Gly Thr Gly Ala Arg Ile Thr Leu Ser Asn
100 105 110

Leu Tyr Gly Gln Ser Pro Leu Thr Val Thr His Ala Ser Ile Ala Leu
115 120 125

```
Ala Ala Gly Pro Asp Thr Ala Ala Ala Ile Ala Asp Thr Met Arg Arg
    130                 135                 140

Leu Thr Phe Gly Gly Ser Ala Arg Val Ile Ile Pro Ala Gly Gly Gln
145                 150                 155                 160

Val Met Ser Asp Thr Ala Arg Leu Ala Ile Pro Tyr Gly Ala Asn Val
                165                 170                 175

Leu Val Thr Thr Tyr Ser Pro Ile Pro Ser Gly Pro Val Thr Tyr His
            180                 185                 190

Pro Gln Ala Arg Gln Thr Ser Tyr Leu Ala Asp Gly Asp Arg Thr Ala
        195                 200                 205

Asp Val Thr Ala Val Ala Tyr Thr Thr Pro Thr Pro Tyr Trp Arg Tyr
    210                 215                 220

Leu Thr Ala Leu Asp Val Leu Ser His Glu Ala Asp Gly Thr Val Val
225                 230                 235                 240

Ala Phe Gly Asp Ser Ile Thr Asp Gly Ala Arg Ser Gln Ser Asp Ala
                245                 250                 255

Asn His Arg Trp Thr Asp Val Leu Ala Ala Arg Leu His Glu Ala Ala
            260                 265                 270

Gly Asp Gly Arg Asp Thr Pro Arg Tyr Ser Val Val Asn Glu Gly Ile
        275                 280                 285

Ser Gly Asn Arg Leu Leu Thr Ser Arg Pro Gly Arg Pro Ala Asp Asn
    290                 295                 300

Pro Ser Gly Leu Ser Arg Phe Gln Arg Asp Val Leu Glu Arg Thr Asn
305                 310                 315                 320

Val Lys Ala Val Val Val Val Leu Gly Val Asn Asp Val Leu Asn Ser
                325                 330                 335

Pro Glu Leu Ala Asp Arg Asp Ala Ile Leu Thr Gly Leu Arg Thr Leu
            340                 345                 350

Val Asp Arg Ala His Ala Arg Gly Leu Arg Val Val Gly Ala Thr Ile
        355                 360                 365

Thr Pro Phe Gly Gly Tyr Gly Gly Tyr Thr Glu Ala Arg Glu Thr Met
    370                 375                 380

Arg Gln Glu Val Asn Glu Glu Ile Arg Ser Gly Arg Val Phe Asp Thr
```

385 390 395 400

Val Val Asp Phe Asp Lys Ala Leu Arg Asp Pro Tyr Asp Pro Arg Arg
405 410 415

Met Arg Ser Asp Tyr Asp Ser Gly Asp His Leu His Pro Gly Asp Lys
420 425 430

Gly Tyr Ala Arg Met Gly Ala Val Ile Asp Leu Ala Ala Leu Lys Gly
435 440 445

Ala Ala Pro Val Lys Ala
450

<210> 12
<211> 340
<212> PRT
<213> Streptomyces coelicolor

<400> 12

```
Met Thr Ser Met Ser Arg Ala Arg Val Ala Arg Arg Ile Ala Ala Gly
1               5                   10                  15

Ala Ala Tyr Gly Gly Gly Gly Ile Gly Leu Ala Gly Ala Ala Ala Val
            20                  25                  30

Gly Leu Val Val Ala Glu Val Gln Leu Ala Arg Arg Arg Val Gly Val
        35                  40                  45

Gly Thr Pro Thr Arg Val Pro Asn Ala Gln Gly Leu Tyr Gly Gly Thr
    50                  55                  60

Leu Pro Thr Ala Gly Asp Pro Pro Leu Arg Leu Met Met Leu Gly Asp
65                  70                  75                  80

Ser Thr Ala Ala Gly Gln Gly Val His Arg Ala Gly Gln Thr Pro Gly
                85                  90                  95

Ala Leu Leu Ala Ser Gly Leu Ala Ala Val Ala Glu Arg Pro Val Arg
            100                 105                 110

Leu Gly Ser Val Ala Gln Pro Gly Ala Cys Ser Asp Asp Leu Asp Arg
        115                 120                 125

Gln Val Ala Leu Val Leu Ala Glu Pro Asp Arg Val Pro Asp Ile Cys
    130                 135                 140

Val Ile Met Val Gly Ala Asn Asp Val Thr His Arg Met Pro Ala Thr
145                 150                 155                 160
```

```
Arg Ser Val Arg His Leu Ser Ser Ala Val Arg Arg Leu Arg Thr Ala
                165                 170                 175

Gly Ala Glu Val Val Val Gly Thr Cys Pro Asp Leu Gly Thr Ile Glu
            180                 185                 190

Arg Val Arg Gln Pro Leu Arg Trp Leu Ala Arg Arg Ala Ser Arg Gln
        195                 200                 205

Leu Ala Ala Ala Gln Thr Ile Gly Ala Val Glu Gln Gly Gly Arg Thr
    210                 215                 220

Val Ser Leu Gly Asp Leu Leu Gly Pro Glu Phe Ala Gln Asn Pro Arg
225                 230                 235                 240

Glu Leu Phe Gly Pro Asp Asn Tyr His Pro Ser Ala Glu Gly Tyr Ala
                245                 250                 255

Thr Ala Ala Met Ala Val Leu Pro Ser Val Cys Ala Ala Leu Gly Leu
            260                 265                 270

Trp Pro Ala Asp Glu Glu His Pro Asp Ala Leu Arg Arg Glu Gly Phe
        275                 280                 285

Leu Pro Val Ala Arg Ala Ala Ala Glu Ala Ala Ser Glu Ala Gly Thr
    290                 295                 300

Glu Val Ala Ala Ala Met Pro Thr Gly Pro Arg Gly Pro Trp Ala Leu
305                 310                 315                 320

Leu Lys Arg Arg Arg Arg Arg Arg Val Ser Glu Ala Glu Pro Ser Ser
                325                 330                 335

Pro Ser Gly Val
            340
```

<210> 13
<211> 305
<212> PRT
<213> Streptomyces coelicolor

<400> 13

Met Gly Arg Gly Thr Asp Gln Arg Thr Arg Tyr Gly Arg Arg Arg Ala
1               5                   10                  15

Arg Val Ala Leu Ala Ala Leu Thr Ala Ala Val Leu Gly Val Gly Val
            20                  25                  30

Ala Gly Cys Asp Ser Val Gly Gly Asp Ser Pro Ala Pro Ser Gly Ser

```
        35                  40                  45

Pro Ser Lys Arg Thr Arg Thr Ala Pro Ala Trp Asp Thr Ser Pro Ala
    50                  55                  60

Ser Val Ala Ala Val Gly Asp Ser Ile Thr Arg Gly Phe Asp Ala Cys
65                  70                  75                  80

Ala Val Leu Ser Asp Cys Pro Glu Val Ser Trp Ala Thr Gly Ser Ser
                85                  90                  95

Ala Lys Val Asp Ser Leu Ala Val Arg Leu Leu Gly Lys Ala Asp Ala
            100                 105                 110

Ala Glu His Ser Trp Asn Tyr Ala Val Thr Gly Ala Arg Met Ala Asp
        115                 120                 125

Leu Thr Ala Gln Val Thr Arg Ala Ala Gln Arg Glu Pro Glu Leu Val
    130                 135                 140

Ala Val Met Ala Gly Ala Asn Asp Ala Cys Arg Ser Thr Thr Ser Ala
145                 150                 155                 160

Met Thr Pro Val Ala Asp Phe Arg Ala Gln Phe Glu Glu Ala Met Ala
                165                 170                 175

Thr Leu Arg Lys Lys Leu Pro Lys Ala Gln Val Tyr Val Ser Ser Ile
            180                 185                 190

Pro Asp Leu Lys Arg Leu Trp Ser Gln Gly Arg Thr Asn Pro Leu Gly
        195                 200                 205

Lys Gln Val Trp Lys Leu Gly Leu Cys Pro Ser Met Leu Gly Asp Ala
    210                 215                 220

Asp Ser Leu Asp Ser Ala Ala Thr Leu Arg Arg Asn Thr Val Arg Asp
225                 230                 235                 240

Arg Val Ala Asp Tyr Asn Glu Val Leu Arg Glu Val Cys Ala Lys Asp
                245                 250                 255

Arg Arg Cys Arg Ser Asp Asp Gly Ala Val His Glu Phe Arg Phe Gly
            260                 265                 270

Thr Asp Gln Leu Ser His Trp Asp Trp Phe His Pro Ser Val Asp Gly
        275                 280                 285

Gln Ala Arg Leu Ala Glu Ile Ala Tyr Arg Ala Val Thr Ala Lys Asn
    290                 295                 300
```

Pro
305

<210> 14
<211> 268
<212> PRT
<213> Streptomyces rimosus

<400> 14

Met Arg Leu Ser Arg Arg Ala Ala Thr Ala Ser Ala Leu Leu Leu Thr
1 5 10 15

Pro Ala Leu Ala Leu Phe Gly Ala Ser Ala Ala Val Ser Ala Pro Arg
20 25 30

Ile Gln Ala Thr Asp Tyr Val Ala Leu Gly Asp Ser Tyr Ser Ser Gly
35 40 45

Val Gly Ala Gly Ser Tyr Asp Ser Ser Ser Gly Ser Cys Lys Arg Ser
50 55 60

Thr Lys Ser Tyr Pro Ala Leu Trp Ala Ala Ser His Thr Gly Thr Arg
65 70 75 80

Phe Asn Phe Thr Ala Cys Ser Gly Ala Arg Thr Gly Asp Val Leu Ala
85 90 95

Lys Gln Leu Thr Pro Val Asn Ser Gly Thr Asp Leu Val Ser Ile Thr
100 105 110

Ile Gly Gly Asn Asp Ala Gly Phe Ala Asp Thr Met Thr Thr Cys Asn
115 120 125

Leu Gln Gly Glu Ser Ala Cys Leu Ala Arg Ile Ala Lys Ala Arg Ala
130 135 140

Tyr Ile Gln Gln Thr Leu Pro Ala Gln Leu Asp Gln Val Tyr Asp Ala
145 150 155 160

Ile Asp Ser Arg Ala Pro Ala Ala Gln Val Val Val Leu Gly Tyr Pro
165 170 175

Arg Phe Tyr Lys Leu Gly Gly Ser Cys Ala Val Gly Leu Ser Glu Lys
180 185 190

Ser Arg Ala Ala Ile Asn Ala Ala Ala Asp Asp Ile Asn Ala Val Thr
195 200 205

```
Ala Lys Arg Ala Ala Asp His Gly Phe Ala Phe Gly Asp Val Asn Thr
    210                 215                 220

Thr Phe Ala Gly His Glu Leu Cys Ser Gly Ala Pro Trp Leu His Ser
225                 230                 235                 240

Val Thr Leu Pro Val Glu Asn Ser Tyr His Pro Thr Ala Asn Gly Gln
                245                 250                 255

Ser Lys Gly Tyr Leu Pro Val Leu Asn Ser Ala Thr
            260                 265
```

<210> 15
<211> 336
<212> PRT
<213> Aeromonas salmonicida subsp. Salmonicida

<400> 15

Met Lys Lys Trp Phe Val Cys Leu Leu Gly Leu Ile Ala Leu Thr Val
1               5                   10                  15

Gln Ala Ala Asp Thr Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

```
Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Glu Thr Gln Tyr Glu Phe Leu Ala His Gly
                325                 330                 335
```

<210> 16
<211> 318
<212> PRT
<213> Aeromonas salmonicida

<400> 16

```
Ala Asp Thr Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Lys Gln Phe Pro Gly Leu Thr Ile Ala Asn Glu
    50                  55                  60
```

```
Ala Glu Gly Gly Ala Thr Ala Val Ala Tyr Asn Lys Ile Ser Trp Asp
65                  70                  75                  80

Pro Lys Tyr Gln Val Ile Asn Asn Leu Asp Tyr Glu Val Thr Gln Phe
                85                  90                  95

Leu Gln Lys Asp Ser Phe Lys Pro Asp Asp Leu Val Ile Leu Trp Val
            100                 105                 110

Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Gln Ile Leu Leu Phe Asn Leu Pro Asp Leu Gly Gln
145                 150                 155                 160

Asn Pro Ser Ala Arg Ser Gln Lys Val Val Glu Ala Val Ser His Val
                165                 170                 175

Ser Ala Tyr His Asn Lys Leu Leu Leu Asn Leu Ala Arg Gln Leu Ala
            180                 185                 190

Pro Thr Gly Met Val Lys Leu Phe Glu Ile Asp Lys Gln Phe Ala Glu
        195                 200                 205

Met Leu Arg Asp Pro Gln Asn Phe Gly Leu Ser Asp Val Glu Asn Pro
    210                 215                 220

Cys Tyr Asp Gly Gly Tyr Val Trp Lys Pro Phe Ala Thr Arg Ser Val
225                 230                 235                 240

Ser Thr Asp Arg Gln Leu Ser Ala Phe Ser Pro Gln Glu Arg Leu Ala
                245                 250                 255

Ile Ala Gly Asn Pro Leu Leu Ala Gln Ala Val Ala Ser Pro Met Ala
            260                 265                 270

Arg Arg Ser Ala Ser Pro Leu Asn Cys Glu Gly Lys Met Phe Trp Asp
        275                 280                 285

Gln Val His Pro Thr Thr Val Val His Ala Ala Leu Ser Glu Arg Ala
    290                 295                 300

Ala Thr Phe Ile Glu Thr Gln Tyr Glu Phe Leu Ala His Gly
305                 310                 315
```

<210> 17
<211> 465
<212> PRT
<213> Candida parapsilosis

<400> 17

```
Met Arg Tyr Phe Ala Ile Ala Phe Leu Leu Ile Asn Thr Ile Ser Ala
1               5                   10                  15

Phe Val Leu Ala Pro Lys Lys Pro Ser Gln Asp Asp Phe Tyr Thr Pro
            20                  25                  30

Pro Gln Gly Tyr Glu Ala Gln Pro Leu Gly Ser Ile Leu Lys Thr Arg
        35                  40                  45

Asn Val Pro Asn Pro Leu Thr Asn Val Phe Thr Pro Val Lys Val Gln
    50                  55                  60

Asn Ala Trp Gln Leu Leu Val Arg Ser Glu Asp Thr Phe Gly Asn Pro
65                  70                  75                  80

Asn Ala Ile Val Thr Thr Ile Ile Gln Pro Phe Asn Ala Lys Lys Asp
                85                  90                  95

Lys Leu Val Ser Tyr Gln Thr Phe Glu Asp Ser Gly Lys Leu Asp Cys
            100                 105                 110

Ala Pro Ser Tyr Ala Ile Gln Tyr Gly Ser Asp Ile Ser Thr Leu Thr
        115                 120                 125

Thr Gln Gly Glu Met Tyr Tyr Ile Ser Ala Leu Leu Asp Gln Gly Tyr
    130                 135                 140

Tyr Val Val Thr Pro Asp Tyr Glu Gly Pro Lys Ser Thr Phe Thr Val
145                 150                 155                 160

Gly Leu Gln Ser Gly Arg Ala Thr Leu Asn Ser Leu Arg Ala Thr Leu
                165                 170                 175

Lys Ser Gly Asn Leu Thr Gly Val Ser Ser Asp Ala Glu Thr Leu Leu
            180                 185                 190

Trp Gly Tyr Ser Gly Gly Ser Leu Ala Ser Gly Trp Ala Ala Ala Ile
        195                 200                 205

Gln Lys Glu Tyr Ala Pro Glu Leu Ser Lys Asn Leu Leu Gly Ala Ala
    210                 215                 220

Leu Gly Gly Phe Val Thr Asn Ile Thr Ala Thr Ala Glu Ala Val Asp
225                 230                 235                 240
```

Ser Gly Pro Phe Ala Gly Ile Ile Ser Asn Ala Leu Ala Gly Ile Gly
245 250 255

Asn Glu Tyr Pro Asp Phe Lys Asn Tyr Leu Leu Lys Lys Val Ser Pro
260 265 270

Leu Leu Ser Ile Thr Tyr Arg Leu Gly Asn Thr His Cys Leu Leu Asp
275 280 285

Gly Gly Ile Ala Tyr Phe Gly Lys Ser Phe Phe Ser Arg Ile Ile Arg
290 295 300

Tyr Phe Pro Asp Gly Trp Asp Leu Val Asn Gln Glu Pro Ile Lys Thr
305 310 315 320

Ile Leu Gln Asp Asn Gly Leu Val Tyr Gln Pro Lys Asp Leu Thr Pro
325 330 335

Gln Ile Pro Leu Phe Ile Tyr His Gly Thr Leu Asp Ala Ile Val Pro
340 345 350

Ile Val Asn Ser Arg Lys Thr Phe Gln Gln Trp Cys Asp Trp Gly Leu
355 360 365

Lys Ser Gly Glu Tyr Asn Glu Asp Leu Thr Asn Gly His Ile Thr Glu
370 375 380

Ser Ile Val Gly Ala Pro Ala Ala Leu Thr Trp Ile Ile Asn Arg Phe
385 390 395 400

Asn Gly Gln Pro Pro Val Asp Gly Cys Gln His Asn Val Arg Ala Ser
405 410 415

Asn Leu Glu Tyr Pro Gly Thr Pro Gln Ser Ile Lys Asn Tyr Phe Glu
420 425 430

Ala Ala Leu His Ala Ile Leu Gly Phe Asp Leu Gly Pro Asp Val Lys
435 440 445

Arg Asp Lys Val Thr Leu Gly Gly Leu Leu Lys Leu Glu Arg Phe Ala
450 455 460

Phe
465

<210> 18
<211> 471

<212> PRT
<213> Candida parapsilosis

<400> 18

```
Met Arg Tyr Phe Ala Ile Ala Phe Leu Leu Ile Asn Thr Ile Ser Ala
1               5                   10                  15

Phe Val Leu Ala Pro Lys Lys Pro Ser Gln Asp Asp Phe Tyr Thr Pro
            20                  25                  30

Pro Gln Gly Tyr Glu Ala Gln Pro Leu Gly Ser Ile Leu Lys Thr Arg
        35                  40                  45

Asn Val Pro Asn Pro Leu Thr Asn Val Phe Thr Pro Val Lys Val Gln
    50                  55                  60

Asn Ala Trp Gln Leu Leu Val Arg Ser Glu Asp Thr Phe Gly Asn Pro
65                  70                  75                  80

Asn Ala Ile Val Thr Thr Ile Ile Gln Pro Phe Asn Ala Lys Lys Asp
                85                  90                  95

Lys Leu Val Ser Tyr Gln Thr Phe Glu Asp Ser Gly Lys Leu Asp Cys
            100                 105                 110

Ala Pro Ser Tyr Ala Ile Gln Tyr Gly Ser Asp Ile Ser Thr Leu Thr
        115                 120                 125

Thr Gln Gly Glu Met Tyr Tyr Ile Ser Ala Leu Leu Asp Gln Gly Tyr
    130                 135                 140

Tyr Val Val Thr Pro Asp Tyr Glu Gly Pro Lys Ser Thr Phe Thr Val
145                 150                 155                 160

Gly Leu Gln Ser Gly Arg Ala Thr Leu Asn Ser Leu Arg Ala Thr Leu
                165                 170                 175

Lys Ser Gly Asn Leu Thr Gly Val Ser Ser Asp Ala Glu Thr Leu Leu
            180                 185                 190

Trp Gly Tyr Ser Gly Gly Ser Leu Ala Ser Gly Trp Ala Ala Ala Ile
        195                 200                 205

Gln Lys Glu Tyr Ala Pro Glu Leu Ser Lys Asn Leu Leu Gly Ala Ala
    210                 215                 220

Leu Gly Gly Phe Val Thr Asn Ile Thr Ala Thr Ala Glu Ala Val Asp
225                 230                 235                 240

Ser Gly Pro Phe Ala Gly Ile Ile Ser Asn Ala Leu Ala Gly Ile Gly
                245                 250                 255
```

```
Asn Glu Tyr Pro Asp Phe Lys Asn Tyr Leu Leu Lys Lys Val Ser Pro
            260                 265                 270

Leu Leu Ser Ile Thr Tyr Arg Leu Gly Asn Thr His Cys Leu Leu Asp
        275                 280                 285

Gly Gly Ile Ala Tyr Phe Gly Lys Ser Phe Phe Ser Arg Ile Ile Arg
    290                 295                 300

Tyr Phe Pro Asp Gly Trp Asp Leu Val Asn Gln Glu Pro Ile Lys Thr
305                 310                 315                 320

Ile Leu Gln Asp Asn Gly Leu Val Tyr Gln Pro Lys Asp Leu Thr Pro
                325                 330                 335

Gln Ile Pro Leu Phe Ile Tyr His Gly Thr Leu Asp Ala Ile Val Pro
            340                 345                 350

Ile Val Asn Ser Arg Lys Thr Phe Gln Gln Trp Cys Asp Trp Gly Leu
        355                 360                 365

Lys Ser Gly Glu Tyr Asn Glu Asp Leu Thr Asn Gly His Ile Thr Glu
    370                 375                 380

Ser Ile Val Gly Ala Pro Ala Ala Leu Thr Trp Ile Ile Asn Arg Phe
385                 390                 395                 400

Asn Gly Gln Pro Pro Val Asp Gly Cys Gln His Asn Val Arg Ala Ser
                405                 410                 415

Asn Leu Glu Tyr Pro Gly Thr Pro Gln Ser Ile Lys Asn Tyr Phe Glu
            420                 425                 430

Ala Ala Leu His Ala Ile Leu Gly Phe Asp Leu Gly Pro Asp Val Lys
        435                 440                 445

Arg Asp Lys Val Thr Leu Gly Gly Leu Leu Lys Leu Glu Arg Phe Ala
    450                 455                 460

Phe His His His His His His
465                 470
```

<210> 19
<211> 261
<212> PRT
<213> Streptomyces coelicolor

<400> 19

```
Met Ile Gly Ser Tyr Val Ala Val Gly Asp Ser Phe Thr Glu Gly Val
1               5                   10                  15

Gly Asp Pro Gly Pro Asp Gly Ala Phe Val Gly Trp Ala Asp Arg Leu
            20                  25                  30

Ala Val Leu Leu Ala Asp Arg Arg Pro Glu Gly Asp Phe Thr Tyr Thr
        35                  40                  45

Asn Leu Ala Val Arg Gly Arg Leu Leu Asp Gln Ile Val Ala Glu Gln
    50                  55                  60

Val Pro Arg Val Val Gly Leu Ala Pro Asp Leu Val Ser Phe Ala Ala
65                  70                  75                  80

Gly Gly Asn Asp Ile Ile Arg Pro Gly Thr Asp Pro Asp Glu Val Ala
                85                  90                  95

Glu Arg Phe Glu Leu Ala Val Ala Ala Leu Thr Ala Ala Ala Gly Thr
            100                 105                 110

Val Leu Val Thr Thr Gly Phe Asp Thr Arg Gly Val Pro Val Leu Lys
        115                 120                 125

His Leu Arg Gly Lys Ile Ala Thr Tyr Asn Gly His Val Arg Ala Ile
    130                 135                 140

Ala Asp Arg Tyr Gly Cys Pro Val Leu Asp Leu Trp Ser Leu Arg Ser
145                 150                 155                 160

Val Gln Asp Arg Arg Ala Trp Asp Ala Asp Arg Leu His Leu Ser Pro
                165                 170                 175

Glu Gly His Thr Arg Val Ala Leu Arg Ala Gly Gln Ala Leu Gly Leu
            180                 185                 190

Arg Val Pro Ala Asp Pro Asp Gln Pro Trp Pro Pro Leu Pro Pro Arg
        195                 200                 205

Gly Thr Leu Asp Val Arg Arg Asp Asp Val His Trp Ala Arg Glu Tyr
    210                 215                 220

Leu Val Pro Trp Ile Gly Arg Arg Leu Arg Gly Glu Ser Ser Gly Asp
225                 230                 235                 240

His Val Thr Ala Lys Gly Thr Leu Ser Pro Asp Ala Ile Lys Thr Arg
                245                 250                 255

Ile Ala Ala Val Ala
```

260

<210> 20
<211> 5
<212> PRT
<213> Artificial Sequence

<220>
<223> Sequence motif

<220>
<221> VARIANT
<222> (2)..(2)
<223> Xaa may be Ala or Gly

<220>
<221> VARIANT
<222> (5)..(5)
<223> Xaa may be Ala, Leu or Tyr

<400> 20

Gly Xaa Asn Asp Xaa
1               5

<210> 21
<211> 18
<212> PRT
<213> Aeromonas sp.

<400> 21

Met Lys Lys Trp Phe Val Cys Leu Leu Gly Leu Ile Ala Leu Thr Val
1               5                   10                  15

Gln Ala

<210> 22
<211> 29
<212> PRT
<213> Bacillus subtilis

<400> 22

Met Arg Ser Lys Lys Leu Trp Ile Ser Leu Leu Phe Ala Leu Thr Leu
1               5                   10                  15

Ile Phe Thr Met Ala Phe Ser Asn Met Ser Ala Gln Ala
            20                  25

<210> 23
<211> 29
<212> PRT
<213> Bacillus licheniformis

<400> 23

```
Met Met Arg Lys Lys Ser Phe Trp Phe Gly Met Leu Thr Ala Phe Met
1               5                   10                  15

Leu Val Phe Thr Met Glu Phe Ser Asp Ser Ala Ser Ala
            20                  25
```

<210> 24
<211> 1047
<212> DNA
<213> Aeromonas hydrophila

<400> 24

```
atgtttaagt ttaaaaagaa tttcttagtt ggattatcgg cagctttaat gagtattagc      60
ttgttttcgg caaccgcctc tgcagctagc gccgacagcc gtcccgcctt ttcccggatc     120
gtgatgttcg gcgacagcct ctccgatacc ggcaaaatgt acagcaagat gcgcggttac     180
ctcccctcca gcccgcccta ctatgagggc cgtttctcca acggacccgt ctggctggag     240
cagctgacca aacagttccc gggtctgacc atcgccaacg aagcggaagg cggtgccact     300
gccgtggctt acaacaagat ctcctggaat cccaagtatc aggtcatcaa caacctggac     360
tacgaggtca cccagttctt gcagaaagac agcttcaagc cggacgatct ggtgatcctc     420
tgggtcggtg ccaatgacta tctggcctat ggctggaaca cggagcagga tgccaagcgg     480
gttcgcgatg ccatcagcga tgcggccaac cgcatggtac tgaacggtgc caagcagata     540
ctgctgttca acctgccgga tctgggccag aacccgtcag ctcgcagtca gaaggtggtc     600
gaggcggtca gccatgtctc cgcctatcac aaccagctgc tgctgaacct ggcacgccag     660
ctggccccca ccggcatggt aaagctgttc gagatcgaca agcaatttgc cgagatgctg     720
cgtgatccgc agaacttcgg cctgagcgac gtcgagaacc cctgctacga cggcggctat     780
gtgtggaagc cgtttgccac ccgcagcgtc agcaccgacc gccagctctc cgccttcagt     840
ccgcaggaac gcctcgccat cgccggcaac ccgctgctgg cacaggccgt tgccagtcct     900
atggcccgcc gcagcgccag cccccctcaac tgtgagggca agatgttctg ggatcaggta     960
cacccgacca ctgtcgtgca cgcagccctg agcgagcgcg ccgccacctt catcgcgaac    1020
cagtacgagt tcctcgccca ctgatga                                        1047
```

<210> 25
<211> 347
<212> PRT
<213> Artificial Sequence

<220>
<223> Fusion construct used for mutagenesis

<400> 25

```
Met Phe Lys Phe Lys Lys Asn Phe Leu Val Gly Leu Ser Ala Ala Leu
1               5                   10                  15
```

```
Met Ser Ile Ser Leu Phe Ser Ala Thr Ala Ser Ala Ala Ser Ala Asp
            20                  25                  30

Ser Arg Pro Ala Phe Ser Arg Ile Val Met Phe Gly Asp Ser Leu Ser
        35                  40                  45

Asp Thr Gly Lys Met Tyr Ser Lys Met Arg Gly Tyr Leu Pro Ser Ser
    50                  55                  60

Pro Pro Tyr Tyr Glu Gly Arg Phe Ser Asn Gly Pro Val Trp Leu Glu
65                  70                  75                  80

Gln Leu Thr Lys Gln Phe Pro Gly Leu Thr Ile Ala Asn Glu Ala Glu
                85                  90                  95

Gly Gly Ala Thr Ala Val Ala Tyr Asn Lys Ile Ser Trp Asn Pro Lys
            100                 105                 110

Tyr Gln Val Ile Asn Asn Leu Asp Tyr Glu Val Thr Gln Phe Leu Gln
        115                 120                 125

Lys Asp Ser Phe Lys Pro Asp Asp Leu Val Ile Leu Trp Val Gly Ala
    130                 135                 140

Asn Asp Tyr Leu Ala Tyr Gly Trp Asn Thr Glu Gln Asp Ala Lys Arg
145                 150                 155                 160

Val Arg Asp Ala Ile Ser Asp Ala Ala Asn Arg Met Val Leu Asn Gly
                165                 170                 175

Ala Lys Gln Ile Leu Leu Phe Asn Leu Pro Asp Leu Gly Gln Asn Pro
            180                 185                 190

Ser Ala Arg Ser Gln Lys Val Val Glu Ala Val Ser His Val Ser Ala
        195                 200                 205

Tyr His Asn Gln Leu Leu Leu Asn Leu Ala Arg Gln Leu Ala Pro Thr
    210                 215                 220

Gly Met Val Lys Leu Phe Glu Ile Asp Lys Gln Phe Ala Glu Met Leu
225                 230                 235                 240

Arg Asp Pro Gln Asn Phe Gly Leu Ser Asp Val Glu Asn Pro Cys Tyr
                245                 250                 255

Asp Gly Gly Tyr Val Trp Lys Pro Phe Ala Thr Arg Ser Val Ser Thr
            260                 265                 270

Asp Arg Gln Leu Ser Ala Phe Ser Pro Gln Glu Arg Leu Ala Ile Ala
```

```
                275                 280                 285

        Gly Asn Pro Leu Leu Ala Gln Ala Val Ala Ser Pro Met Ala Arg Arg
            290                 295                 300

        Ser Ala Ser Pro Leu Asn Cys Glu Gly Lys Met Phe Trp Asp Gln Val
        305                 310                 315                 320

        His Pro Thr Thr Val Val His Ala Ala Leu Ser Glu Arg Ala Ala Thr
                        325                 330                 335

        Phe Ile Ala Asn Gln Tyr Glu Phe Leu Ala His
                    340                 345
```

<210> 26
<211> 267
<212> PRT
<213> Streptomyces sp.

<400> 26

Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30

Ala Tyr Val Ala Leu Gly Asp Ser Tyr Ser Ser Gly Asn Gly Ala Gly
        35                  40                  45

Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                  80

Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160

Ala Pro Asn Ala Arg Val Val Val Leu Gly Tyr Pro Arg Met Tyr Leu
165 170 175

Ala Ser Asn Pro Trp Tyr Cys Leu Gly Leu Ser Asn Thr Lys Arg Ala
180 185 190

Ala Ile Asn Thr Thr Ala Asp Thr Leu Asn Ser Val Ile Ser Ser Arg
195 200 205

Ala Thr Ala His Gly Phe Arg Phe Gly Asp Val Arg Pro Thr Phe Asn
210 215 220

Asn His Glu Leu Phe Phe Gly Asn Asp Trp Leu His Ser Leu Thr Leu
225 230 235 240

Pro Val Trp Glu Ser Tyr His Pro Thr Ser Thr Gly His Gln Ser Gly
245 250 255

Tyr Leu Pro Val Leu Asn Ala Asn Ser Ser Thr
260 265

<210> 27
<211> 548
<212> PRT
<213> Thermobifida sp.

<400> 27

```
Met Leu Pro His Pro Ala Gly Glu Arg Gly Glu Val Gly Ala Phe Phe
1               5                   10                  15

Ala Leu Leu Val Gly Thr Pro Gln Asp Arg Arg Leu Arg Leu Glu Cys
            20                  25                  30

His Glu Thr Arg Pro Leu Arg Gly Arg Cys Gly Cys Gly Glu Arg Arg
        35                  40                  45

Val Pro Pro Leu Thr Leu Pro Gly Asp Gly Val Leu Cys Thr Thr Ser
    50                  55                  60

Ser Thr Arg Asp Ala Glu Thr Val Trp Arg Lys His Leu Gln Pro Arg
65                  70                  75                  80

Pro Asp Gly Gly Phe Arg Pro His Leu Gly Val Gly Cys Leu Leu Ala
                85                  90                  95

Gly Gln Gly Ser Pro Gly Val Leu Trp Cys Gly Arg Glu Gly Cys Arg
            100                 105                 110

Phe Glu Val Cys Arg Arg Asp Thr Pro Gly Leu Ser Arg Thr Arg Asn
```

```
        115                 120                 125

Gly Asp Ser Ser Pro Pro Phe Arg Ala Gly Trp Ser Leu Pro Pro Lys
    130                 135                 140

Cys Gly Glu Ile Ser Gln Ser Ala Arg Lys Thr Pro Ala Val Pro Arg
145                 150                 155                 160

Tyr Ser Leu Leu Arg Thr Asp Arg Pro Asp Gly Pro Arg Gly Arg Phe
                165                 170                 175

Val Gly Ser Gly Pro Arg Ala Ala Thr Arg Arg Arg Leu Phe Leu Gly
            180                 185                 190

Ile Pro Ala Leu Val Leu Val Thr Ala Leu Thr Leu Val Leu Ala Val
        195                 200                 205

Pro Thr Gly Arg Glu Thr Leu Trp Arg Met Trp Cys Glu Ala Thr Gln
    210                 215                 220

Asp Trp Cys Leu Gly Val Pro Val Asp Ser Arg Gly Gln Pro Ala Glu
225                 230                 235                 240

Asp Gly Glu Phe Leu Leu Leu Ser Pro Val Gln Ala Ala Thr Trp Gly
                245                 250                 255

Asn Tyr Tyr Ala Leu Gly Asp Ser Tyr Ser Ser Gly Asp Gly Ala Arg
            260                 265                 270

Asp Tyr Tyr Pro Gly Thr Ala Val Lys Gly Gly Cys Trp Arg Ser Ala
        275                 280                 285

Asn Ala Tyr Pro Glu Leu Val Ala Glu Ala Tyr Asp Phe Ala Gly His
    290                 295                 300

Leu Ser Phe Leu Ala Cys Ser Gly Gln Arg Gly Tyr Ala Met Leu Asp
305                 310                 315                 320

Ala Ile Asp Glu Val Gly Ser Gln Leu Asp Trp Asn Ser Pro His Thr
                325                 330                 335

Ser Leu Val Thr Ile Gly Ile Gly Gly Asn Asp Leu Gly Phe Ser Thr
            340                 345                 350

Val Leu Lys Thr Cys Met Val Arg Val Pro Leu Leu Asp Ser Lys Ala
        355                 360                 365

Cys Thr Asp Gln Glu Asp Ala Ile Arg Lys Arg Met Ala Lys Phe Glu
    370                 375                 380
```

```
Thr Thr Phe Glu Glu Leu Ile Ser Glu Val Arg Thr Arg Ala Pro Asp
385                 390                 395                 400

Ala Arg Ile Leu Val Val Gly Tyr Pro Arg Ile Phe Pro Glu Glu Pro
                405                 410                 415

Thr Gly Ala Tyr Tyr Thr Leu Thr Ala Ser Asn Gln Arg Trp Leu Asn
            420                 425                 430

Glu Thr Ile Gln Glu Phe Asn Gln Gln Leu Ala Glu Ala Val Ala Val
        435                 440                 445

His Asp Glu Glu Ile Ala Ala Ser Gly Gly Val Gly Ser Val Glu Phe
    450                 455                 460

Val Asp Val Tyr His Ala Leu Asp Gly His Glu Ile Gly Ser Asp Glu
465                 470                 475                 480

Pro Trp Val Asn Gly Val Gln Leu Arg Asp Leu Ala Thr Gly Val Thr
                485                 490                 495

Val Asp Arg Ser Thr Phe His Pro Asn Ala Ala Gly His Arg Ala Val
            500                 505                 510

Gly Glu Arg Val Ile Glu Gln Ile Glu Thr Gly Pro Gly Arg Pro Leu
        515                 520                 525

Tyr Ala Thr Phe Ala Val Val Ala Gly Ala Thr Val Asp Thr Leu Ala
    530                 535                 540

Gly Glu Val Gly
545
```

<210> 28
<211> 372
<212> PRT
<213> Thermobifida sp.

<400> 28

```
Met Gly Ser Gly Pro Arg Ala Ala Thr Arg Arg Arg Leu Phe Leu Gly
1               5                   10                  15

Ile Pro Ala Leu Val Leu Val Thr Ala Leu Thr Leu Val Leu Ala Val
            20                  25                  30

Pro Thr Gly Arg Glu Thr Leu Trp Arg Met Trp Cys Glu Ala Thr Gln
        35                  40                  45
```

```
Asp Trp Cys Leu Gly Val Pro Val Asp Ser Arg Gly Gln Pro Ala Glu
    50                  55                  60

Asp Gly Glu Phe Leu Leu Leu Ser Pro Val Gln Ala Ala Thr Trp Gly
65                  70                  75                  80

Asn Tyr Tyr Ala Leu Gly Asp Ser Tyr Ser Ser Gly Asp Gly Ala Arg
                85                  90                  95

Asp Tyr Tyr Pro Gly Thr Ala Val Lys Gly Gly Cys Trp Arg Ser Ala
            100                 105                 110

Asn Ala Tyr Pro Glu Leu Val Ala Glu Ala Tyr Asp Phe Ala Gly His
        115                 120                 125

Leu Ser Phe Leu Ala Cys Ser Gly Gln Arg Gly Tyr Ala Met Leu Asp
    130                 135                 140

Ala Ile Asp Glu Val Gly Ser Gln Leu Asp Trp Asn Ser Pro His Thr
145                 150                 155                 160

Ser Leu Val Thr Ile Gly Ile Gly Gly Asn Asp Leu Gly Phe Ser Thr
                165                 170                 175

Val Leu Lys Thr Cys Met Val Arg Val Pro Leu Leu Asp Ser Lys Ala
            180                 185                 190

Cys Thr Asp Gln Glu Asp Ala Ile Arg Lys Arg Met Ala Lys Phe Glu
        195                 200                 205

Thr Thr Phe Glu Glu Leu Ile Ser Glu Val Arg Thr Arg Ala Pro Asp
    210                 215                 220

Ala Arg Ile Leu Val Val Gly Tyr Pro Arg Ile Phe Pro Glu Glu Pro
225                 230                 235                 240

Thr Gly Ala Tyr Tyr Thr Leu Thr Ala Ser Asn Gln Arg Trp Leu Asn
                245                 250                 255

Glu Thr Ile Gln Glu Phe Asn Gln Gln Leu Ala Glu Ala Val Ala Val
            260                 265                 270

His Asp Glu Glu Ile Ala Ala Ser Gly Gly Val Gly Ser Val Glu Phe
        275                 280                 285

Val Asp Val Tyr His Ala Leu Asp Gly His Glu Ile Gly Ser Asp Glu
    290                 295                 300

Pro Trp Val Asn Gly Val Gln Leu Arg Asp Leu Ala Thr Gly Val Thr
```

305 310 315 320

Val Asp Arg Ser Thr Phe His Pro Asn Ala Ala Gly His Arg Ala Val
325 330 335

Gly Glu Arg Val Ile Glu Gln Ile Glu Thr Gly Pro Gly Arg Pro Leu
340 345 350

Tyr Ala Thr Phe Ala Val Val Ala Gly Ala Thr Val Asp Thr Leu Ala
355 360 365

Gly Glu Val Gly
370

<210> 29
<211> 300
<212> PRT
<213> Corynebacterium efficiens

<400> 29

```
Met Arg Thr Thr Val Ile Ala Ala Ser Ala Leu Leu Leu Leu Ala Gly
1               5                   10                  15

Cys Ala Asp Gly Ala Arg Glu Glu Thr Ala Gly Ala Pro Pro Gly Glu
            20                  25                  30

Ser Ser Gly Gly Ile Arg Glu Glu Gly Ala Glu Ala Ser Thr Ser Ile
        35                  40                  45

Thr Asp Val Tyr Ile Ala Leu Gly Asp Ser Tyr Ala Ala Met Gly Gly
    50                  55                  60

Arg Asp Gln Pro Leu Arg Gly Glu Pro Phe Cys Leu Arg Ser Ser Gly
65                  70                  75                  80

Asn Tyr Pro Glu Leu Leu His Ala Glu Val Thr Asp Leu Thr Cys Gln
                85                  90                  95

Gly Ala Val Thr Gly Asp Leu Leu Glu Pro Arg Thr Leu Gly Glu Arg
            100                 105                 110

Thr Leu Pro Ala Gln Val Asp Ala Leu Thr Glu Asp Thr Thr Leu Val
        115                 120                 125

Thr Leu Ser Ile Gly Gly Asn Asp Leu Gly Phe Gly Glu Val Ala Gly
    130                 135                 140

Cys Ile Arg Glu Arg Ile Ala Gly Glu Asn Ala Asp Asp Cys Val Asp
145                 150                 155                 160
```

```
Leu Leu Gly Glu Thr Ile Gly Glu Gln Leu Asp Gln Leu Pro Pro Gln
                165                 170                 175

Leu Asp Arg Val His Glu Ala Ile Arg Asp Arg Ala Gly Asp Ala Gln
            180                 185                 190

Val Val Val Thr Gly Tyr Leu Pro Leu Val Ser Ala Gly Asp Cys Pro
        195                 200                 205

Glu Leu Gly Asp Val Ser Glu Ala Asp Arg Arg Trp Ala Val Glu Leu
    210                 215                 220

Thr Gly Gln Ile Asn Glu Thr Val Arg Glu Ala Ala Glu Arg His Asp
225                 230                 235                 240

Ala Leu Phe Val Leu Pro Asp Asp Ala Asp Glu His Thr Ser Cys Ala
                245                 250                 255

Pro Pro Gln Gln Arg Trp Ala Asp Ile Gln Gly Gln Gln Thr Asp Ala
            260                 265                 270

Tyr Pro Leu His Pro Thr Ser Ala Gly His Glu Ala Met Ala Ala Ala
        275                 280                 285

Val Arg Asp Ala Leu Gly Leu Glu Pro Val Gln Pro
    290                 295                 300
```

<210> 30
<211> 284
<212> PRT
<213> Novosphingobium aromaticivorans

<400> 30

Met Gly Gln Val Lys Leu Phe Ala Arg Arg Cys Ala Pro Val Leu Leu
1 5 10 15

Ala Leu Ala Gly Leu Ala Pro Ala Ala Thr Val Ala Arg Glu Ala Pro
20 25 30

Leu Ala Glu Gly Ala Arg Tyr Val Ala Leu Gly Ser Ser Phe Ala Ala
35 40 45

Gly Pro Gly Val Gly Pro Asn Ala Pro Gly Ser Pro Glu Arg Cys Gly
50 55 60

Arg Gly Thr Leu Asn Tyr Pro His Leu Leu Ala Glu Ala Leu Lys Leu
65 70 75 80

Asp Leu Val Asp Ala Thr Cys Ser Gly Ala Thr Thr His His Val Leu

```
                85                  90                  95

Gly Pro Trp Asn Glu Val Pro Pro Gln Ile Asp Ser Val Asn Gly Asp
            100                 105                 110

Thr Arg Leu Val Thr Leu Thr Ile Gly Gly Asn Asp Val Ser Phe Val
        115                 120                 125

Gly Asn Ile Phe Ala Ala Ala Cys Glu Lys Met Ala Ser Pro Asp Pro
    130                 135                 140

Arg Cys Gly Lys Trp Arg Glu Ile Thr Glu Glu Glu Trp Gln Ala Asp
145                 150                 155                 160

Glu Glu Arg Met Arg Ser Ile Val Arg Gln Ile His Ala Arg Ala Pro
                165                 170                 175

Leu Ala Arg Val Val Val Val Asp Tyr Ile Thr Val Leu Pro Pro Ser
            180                 185                 190

Gly Thr Cys Ala Ala Met Ala Ile Ser Pro Asp Arg Leu Ala Gln Ser
        195                 200                 205

Arg Ser Ala Ala Lys Arg Leu Ala Arg Ile Thr Ala Arg Val Ala Arg
    210                 215                 220

Glu Glu Gly Ala Ser Leu Leu Lys Phe Ser His Ile Ser Arg Arg His
225                 230                 235                 240

His Pro Cys Ser Ala Lys Pro Trp Ser Asn Gly Leu Ser Ala Pro Ala
                245                 250                 255

Asp Asp Gly Ile Pro Val His Pro Asn Arg Leu Gly His Ala Glu Ala
            260                 265                 270

Ala Ala Ala Leu Val Lys Leu Val Lys Leu Met Lys
        275                 280
```

<210> 31
<211> 268
<212> PRT
<213> Streptomyces coelicolor

<400> 31

```
Met Arg Arg Phe Arg Leu Val Gly Phe Leu Ser Ser Leu Val Leu Ala
1               5                   10                  15

Ala Gly Ala Ala Leu Thr Gly Ala Ala Thr Ala Gln Ala Ala Gln Pro
            20                  25                  30
```

```
Ala Ala Ala Asp Gly Tyr Val Ala Leu Gly Asp Ser Tyr Ser Ser Gly
        35                  40                  45

Val Gly Ala Gly Ser Tyr Ile Ser Ser Ser Gly Asp Cys Lys Arg Ser
    50                  55                  60

Thr Lys Ala His Pro Tyr Leu Trp Ala Ala Ala His Ser Pro Ser Thr
65                  70                  75                  80

Phe Asp Phe Thr Ala Cys Ser Gly Ala Arg Thr Gly Asp Val Leu Ser
                85                  90                  95

Gly Gln Leu Gly Pro Leu Ser Ser Gly Thr Gly Leu Val Ser Ile Ser
            100                 105                 110

Ile Gly Gly Asn Asp Ala Gly Phe Ala Asp Thr Met Thr Thr Cys Val
        115                 120                 125

Leu Gln Ser Glu Ser Ser Cys Leu Ser Arg Ile Ala Thr Ala Glu Ala
    130                 135                 140

Tyr Val Asp Ser Thr Leu Pro Gly Lys Leu Asp Gly Val Tyr Ser Ala
145                 150                 155                 160

Ile Ser Asp Lys Ala Pro Asn Ala His Val Val Val Ile Gly Tyr Pro
                165                 170                 175

Arg Phe Tyr Lys Leu Gly Thr Thr Cys Ile Gly Leu Ser Glu Thr Lys
            180                 185                 190

Arg Thr Ala Ile Asn Lys Ala Ser Asp His Leu Asn Thr Val Leu Ala
        195                 200                 205

Gln Arg Ala Ala Ala His Gly Phe Thr Phe Gly Asp Val Arg Thr Thr
    210                 215                 220

Phe Thr Gly His Glu Leu Cys Ser Gly Ser Pro Trp Leu His Ser Val
225                 230                 235                 240

Asn Trp Leu Asn Ile Gly Glu Ser Tyr His Pro Thr Ala Ala Gly Gln
                245                 250                 255

Ser Gly Gly Tyr Leu Pro Val Leu Asn Gly Ala Ala
            260                 265
```

<210> 32
<211> 269

<212> PRT
<213> Streptomyces avermitilis

<400> 32

```
Met Arg Arg Ser Arg Ile Thr Ala Tyr Val Thr Ser Leu Leu Leu Ala
1               5                   10                  15

Val Gly Cys Ala Leu Thr Gly Ala Ala Thr Ala Gln Ala Ser Pro Ala
            20                  25                  30

Ala Ala Ala Thr Gly Tyr Val Ala Leu Gly Asp Ser Tyr Ser Ser Gly
        35                  40                  45

Val Gly Ala Gly Ser Tyr Leu Ser Ser Ser Gly Asp Cys Lys Arg Ser
    50                  55                  60

Ser Lys Ala Tyr Pro Tyr Leu Trp Gln Ala Ala His Ser Pro Ser Ser
65                  70                  75                  80

Phe Ser Phe Met Ala Cys Ser Gly Ala Arg Thr Gly Asp Val Leu Ala
                85                  90                  95

Asn Gln Leu Gly Thr Leu Asn Ser Ser Thr Gly Leu Val Ser Leu Thr
            100                 105                 110

Ile Gly Gly Asn Asp Ala Gly Phe Ser Asp Val Met Thr Thr Cys Val
        115                 120                 125

Leu Gln Ser Asp Ser Ala Cys Leu Ser Arg Ile Asn Thr Ala Lys Ala
    130                 135                 140

Tyr Val Asp Ser Thr Leu Pro Gly Gln Leu Asp Ser Val Tyr Thr Ala
145                 150                 155                 160

Ile Ser Thr Lys Ala Pro Ser Ala His Val Ala Val Leu Gly Tyr Pro
                165                 170                 175

Arg Phe Tyr Lys Leu Gly Gly Ser Cys Leu Ala Gly Leu Ser Glu Thr
            180                 185                 190

Lys Arg Ser Ala Ile Asn Asp Ala Ala Asp Tyr Leu Asn Ser Ala Ile
        195                 200                 205

Ala Lys Arg Ala Ala Asp His Gly Phe Thr Phe Gly Asp Val Lys Ser
    210                 215                 220

Thr Phe Thr Gly His Glu Ile Cys Ser Ser Ser Thr Trp Leu His Ser
225                 230                 235                 240

Leu Asp Leu Leu Asn Ile Gly Gln Ser Tyr His Pro Thr Ala Ala Gly
                245                 250                 255
```

Gln Ser Gly Gly Tyr Leu Pro Val Met Asn Ser Val Ala
260 265

<210> 33
<211> 267
<212> PRT
<213> Streptomyces sp.

<400> 33

Met Arg Leu Thr Arg Ser Leu Ser Ala Ala Ser Val Ile Val Phe Ala
1 5 10 15

Leu Leu Leu Ala Leu Leu Gly Ile Ser Pro Ala Gln Ala Ala Gly Pro
20 25 30

Ala Tyr Val Ala Leu Gly Asp Ser Tyr Ser Ser Gly Asn Gly Ala Gly
35 40 45

Ser Tyr Ile Asp Ser Ser Gly Asp Cys His Arg Ser Asn Asn Ala Tyr
50 55 60

Pro Ala Arg Trp Ala Ala Ala Asn Ala Pro Ser Ser Phe Thr Phe Ala
65 70 75 80

Ala Cys Ser Gly Ala Val Thr Thr Asp Val Ile Asn Asn Gln Leu Gly
85 90 95

Ala Leu Asn Ala Ser Thr Gly Leu Val Ser Ile Thr Ile Gly Gly Asn
100 105 110

Asp Ala Gly Phe Ala Asp Ala Met Thr Thr Cys Val Thr Ser Ser Asp
115 120 125

Ser Thr Cys Leu Asn Arg Leu Ala Thr Ala Thr Asn Tyr Ile Asn Thr
130 135 140

Thr Leu Leu Ala Arg Leu Asp Ala Val Tyr Ser Gln Ile Lys Ala Arg
145 150 155 160

Ala Pro Asn Ala Arg Val Val Val Leu Gly Tyr Pro Arg Met Tyr Leu
165 170 175

Ala Ser Asn Pro Trp Tyr Cys Leu Gly Leu Ser Asn Thr Lys Arg Ala
180 185 190

Ala Ile Asn Thr Thr Ala Asp Thr Leu Asn Ser Val Ile Ser Ser Arg
195 200 205

Ala Thr Ala His Gly Phe Arg Phe Gly Asp Val Arg Pro Thr Phe Asn
210 215 220

Asn His Glu Leu Phe Phe Gly Asn Asp Trp Leu His Ser Leu Thr Leu
225 230 235 240

Pro Val Trp Glu Ser Tyr His Pro Thr Ser Thr Gly His Gln Ser Gly
245 250 255

Tyr Leu Pro Val Leu Asn Ala Asn Ser Ser Thr
260 265

<210> 34
<211> 317
<212> PRT
<213> Aeromonas hydrophila

<400> 34

```
Ala Asp Ser Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Lys Gln Phe Pro Gly Leu Thr Ile Ala Asn Glu
    50                  55                  60

Ala Glu Gly Gly Ala Thr Ala Val Ala Tyr Asn Lys Ile Ser Trp Asn
65                  70                  75                  80

Pro Lys Tyr Gln Val Ile Asn Asn Leu Asp Tyr Glu Val Thr Gln Phe
                85                  90                  95

Leu Gln Lys Asp Ser Phe Lys Pro Asp Asp Leu Val Ile Leu Trp Val
            100                 105                 110

Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Gln Ile Leu Leu Phe Asn Leu Pro Asp Leu Gly Gln
145                 150                 155                 160

Asn Pro Ser Ala Arg Ser Gln Lys Val Val Glu Ala Val Ser His Val
                165                 170                 175
```

```
Ser Ala Tyr His Asn Gln Leu Leu Leu Asn Leu Ala Arg Gln Leu Ala
            180                 185                 190

Pro Thr Gly Met Val Lys Leu Phe Glu Ile Asp Lys Gln Phe Ala Glu
        195                 200                 205

Met Leu Arg Asp Pro Gln Asn Phe Gly Leu Ser Asp Val Glu Asn Pro
    210                 215                 220

Cys Tyr Asp Gly Gly Tyr Val Trp Lys Pro Phe Ala Thr Arg Ser Val
225                 230                 235                 240

Ser Thr Asp Arg Gln Leu Ser Ala Phe Ser Pro Gln Glu Arg Leu Ala
                245                 250                 255

Ile Ala Gly Asn Pro Leu Leu Ala Gln Ala Val Ala Ser Pro Met Ala
            260                 265                 270

Arg Arg Ser Ala Ser Pro Leu Asn Cys Glu Gly Lys Met Phe Trp Asp
        275                 280                 285

Gln Val His Pro Thr Thr Val Val His Ala Ala Leu Ser Glu Arg Ala
    290                 295                 300

Ala Thr Phe Ile Ala Asn Gln Tyr Glu Phe Leu Ala His
305                 310                 315
```

<210> 35
<211> 318
<212> PRT
<213> Aeromonas salmonicida

<400> 35

```
Ala Asp Thr Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Lys Gln Phe Pro Gly Leu Thr Ile Ala Asn Glu
    50                  55                  60

Ala Glu Gly Gly Ala Thr Ala Val Ala Tyr Asn Lys Ile Ser Trp Asn
65                  70                  75                  80
```

```
Pro Lys Tyr Gln Val Ile Asn Asn Leu Asp Tyr Glu Val Thr Gln Phe
                85                  90                  95

Leu Gln Lys Asp Ser Phe Lys Pro Asp Asp Leu Val Ile Leu Trp Val
            100                 105                 110

Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Gln Ile Leu Leu Phe Asn Leu Pro Asp Leu Gly Gln
145                 150                 155                 160

Asn Pro Ser Ala Arg Ser Gln Lys Val Val Glu Ala Val Ser His Val
                165                 170                 175

Ser Ala Tyr His Asn Lys Leu Leu Leu Asn Leu Ala Arg Gln Leu Ala
            180                 185                 190

Pro Thr Gly Met Val Lys Leu Phe Glu Ile Asp Lys Gln Phe Ala Glu
        195                 200                 205

Met Leu Arg Asp Pro Gln Asn Phe Gly Leu Ser Asp Val Glu Asn Pro
    210                 215                 220

Cys Tyr Asp Gly Gly Tyr Val Trp Lys Pro Phe Ala Thr Arg Ser Val
225                 230                 235                 240

Ser Thr Asp Arg Gln Leu Ser Ala Phe Ser Pro Gln Glu Arg Leu Ala
                245                 250                 255

Ile Ala Gly Asn Pro Leu Leu Ala Gln Ala Val Ala Ser Pro Met Ala
            260                 265                 270

Arg Arg Ser Ala Ser Pro Leu Asn Cys Glu Gly Lys Met Phe Trp Asp
        275                 280                 285

Gln Val His Pro Thr Thr Val Val His Ala Ala Leu Ser Glu Arg Ala
    290                 295                 300

Ala Thr Phe Ile Glu Thr Gln Tyr Glu Phe Leu Ala His Gly
305                 310                 315
```

<210> 36
<211> 1371

<212> DNA
<213> Streptomyces thermosacchari

<400> 36

```
acaggccgat gcacggaacc gtacctttcc gcagtgaagc gctctccccc catcgttcgc      60
cgggacttca tccgcgattt tggcatgaac acttccttca acgcgcgtag cttgctacaa     120
gtgcggcagc agacccgctc gttggaggct cagtgagatt gacccgatcc ctgtcggccg     180
catccgtcat cgtcttcgcc ctgctgctcg cgctgctggg catcagcccg gcccaggcag     240
ccggcccggc ctatgtggcc ctgggggatt cctattcctc gggcaacggc gccggaagtt     300
acatcgattc gagcggtgac tgtcaccgca gcaacaacgc gtaccccgcc cgctgggcgg     360
cggccaacgc accgtcctcc ttcaccttcg cggcctgctc gggagcggtg accacggatg     420
tgatcaacaa tcagctgggc gccctcaacg cgtccaccgg cctggtgagc atcaccatcg     480
gcggcaatga cgcgggcttc gcggacgcga tgaccacctg cgtcaccagc tcggacagca     540
cctgcctcaa ccggctggcc accgccacca actacatcaa caccaccctg ctcgcccggc     600
tcgacgcggt ctacagccag atcaaggccc gtgcccccaa cgcccgcgtg gtcgtcctcg     660
gctacccgcg catgtacctg gcctcgaacc cctggtactg cctgggcctg agcaacacca     720
agcgcgcggc catcaacacc accgccgaca ccctcaactc ggtgatctcc tcccgggcca     780
ccgcccacgg attccgattc ggcgatgtcc gcccgacctt caacaaccac gaactgttct     840
tcggcaacga ctggctgcac tcactcaccc tgccggtgtg ggagtcgtac caccccacca     900
gcacgggcca tcagagcggc tatctgccgg tcctcaacgc caacagctcg acctgatcaa     960
cgcacggccg tgcccgcccc gcgcgtcacg ctcggcgcgg gcgccgcagc gcgttgatca    1020
gcccacagtg ccggtgacgg tcccaccgtc acggtcgagg gtgtacgtca cggtggcgcc    1080
gctccagaag tggaacgtca gcaggaccgt ggagccgtcc ctgacctcgt cgaagaactc    1140
cggggtcagc gtgatcaccc ctcccccgta gccgggggcg aaggcggcgc cgaactcctt    1200
gtaggacgtc cagtcgtgcg gcccggcgtt gccaccgtcc gcgtagaccg cttccatggt    1260
cgccagccgg tccccgcgga actcggtggg gatgtccgtg cccaaggtgg tcccggtggt    1320
gtccgagagc accgggggct cgtaccggat gatgtgcaga tccaaagaat t              1371
```

<210> 37
<211> 267
<212> PRT
<213> Streptomyces thermosacchari

<400> 37

```
Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30

Ala Tyr Val Ala Leu Gly Asp Ser Tyr Ser Ser Gly Asn Gly Ala Gly
        35                  40                  45
```

```
Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                  80

Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160

Ala Pro Asn Ala Arg Val Val Val Leu Gly Tyr Pro Arg Met Tyr Leu
                165                 170                 175

Ala Ser Asn Pro Trp Tyr Cys Leu Gly Leu Ser Asn Thr Lys Arg Ala
            180                 185                 190

Ala Ile Asn Thr Thr Ala Asp Thr Leu Asn Ser Val Ile Ser Ser Arg
        195                 200                 205

Ala Thr Ala His Gly Phe Arg Phe Gly Asp Val Arg Pro Thr Phe Asn
    210                 215                 220

Asn His Glu Leu Phe Phe Gly Asn Asp Trp Leu His Ser Leu Thr Leu
225                 230                 235                 240

Pro Val Trp Glu Ser Tyr His Pro Thr Ser Thr Gly His Gln Ser Gly
                245                 250                 255

Tyr Leu Pro Val Leu Asn Ala Asn Ser Ser Thr
            260                 265
```

<210> 38
<211> 548
<212> PRT
<213> Thermobifida fusca

<400> 38

```
Met Leu Pro His Pro Ala Gly Glu Arg Gly Glu Val Gly Ala Phe Phe
1               5                   10                  15

Ala Leu Leu Val Gly Thr Pro Gln Asp Arg Arg Leu Arg Leu Glu Cys
            20                  25                  30

His Glu Thr Arg Pro Leu Arg Gly Arg Cys Gly Cys Gly Glu Arg Arg
        35                  40                  45

Val Pro Pro Leu Thr Leu Pro Gly Asp Gly Val Leu Cys Thr Thr Ser
    50                  55                  60

Ser Thr Arg Asp Ala Glu Thr Val Trp Arg Lys His Leu Gln Pro Arg
65                  70                  75                  80

Pro Asp Gly Gly Phe Arg Pro His Leu Gly Val Gly Cys Leu Leu Ala
                85                  90                  95

Gly Gln Gly Ser Pro Gly Val Leu Trp Cys Gly Arg Glu Gly Cys Arg
            100                 105                 110

Phe Glu Val Cys Arg Arg Asp Thr Pro Gly Leu Ser Arg Thr Arg Asn
        115                 120                 125

Gly Asp Ser Ser Pro Pro Phe Arg Ala Gly Trp Ser Leu Pro Pro Lys
    130                 135                 140

Cys Gly Glu Ile Ser Gln Ser Ala Arg Lys Thr Pro Ala Val Pro Arg
145                 150                 155                 160

Tyr Ser Leu Leu Arg Thr Asp Arg Pro Asp Gly Pro Arg Gly Arg Phe
                165                 170                 175

Val Gly Ser Gly Pro Arg Ala Ala Thr Arg Arg Arg Leu Phe Leu Gly
            180                 185                 190

Ile Pro Ala Leu Val Leu Val Thr Ala Leu Thr Leu Val Leu Ala Val
        195                 200                 205

Pro Thr Gly Arg Glu Thr Leu Trp Arg Met Trp Cys Glu Ala Thr Gln
    210                 215                 220

Asp Trp Cys Leu Gly Val Pro Val Asp Ser Arg Gly Gln Pro Ala Glu
225                 230                 235                 240

Asp Gly Glu Phe Leu Leu Leu Ser Pro Val Gln Ala Ala Thr Trp Gly
                245                 250                 255

Asn Tyr Tyr Ala Leu Gly Asp Ser Tyr Ser Ser Gly Asp Gly Ala Arg
```

260 265 270

Asp Tyr Tyr Pro Gly Thr Ala Val Lys Gly Gly Cys Trp Arg Ser Ala
275 280 285

Asn Ala Tyr Pro Glu Leu Val Ala Glu Ala Tyr Asp Phe Ala Gly His
290 295 300

Leu Ser Phe Leu Ala Cys Ser Gly Gln Arg Gly Tyr Ala Met Leu Asp
305 310 315 320

Ala Ile Asp Glu Val Gly Ser Gln Leu Asp Trp Asn Ser Pro His Thr
325 330 335

Ser Leu Val Thr Ile Gly Ile Gly Gly Asn Asp Leu Gly Phe Ser Thr
340 345 350

Val Leu Lys Thr Cys Met Val Arg Val Pro Leu Leu Asp Ser Lys Ala
355 360 365

Cys Thr Asp Gln Glu Asp Ala Ile Arg Lys Arg Met Ala Lys Phe Glu
370 375 380

Thr Thr Phe Glu Glu Leu Ile Ser Glu Val Arg Thr Arg Ala Pro Asp
385 390 395 400

Ala Arg Ile Leu Val Val Gly Tyr Pro Arg Ile Phe Pro Glu Glu Pro
405 410 415

Thr Gly Ala Tyr Tyr Thr Leu Thr Ala Ser Asn Gln Arg Trp Leu Asn
420 425 430

Glu Thr Ile Gln Glu Phe Asn Gln Gln Leu Ala Glu Ala Val Ala Val
435 440 445

His Asp Glu Glu Ile Ala Ala Ser Gly Gly Val Gly Ser Val Glu Phe
450 455 460

Val Asp Val Tyr His Ala Leu Asp Gly His Glu Ile Gly Ser Asp Glu
465 470 475 480

Pro Trp Val Asn Gly Val Gln Leu Arg Asp Leu Ala Thr Gly Val Thr
485 490 495

Val Asp Arg Ser Thr Phe His Pro Asn Ala Ala Gly His Arg Ala Val
500 505 510

Gly Glu Arg Val Ile Glu Gln Ile Glu Thr Gly Pro Gly Arg Pro Leu
515 520 525

Tyr Ala Thr Phe Ala Val Val Ala Gly Ala Thr Val Asp Thr Leu Ala
    530                 535                 540

Gly Glu Val Gly
545

<210> 39
<211> 3000
<212> DNA
<213> Thermobifida fusca

<400> 39

ggtggtgaac cagaacaccc ggtcgtcggc gtgggcgtcc aggtgcaggt gcaggttctt 60

caactgctcc agcaggatgc cgccgtggcc gtgcacgatg gccttgggca ggcctgtggt 120

ccccgacgag tacagcaccc atagcggatg gtcgaacggc agcggggtga actccagttc 180

cgcgccttcg cccgcggctt cgaactccgc ccaggacagg gtgtcggcga cagggccgca 240

gcccaggtac ggcaggacga cggtgtgctg caggctgggc atgccgtcgc gcagggcttt 300

gagcacgtca cggcggtcga agtccttacc gccgtagcgg tagccgtcca cggccagcag 360

cactttcggt tcgatctgcg cgaaccggtc gaggacgctg cgcaccccga agtcggggga 420

acaggacgac caggtcgcac cgatcgcggc gcaggcgagg aatgcggccg tcgcctcggc 480

gatgttcggc aggtaggcca cgacccggtc gccggggccc accccgaggc tgcggagggc 540

cgcagcgatc gcggcggtgc gggtccgcag ttctccccag gtccactcgg tcaacggccg 600

gagttcggac gcgtgccgga tcgccacggc tgatgggtca cggtcgcgga agatgtgctc 660

ggcgtagttg agggtggcgc cggggaacca gacggcgccg ggcatggcgt cggaggcgag 720

cactgtggtg tacggggtgg cggcgcgcac ccggtagtac tcccagatcg cggaccagaa 780

tccttcgagg tcggttaccg accagcgcca cagtgcctcg tagtccggtg cgtccacacc 840

gcggtgctcc cgcacccagc gggtgaacgc ggtgaggttg gcgcgttctt tgcgctcctc 900

gtcgggactc cacaggatcg gcggctgcgg cttgagtgtc atgaaacgcg acccccttcgt 960

ggacggtgcg gatgcggtga gcgtcgggtg cctcccctaa cgctccccgg tgacggagtg 1020

ttgtgcacca catctagcac gcgggacgcg gaaaccgtat ggagaaaaca cctacaaccc 1080

cggccggacg gtgggtttcg gccacactta ggggtcgggt gcctgcttgc cgggcagggc 1140

agtcccgggg tgctgtggtg cgggcgggag ggctgtcgct tcgaggtgtg ccggcgggac 1200

actccgggcc tcagccgtac ccgcaacggg gacagttctc ctcccttccg ggctggatgg 1260

tcccttcccc cgaaatgcgg cgagatctcc cagtcagccc ggaaaacacc cgctgtgccc 1320

aggtactctt tgcttcgaac agacaggccg gacggtccac gggggaggtt tgtgggcagc 1380

ggaccacgtg cggcgaccag acgacggttg ttcctcggta tccccgctct tgtacttgtg 1440

acagcgctca cgctggtctt ggctgtcccg acggggcgcg agacgctgtg gcgcatgtgg 1500

```
tgtgaggcca cccaggactg gtgcctgggg gtgccggtcg actcccgcgg acagcctgcg      1560
gaggacggcg agtttctgct gctttctccg gtccaggcag cgacctgggg gaactattac      1620
gcgctcgggg attcgtactc ttcgggggac ggggcccgcg actactatcc cggcaccgcg      1680
gtgaagggcg gttgctggcg gtccgctaac gcctatccgg agctggtcgc cgaagcctac      1740
gacttcgccg gacacttgtc gttcctggcc tgcagcggcc agcgcggcta cgccatgctt      1800
gacgctatcg acgaggtcgg ctcgcagctg gactggaact cccctcacac gtcgctggtg      1860
acgatcggga tcggcggcaa cgatctgggg ttctccacgg ttttgaagac ctgcatggtg      1920
cgggtgccgc tgctggacag caaggcgtgc acggaccagg aggacgctat ccgcaagcgg      1980
atggcgaaat tcgagacgac gtttgaagag ctcatcagcg aagtgcgcac ccgcgcgccg      2040
gacgcccgga tccttgtcgt gggctacccc cggatttttc cggaggaacc gaccggcgcc      2100
tactacacgc tgaccgcgag caaccagcgg tggctcaacg aaaccattca ggagttcaac      2160
cagcagctcg ccgaggctgt cgcggtccac gacgaggaga ttgccgcgtc gggcggggtg      2220
ggcagcgtgg agttcgtgga cgtctaccac gcgttggacg gccacgagat cggctcggac      2280
gagccgtggg tgaacggggt gcagttgcgg gacctcgcca ccggggtgac tgtggaccgc      2340
agtaccttcc accccaacgc cgctgggcac cgggcggtcg gtgagcgggt catcgagcag      2400
atcgaaaccg gcccgggccg tccgctctat gccactttcg cggtggtggc gggggcgacc      2460
gtggacactc tcgcgggcga ggtggggtga cccggcttac cgtccggccc gcaggtctgc      2520
gagcactgcg gcgatctggt ccactgccca gtgcagttcg tcttcggtga tgaccagcgg      2580
cggggagagc cggatcgttg agccgtgcgt gtctttgacg agcacacccc gctgcaggag      2640
ccgttcgcac agttctcttc cggtggccag agtcgggtcg acgtcgatcc cagcccacag      2700
gccgatgctg cgggccgcga ccacgccgtt gccgaccagt tggtcgaggc gggcgcgcag      2760
cacgggggcg agggcgcgga catggtccag gtaagggccg tcgcggacga ggctcaccac      2820
ggcagtgccg accgcgcagg cgagggcgtt gccgccgaag gtgctgccgt gctggccggg      2880
gcggatcacg tcgaagactt ccgcgtcgcc taccgccgcc gccacgggca ggatgccgcc      2940
gcccagcgct ttgccgaaca ggtagatatc ggcgtcgact ccgctgtggt cgcaggcccg      3000
```

<210> 40
<211> 372
<212> PRT
<213> Thermobifida fusca

<400> 40

```
Val Gly Ser Gly Pro Arg Ala Ala Thr Arg Arg Arg Leu Phe Leu Gly
1               5                   10                  15

Ile Pro Ala Leu Val Leu Val Thr Ala Leu Thr Leu Val Leu Ala Val
            20                  25                  30
```

Pro Thr Gly Arg Glu Thr Leu Trp Arg Met Trp Cys Glu Ala Thr Gln
35 40 45

Asp Trp Cys Leu Gly Val Pro Val Asp Ser Arg Gly Gln Pro Ala Glu
50 55 60

Asp Gly Glu Phe Leu Leu Leu Ser Pro Val Gln Ala Ala Thr Trp Gly
65 70 75 80

Asn Tyr Tyr Ala Leu Gly Asp Ser Tyr Ser Ser Gly Asp Gly Ala Arg
85 90 95

Asp Tyr Tyr Pro Gly Thr Ala Val Lys Gly Gly Cys Trp Arg Ser Ala
100 105 110

Asn Ala Tyr Pro Glu Leu Val Ala Glu Ala Tyr Asp Phe Ala Gly His
115 120 125

Leu Ser Phe Leu Ala Cys Ser Gly Gln Arg Gly Tyr Ala Met Leu Asp
130 135 140

Ala Ile Asp Glu Val Gly Ser Gln Leu Asp Trp Asn Ser Pro His Thr
145 150 155 160

Ser Leu Val Thr Ile Gly Ile Gly Gly Asn Asp Leu Gly Phe Ser Thr
165 170 175

Val Leu Lys Thr Cys Met Val Arg Val Pro Leu Leu Asp Ser Lys Ala
180 185 190

Cys Thr Asp Gln Glu Asp Ala Ile Arg Lys Arg Met Ala Lys Phe Glu
195 200 205

Thr Thr Phe Glu Glu Leu Ile Ser Glu Val Arg Thr Arg Ala Pro Asp
210 215 220

Ala Arg Ile Leu Val Val Gly Tyr Pro Arg Ile Phe Pro Glu Glu Pro
225 230 235 240

Thr Gly Ala Tyr Tyr Thr Leu Thr Ala Ser Asn Gln Arg Trp Leu Asn
245 250 255

Glu Thr Ile Gln Glu Phe Asn Gln Gln Leu Ala Glu Ala Val Ala Val
260 265 270

His Asp Glu Glu Ile Ala Ala Ser Gly Gly Val Gly Ser Val Glu Phe
275 280 285

Val Asp Val Tyr His Ala Leu Asp Gly His Glu Ile Gly Ser Asp Glu
290 295 300

Pro Trp Val Asn Gly Val Gln Leu Arg Asp Leu Ala Thr Gly Val Thr
305 310 315 320

Val Asp Arg Ser Thr Phe His Pro Asn Ala Ala Gly His Arg Ala Val
325 330 335

Gly Glu Arg Val Ile Glu Gln Ile Glu Thr Gly Pro Gly Arg Pro Leu
340 345 350

Tyr Ala Thr Phe Ala Val Val Ala Gly Ala Thr Val Asp Thr Leu Ala
355 360 365

Gly Glu Val Gly
370

<210> 41
<211> 300
<212> PRT
<213> Corynebacterium efficiens

<400> 41

```
Met Arg Thr Thr Val Ile Ala Ala Ser Ala Leu Leu Leu Leu Ala Gly
1               5                   10                  15

Cys Ala Asp Gly Ala Arg Glu Glu Thr Ala Gly Ala Pro Pro Gly Glu
            20                  25                  30

Ser Ser Gly Gly Ile Arg Glu Glu Gly Ala Glu Ala Ser Thr Ser Ile
        35                  40                  45

Thr Asp Val Tyr Ile Ala Leu Gly Asp Ser Tyr Ala Ala Met Gly Gly
    50                  55                  60

Arg Asp Gln Pro Leu Arg Gly Glu Pro Phe Cys Leu Arg Ser Ser Gly
65                  70                  75                  80

Asn Tyr Pro Glu Leu Leu His Ala Glu Val Thr Asp Leu Thr Cys Gln
                85                  90                  95

Gly Ala Val Thr Gly Asp Leu Leu Glu Pro Arg Thr Leu Gly Glu Arg
            100                 105                 110

Thr Leu Pro Ala Gln Val Asp Ala Leu Thr Glu Asp Thr Thr Leu Val
        115                 120                 125

Thr Leu Ser Ile Gly Gly Asn Asp Leu Gly Phe Gly Glu Val Ala Gly
```

```
        130                 135                 140

Cys Ile Arg Glu Arg Ile Ala Gly Glu Asn Ala Asp Asp Cys Val Asp
145                 150                 155                 160

Leu Leu Gly Glu Thr Ile Gly Glu Gln Leu Asp Gln Leu Pro Pro Gln
                165                 170                 175

Leu Asp Arg Val His Glu Ala Ile Arg Asp Arg Ala Gly Asp Ala Gln
            180                 185                 190

Val Val Val Thr Gly Tyr Leu Pro Leu Val Ser Ala Gly Asp Cys Pro
        195                 200                 205

Glu Leu Gly Asp Val Ser Glu Ala Asp Arg Arg Trp Ala Val Glu Leu
    210                 215                 220

Thr Gly Gln Ile Asn Glu Thr Val Arg Glu Ala Ala Glu Arg His Asp
225                 230                 235                 240

Ala Leu Phe Val Leu Pro Asp Asp Ala Asp Glu His Thr Ser Cys Ala
                245                 250                 255

Pro Pro Gln Gln Arg Trp Ala Asp Ile Gln Gly Gln Gln Thr Asp Ala
            260                 265                 270

Tyr Pro Leu His Pro Thr Ser Ala Gly His Glu Ala Met Ala Ala Ala
        275                 280                 285

Val Arg Asp Ala Leu Gly Leu Glu Pro Val Gln Pro
    290                 295                 300
```

<210> 42
<211> 3000
<212> DNA
<213> Corynebacterium efficiens

<400> 42

```
ttctggggtg ttatggggtt gttatcggct cgtcctgggt ggatcccgcc aggtggggta      60
ttcacggggg acttttgtgt ccaacagccg agaatgagtg ccctgagcgg tgggaatgag     120
gtgggcgggg ctgtgtcgcc atgaggggc ggcgggctct gtggtgcccc gcgacccccg     180
gccccggtga gcggtgaatg aaatccggct gtaatcagca tcccgtgccc accccgtcgg     240
ggaggtcagc gcccggagtg tctacgcagt cggatcctct cggactcggc catgctgtcg     300
gcagcatcgc gctcccgggt cttggcgtcc ctcggctgtt ctgcctgctg tccctggaag     360
gcgaaatgat caccggggag tgatacaccg gtggtctcat cccggatgcc cacttcggcg     420
ccatccggca attcgggcag ctccgggtgg aagtaggtgg catccgatgc gtcggtgacg     480
```

ccatagtggg cgaagatctc atcctgctcg agggtgctca ggccactctc cggatcgata 540
tcgggggcgt ccttgatggc gtccttgctg aaaccgaggt gcagcttgtg ggcttccaat 600
ttcgcaccac ggagcgggac gaggctggaa tgacggccga agagcccgtg gtggacctca 660
acgaaggtgg gtagtcccgt gtcatcattg aggaacacgc cctccaccgc acccagcttg 720
tggccggagt tgtcgtaggc gctggcatcc agaagggaaa cgatctcata tttgtcggtg 780
tgctcagaca tgatcttcct ttgctgtcgg tgtctggtac taccacggta gggctgaatg 840
caactgttat ttttctgtta ttttaggaat tggtccatat cccacaggct ggctgtggtc 900
aaatcgtcat caagtaatcc ctgtcacaca aaatgggtgg tgggagccct ggtcgcggtt 960
ccgtgggagg cgccgtgccc cgcaggatcg tcggcatcgg cggatctggc cggtaccccg 1020
cggtgaataa aatcattctg taaccttcat cacggttggt tttaggtatc cgcccctttc 1080
gtcctgaccc cgtccccggc gcgcgggagc ccgcgggttg cggtagacag gggagacgtg 1140
gacaccatga ggacaacggt catcgcagca agcgcattac tccttctcgc cggatgcgcg 1200
gatggggccc gggaggagac cgccggtgca ccgccgggtg agtcctccgg gggcatccgg 1260
gaggaggggg cggaggcgtc gacaagcatc accgacgtct acatcgccct cggggattcc 1320
tatgcggcga tgggcgggcg ggatcagccg ttacggggtg agccgttctg cctgcgctcg 1380
tccggtaatt acccggaact cctccacgca gaggtcaccg atctcacctg ccaggggggcg 1440
gtgaccgggg atctgctcga acccaggacg ctgggggagc gcacgctgcc ggcgcaggtg 1500
gatgcgctga cggaggacac caccctggtc accctctcca tcgggggcaa tgacctcgga 1560
ttcggggagg tggcgggatg catccgggaa cggatcgccg gggagaacgc tgatgattgc 1620
gtggacctgc tgggggaaac catcggggag cagctcgatc agcttccccc gcagctggac 1680
cgcgtgcacg aggctatccg ggaccgcgcc ggggacgcgc aggttgtggt caccggttac 1740
ctgccgctcg tgtctgccgg ggactgcccc gaactggggg atgtctccga ggcggatcgt 1800
cgttgggcgg ttgagctgac cgggcagatc aacgagaccg tgcgcgaggc ggccgaacga 1860
cacgatgccc tctttgtcct gcccgacgat gccgatgagc acaccagttg tgcacccccca 1920
cagcagcgct gggcggatat ccagggccaa cagaccgatg cctatccgct gcacccgacc 1980
tccgccggcc atgaggcgat ggccgccgcc gtccgggacg cgctgggcct ggaaccggtc 2040
cagccgtagc gccgggcgcg cgcttgtcga cgaccaaccc atgccaggct gcagtcacat 2100
ccgcacatag cgcgcgcggg cgatggagta cgcaccatag aggatgagcc cgatgccgac 2160
gatgatgagc agcacactgc cgaagggttg ttccccgagg gtgcgcagag ccgagtccag 2220
acctgcggcc tgctccggat catgggccca accggcgatg acgatcaaca cccccaggat 2280
cccgaaggcg ataccacggg cgacataacc ggctgttccg gtgatgatga tcgcggtccc 2340
gacctgccct gaccccgcac ccgcctccag atcctcccgg aaatcccggg tggccccctt 2400
ccagaggttg tagacacccg cccccagtac caccagcccg gcgaccacaa ccagcaccac 2460

```
accccagggt tgggatagga cggtggcggt gacatcggtg gcggtctccc catcggaggt     2520
gctgccgccc cgggcgaagg tggaggtggt caccgccagg gagaagtaga ccatggccat     2580
gaccgccccc ttggcccttt ccttgaggtc ctcgcccgcc agcagctggc tcaattgcca     2640
gagtcccagg gccgccaggg cgatgacggc aacccacagg aggaactgcc cacccggagc     2700
ctccgcgatg gtggccaggg cacctgaatt cgaggcctca tcacccgaac cgccggatcc     2760
agtggcgatg cgcaccgcga tccacccgat gaggatgtgc agtatgccca ggacaatgaa     2820
accacctctg gccagggtgg tcagcgcggg gtggtcctcg gcctggtcgg cagcccgttc     2880
gatcgtccgt ttcgcggatc tggtgtcgcc cttatccata gctcccattg aaccgccttg     2940
aggggtgggc ggccactgtc agggcggatt gtgatctgaa ctgtgatgtt ccatcaaccc     3000
```

<210> 43
<211> 268
<212> PRT
<213> Streptomyces coelicolor

<400> 43

```
Met Arg Arg Phe Arg Leu Val Gly Phe Leu Ser Ser Leu Val Leu Ala
1               5                   10                  15

Ala Gly Ala Ala Leu Thr Gly Ala Ala Thr Ala Gln Ala Ala Gln Pro
            20                  25                  30

Ala Ala Ala Asp Gly Tyr Val Ala Leu Gly Asp Ser Tyr Ser Ser Gly
        35                  40                  45

Val Gly Ala Gly Ser Tyr Ile Ser Ser Ser Gly Asp Cys Lys Arg Ser
    50                  55                  60

Thr Lys Ala His Pro Tyr Leu Trp Ala Ala Ala His Ser Pro Ser Thr
65                  70                  75                  80

Phe Asp Phe Thr Ala Cys Ser Gly Ala Arg Thr Gly Asp Val Leu Ser
                85                  90                  95

Gly Gln Leu Gly Pro Leu Ser Ser Gly Thr Gly Leu Val Ser Ile Ser
            100                 105                 110

Ile Gly Gly Asn Asp Ala Gly Phe Ala Asp Thr Met Thr Thr Cys Val
        115                 120                 125

Leu Gln Ser Glu Ser Ser Cys Leu Ser Arg Ile Ala Thr Ala Glu Ala
    130                 135                 140

Tyr Val Asp Ser Thr Leu Pro Gly Lys Leu Asp Gly Val Tyr Ser Ala
145                 150                 155                 160
```

```
Ile Ser Asp Lys Ala Pro Asn Ala His Val Val Val Ile Gly Tyr Pro
                165                 170                 175

Arg Phe Tyr Lys Leu Gly Thr Thr Cys Ile Gly Leu Ser Glu Thr Lys
            180                 185                 190

Arg Thr Ala Ile Asn Lys Ala Ser Asp His Leu Asn Thr Val Leu Ala
        195                 200                 205

Gln Arg Ala Ala Ala His Gly Phe Thr Phe Gly Asp Val Arg Thr Thr
    210                 215                 220

Phe Thr Gly His Glu Leu Cys Ser Gly Ser Pro Trp Leu His Ser Val
225                 230                 235                 240

Asn Trp Leu Asn Ile Gly Glu Ser Tyr His Pro Thr Ala Ala Gly Gln
                245                 250                 255

Ser Gly Gly Tyr Leu Pro Val Leu Asn Gly Ala Ala
            260                 265
```

<210> 44
<211> 2000
<212> DNA
<213> Streptomyces coelicolor

<400> 44

```
cccggcggcc cgtgcaggag cagcagccgg cccgcgatgt cctcgggcgt cgtcttcatc      60
aggccgtcca tcgcgtcggc gaccggcgcc gtgtagttgg cccggacctc gtcccaggtg     120
cccgcggcga tctggcgggt ggtgcggtgc gggccgcgcc gaggggagac gtaccagaag     180
cccatcgtca cgttctccgg ctgcggttcg ggctcgtccg ccgctccgtc cgtcgcctcg     240
ccgagcacct tctcggcgag gtcggcgctg gtcgccgtca ccgtgacgtc ggcgccccgg     300
ctccagcgcg agatcagcag cgtccagccg tcgccctccg ccagcgtcgc gctgcggtcg     360
tcgtcgcggg cgatccgcag cacgcgcgcg ccgggcggca gcagcgtggc gccggaccgt     420
acgcggtcga tgttcgccgc gtgcgagtac ggctgctcac ccgtggcgaa acggccgagg     480
aacagcgcgt cgacgacgtc ggacggggag tcgctgtcgt ccacgttgag ccggatcggc     540
agggcttcgt gcgggttcac ggacatgtcg ccatgatcgg gcacccggcc gccgcgtgca     600
cccgctttcc cgggcacgca cgacaggggc tttctcgccg tcttccgtcc gaacttgaac     660
gagtgtcagc catttcttgg catggacact tccagtcaac gcgcgtagct gctaccacgg     720
ttgtggcagc aatcctgcta agggaggttc catgagacgt ttccgacttg tcggcttcct     780
gagttcgctc gtcctcgccg ccggcgccgc cctcaccggg gcagcgaccg cccaggcggc     840
ccaacccgcc gccgccgacg gctatgtggc cctcggcgac tcctactcct ccggggtcgg     900
```

agcgggcagc tacatcagct cgagcggcga ctgcaagcgc agcacgaagg cccatcccta 960

cctgtgggcg gccgcccact cgccctccac gttcgacttc accgcctgtt ccggcgcccg 1020

tacgggtgat gttctctccg gacagctcgg cccgctcagc tccggcaccg gcctcgtctc 1080

gatcagcatc ggcggcaacg acgccggttt cgccgacacc atgacgacct gtgtgctcca 1140

gtccgagagc tcctgcctgt cgcggatcgc caccgccgag gcgtacgtcg actcgacgct 1200

gcccggcaag ctcgacggcg tctactcggc aatcagcgac aaggcgccga acgcccacgt 1260

cgtcgtcatc ggctacccgc gcttctacaa gctcggcacc acctgcatcg gcctgtccga 1320

gaccaagcgg acggcgatca acaaggcctc cgaccacctc aacaccgtcc tcgcccagcg 1380

cgccgccgcc cacggcttca ccttcggcga cgtacgcacc accttcaccg gccacgagct 1440

gtgctccggc agcccctggc tgcacagcgt caactggctg aacatcggcg agtcgtacca 1500

ccccaccgcg gccggccagt ccggtggcta cctgccggtc ctcaacggcg ccgcctgacc 1560

tcaggcggaa ggagaagaag aaggagcgga gggagacgag gagtgggagg ccccgcccga 1620

cggggtcccc gtccccgtct ccgtctccgt cccggtcccg caagtcaccg agaacgccac 1680

cgcgtcggac gtggcccgca ccggactccg cacctccacg cgcacggcac tctcgaacgc 1740

gccggtgtcg tcgtgcgtcg tcaccaccac gccgtcctgg cgcgagcgct cgccgcccga 1800

cgggaaggac agcgtccgcc accccggatc ggagaccgac ccgtccgcgg tcacccaccg 1860

gtagccgacc tccgcgggca gccgcccgac cgtgaacgtc gccgtgaacg cgggtgcccg 1920

gtcgtgcggc ggcggacagg cccccgagta gtgggtgcgc gagcccacca cggtcacctc 1980

caccgactgc gctgcggggc 2000

<210> 45
<211> 269
<212> PRT
<213> Streptomyces avermitilis

<400> 45

```
Met Arg Arg Ser Arg Ile Thr Ala Tyr Val Thr Ser Leu Leu Leu Ala
1               5                   10                  15

Val Gly Cys Ala Leu Thr Gly Ala Ala Thr Ala Gln Ala Ser Pro Ala
            20                  25                  30

Ala Ala Ala Thr Gly Tyr Val Ala Leu Gly Asp Ser Tyr Ser Ser Gly
        35                  40                  45

Val Gly Ala Gly Ser Tyr Leu Ser Ser Ser Gly Asp Cys Lys Arg Ser
    50                  55                  60

Ser Lys Ala Tyr Pro Tyr Leu Trp Gln Ala Ala His Ser Pro Ser Ser
65                  70                  75                  80
```

```
Phe Ser Phe Met Ala Cys Ser Gly Ala Arg Thr Gly Asp Val Leu Ala
                85                  90                  95

Asn Gln Leu Gly Thr Leu Asn Ser Ser Thr Gly Leu Val Ser Leu Thr
            100                 105                 110

Ile Gly Gly Asn Asp Ala Gly Phe Ser Asp Val Met Thr Thr Cys Val
        115                 120                 125

Leu Gln Ser Asp Ser Ala Cys Leu Ser Arg Ile Asn Thr Ala Lys Ala
    130                 135                 140

Tyr Val Asp Ser Thr Leu Pro Gly Gln Leu Asp Ser Val Tyr Thr Ala
145                 150                 155                 160

Ile Ser Thr Lys Ala Pro Ser Ala His Val Ala Val Leu Gly Tyr Pro
                165                 170                 175

Arg Phe Tyr Lys Leu Gly Gly Ser Cys Leu Ala Gly Leu Ser Glu Thr
            180                 185                 190

Lys Arg Ser Ala Ile Asn Asp Ala Ala Asp Tyr Leu Asn Ser Ala Ile
        195                 200                 205

Ala Lys Arg Ala Ala Asp His Gly Phe Thr Phe Gly Asp Val Lys Ser
    210                 215                 220

Thr Phe Thr Gly His Glu Ile Cys Ser Ser Ser Thr Trp Leu His Ser
225                 230                 235                 240

Leu Asp Leu Leu Asn Ile Gly Gln Ser Tyr His Pro Thr Ala Ala Gly
                245                 250                 255

Gln Ser Gly Gly Tyr Leu Pro Val Met Asn Ser Val Ala
            260                 265
```

<210> 46
<211> 1980
<212> DNA
<213> Streptomyces avermitilis

<400> 46

ccaccgccgg gtcggcggcg agtctcctgg cctcggtcgc ggagaggttg gccgtgtagc 60

cgttcagcgc ggcgccgaac gtcttcttca ccgtgccgcc gtactcgttg atcaggccct 120

tgcccttgct cgacgcggcc ttgaagccgg tgcccttctt gagcgtgacg atgtagctgc 180

ccttgatcgc ggtgggggag ccggcggcga gcaccgtgcc ctcggccggg gtggcctggg 240

cgggcagtgc ggtgaatccg cccacgaggg cgccggtcgc cacggcggtt atcgcggcga 300

```
tccggatctt cttgctacgc agctgtgcca tacgagggag tcctcctctg ggcagcggcg      360
cgcctgggtg gggcgcacgg ctgtgggggg tgcgcgcgtc atcacgcaca cggccctgga      420
gcgtcgtgtt ccgccctggg ttgagtaaag cctcggccat ctacgggggt ggctcaaggg      480
agttgagacc ctgtcatgag tctgacatga gcacgcaatc aacggggccg tgagcacccc      540
ggggcgaccc cggaaagtgc cgagaagtct tggcatggac acttcctgtc aacacgcgta      600
gctggtacga cggttacggc agagatcctg ctaaagggag gttccatgag acgttcccga      660
attacggcat acgtgacctc actcctcctc gccgtcggct gcgccctcac cggggcagcg      720
acggcgcagg cgtccccagc cgccgcggcc acgggctatg tggccctcgg cgactcgtac      780
tcgtccggtg tcggcgccgg cagctacctc agctccagcg gcgactgcaa gcgcagttcg      840
aaggcctatc cgtacctctg gcaggccgcg cattcaccct cgtcgttcag tttcatggct      900
tgctcgggcg ctcgtacggg tgatgtcctg gccaatcagc tcggcaccct gaactcgtcc      960
accggcctgg tctccctcac catcggaggc aacgacgcgg gcttctccga cgtcatgacg     1020
acctgtgtgc tccagtccga cagcgcctgc ctctcccgca tcaacacggc gaaggcgtac     1080
gtcgactcca ccctgcccgg ccaactcgac agcgtgtaca cggcgatcag cacgaaggcc     1140
ccgtcggccc atgtggccgt gctgggctac ccccgcttct acaaactggg cggctcctgc     1200
ctcgcgggcc tctcggagac caagcggtcc gccatcaacg acgcggccga ctatctgaac     1260
agcgccatcg ccaagcgcgc cgccgaccac ggcttcacct tcggcgacgt caagagcacc     1320
ttcaccggcc atgagatctg ctccagcagc acctggctgc acagtctcga cctgctgaac     1380
atcggccagt cctaccaccc gaccgcggcc ggccagtccg gcggctatct gccggtcatg     1440
aacagcgtgg cctgagctcc cacggcctga atttttaagg cctgaatttt taaggcgaag     1500
gtgaaccgga agcggaggcc ccgtccgtcg ggtctccgt cgcacaggtc accgagaacg      1560
gcacggagtt ggacgtcgtg cgcaccgggt cgcgcacctc gacggcgatc tcgttcgaga     1620
tcgttccgct cgtgtcgtac gtggtgacga acacctgctt ctgctgggtc tttccgccgc     1680
tcgccgggaa ggacagcgtc ttccagcccg gatccgggac ctcgcccttc ttggtcaccc     1740
agcggtactc cacctcgacc ggcacccggc ccaccgtgaa ggtcgccgtg aacgtgggcg     1800
cctgggcggt gggcggcggg caggcaccgg agtagtcggt gtgcacgccg gtgaccgtca     1860
ccttcacgga ctgggccggc ggggtcgtcg taccgccgcc gccaccgccg cctcccggag     1920
tggagcccga gctgtggtcg cccccgccgt cggcgttgtc gtcctcgggg gttttcgaac     1980
```

<210> 47
<211> 372
<212> PRT
<213> Thermobifida fusca

<400> 47

```
Met Gly Ser Gly Pro Arg Ala Ala Thr Arg Arg Arg Leu Phe Leu Gly
1               5                   10                  15

Ile Pro Ala Leu Val Leu Val Thr Ala Leu Thr Leu Val Leu Ala Val
            20                  25                  30

Pro Thr Gly Arg Glu Thr Leu Trp Arg Met Trp Cys Glu Ala Thr Gln
        35                  40                  45

Asp Trp Cys Leu Gly Val Pro Val Asp Ser Arg Gly Gln Pro Ala Glu
    50                  55                  60

Asp Gly Glu Phe Leu Leu Leu Ser Pro Val Gln Ala Ala Thr Trp Gly
65                  70                  75                  80

Asn Tyr Tyr Ala Leu Gly Asp Ser Tyr Ser Ser Gly Asp Gly Ala Arg
                85                  90                  95

Asp Tyr Tyr Pro Gly Thr Ala Val Lys Gly Gly Cys Trp Arg Ser Ala
            100                 105                 110

Asn Ala Tyr Pro Glu Leu Val Ala Glu Ala Tyr Asp Phe Ala Gly His
        115                 120                 125

Leu Ser Phe Leu Ala Cys Ser Gly Gln Arg Gly Tyr Ala Met Leu Asp
    130                 135                 140

Ala Ile Asp Glu Val Gly Ser Gln Leu Asp Trp Asn Ser Pro His Thr
145                 150                 155                 160

Ser Leu Val Thr Ile Gly Ile Gly Gly Asn Asp Leu Gly Phe Ser Thr
                165                 170                 175

Val Leu Lys Thr Cys Met Val Arg Val Pro Leu Leu Asp Ser Lys Ala
            180                 185                 190

Cys Thr Asp Gln Glu Asp Ala Ile Arg Lys Arg Met Ala Lys Phe Glu
        195                 200                 205

Thr Thr Phe Glu Glu Leu Ile Ser Glu Val Arg Thr Arg Ala Pro Asp
    210                 215                 220

Ala Arg Ile Leu Val Val Gly Tyr Pro Arg Ile Phe Pro Glu Glu Pro
225                 230                 235                 240

Thr Gly Ala Tyr Tyr Thr Leu Thr Ala Ser Asn Gln Arg Trp Leu Asn
                245                 250                 255

Glu Thr Ile Gln Glu Phe Asn Gln Gln Leu Ala Glu Ala Val Ala Val
```

```
            260                 265                 270

His Asp Glu Glu Ile Ala Ala Ser Gly Gly Val Gly Ser Val Glu Phe
        275                 280                 285

Val Asp Val Tyr His Ala Leu Asp Gly His Glu Ile Gly Ser Asp Glu
    290                 295                 300

Pro Trp Val Asn Gly Val Gln Leu Arg Asp Leu Ala Thr Gly Val Thr
305                 310                 315                 320

Val Asp Arg Ser Thr Phe His Pro Asn Ala Ala Gly His Arg Ala Val
                325                 330                 335

Gly Glu Arg Val Ile Glu Gln Ile Glu Thr Gly Pro Gly Arg Pro Leu
            340                 345                 350

Tyr Ala Thr Phe Ala Val Val Ala Gly Ala Thr Val Asp Thr Leu Ala
        355                 360                 365

Gly Glu Val Gly
    370
```

<210> 48
<211> 968
<212> DNA
<213> Thermobifida fusca

<400> 48

ctgcagacac ccgccccgcc ttctcccgga tcgtcatgtt cggcgactcc ctcagcgaca 60
ccggcaagat gtactccaag atgcgcggct acctgccgtc ctccccgccg tactacgagg 120
gccgcttctc gaacggcccg gtctggctgg agcagctgac gaagcagttc cccggcctga 180
cgatcgccaa cgaggccgag gggggcgcga ccgcagtcgc ctacaacaag atctcctgga 240
acccgaagta ccaggtcatt aacaacctcg actacgaggt cacccagttc ttgcagaagg 300
actcgttcaa gcccgacgac ctggtcatcc tgtgggtggg cgccaacgac tacctggcct 360
acggttggaa cacggagcag gacgccaagc gggtgcgcga cgccatctcg gacgcggcaa 420
accgcatggt cctgaacggc gcgaagcaga tcctgctgtt caacctgccc gacctgggcc 480
agaacccgtc cgcccgctcc cagaaggtcg tcgaggccgt ctcgcacgtg tccgcctacc 540
acaacaagct gctcctcaac ctcgcccggc agctcgcccc gacgggcatg gtcaagctgt 600
tcgagatcga caagcagttc gcggagatgc tgcgcgaccc ccagaacttc ggcctgagcg 660
acgtggagaa cccgtgctac gacggcggct acgtgtggaa gccgttcgcc acccggtccg 720
tctcgaccga ccggcagctg tcggccttct cgccccagga gcgcctggcg atcgctggca 780
acccgctcct ggcacaggcg gtagcttcgc cgatggcccg ccgctcggcc tcgcccctca 840
actgcgaggg caagatgttc tgggaccagg tccaccccac caccgtggtc cacgccgccc 900
tctcggagcg cgccgccacc ttcatcgaga cccagtacga gttcctcgcc cactagtcta 960
gaggatcc 968

<210> 49
<211> 1044
<212> DNA
<213> Aeromonas salmonicida

<400> 49

atgaaacaac aaaaacggct ttacgcccga ttgctgacgc tgttatttgc gctcatcttc 60
ttgctgcctc attctgcagc ttcagcagca gatacaagac cggcgtttag ccggatcgtc 120
atgtttggag atagcctgag cgatacgggc aaaatgtata gcaaaatgag aggctatctt 180
ccgtcaagcc cgccgtatta tgaaggccgc tttagcaatg gaccggtctg gctggaacaa 240
ctgacgaaac aatttccggg actgacgatc gctaatgaag cagaaggagg agcaacagcg 300
gtcgcctata acaaaatcag ctgggacccg aaatatcagg tcatcaacaa cctggactat 360
gaagtcacac agtttcttca gaaagacagc tttaaaccgg atgatctggt catcctttgg 420
gtcggcgcca atgattatct ggcgtatggc tggaacacag aacaagatgc caaaagagtc 480
agagatgcca tcagcgatgc cgctaataga atggtcctga acggcgccaa acaaatcctg 540
ctgtttaacc tgccggatct gggacaaaat ccgagcgcca gaagccaaaa agtcgtcgaa 600
gcagtcagcc atgtcagcgc ctatcataac aaactgctgc tgaacctggc aagacaattg 660
gcaccgacgg gaatggttaa attgtttgaa attgacaaac agtttgccga aatgctgaga 720
gatccgcaaa attttggcct gagcgatgtc gaaaacccgt gctatgatgg cggatatgtc 780
tggaaaccgt ttgccacaag aagcgtcagc acggatagac aactgtcagc gtttagcccg 840
caagaaagac tggcaatcgc cggaaatccg cttttggcac aagcagttgc ttcaccgatg 900
gcaagaagat cagcaagccc gctgaattgc gaaggcaaaa tgttttggga tcaggtccat 960
ccgacaacag ttgtccatgc tgccctttca gaaagagcgg cgacgtttat cgaaacacag 1020
tatgaatttc tggcccatgg ctga 1044

<210> 50
<211> 1005
<212> DNA
<213> Aeromonas hydrophila

<400> 50

atgaaaaaat ggtttgtgtg tttattggga ttggtcgcgc tgacagttca ggcagccgac 60
agccgtcccg ccttctcccg gatcgtgatg tttggcgaca gcctctccga taccggcaag 120
atgtacagca agatgcgcgg ttacctcccc tccagccccc cctactatga gggccgcttc 180
tccaacgggc ccgtctggct ggagcagctg accaacgagt tcccgggcct gaccatagcc 240
aacgaggcgg aaggcggacc gaccgccgtg gcttacaaca agatctcctg gaatcccaag 300

```
tatcaggtca tcaacaacct ggactacgag gtcacccagt tcctgcaaaa agacagcttc      360
aagccggacg atctggtgat cctctgggtc ggcgccaacg actatctggc ctatggctgg      420
aacacagagc aggatgccaa gcgggtgcgc gacgccatca gcgatgcggc caaccgcatg      480
gtgctgaacg gcgccaagga gatactgctg ttcaacctgc cggatctggg ccagaacccc      540
tcggcccgca gccagaaggt ggtcgaggcg gccagccatg tctccgccta ccacaaccag      600
ctgctgctga acctggcacg ccagctggct cccaccggca tggtgaagct gttcgagatc      660
gacaagcagt ttgccgagat gctgcgtgat ccgcagaact tcggcctgag cgaccagagg      720
aacgcctgct acggtggcag ctatgtatgg aagccgtttg cctcccgcag cgccagcacc      780
gacagccagc tctccgcctt caacccgcag gagcgcctcg ccatcgccgg caacccgctg      840
ctggcccagg ccgtcgccag ccccatggct gcccgcagcg ccagcaccct caactgtgag      900
ggcaagatgt tctgggatca ggtccacccc accactgtcg tgcacgccgc cctgagcgag      960
cccgccgcca ccttcatcga gagccagtac gagttcctcg cccac                     1005
```

<210> 51
<211> 1011
<212> DNA
<213> Aeromonas salmonicida

<400> 51

```
atgaaaaaat ggtttgtttg tttattgggg ttgatcgcgc tgacagttca ggcagccgac      60
actcgccccg ccttctcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaagcagt tcccgggtct gaccatcgcc     240
aacgaagcgg aaggcggtgc cactgccgtg gcttacaaca agatctcctg gaatcccaag     300
tatcaggtct acaacaacct ggactacgag gtcacccagt tcttgcagaa agacagcttc     360
aagccggacg atctggtgat cctctgggtc ggtgccaatg actatctggc atatggctgg     420
aatacggagc aggatgccaa gcgagttcgc gatgccatca gcgatgcggc caaccgcatg     480
gtactgaacg gtgccaagca gatactgctg ttcaacctgc cggatctggg ccagaacccg     540
tcagcccgca gtcagaaggt ggtcgaggcg gtcagccatg tctccgccta tcacaacaag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagcccccct caactgtgag     900
ggcaagatgt tctgggatca ggtacacccg accactgtcg tgcacgcagc cctgagcgag     960
cgcgccgcca ccttcatcga gacccagtac gagttcctcg cccacggatg a             1011
```

<210> 52
<211> 888
<212> DNA
<213> Streptomyces coelicolor

<400> 52

atgccgaagc ctgcccttcg ccgtgtcatg accgcgacag tcgccgccgt cggcacgctc 60
gccctcggcc tcaccgacgc caccgcccac gccgcgcccg cccaggccac tccgaccctg 120
gactacgtcg ccctcggcga cagctacagc gccggctccg gcgtcctgcc cgtcgacccc 180
gccaacctgc tctgtctgcg ctcgacggcc aactaccccc acgtcatcgc ggacacgacg 240
ggcgcccgcc tcacggacgt cacctgcggc gccgcgcaga ccgccgactt cacgcgggcc 300
cagtacccgg gcgtcgcacc ccagttggac gcgctcggca ccggcacgga cctggtcacg 360
ctcaccatcg gcggcaacga caacagcacc ttcatcaacg ccatcacggc ctgcggcacg 420
gcgggtgtcc tcagcggcgg caagggcagc ccctgcaagg acaggcacgg cacctccttc 480
gacgacgaga tcgaggccaa cacgtacccc gcgctcaagg aggcgctgct cggcgtccgc 540
gccagggctc cccacgccag ggtggcggct ctcggctacc cgtggatcac cccggccacc 600
gccgacccgt cctgcttcct gaagctcccc ctcgccgccg gtgacgtgcc ctacctgcgg 660
gccatccagg cacacctcaa cgacgcggtc cggcgggccg ccgaggagac cggagccacc 720
tacgtggact tctccggggt gtccgacggc cacgacgcct gcgaggcccc cggcacccgc 780
tggatcgaac cgctgctctt cgggcacagc ctcgttcccg tccaccccaa cgccctgggc 840
gagcggcgca tggccgagca cacgatggac gtcctcggcc tggactga 888

<210> 53
<211> 888
<212> DNA
<213> Streptomyces coelicolor

<400> 53

tcagtccagg ccgaggacgt ccatcgtgtg ctcggccatg cgccgctcgc ccagggcgtt 60
ggggtggacg ggaacgaggc tgtgcccgaa gagcagcggt tcgatccagc gggtgccggg 120
ggcctcgcag gcgtcgtggc cgtcggacac cccggagaag tccacgtagg tggctccggt 180
ctcctcggcg gcccgccgga ccgcgtcgtt gaggtgtgcc tggatggccc gcaggtaggg 240
cacgtcaccg gcggcgaggg ggagcttcag gaagcaggac gggtcggcgg tggccggggt 300
gatccacggg tagccgagag ccgccaccct ggcgtgggga gccctggcgc ggacgccgag 360
cagcgcctcc ttgagcgcgg ggtacgtgtt ggcctcgatc tcgtcgtcga aggaggtgcc 420
gtgcctgtcc ttgcaggggc tgcccttgcc gccgctgagg acacccgccg tgccgcaggc 480
cgtgatggcg ttgatgaagg tgctgttgtc gttgccgccg atggtgagcg tgaccaggtc 540
cgtgccggtg ccgagcgcgt ccaactgggg tgcgacgccc gggtactggg cccgcgtgaa 600

gtcggcggtc tgcgcggcgc cgcaggtgac gtccgtgagg cgggcgcccg tcgtgtccgc 660

gatgacgtgg gggtagttgg ccgtcgagcg cagacagagc aggttggcgg ggtcgacggg 720

caggacgccg gagccggcgc tgtagctgtc gccgagggcg acgtagtcca gggtcggagt 780

ggcctgggcg ggcgcggcgt gggcggtggc gtcggtgagg ccgagggcga gcgtgccgac 840

ggcggcgact gtcgcggtca tgacacggcg aagggcaggc ttcggcat 888

<210> 54
<211> 717
<212> DNA
<213> Saccharomyces cerevisiae

<400> 54

atggattacg agaagtttct gttatttggg gattccatta ctgaatttgc ttttaatact 60

aggcccattg aagatggcaa agatcagtat gctcttggag ccgcattagt caacgaatat 120

acgagaaaaa tggatattct tcaaagaggg ttcaaagggt acacttctag atgggcgttg 180

aaaatacttc ctgagatttt aaagcatgaa tccaatattg tcatggccac aatatttttg 240

ggtgccaacg atgcatgctc agcaggtccc caaagtgtcc ccctccccga atttatcgat 300

aatattcgtc aaatggtatc tttgatgaag tcttaccata tccgtcctat tataatagga 360

ccggggctag tagatagaga gaagtgggaa aaagaaaaat ctgaagaaat agctctcgga 420

tacttccgta ccaacgagaa ctttgccatt tattccgatg ccttagcaaa actagccaat 480

gaggaaaaag ttcccttcgt ggctttgaat aaggcgtttc aacaggaagg tggtgatgct 540

tggcaacaac tgctaacaga tggactgcac ttttccggaa aagggtacaa aatttttcat 600

gacgaattat tgaaggtcat tgagacattc tacccccaat atcatcccaa aaacatgcag 660

tacaaactga aagattggag agatgtgcta gatgatggat ctaacataat gtcttga 717

<210> 55
<211> 1044
<212> DNA
<213> Ralstonia sp.

<400> 55

atgaacctgc gtcaatggat gggcgccgcc acggctgccc ttgccttggg cttggccgcg 60

tgcgggggcg gtgggaccga ccagagcggc aatcccaatg tcgccaaggt gcagcgcatg 120

gtggtgttcg gcgacagcct gagcgatatc ggcacctaca cccccgtcgc gcaggcggtg 180

ggcggcggca agttcaccac caacccgggc ccgatctggg ccgagaccgt ggccgcgcaa 240

ctgggcgtga cgctcacgcc ggcggtgatg ggctacgcca cctccgtgca gaattgcccc 300

aaggccggct gcttcgacta tgcgcagggc ggctcgcgcg tgaccgatcc gaacggcatc 360

ggccacaacg gcggcgcggg ggcgctgacc tacccggttc agcagcagct cgccaacttc 420

tacgcggcca gcaacaacac attcaacggc aataacgatg tcgtcttcgt gctggccggc 480

agcaacgaca ttttcttctg gaccactgcg gcggccacca gcggctccgg cgtgacgccc 540

gccattgcca cggcccaggt gcagcaggcc gcgacggacc tggtcggcta tgtcaaggac 600

atgatcgcca agggtgcgac gcaggtctac gtgttcaacc tgcccgacag cagcctgacg 660

ccggacggcg tggcaagcgg cacgaccggc caggcgctgc tgcacgcgct ggtgggcacg 720

ttcaacacga cgctgcaaag cgggctggcc ggcacctcgg cgcgcatcat cgacttcaac 780

gcacaactga ccgcggcgat ccagaatggc gcctcgttcg gcttcgccaa caccagcgcc 840

cgggcctgcg acgccaccaa gatcaatgcc ctggtgccga gcgccggcgg cagctcgctg 900

ttctgctcgg ccaacacgct ggtggcttcc ggtgcggacc agagctacct gttcgccgac 960

ggcgtgcacc cgaccacggc cggccatcgc ctgatcgcca gcaacgtgct ggcgcgcctg 1020

ctggcggata acgtcgcgca ctga 1044

<210> 56
<211> 786
<212> DNA
<213> Streptomyces coelicolor

<400> 56

```
gtgatcgggt cgtacgtggc ggtgggggac agcttcaccg agggcgtcgg cgaccccggc        60
cccgacgggg cgttcgtcgg ctgggccgac cggctcgccg tactgctcgc ggaccggcgc       120
cccgagggcg acttcacgta cacgaacctc gccgtgcgcg gcaggctcct cgaccagatc       180
gtggcggaac aggtcccgcg ggtcgtcgga ctcgcgcccg acctcgtctc gttcgcggcg       240
ggcggcaacg acatcatccg gcccggcacc gatcccgacg aggtcgccga gcggttcgag       300
ctggcggtgg ccgcgctgac cgccgcggcc ggaaccgtcc tggtgaccac cgggttcgac       360
acccgggggg tgcccgtcct caagcacctg cgcggcaaga tcgccacgta caacgggcac       420
gtccgcgcca tcgccgaccg ctacggctgc ccggtgctcg acctgtggtc gctgcggagc       480
gtccaggacc gcagggcgtg ggacgccgac cggctgcacc tgtcgccgga ggggcacacc       540
cgggtggcgc tgcgcgcggg gcaggccctg ggcctgcgcg tcccggccga ccctgaccag       600
ccctggccgc ccctgccgcc gcgcggcacg ctcgacgtcc ggcgcgacga cgtgcactgg       660
gcgcgcgagt acctggtgcc gtggatcggg cgccggctgc ggggcgagtc gtcgggcgac       720
cacgtgacgg ccaaggggac gctgtcgccg gacgccatca agacgcggat cgccgcggtg       780
gcctga                                                                  786
```

<210> 57
<211> 783
<212> DNA
<213> Streptomyces coelicolor

<400> 57

```
atgcagacga accccgcgta caccagtctc gtcgccgtcg gcgactcctt caccgagggc        60
atgtcggacc tgctgcccga cggctcctac cgtggctggg ccgacctcct cgccacccgg       120
```

atggcggccc gctcccccgg cttccggtac gccaacctgg cggtgcgcgg gaagctgatc 180
ggacagatcg tcgacgagca ggtggacgtg gccgccgcca tgggagccga cgtgatcacg 240
ctggtcggcg ggctcaacga cacgctgcgg cccaagtgcg acatggcccg ggtgcgggac 300
ctgctgaccc aggccgtgga acggctcgcc ccgcactgcg agcagctggt gctgatgcgc 360
agtcccggtc gccagggtcc ggtgctggag cgcttccggc cccgcatgga ggccctgttc 420
gccgtgatcg acgacctggc cgggcggcac ggcgccgtgg tcgtcgacct gtacggggcc 480
cagtcgctgg ccgaccctcg gatgtgggac gtggaccggc tgcacctgac cgccgagggc 540
caccgccggg tcgcggaggc ggtgtggcag tcgctcggcc acgagcccga ggaccccgag 600
tggcacgcgc cgatcccggc gacgccgccg ccggggtggg tgacgcgcag gaccgcggac 660
gtccggttcg cccggcagca cctgctgccc tggataggcc gcaggctgac cgggcgctcg 720
tccggggacg gcctgccggc caagcgcccg gacctgctgc cctacgagga ccccgcacgg 780
tga 783

<210> 58
<211> 1365
<212> DNA
<213> Streptomyces coelicolor

<400> 58

```
atgacccggg gtcgtgacgg gggtgcgggg gcgcccccca ccaagcaccg tgccctgctc      60
gcggcgatcg tcaccctgat agtggcgatc tccgcggcca tatacgccgg agcgtccgcg     120
gacgacggca gcagggacca cgcgctgcag gccggaggcc gtctcccacg aggagacgcc     180
gcccccgcgt ccaccggtgc ctgggtgggc gcctgggcca ccgcaccggc cgcggccgag     240
ccgggcaccg agacgaccgg cctggcgggc cgctccgtgc gcaacgtcgt gcacacctcg     300
gtcggcggca ccggcgcgcg gatcaccctc tcgaacctgt acgggcagtc gccgctgacc     360
gtcacacacg cctcgatcgc cctggccgcc gggcccgaca ccgccgccgc gatcgccgac     420
accatgcgcc ggctcacctt cggcggcagc gcccgggtga tcatcccggc gggcggccag     480
gtgatgagcg acaccgcccg cctcgccatc ccctacgggg cgaacgtcct ggtcaccacg     540
tactccccca tcccgtccgg gccggtgacc taccatccgc aggcccggca gaccagctac     600
ctggccgacg gcgaccgcac ggcggacgtc accgccgtcg cgtacaccac cccacgccc      660
tactggcgct acctgaccgc cctcgacgtg ctgagccacg aggccgacgg cacggtcgtg     720
gcgttcggcg actccatcac cgacggcgcc cgctcgcaga gcgacgccaa ccaccgctgg     780
accgacgtcc tcgccgcacg cctgcacgag gcggcgggcg acggccggga cacgccccgc     840
tacagcgtcg tcaacgaggg catcagcggc aaccggctcc tgaccagcag gccggggcgg     900
ccggccgaca acccgagcgg actgagccgg ttccagcggg acgtgctgga acgcaccaac     960
gtcaaggccg tcgtcgtcgt cctcggcgtc aacgacgtcc tgaacagccc ggaactcgcc    1020
gaccgcgacg ccatcctgac cggcctgcgc accctcgtcg accgggcgca cgcccgggga    1080
ctgcgggtcg tcggcgccac gatcacgccg ttcggcggct acggcggcta caccgaggcc    1140
cgcgagacga tgcggcagga ggtcaacgag gagatccgct ccggccgggt cttcgacacg    1200
gtcgtcgact tcgacaaggc cctgcgcgac ccgtacgacc cgcgccggat gcgctccgac    1260
tacgacagcg gcgaccacct gcaccccggc gacaaggggt acgcgcgcat gggcgcggtc    1320
atcgacctgg ccgcgctgaa gggcgcggcg ccggtcaagg cgtag                    1365
```

<210> 59
<211> 1023
<212> DNA
<213> Streptomyces coelicolor

<400> 59

```
atgacgagca tgtcgagggc gagggtggcg cggcggatcg cggccggcgc ggcgtacggc      60
ggcggcggca tcggcctggc gggagcggcg gcggtcggtc tggtggtggc cgaggtgcag     120
ctggccagac gcagggtggg ggtgggcacg ccgacccggg tgccgaacgc gcagggactg     180
tacggcggca ccctgcccac ggccggcgac ccgccgctgc ggctgatgat gctgggcgac     240
tccacggccg ccgggcaggg cgtgcaccgg gccgggcaga cgccgggcgc gctgctggcg     300
tccgggctcg cggcggtggc ggagcggccg gtgcggctgg ggtcggtcgc ccagccgggg     360
gcgtgctcgg acgacctgga ccggcaggtg gcgctggtgc tcgccgagcc ggaccgggtg     420
cccgacatct gcgtgatcat ggtcggcgcc aacgacgtca cccaccggat gccggcgacc     480
cgctcggtgc ggcacctgtc ctcggcggta cggcggctgc gcacggccgg tgcggaggtg     540
gtggtcggca cctgtccgga cctgggcacg atcgagcggg tgcggcagcc gctgcgctgg     600
ctggcccggc gggcctcacg gcagctcgcg gcggcacaga ccatcggcgc cgtcgagcag     660
ggcgggcgca cggtgtcgct gggcgacctg ctgggtccgg agttcgcgca gaacccgcgg     720
gagctcttcg gccccgacaa ctaccacccc tccgccgagg ggtacgccac ggccgcgatg     780
gcggtactgc cctcggtgtg cgccgcgctc ggcctgtggc cggccgacga ggagcacccg     840
gacgcgctgc gccgcgaggg cttcctgccg gtggcgcgcg cggcggcgga ggcggcgtcc     900
gaggcgggta cggaggtcgc cgccgccatg cctacggggc ctcgggggcc ctgggcgctg     960
ctgaagcgcc ggagacggcg tcgggtgtcg gaggcggaac cgtccagccc gtccggcgtt    1020
tga                                                                  1023
```

<210> 60
<211> 918
<212> DNA
<213> Streptomyces coelicolor

<400> 60

```
atgggtcgag ggacggacca gcggacgcgg tacggccgtc gccgggcgcg tgtcgcgctc      60
gccgccctga ccgccgccgt cctggcgtg ggcgtggcgg gctgcgactc cgtgggcggc     120
```

gactcacccg ctccttccgg cagcccgtcg aagcggacga ggacggcgcc cgcctgggac 180
accagcccgg cgtccgtcgc cgccgtgggc gactccatca cgcgcggctt cgacgcctgt 240
gcggtgctgt cggactgccc ggaggtgtcg tgggcgaccg gcagcagcgc gaaggtcgac 300
tcgctggccg tacggctgct ggggaaggcg gacgcggccg agcacagctg gaactacgcg 360
gtcaccgggg cccggatggc ggacctgacc gctcaggtga cgcgggcggc gcagcgcgag 420
ccggagctgg tggcggtgat ggccggggcg aacgacgcgt gccggtccac gacctcggcg 480
atgacgccgg tggcggactt ccgggcgcag ttcgaggagg cgatggccac cctgcgcaag 540
aagctcccca aggcgcaggt gtacgtgtcg agcatcccgg acctcaagcg gctctggtcc 600
cagggccgca ccaacccgct gggcaagcag gtgtggaagc tcggcctgtg cccgtcgatg 660
ctgggcgacg cggactccct ggactcggcg gcgaccctgc ggcgcaacac ggtgcgcgac 720
cgggtggcgg actacaacga ggtgctgcgg gaggtctgcg cgaaggaccg gcggtgccgc 780
agcgacgacg gcgcggtgca cgagttccgg ttcggcacgg accagttgag ccactgggac 840
tggttccacc cgagtgtgga cggccaggcc cggctggcgg agatcgccta ccgcgcggtc 900
accgcgaaga atccctga 918

<210> 61
<211> 1068
<212> DNA
<213> Streptomyces rimosus

<400> 61

ttcatcacaa cgatgtcaca acaccggcca tccgggtcat ccctgatcgt gggaatgggt 60
gacaagcctt cccgtgacga aagggtcctg ctacatcaga aatgacagaa atcctgctca 120
gggaggttcc atgagactgt cccgacgcgc ggccacggcg tccgcgctcc tcctcacccc 180
ggcgctcgcg ctcttcggcg cgagcgccgc cgtgtccgcg ccgcgaatcc aggccaccga 240
ctacgtggcc ctcggcgact cctactcctc ggggggtcggc gcgggcagct acgacagcag 300
cagtggctcc tgtaagcgca gcaccaagtc ctacccggcc ctgtgggccg cctcgcacac 360
cggtacgcgg ttcaacttca ccgcctgttc gggcgcccgc acaggagacg tgctggccaa 420
gcagctgacc ccggtcaact ccggcaccga cctggtcagc attaccatcg gcggcaacga 480
cgcgggcttc gccgacacca tgaccacctg caacctccag ggcgagagcg cgtgcctggc 540
gcggatcgcc aaggcgcgcg cctacatcca gcagacgctg cccgcccagc tggaccaggt 600
ctacgacgcc atcgacagcc gggcccccgc agcccaggtc gtcgtcctgg gctacccgcg 660
cttctacaag ctgggcggca gctgcgccgt cggtctctcg gagaagtccc gcgcggccat 720
caacgccgcc gccgacgaca tcaacgccgt caccgccaag cgcgccgccg accacggctt 780
cgccttcggg gacgtcaaca cgaccttcgc cgggcacgag ctgtgctccg gcgcccctg 840
gctgcacagc gtcacccttc ccgtggagaa ctcctaccac cccacggcca acggacagtc 900

caagggctac ctgcccgtcc tgaactccgc cacctgatct cgcggctact ccgcccctga 960
cgaagtcccg cccccgggcg gggcttcgcc gtaggtgcgc gtaccgccgt cgcccgtcgc 1020
gccggtggcc ccgccgtacg tgccgccgcc cccggacgcg gtcggttc 1068

<210> 62
<211> 1008
<212> DNA
<213> Aeromonas hydrophila

<400> 62

```
atgaaaaaat ggtttgtgtg tttattggga ttggtcgcgc tgacagttca ggcagccgac        60
agtcgccccg ccttttcccg gatcgtgatg ttcggcgaca gcctctccga taccggcaaa       120
atgtacagca agatgcgcgg ttacctcccc tccagcccgc cctactatga gggccgtttc       180
tccaacggac ccgtctggct ggagcagctg accaaacagt tcccgggtct gaccatcgcc       240
aacgaagcgg aaggcggtgc cactgccgtg gcttacaaca agatctcctg gaatcccaag       300
tatcaggtca tcaacaacct ggactacgag gtcacccagt tcttgcagaa agacagcttc       360
aagccggacg atctggtgat cctctgggtc ggtgccaatg actatctggc ctatggctgg       420
aacacggagc aggatgccaa gcgggttcgc gatgccatca gcgatgcggc caaccgcatg       480
gtactgaacg gtgccaagca gatactgctg ttcaacctgc cggatctggg ccagaacccg       540
tcagctcgca gtcagaaggt ggtcgaggcg gtcagccatg tctccgccta tcacaaccag       600
ctgctgctga acctggcacg ccagctggcc cccaccggca tggtaaagct gttcgagatc       660
gacaagcaat ttgccgagat gctgcgtgat ccgcagaact tcggcctgag cgacgtcgag       720
aacccctgct acgacggcgg ctatgtgtgg aagccgtttg ccacccgcag cgtcagcacc       780
gaccgccagc tctccgcctt cagtccgcag gaacgcctcg ccatcgccgg caacccgctg       840
ctggcacagg ccgttgccag tcctatggcc cgccgcagcg ccagccccct caactgtgag       900
ggcaagatgt tctgggatca ggtacacccg accactgtcg tgcacgcagc cctgagcgag       960
cgcgccgcca ccttcatcgc gaaccagtac gagttcctcg cccactga                   1008
```

<210> 63
<211> 1011
<212> DNA
<213> Aeromonas salmonicida subsp. Salmonicida

<400> 63

```
atgaaaaaat ggtttgtttg tttattgggg ttgatcgcgc tgacagttca ggcagccgac        60
actcgccccg ccttctcccg gatcgtgatg ttcggcgaca gcctctccga taccggcaaa       120
atgtacagca agatgcgcgg ttacctcccc tccagcccgc cctactatga gggccgtttc       180
tccaacggac ccgtctggct ggagcagctg accaagcagt tcccgggtct gaccatcgcc       240
aacgaagcgg aaggcggtgc cactgccgtg gcttacaaca agatctcctg gaatcccaag       300
```

tatcaggtca tcaacaacct ggactacgag gtcacccagt tcttgcagaa agacagcttc 360
aagccggacg atctggtgat cctctgggtc ggtgccaatg actatctggc atatggctgg 420
aatacggagc aggatgccaa gcgagttcgc gatgccatca gcgatgcggc caaccgcatg 480
gtactgaacg gtgccaagca gatactgctg ttcaacctgc cggatctggg ccagaacccg 540
tcagcccgca gtcagaaggt ggtcgaggcg gtcagccatg tctccgccta tcacaacaag 600
ctgctgctga acctggcacg ccagctggcc cccaccggca tggtaaagct gttcgagatc 660
gacaagcaat ttgccgagat gctgcgtgat ccgcagaact tcggcctgag cgacgtcgag 720
aacccctgct acgacggcgg ctatgtgtgg aagccgtttg ccacccgcag cgtcagcacc 780
gaccgccagc tctccgcctt cagtccgcag gaacgcctcg ccatcgccgg caacccgctg 840
ctggcacagg ccgttgccag tcctatggcc cgccgcagcg ccagccccct caactgtgag 900
ggcaagatgt tctgggatca ggtacacccg accactgtcg tgcacgcagc cctgagcgag 960
cgcgccgcca ccttcatcga gacccagtac gagttcctcg cccacggatg a 1011

<210> 64
<211> 51
<212> DNA
<213> Artificial Sequence

<220>
<223> alpha-amylase terminator sequence

<400> 64
cgggacttac cgaaagaaac catcaatgat ggtttctttt ttgttcataa a 51

<210> 65
<211> 59
<212> DNA
<213> Artificial Sequence

<220>
<223> alkaline protease terminator sequence

<400> 65
caagactaaa gaccgttcgc ccgtttttgc aataagcggg cgaatcttac ataaaaata 59

<210> 66
<211> 61
<212> DNA
<213> Artificial Sequence

<220>
<223> glutamic-acid specific terminator sequence

<400> 66

acggccgtta gatgtgacag cccgttccaa aaggaagcgg gctgtcttcg tgtattattg 60

t 61

<210> 67
<211> 54
<212> DNA
<213> Artificial Sequence

<220>
<223> levanase terminator sequence

<400> 67
tcttttaaag gaaaggctgg aatgcccggc attccagcca catgatcatc gttt 54

<210> 68
<211> 280
<212> PRT
<213> Aeromonas salmonicida

<400> 68

```
Ala Asp Thr Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Lys Gln Phe Pro Gly Leu Thr Ile Ala Asn Glu
    50                  55                  60

Ala Glu Gly Gly Ala Thr Ala Val Ala Tyr Asn Lys Ile Ser Trp Asp
65                  70                  75                  80

Pro Lys Tyr Gln Val Ile Asn Asn Leu Asp Tyr Glu Val Thr Gln Phe
                85                  90                  95

Leu Gln Lys Asp Ser Phe Lys Pro Asp Asp Leu Val Ile Leu Trp Val
            100                 105                 110

Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Gln Ile Leu Leu Phe Asn Leu Pro Asp Leu Gly Gln
145                 150                 155                 160

Asn Pro Ser Ala Arg Ser Gln Lys Val Val Glu Ala Val Ser His Val
                165                 170                 175

Ser Ala Tyr His Asn Lys Leu Leu Leu Asn Leu Ala Arg Gln Leu Ala
            180                 185                 190
```

Pro Thr Gly Met Val Lys Leu Phe Glu Ile Asp Lys Gln Phe Ala Glu
195 200 205

Met Leu Arg Asp Pro Gln Asn Phe Gly Leu Ser Asp Val Glu Asn Pro
210 215 220

Cys Tyr Asp Gly Gly Tyr Val Trp Lys Pro Phe Arg Ser Ala Ser Pro
225 230 235 240

Leu Asn Cys Glu Gly Lys Met Phe Trp Asp Gln Val His Pro Thr Thr
245 250 255

Val Val His Ala Ala Leu Ser Glu Arg Ala Ala Thr Phe Ile Glu Thr
260 265 270

Gln Tyr Glu Phe Leu Ala His Gly
275 280

<210> 69
<211> 4
<212> PRT
<213> Artificial Sequence

<220>
<223> Sequence motif

<220>
<221> VARIANT
<222> (4)..(4)
<223> Xaa may be any of the following amino acid residues Leu, Ala, Val, Ile, Phe, Tyr, His, Gln, Thr, Asn, Met or Ser.

<400> 69

Gly Asp Ser Xaa
1

<210> 70
<211> 8
<212> PRT
<213> Artificial Sequence

<220>
<223> Consensus sequence

<400> 70

Met Arg Arg Ser Arg Phe Leu Ala
1 5

<210> 71
<211> 8
<212> PRT
<213> Artificial Sequence

<220>
<223> Consensus sequence

<400> 71

Ala Leu Ile Leu Leu Thr Leu Ala
1 5

<210> 72
<211> 5
<212> PRT
<213> Artificial Sequence

<220>
<223> Consensus sequence

<400> 72

Ala Arg Ala Ala Pro
1 5

<210> 73
<211> 11
<212> PRT
<213> Artificial Sequence

<220>
<223> Consensus sequence

<400> 73

Tyr Val Ala Leu Gly Asp Ser Tyr Ser Ser Gly
1 5 10

<210> 74
<211> 5
<212> PRT
<213> Artificial Sequence

<220>
<223> Consensus sequence

<400> 74

Gly Ala Gly Ser Tyr
1 5

<210> 75
<211> 4
<212> PRT
<213> Artificial Sequence

<220>
<223> Consensus sequence

<400> 75

Ser Ser Gly Asp
1

<210> 76
<211> 15
<212> PRT
<213> Artificial Sequence

<220>
<223> Consensus sequence

<400> 76

Arg Ser Thr Lys Ala Tyr Pro Ala Leu Trp Ala Ala Ala His Ala
1 5 10 15

<210> 77
<211> 5
<212> PRT
<213> Artificial Sequence

<220>
<223> Consensus sequence

<400> 77

Ser Ser Phe Ser Phe
1 5

<210> 78
<211> 12
<212> PRT
<213> Artificial Sequence

<220>
<223> Consensus sequence

<400> 78

Ala Cys Ser Gly Ala Arg Thr Tyr Asp Val Leu Ala
1 5 10

<210> 79
<211> 15
<212> PRT
<213> Artificial Sequence

<220>
<223> Consensus sequence

<400> 79

Leu Val Ser Ile Thr Ile Gly Gly Asn Asp Ala Gly Phe Ala Asp
1 5 10 15

<210> 80
<211> 6
<212> PRT

<213> Artificial Sequence

<220>
<223> Consensus sequence

<400> 80

Met Thr Thr Cys Val Leu
1 5

<210> 81
<211> 6
<212> PRT
<213> Artificial Sequence

<220>
<223> Consensus sequence

<400> 81

Ser Asp Ser Ala Cys Leu
1 5

<210> 82
<211> 4
<212> PRT
<213> Artificial Sequence

<220>
<223> Consensus sequence

<400> 82

Thr Leu Pro Ala
1

<210> 83
<211> 9
<212> PRT
<213> Artificial Sequence

<220>
<223> Consensus sequence

<400> 83

Arg Leu Asp Ser Val Tyr Ser Ala Ile
1 5

<210> 84
<211> 4
<212> PRT
<213> Artificial Sequence

<220>
<223> Consensus sequence

<400> 84

Thr Arg Ala Pro
1

<210> 85
<211> 12
<212> PRT
<213> Artificial Sequence

<220>
<223> Consensus sequence

<400> 85

Ala Arg Val Val Val Leu Gly Tyr Pro Arg Ile Tyr
1 5 10

<210> 86
<211> 4
<212> PRT
<213> Artificial Sequence

<220>
<223> Consensus sequence

<400> 86

Leu Gly Leu Ser
1

<210> 87
<211> 11
<212> PRT
<213> Artificial Sequence

<220>
<223> Consensus sequence

<400> 87

Thr Lys Arg Ala Ala Ile Asn Asp Ala Ala Asp
1 5 10

<210> 88
<211> 12
<212> PRT
<213> Artificial Sequence

<220>
<223> Consensus sequence

<400> 88

Leu Asn Ser Val Ile Ala Lys Arg Ala Ala Asp His
1 5 10

<210> 89
<211> 7
<212> PRT

<213> Artificial Sequence

<220>
<223> Consensus sequence

<400> 89

Gly Phe Thr Phe Gly Asp Val
1 5

<210> 90
<211> 7
<212> PRT
<213> Artificial Sequence

<220>
<223> Consensus sequence

<400> 90

Gly His Glu Leu Cys Ser Ala
1 5

<210> 91
<211> 9
<212> PRT
<213> Artificial Sequence

<220>
<223> Consensus sequence

<400> 91

Pro Trp Leu His Ser Leu Thr Leu Pro
1 5

<210> 92
<211> 6
<212> PRT
<213> Artificial Sequence

<220>
<223> Consensus sequence

<400> 92

Ser Tyr His Pro Thr Ala
1 5

<210> 93
<211> 13
<212> PRT
<213> Artificial Sequence

<220>
<223> Consensus sequence

<400> 93

Gly His Ala Ala Gly Tyr Leu Pro Val Leu Asn Ser Ile
1 5 10

<210> 94
<211> 230
<212> PRT
<213> Aspergillus aculeatus

<400> 94

Thr Thr Val Tyr Leu Ala Gly Asp Ser Thr Met Ala Lys Asn Gly Gly
1 5 10 15

```
Gly Ser Gly Thr Asn Gly Trp Gly Glu Tyr Leu Ala Ser Tyr Leu Ser
            20                  25                  30

Ala Thr Val Val Asn Asp Ala Val Ala Gly Arg Ser Ala Arg Ser Tyr
        35                  40                  45

Thr Arg Glu Gly Arg Phe Glu Asn Ile Ala Asp Val Val Thr Ala Gly
    50                  55                  60

Asp Tyr Val Ile Val Glu Phe Gly His Asn Asp Gly Gly Ser Leu Ser
65                  70                  75                  80

Thr Asp Asn Gly Arg Thr Asp Cys Ser Gly Thr Gly Ala Glu Val Cys
                85                  90                  95

Tyr Ser Val Tyr Asp Gly Val Asn Glu Thr Ile Leu Thr Phe Pro Ala
            100                 105                 110

Tyr Leu Glu Asn Ala Ala Lys Leu Phe Thr Ala Lys Gly Ala Lys Val
        115                 120                 125

Ile Leu Ser Ser Gln Thr Pro Asn Asn Pro Trp Glu Thr Gly Thr Phe
    130                 135                 140

Val Asn Ser Pro Thr Arg Phe Val Glu Tyr Ala Glu Leu Ala Ala Glu
145                 150                 155                 160

Val Ala Gly Val Glu Tyr Val Asp His Trp Ser Tyr Val Asp Ser Ile
                165                 170                 175

Tyr Glu Thr Leu Gly Asn Ala Thr Val Asn Ser Tyr Phe Pro Ile Asp
            180                 185                 190

His Thr His Thr Ser Pro Ala Gly Ala Glu Val Val Ala Glu Ala Phe
        195                 200                 205

Leu Lys Ala Val Val Cys Thr Gly Thr Ser Leu Lys Ser Val Leu Thr
    210                 215                 220

Thr Thr Ser Phe Glu Gly
225                 230
```

<210> 95
<211> 184
<212> PRT
<213> Escherichia coli

<400> 95

Ala Asp Thr Leu Leu Ile Leu Gly Asp Ser Leu Ser Ala Gly Tyr Arg
1 5 10 15

Met Ser Ala Ser Ala Ala Trp Pro Ala Leu Leu Asn Asp Lys Trp Gln
20 25 30

Ser Lys Thr Ser Val Val Asn Ala Ser Ile Ser Gly Asp Thr Ser Gln
35 40 45

Gln Gly Leu Ala Arg Leu Pro Ala Leu Leu Lys Gln His Gln Pro Arg
50 55 60

Trp Val Leu Val Glu Leu Gly Gly Asn Asp Gly Leu Arg Gly Phe Gln
65 70 75 80

Pro Gln Gln Thr Glu Gln Thr Leu Arg Gln Ile Leu Gln Asp Val Lys
85 90 95

Ala Ala Asn Ala Glu Pro Leu Leu Met Gln Ile Arg Leu Pro Ala Asn
100 105 110

Tyr Gly Arg Arg Tyr Asn Glu Ala Phe Ser Ala Ile Tyr Pro Lys Leu
115 120 125

Ala Lys Glu Phe Asp Val Pro Leu Leu Pro Phe Phe Met Glu Glu Val
130 135 140

Tyr Leu Lys Pro Gln Trp Met Gln Asp Asp Gly Ile His Pro Asn Arg
145 150 155 160

Asp Ala Gln Pro Phe Ile Ala Asp Trp Met Ala Lys Gln Leu Gln Pro
165 170 175

Leu Val Asn His Asp Ser Leu Glu
180

<210> 96
<211> 308
<212> PRT
<213> Aeromonas hydrophila

<400> 96

```
Ile Val Met Phe Gly Asp Ser Leu Ser Asp Thr Gly Lys Met Tyr Ser
1               5                   10                  15

Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Asn Glu Phe Pro
        35                  40                  45
```

Gly Leu Thr Ile Ala Asn Glu Ala Glu Gly Gly Pro Thr Ala Val Ala
    50                  55                  60

Tyr Asn Lys Ile Ser Trp Asn Pro Lys Tyr Gln Val Ile Asn Asn Leu
65                  70                  75                  80

Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Glu Ile Leu Leu Phe
    130                 135                 140

Asn Leu Pro Asp Leu Gly Gln Asn Pro Ser Ala Arg Ser Gln Lys Val
145                 150                 155                 160

Val Glu Ala Ala Ser His Val Ser Ala Tyr His Asn Gln Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Gln Arg Asn Ala Cys Tyr Gly Gly Ser Tyr Val Trp Lys
    210                 215                 220

Pro Phe Ala Ser Arg Ser Ala Ser Thr Asp Ser Gln Leu Ser Ala Phe
225                 230                 235                 240

Asn Pro Gln Glu Arg Leu Ala Ile Ala Gly Asn Pro Leu Leu Ala Gln
                245                 250                 255

Ala Val Ala Ser Pro Met Ala Ala Arg Ser Ala Ser Thr Leu Asn Cys
            260                 265                 270

Glu Gly Lys Met Phe Trp Asp Gln Val His Pro Thr Thr Val Val His
        275                 280                 285

Ala Ala Leu Ser Glu Pro Ala Ala Thr Phe Ile Glu Ser Gln Tyr Glu
    290                 295                 300

Phe Leu Ala His
305

<210> 97
<211> 232
<212> PRT
<213> Aspergillus aculeatus

<400> 97

```
Thr Thr Val Tyr Leu Ala Gly Asp Ser Thr Met Ala Lys Asn Gly Gly
1               5                   10                  15

Gly Ser Gly Thr Asn Gly Trp Gly Glu Tyr Leu Ala Ser Tyr Leu Ser
            20                  25                  30

Ala Thr Val Val Asn Asp Ala Val Ala Gly Arg Ser Ala Arg Ser Tyr
        35                  40                  45

Thr Arg Glu Gly Arg Phe Glu Asn Ile Ala Asp Val Val Thr Ala Gly
    50                  55                  60

Asp Tyr Val Ile Val Glu Phe Gly His Asn Asp Gly Gly Ser Leu Ser
65                  70                  75                  80

Thr Asp Asn Gly Arg Thr Asp Cys Ser Gly Thr Gly Ala Glu Val Cys
                85                  90                  95

Tyr Ser Val Tyr Asp Gly Val Asn Glu Thr Ile Leu Thr Phe Pro Ala
            100                 105                 110

Tyr Leu Glu Asn Ala Ala Lys Leu Phe Thr Ala Lys Gly Ala Lys Val
        115                 120                 125

Ile Leu Ser Ser Gln Thr Pro Asn Asn Pro Trp Glu Thr Gly Thr Phe
    130                 135                 140

Val Asn Ser Pro Thr Arg Phe Val Glu Tyr Ala Glu Leu Ala Ala Glu
145                 150                 155                 160

Val Ala Gly Val Glu Tyr Val Asp His Trp Ser Tyr Val Asp Ser Ile
                165                 170                 175

Tyr Glu Thr Leu Gly Asn Ala Thr Val Asn Ser Tyr Phe Pro Ile Asp
            180                 185                 190

His Thr His Thr Ser Pro Ala Gly Ala Glu Val Val Ala Glu Ala Phe
        195                 200                 205

Leu Lys Ala Val Val Cys Thr Gly Thr Ser Leu Lys Ser Val Leu Thr

            210                 215                 220

        Thr Thr Ser Phe Glu Gly Thr Cys
        225                 230
```

<210> 98

<211> 167
<212> PRT
<213> Escherichia coli

<400> 98

```
Leu Leu Ile Leu Gly Asp Ser Leu Ser Ala Gly Tyr Arg Met Ser Ala
1               5                   10                  15

Ser Ala Ala Trp Pro Ala Leu Leu Asn Asp Lys Trp Gln Ser Lys Thr
            20                  25                  30

Ser Val Val Asn Ala Ser Ile Ser Gly Asp Thr Ser Gln Gln Gly Leu
        35                  40                  45

Ala Arg Leu Pro Ala Leu Leu Lys Gln His Gln Pro Arg Trp Val Leu
    50                  55                  60

Val Glu Leu Gly Gly Asn Asp Gly Leu Arg Gly Phe Gln Pro Gln Gln
65                  70                  75                  80

Thr Glu Gln Thr Leu Arg Gln Ile Leu Gln Asp Val Lys Ala Ala Asn
                85                  90                  95

Ala Glu Pro Leu Leu Met Gln Ile Arg Leu Pro Ala Asn Tyr Gly Arg
            100                 105                 110

Arg Tyr Asn Glu Ala Phe Ser Ala Ile Tyr Pro Lys Leu Ala Lys Glu
        115                 120                 125

Phe Asp Val Pro Leu Leu Pro Phe Phe Met Glu Glu Val Tyr Leu Lys
    130                 135                 140

Pro Gln Trp Met Gln Asp Asp Gly Ile His Pro Asn Arg Asp Ala Gln
145                 150                 155                 160

Pro Phe Ile Ala Asp Trp Met
                165
```

<210> 99
<211> 295
<212> PRT
<213> Aeromonas hydrophila

<400> 99

```
Ile Val Met Phe Gly Asp Ser Leu Ser Asp Thr Gly Lys Met Tyr Ser
1               5                   10                  15

Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Asn Glu Phe Pro
        35                  40                  45

Gly Leu Thr Ile Ala Asn Glu Ala Glu Gly Gly Pro Thr Ala Val Ala
    50                  55                  60

Tyr Asn Lys Ile Ser Trp Asn Pro Lys Tyr Gln Val Ile Asn Asn Leu
65                  70                  75                  80

Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Glu Ile Leu Leu Phe
    130                 135                 140

Asn Leu Pro Asp Leu Gly Gln Asn Pro Ser Ala Arg Ser Gln Lys Val
145                 150                 155                 160

Val Glu Ala Ala Ser His Val Ser Ala Tyr His Asn Gln Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Gln Arg Asn Ala Cys Tyr Gly Gly Ser Tyr Val Trp Lys
    210                 215                 220

Pro Phe Ala Ser Arg Ser Ala Ser Thr Asp Ser Gln Leu Ser Ala Phe
225                 230                 235                 240

Asn Pro Gln Glu Arg Leu Ala Ile Ala Gly Asn Pro Leu Leu Ala Gln
                245                 250                 255

Ala Val Ala Ser Pro Met Ala Ala Arg Ser Ala Ser Thr Leu Asn Cys
```

260 265 270

Glu Gly Lys Met Phe Trp Asp Gln Val His Pro Thr Thr Val Val His
275 280 285

Ala Ala Leu Ser Glu Pro Ala
290 295

<210> 100
<211> 50
<212> PRT
<213> Artificial Sequence

<220>
<223> Consensus sequence

<400> 100

Arg Pro Ala Phe Ser Arg Ile Val Met Phe Gly Asp Ser Leu Ser Asp
1 5 10 15

Thr Gly Lys Met Tyr Ser Lys Met Arg Gly Tyr Leu Pro Ser Ser Pro
20 25 30

Pro Tyr Tyr Glu Gly Arg Phe Ser Asn Gly Pro Val Trp Leu Glu Gln
35 40 45

Leu Thr
50

<210> 101
<211> 13
<212> PRT
<213> Artificial Sequence

<220>
<223> Consensus sequence

<400> 101

Phe Pro Gly Leu Thr Ile Ala Asn Glu Ala Glu Gly Gly
1 5 10

<210> 102
<211> 79
<212> PRT
<213> Artificial Sequence

<220>
<223> Consensus sequence

<400> 102

Thr Ala Val Ala Tyr Asn Lys Ile Ser Trp Asn Pro Lys Tyr Gln Val
1 5 10 15

Ile Asn Asn Leu Asp Tyr Glu Val Thr Gln Phe Leu Gln Lys Asp Ser
20 25 30

Phe Lys Pro Asp Asp Leu Val Ile Leu Trp Val Gly Ala Asn Asp Tyr
35 40 45

Leu Ala Tyr Gly Trp Asn Thr Glu Gln Asp Ala Lys Arg Val Arg Asp
50 55 60

Ala Ile Ser Asp Ala Ala Asn Arg Met Val Leu Asn Gly Ala Lys
65 70 75

<210> 103
<211> 23
<212> PRT
<213> Artificial Sequence

<220>
<223> Consensus sequence

<400> 103

Ile Leu Leu Phe Asn Leu Pro Asp Leu Gly Gln Asn Pro Ser Ala Arg
1 5 10 15

Ser Gln Lys Val Val Glu Ala
20

<210> 104
<211> 8
<212> PRT
<213> Artificial Sequence

<220>
<223> Consensus sequence

<400> 104

Ser His Val Ser Ala Tyr His Asn
1 5

<210> 105
<211> 38
<212> PRT
<213> Artificial Sequence

<220>
<223> Consensus sequence

<400> 105

Leu Leu Leu Asn Leu Ala Arg Gln Leu Ala Pro Thr Gly Met Val Lys
1 5 10 15

Leu Phe Glu Ile Asp Lys Gln Phe Ala Glu Met Leu Arg Asp Pro Gln
20 25 30

Asn Phe Gly Leu Ser Asp
35

<210> 106
<211> 7
<212> PRT
<213> Artificial Sequence

<220>
<223> Consensus sequence

<400> 106

Tyr Val Trp Lys Pro Phe Ala
1 5

<210> 107
<211> 5
<212> PRT
<213> Artificial Sequence

<220>
<223> Consensus sequence

<400> 107

Gln Leu Ser Ala Phe
1 5

<210> 108
<211> 22
<212> PRT
<213> Artificial Sequence

<220>
<223> Consensus sequence

<400> 108

Pro Gln Glu Arg Leu Ala Ile Ala Gly Asn Pro Leu Leu Ala Gln Ala
1 5 10 15

Val Ala Ser Pro Met Ala
20

<210> 109
<211> 4
<212> PRT
<213> Artificial Sequence

<220>
<223> Consensus sequence

<400> 109

Arg Ser Ala Ser
1

<210> 110
<211> 24
<212> PRT
<213> Artificial Sequence

<220>
<223> Consensus sequence

<400> 110

Leu Asn Cys Glu Gly Lys Met Phe Trp Asp Gln Val His Pro Thr Thr
1 5 10 15

Val Val His Ala Ala Leu Ser Glu
20

<210> 111
<211> 5
<212> PRT
<213> Artificial Sequence

<220>
<223> Consensus sequence

<400> 111

Ala Ala Thr Phe Ile
1 5

<210> 112
<211> 7
<212> PRT
<213> Artificial Sequence

<220>
<223> Consensus sequence

<400> 112

Gln Tyr Glu Phe Leu Ala His
1 5

<210> 113
<211> 1225
<212> DNA
<213> Artificial Sequence

<220>
<223> XhoI insert containing the LAT-KLM3’ precursor gene

<220>
<221> CDS
<222> (101)..(1144)

<400> 113

```
gcttttcttt tggaagaaaa tatagggaaa atggtacttg ttaaaaattc ggaatattta       60

tacaatatca tatgtttcac attgaaaggg gaggagaatc atg aaa caa caa aaa        115
                                            Met Lys Gln Gln Lys
                                            1               5
```

```
cgg ctt tac gcc cga ttg ctg acg ctg tta ttt gcg ctc atc ttc ttg        163
Arg Leu Tyr Ala Arg Leu Leu Thr Leu Leu Phe Ala Leu Ile Phe Leu
                10                  15                  20

ctg cct cat tct gca gct tca gca gca gat aca aga ccg gcg ttt agc        211
Leu Pro His Ser Ala Ala Ser Ala Ala Asp Thr Arg Pro Ala Phe Ser
            25                  30                  35

cgg atc gtc atg ttt gga gat agc ctg agc gat acg ggc aaa atg tat        259
Arg Ile Val Met Phe Gly Asp Ser Leu Ser Asp Thr Gly Lys Met Tyr
        40                  45                  50

agc aaa atg aga ggc tat ctt ccg tca agc ccg ccg tat tat gaa ggc        307
Ser Lys Met Arg Gly Tyr Leu Pro Ser Ser Pro Pro Tyr Tyr Glu Gly
    55                  60                  65

cgc ttt agc aat gga ccg gtc tgg ctg gaa caa ctg acg aaa caa ttt        355
Arg Phe Ser Asn Gly Pro Val Trp Leu Glu Gln Leu Thr Lys Gln Phe
70                  75                  80                  85

ccg gga ctg acg atc gct aat gaa gca gaa gga gga gca aca gcg gtc        403
Pro Gly Leu Thr Ile Ala Asn Glu Ala Glu Gly Gly Ala Thr Ala Val
                90                  95                  100

gcc tat aac aaa atc agc tgg gac ccg aaa tat cag gtc atc aac aac        451
Ala Tyr Asn Lys Ile Ser Trp Asp Pro Lys Tyr Gln Val Ile Asn Asn
            105                 110                 115

ctg gac tat gaa gtc aca cag ttt ctt cag aaa gac agc ttt aaa ccg        499
Leu Asp Tyr Glu Val Thr Gln Phe Leu Gln Lys Asp Ser Phe Lys Pro
        120                 125                 130

gat gat ctg gtc atc ctt tgg gtc ggc gcc aat gat tat ctg gcg tat        547
Asp Asp Leu Val Ile Leu Trp Val Gly Ala Asn Asp Tyr Leu Ala Tyr
    135                 140                 145

ggc tgg aac aca gaa caa gat gcc aaa aga gtc aga gat gcc atc agc        595
Gly Trp Asn Thr Glu Gln Asp Ala Lys Arg Val Arg Asp Ala Ile Ser
150                 155                 160                 165

gat gcc gct aat aga atg gtc ctg aac ggc gcc aaa caa atc ctg ctg        643
Asp Ala Ala Asn Arg Met Val Leu Asn Gly Ala Lys Gln Ile Leu Leu
                170                 175                 180

ttt aac ctg ccg gat ctg gga caa aat ccg agc gcc aga agc caa aaa        691
Phe Asn Leu Pro Asp Leu Gly Gln Asn Pro Ser Ala Arg Ser Gln Lys
            185                 190                 195

gtc gtc gaa gca gtc agc cat gtc agc gcc tat cat aac aaa ctg ctg        739
Val Val Glu Ala Val Ser His Val Ser Ala Tyr His Asn Lys Leu Leu
        200                 205                 210

ctg aac ctg gca aga caa ttg gca ccg acg gga atg gtt aaa ttg ttt        787
Leu Asn Leu Ala Arg Gln Leu Ala Pro Thr Gly Met Val Lys Leu Phe
    215                 220                 225

gaa att gac aaa cag ttt gcc gaa atg ctg aga gat ccg caa aat ttt        835
Glu Ile Asp Lys Gln Phe Ala Glu Met Leu Arg Asp Pro Gln Asn Phe
230                 235                 240                 245

ggc ctg agc gat gtc gaa aac ccg tgc tat gat ggc gga tat gtc tgg        883
Gly Leu Ser Asp Val Glu Asn Pro Cys Tyr Asp Gly Gly Tyr Val Trp
                250                 255                 260
```

```
aaa ccg ttt gcc aca aga agc gtc agc acg gat aga caa ctg tca gcg         931
Lys Pro Phe Ala Thr Arg Ser Val Ser Thr Asp Arg Gln Leu Ser Ala
            265                 270                 275

ttt agc ccg caa gaa aga ctg gca atc gcc gga aat ccg ctt ttg gca         979
Phe Ser Pro Gln Glu Arg Leu Ala Ile Ala Gly Asn Pro Leu Leu Ala
        280                 285                 290

caa gca gtt gct tca ccg atg gca aga aga tca gca agc ccg ctg aat        1027
Gln Ala Val Ala Ser Pro Met Ala Arg Arg Ser Ala Ser Pro Leu Asn
    295                 300                 305

tgc gaa ggc aaa atg ttt tgg gat cag gtc cat ccg aca aca gtt gtc        1075
Cys Glu Gly Lys Met Phe Trp Asp Gln Val His Pro Thr Thr Val Val
310                 315                 320                 325

cat gct gcc ctt tca gaa aga gcg gcg acg ttt atc gaa aca cag tat        1123
His Ala Ala Leu Ser Glu Arg Ala Ala Thr Phe Ile Glu Thr Gln Tyr
                330                 335                 340

gaa ttt ctg gcc cat ggc tga gttaacagag gacggatttc ctgaaggaaa           1174
Glu Phe Leu Ala His Gly
            345

tccgtttttt tattttaagc ttggagacaa ggtaaaggat aaaacctcga g               1225
```

<210> 114
<211> 347
<212> PRT
<213> Artificial Sequence

<220>
<223> Synthetic Construct

<400> 114

```
Met Lys Gln Gln Lys Arg Leu Tyr Ala Arg Leu Leu Thr Leu Leu Phe
1               5                   10                  15

Ala Leu Ile Phe Leu Leu Pro His Ser Ala Ala Ser Ala Ala Asp Thr
            20                  25                  30

Arg Pro Ala Phe Ser Arg Ile Val Met Phe Gly Asp Ser Leu Ser Asp
        35                  40                  45

Thr Gly Lys Met Tyr Ser Lys Met Arg Gly Tyr Leu Pro Ser Ser Pro
    50                  55                  60

Pro Tyr Tyr Glu Gly Arg Phe Ser Asn Gly Pro Val Trp Leu Glu Gln
65                  70                  75                  80

Leu Thr Lys Gln Phe Pro Gly Leu Thr Ile Ala Asn Glu Ala Glu Gly
                85                  90                  95

Gly Ala Thr Ala Val Ala Tyr Asn Lys Ile Ser Trp Asp Pro Lys Tyr
            100                 105                 110
```

```
Gln Val Ile Asn Asn Leu Asp Tyr Glu Val Thr Gln Phe Leu Gln Lys
        115                 120                 125

Asp Ser Phe Lys Pro Asp Asp Leu Val Ile Leu Trp Val Gly Ala Asn
    130                 135                 140

Asp Tyr Leu Ala Tyr Gly Trp Asn Thr Glu Gln Asp Ala Lys Arg Val
145                 150                 155                 160

Arg Asp Ala Ile Ser Asp Ala Ala Asn Arg Met Val Leu Asn Gly Ala
                165                 170                 175

Lys Gln Ile Leu Leu Phe Asn Leu Pro Asp Leu Gly Gln Asn Pro Ser
            180                 185                 190

Ala Arg Ser Gln Lys Val Val Glu Ala Val Ser His Val Ser Ala Tyr
        195                 200                 205

His Asn Lys Leu Leu Leu Asn Leu Ala Arg Gln Leu Ala Pro Thr Gly
    210                 215                 220

Met Val Lys Leu Phe Glu Ile Asp Lys Gln Phe Ala Glu Met Leu Arg
225                 230                 235                 240

Asp Pro Gln Asn Phe Gly Leu Ser Asp Val Glu Asn Pro Cys Tyr Asp
                245                 250                 255

Gly Gly Tyr Val Trp Lys Pro Phe Ala Thr Arg Ser Val Ser Thr Asp
            260                 265                 270

Arg Gln Leu Ser Ala Phe Ser Pro Gln Glu Arg Leu Ala Ile Ala Gly
        275                 280                 285

Asn Pro Leu Leu Ala Gln Ala Val Ala Ser Pro Met Ala Arg Arg Ser
    290                 295                 300

Ala Ser Pro Leu Asn Cys Glu Gly Lys Met Phe Trp Asp Gln Val His
305                 310                 315                 320

Pro Thr Thr Val Val His Ala Ala Leu Ser Glu Arg Ala Ala Thr Phe
                325                 330                 335

Ile Glu Thr Gln Tyr Glu Phe Leu Ala His Gly
            340                 345
```

<210> 115
<211> 5

<212> PRT
<213> Artificial Sequence

<220>
<223> Sequence motif

<400> 115

Arg Arg Ser Ala Ser
1               5

<210> 116
<211> 4
<212> PRT
<213> Artificial Sequence

<220>
<223> Sequence motif

<220>
<221> misc_feature
<222> (2)..(3)
<223> Xaa can be any naturally occurring amino acid

<400> 116

Asp Xaa Xaa His
1

<210> 117
<211> 102
<212> DNA
<213> Artificial Sequence

<220>
<223> Primer

<400> 117

ccccgctcga ggcttttctt ttggaagaaa atatagggaa aatggtactt gttaaaaatt 60

cggaatattt atacaatatc atatgtttca cattgaaagg gg 102

<210> 118
<211> 35
<212> DNA
<213> Artificial Sequence

<220>
<223> Primer

<400> 118
tggaatctcg aggttttatc ctttaccttg tctcc 35

<210> 119
<211> 56
<212> DNA

<213> Artificial Sequence

<220>
<223> subtilisin E terminator sequence

<400> 119
gctgacaaat aaaaagaagc aggtatggag gaacctgctt ctttttacta ttattg 56

<210> 120

<400> 120
000

<210> 121

<400> 121
000

<210> 122

<400> 122
000

<210> 123

<400> 123
000

<210> 124
<211> 79
<212> PRT
<213> Aeromonas salmonicida

<400> 124

```
Ala Glu Met Leu Arg Asp Pro Gln Asn Phe Gly Leu Ser Asp Val Glu
1               5                   10                  15

Asn Pro Cys Tyr Asp Gly Gly Tyr Val Trp Lys Pro Phe Ala Thr Arg
            20                  25                  30

Ser Val Ser Thr Asp Arg Gln Leu Ser Ala Ser Pro Gln Glu Arg Leu
        35                  40                  45

Ala Ile Ala Gly Asn Pro Leu Leu Ala Gln Ala Val Ala Ser Pro Met
    50                  55                  60

Ala Arg Arg Ser Ala Ser Pro Leu Asn Cys Glu Gly Lys Met Phe
65                  70                  75
```